# EUROPEAN PATENT SPECIFICATION

(11) **EP 4 530 287 B1**
(45) Date of publication and mention of the grant of the patent: **30.09.2026**
(21) Application number: 23839017.3
(22) Date of filing: 13.07.2023
(51) Int. Cl.: C07D 413/12, C07D 413/14, A01N 43/80, A01N 55/00, A01P 5/00, A01P 7/02, A01P 7/04, A01P 9/00

(54) **COMPOUND CONTAINING PYRAZOLE STRUCTURE, APPLICATION, AND INSECTICIDE**
VERBINDUNG MIT PYRAZOLSTRUKTUR, ANWENDUNG UND INSEKTIZID
COMPOSÉ CONTENANT UNE STRUCTURE PYRAZOLE, APPLICATION ET INSECTICIDE

(30) Priority: 13.07.2022 CN 202210826763
(43) Date of publication of application: 02.04.2025
(73) Proprietor: Anhui Shengfeng Biochemical Co., Ltd, Hefei, Anhui 231604 (CN)
(72) Inventor: YANG, Guangfu, Wuhan, Hubei 430079 (CN); HUANG, Wei, Wuhan, Hubei 430079 (CN); MA, Zhepeng, Wuhan, Hubei 430079 (CN); WANG, Zhenyu, Wuhan, Hubei 430079 (CN); WANG, Jinzhen, Wuhan, Hubei 430079 (CN); LI, Jianyou, Hefei, Anhui 231604 (CN)
(74) Representative: Ström & Gulliksson AB
(86) International application number: PCT/CN2023/107234
(87) International publication number: WO 2024/012527

(56) References cited:
- WO-A1-2021/127188
- CN-A- 1 930 136

## Description

### Cross Reference to Related Applications

The present application claims the benefit of Chinese patent application 202210826763.6, filed on 13/07/2022.

### Field of the invention

The present invention relates to the field of pesticides and insecticides, and particularly relates to compound containing pyrazole structure, application, and insecticide.

### Background of the invention

γ-aminobutyric acid (GABA) is an important neurotransmitter in animal body, is mainly present in central nervous system, and is converted from glutamate by enzymatic metabolism in the body.

As an inhibitory neurotransmitter, GABA is released from the presynaptic membrane of a nerve cell, reaches the postsynaptic membrane through a synaptic cleft, and is specifically combined with a GABA receptor on the postsynaptic membrane to promote chloride ion channels on cell membranes to be opened, chloride ions outside the membranes enter cells to generate a hyperpolarization phenomenon, so that the nerve cell is inhibited from exciting, and the physiological action is played.

Isoxazolines are a new class of pesticides that act on specific sites of ionic GABA receptors. The compound noncompetitive blocks the inflow of chlorine ions of nerve cells, interferes with the normal function of the central nervous system of insects, and enables the nerve excitation not to be normally inhibited, thereby leading the insects to die.

The isoxazoline compound has the characteristics of broad spectrum, high activity, high selectivity and the like, and has good biological activity on agricultural pests such as Hemiptera, Thysanoptera, Diptera, Lepidoptera, mites and the like.

Therefore, the isoxazoline compound is a pesticide with great research value and development prospect.

In 2005, Nissan Chemical Corporation disclosed that isoxazolines had insecticidal properties in WO2005085216A1. The representative substances are a compound K1 and a compound K2, and the structures are as follows, and the substances are mainly used for controlling lepidoptera pests in crops.

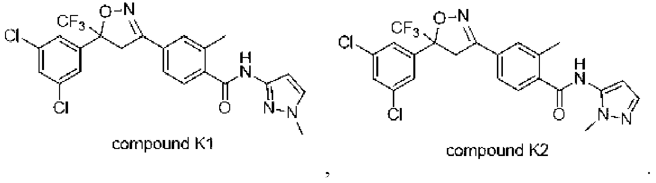

Subsequently, WO2007026965, WO2008108448, WO2011104089, WO2019243262, WO2012007426 and other prior arts disclose a series of compounds containing isoxazoline structures with insecticidal activity on the basis of the prior art. However, the insecticidal or acaricidal activity of the above compounds is still insufficient in practical application, and the activity of the compounds to other target organisms besides whiteflies is not satisfactory.

### Description of the invention

The invention aims to provide a novel pyrazole structure-containing compound which can successfully kill insects, mites, nematodes and piercing-sucking insects at low concentrations.

The inventors of the present invention have unexpectedly found in their studies that a novel compound obtained by shifting the 5-aminopyrazole structure of prior art isoxazolines to 4-aminopyrazole and introducing or not introducing characteristic substituents at the 1, 3, 5-positions has excellent inhibitory activity against pests. Meanwhile, the novel compound has good safety on aquatic organisms such as fish, daphnia, algae and the like. That is, the present invention can provide a novel isoxazoline pesticide having both environmental friendliness and high inhibitory activity against pests. In view of the above, the inventor provides the scheme of the invention.

In order to achieve the above object, a first aspect of the present invention provides a compound having a pyrazole structure, or an agrochemically acceptable salt, hydrate and solvate thereof, the compound having a structure represented by formula (I):

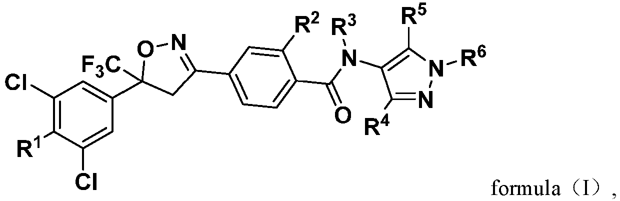

Wherein, in the formula (I),
R¹ is selected from H, halogen, at least one halogen-substituted C₁₋₆ alkyl, at least one halogen-substituted C₁₋₆ alkoxy;
R² is selected from C₁₋₁₂ alkyl, C₁₋₁₂ alkoxy, halogen, at least one halogen-substituted C₁₋₆ alkyl, at least one halogen-substituted C₁₋₆ alkoxy and cyano;
R³ is selected from H, C₁₋₁₂ alkyl, C₁₋₁₂ alkoxy, C₃₋₁₂ cycloalkyl, -CO-R₃₁, -CO-CH₂-R₃₁; R₃₁ is selected from C₁₋₁₂ alkyl, C₃₋₁₂ cycloalkyl and C₁₋₁₂ alkoxy;
R⁴, R⁵ are respectively and independently selected from H, C₁₋₁₂ alkyl, C₁₋₁₂ alkoxy, halogen, at least one halogen-substituted C₁₋₆ alkyl and at least one halogen-substituted C₁₋₆ alkoxy;
R⁶ is selected from the group consisting of H, C₁₋₁₂ alkyl unsubstituted or substituted by at least one group from hydroxyl, halogen, C₁₋₁₂ alkyl, C₁₋₁₂ alkoxy and trimethylsilyl, C₁₋₁₂ alkoxy unsubstituted or substituted by at least one group from hydroxyl, halogen, C₁₋₁₂ alkyl, C₁₋₁₂ alkoxy and trimethylsilyl, C₃₋₁₂ cycloalkyl unsubstituted or substituted by at least one group from hydroxyl, halogen, C₁₋₁₂ alkyl, C₁₋₁₂ alkoxy and trimethylsilyl, phenyl unsubstituted or substituted by at least one group from hydroxyl, halogen, C₁₋₁₂ alkyl, C₁₋₁₂ alkoxy and trimethylsilyl, C₃₋₁₂ cycloalkyl containing at least one heteroatom as a ring-forming atom, C₂₋₁₂ hydrocarbon containing at least one unsaturated carbon-carbon double bond or unsaturated carbon-carbon triple bond which is unsubstituted or substituted by at least one group from hydroxyl, halogen, C₁₋₁₂ alkyl, C₁₋₁₂ alkoxy and trimethylsilyl, -(CH₂)ₙ-R₆₁, -CO-R₆₂, -COO-R₆₂, -CO-N(R₆₃R₆₄), -S(=O)₂-R₆₂, - (CH₂)ₙ-CO-R₆₂, R₆₁ is selected from the group consisting of C₃₋₁₂ cycloalkyl, C₃₋₁₂ cycloalkyl containing at least one heteroatom as a ring-forming atom, phenyl, R₆₂ is selected from the group consisting of C₁₋₁₂ alkyl, phenyl unsubstituted or substituted by at least one of hydroxyl, halogen, C₁₋₁₂ alkyl, C₁₋₁₂ alkoxy and trimethylsilyl, C₃₋₁₂ cycloalkyl, C₃₋₁₂ cycloalkyl containing at least one heteroatom as a ring-forming atom; R₆₃, R₆₄ are independently selected from C₁₋₁₂ alkyl; each of the heteroatoms is independently at least one selected fromN, O, S; each n is independently 1 or 2;

In a second aspect, the present invention provides the non-therapeutic use of the compound containing a pyrazole structure according to the first aspect, or an agrochemically acceptable salt, hydrate, and solvate thereof, for at least one of insect killing, mite killing, nematode killing, and piercing-sucking insects killing.

A third aspect of the present invention provides the non-therapeutic use of the aforementioned pyrazole structure-containing compound or an agrochemically acceptable salt, hydrate and solvate thereof as an agrochemical insecticide. A fourth aspect of the present invention provides an insecticide containing a pyrazole structure or an agrochemically acceptable salt, hydrate and solvate thereof as an active ingredient.

The compound or the agrochemically acceptable salt, hydrate and solvate of the compound has excellent control effect on pests such as *Plutella xylostella, Spodoptera frugiperda, Spodoptera litura, Myzus persicae, Spodoptera exigua, Ostrinia furnacalis, Helicoverpa armigera, Chilo suppressalis, Cnaphalocrocis medinalis, Frankliniella occidentalis,* flea beetle and the like, and has good market development prospect.

The compound or the agrochemically acceptable salt, hydrate and solvate thereof provided by the invention can also effectively control mutant pests which have resistance to the existing insecticide, and has important significance for developing novel insecticides without cross resistance.

### Detailed Description

Some exemplary explanations are provided below for the partial groups of the present invention, and the unrecited parts are explained with reference to the following exemplary explanations without particular description.

"halogen" means fluorine, chlorine, bromine, iodine.

"C₁₋₆ alkyl" refers to a straight or branched chain alkyl group having a total number of carbon atoms of 1, 2, 3, 4, 5 or 6. Such as methyl, ethyl, n-propyl, isopropyl, n-butyl, isobutyl, tert-butyl, and the like. "C₁₋₈ alkyl", "C₁₋₁₀ alkyl", "C₁₋₁₂ alkyl" have similar definitions, except that the total number of carbon atoms is different.

"C₁₋₆ alkoxy" means a straight or branched chain alkoxy group having a total number of carbon atoms of 1, 2, 3, 4, 5 or 6. Such as methoxy, ethoxy, n-propoxy, isopropoxy, n-butoxy, isobutoxy, tert-butoxy and the like. "C₁₋₈ alkoxy", "C₁₋₁₀ alkoxy" and "C₁₋₁₂ alkoxy" have similar definitions, only differing in the total number of carbon atoms.

"at least one halogen-substituted C₁₋₆ alkyl" means that at least one H atom in the "C₁₋₆ alkyl" is substituted by halogen, and the substituted halogen may be one or more. "at least one halogen-substituted C₁₋₆ alkoxy" has a similar definition to that of "C₁₋₆ alkyl" merely replacing it with "C₁₋₆ alkoxy".

"C₃₋₁₀ cycloalkyl" refers to cycloalkyl having 3-10 carbon atoms in total, and the ring-forming atoms are all C atoms, and may be, for example, 3, 4, 5, 6, 7, 8, 9, 10 ring-forming carbon atoms in total, and any position on the "C₃₋₁₀ cycloalkyl" which may be substituted is directly connected to the parent nucleus. "C₃₋₆ cycloalkyl" and "C₃₋₁₂ cycloalkyl" have similar definitions, except for the total number of carbon atoms.

"C₁₋₁₂ alkyl unsubstituted or substituted by at least one group from hydroxyl, halogen, C₁₋₁₂ alkyl, C₁₋₁₂ alkoxy and trimethylsilyl" means that there is no substituent in "C₁₋₁₂ alkyl" or that it is substituted by one or more groups from hydroxyl, halogen, C₁₋₁₂ alkyl, C₁₋₁₂ alkoxy and trimethylsilyl. "C₁₋₁₀ alkyl unsubstituted or substituted by at least one group from hydroxyl, halogen, C₁₋₈ alkyl, C₁₋₈ alkoxy and trimethylsilyl" has a similar definition only for the total number of carbon atoms.

"C₁₋₁₂ alkoxy unsubstituted or substituted by at least one group from hydroxyl, halogen, C₁₋₁₂ alkyl, C₁₋₁₂ alkoxy and trimethylsilyl" means that there are no substituents in "C₁₋₁₂ alkoxy" or that there are substitutions by one or more groups from hydroxyl, halogen, C₁₋₁₂ alkyl, C₁₋₁₂ alkoxy and trimethylsilyl. "alkoxy of C₁₋₁₀ unsubstituted or substituted by at least one group from hydroxyl, halogen, C₁₋₈ alkyl, C₁₋₈ alkoxy and trimethylsilyl" has a similar definition, merely differing in the total number of carbon atoms.

"C₃₋₁₂ cycloalkyl unsubstituted or substituted by at least one group from hydroxyl, halogen, C₁₋₁₂ alkyl, C₁₋₁₂ alkoxy and trimethylsilyl" means the case where "C₃₋₁₂ alkoxy" has no substituent or is substituted by one or more groups from hydroxyl, halogen, C₁₋₁₂ alkyl, C₁₋₁₂ alkoxy and trimethylsilyl. "C₃₋₁₀ cycloalkyl unsubstituted or substituted by at least one group from hydroxyl, halogen, C₁₋₈ alkyl, C₁₋₈ alkoxy and trimethylsilyl" has a similar definition, merely differing in the total number of carbon atoms.

"phenyl unsubstituted or substituted by at least one group from hydroxyl, halogen, C₁₋₁₂ alkyl, C₁₋₁₂ alkoxy and trimethylsilyl" means the case where "phenyl" has no substituent or is substituted by one or more groups from hydroxyl, halogen, C₁₋₁₂ alkyl, C₁₋₁₂ alkoxy and trimethylsilyl.

"C₃₋₁₂ cycloalkyl containing at least one hetero atom as a ring-forming atom" means that at least one of the ring-forming atoms of "C₃₋₁₂ cycloalkyl" is a hetero atom other than carbon, and may be exemplified by an oxygen atom, a sulfur atom and a nitrogen atom. "C₃₋₁₀ cycloalkyl containing at least one hetero atom as a ring-constituting atom" has a similar definition to that of "C₃₋₁₂ cycloalkyl" except that the total number of carbon atoms is different from that of "C₃₋₁₂ cycloalkyl".

"C₂₋₁₂ hydrocarbon containing at least one unsaturated carbon-carbon double bond or unsaturated carbon-carbon triple bond" means that the C₂₋₁₂ hydrocarbon contains at least one unsaturated carbon-carbon double bond or unsaturated carbon-carbon triple bond. "C₂₋₁₀ hydrocarbyl containing at least one unsaturated carbon-carbon double bond or unsaturated carbon-carbon triple bond" has a similar definition, except that the total number of carbon atoms is different.

"C₂₋₁₂ hydrocarbon containing at least one unsaturated carbon-carbon double bond or unsaturated carbon-carbon triple bond which is unsubstituted or substituted by at least one group from hydroxyl, halogen, C₁₋₁₂ alkyl, C₁₋₁₂ alkoxy and trimethylsilyl" means the case where there is no substituent in "C₂₋₁₂ hydrocarbon radical containing at least one unsaturated carbon-carbon double bond or unsaturated carbon-carbon triple bond" or where there is substitution by one or more groups from hydroxyl, halogen, C₁₋₁₂ alkyl, C₁₋₁₂ alkoxy and trimethylsilyl. "C₂₋₁₀ hydrocarbon containing at least one unsaturated carbon-carbon double bond or unsaturated carbon-carbon triple bond, unsubstituted or substituted by at least one group from hydroxyl, halogen, C₁₋₈ alkyl, C₁₋₈ alkoxy and trimethylsilyl" has a similar definition, merely differing in the total number of carbon atoms.

As described hereinbefore, the first aspect of the present invention provides a compound having a pyrazole structure, or an agrochemically acceptable salt, hydrate and solvate thereof, the compound having a structure represented by formula (I):

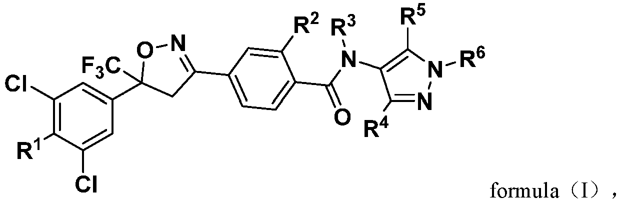

Wherein, in the formula (I),
R' is selected from H, halogen, at least one halogen substituted C₁₋₆ alkyl, at least one halogen substituted C₁₋₆ alkoxy;
R² is selected from C₁₋₁₂ alkyl, C₁₋₁₂ alkoxy, halogen, at least one halogen-substituted C₁₋₆ alkyl, at least one halogen-substituted C₁₋₆ alkoxy and cyano;
R³ is selected from H, C₁₋₁₂ alkyl, C₁₋₁₂ alkoxy, C₃₋₁₂ cycloalkyl, -CO-R₃₁, -CO-CH₂-R₃₁; R₃₁ is selected from C₁₋₁₂ alkyl, C₃₋₁₂ cycloalkyl and C₁₋₁₂ alkoxy;
R⁴, R⁵ are respectively and independently selected from H, C₁₋₁₂ alkyl, C₁₋₁₂ alkoxy, halogen, at least one halogen-substituted C₁₋₆ alkyl and at least one halogen-substituted C₁₋₆ alkoxy;
R⁶ is selected from the group consisting of H, C₁₋₁₂ alkyl unsubstituted or substituted by at least one group from hydroxyl, halogen, C₁₋₁₂ alkyl, C₁₋₁₂ alkoxy and trimethylsilyl, C₁₋₁₂ alkoxy unsubstituted or substituted by at least one group from hydroxyl, halogen, C₁₋₁₂ alkyl, C₁₋₁₂ alkoxy and trimethylsilyl, C₃₋₁₂ cycloalkyl unsubstituted or substituted by at least one group from hydroxyl, halogen, C₁₋₁₂ alkyl, C₁₋₁₂ alkoxy and trimethylsilyl, phenyl unsubstituted or substituted by at least one group from hydroxyl, halogen, C₁₋₁₂ alkyl, C₁₋₁₂ alkoxy and trimethylsilyl, C₃₋₁₂ cycloalkyl containing at least one heteroatom as a ring-forming atom, C₂₋₁₂ hydrocarbon containing at least one unsaturated carbon-carbon double bond or unsaturated carbon-carbon triple bond which is unsubstituted or substituted by at least one group from hydroxyl, halogen, C₁₋₁₂ alkyl, C₁₋₁₂ alkoxy and trimethylsilyl, -(CH₂)ₙ-R₆₁, -CO-R₆₂, -COO-R₆₂, -CO-N(R₆₃R₆₄), -S(=O)₂-R₆₂, - (CH₂)ₙ-CO-R₆₂, R₆₁ is selected from the group consisting of C₃₋₁₂ cycloalkyl, C₃₋₁₂ cycloalkyl containing at least one heteroatom as a ring-forming atom, phenyl, R₆₂ is selected from the group consisting of C₁₋₁₂ alkyl, phenyl unsubstituted or substituted by at least one of hydroxyl, halogen, C₁₋₁₂ alkyl, C₁₋₁₂ alkoxy and trimethylsilyl, C₃₋₁₂ cycloalkyl, C₃₋₁₂ cycloalkyl containing at least one heteroatom as a ring-forming atom; R₆₃, R₆₄ are independently selected from C₁₋₁₂ alkyl; each of the heteroatoms is independently at least one selected from N, O, S; each n is independently 1 or 2;

Preferably, in formula (I), R¹ is selected from H, halogen, at least one halogen-substituted C₁₋₆ alkyl, at least one halogen-substituted C₁₋₆ alkoxy; more preferably, R¹ is selected from H, F, chloro, bromo.

Preferably, in the formula (I), R² is selected from C₁₋₈ alkyl, C₁₋₈ alkoxy, halogen, at least one halogen-substituted C₁₋₆ alkyl, at least one halogen-substituted C₁₋₆ alkoxy and cyano; more preferably, R² is selected from methyl, ethyl, n-propyl, isopropyl, n-butyl, tert-butyl, cyano.

Preferably, in formula (I), R³ is selected from the group consisting of H, C₁₋₈ alkyl, C₁₋₈ alkoxy, C₃₋₁₀ cycloalkyl, - CO-R₃₁, -CO-CH₂-R₃₁; R₃₁ is selected from C₁₋₈ alkyl, C₃₋₁₀ cycloalkyl and C₁₋₈ alkoxy; more preferably, R³ is selected from the group consisting of H, methyl, ethyl, n-propyl, isopropyl, n-butyl, tert-butyl, methoxy, ethoxy, n-propoxy, isopropoxy, n-butoxy, tert-butoxy, cyclopropyl, cyclobutyl, cyclopentyl, cyclohexyl, -CO-R₃₁, -CO-CH₂-R₃₁; R₃₁ is selected from C₁₋₆ alkyl, C₃₋₆ cycloalkyl and C₁₋₆ alkoxy.

Preferably, in the formula (I), R⁴, R⁵ are independently selected from H, C₁₋₈ alkyl, C₁₋₈ alkoxy, halogen, at least one halogen-substituted C₁₋₆ alkyl and at least one halogen-substituted C₁₋₆ alkoxy; more preferably, R⁴, R⁵ are each independently selected from H, methyl, ethyl, n-propyl, isopropyl, n-butyl, tert-butyl.

Preferably, in formula (I), R⁶ is selected from the group consisting of H, C₁₋₁₀ alkyl unsubstituted or substituted by at least one group from hydroxyl, halogen, C₁₋₈ alkyl, C₁₋₈ alkoxy and trimethylsilyl, C₁₋₁₀ alkoxy unsubstituted or substituted by at least one group from hydroxyl, halogen, C₁₋₈ alkyl, C₁₋₈ alkoxy and trimethylsilyl, C₃₋₁₀ cycloalkyl unsubstituted or substituted by at least one group from hydroxyl, halogen, C₁₋₈ alkyl, C₁₋₈ alkoxy and trimethylsilyl, phenyl unsubstituted or substituted by at least one group from hydroxyl, halogen, C₁₋₈ alkyl, C₁₋₈ alkoxy and trimethylsilyl, C₃₋₁₀ cycloalkyl containing at least one heteroatom as a ring-forming atom, C₂₋₁₀ hydrocarbon containing at least one unsaturated carbon-carbon double bond or unsaturated carbon-carbon triple bond which is unsubstituted or substituted by at least one group from hydroxyl, halogen, C₁₋₈ alkyl, C₁₋₈ alkoxy and trimethylsilyl, -(CH₂)ₙ-R₆₁, -CO-R₆₂, -COO-R₆₂, -CO-N(R₆₃R₆₄), - S(=O)₂-R₆₂, -(CH₂)ₙ-CO-R₆₂; R₆₁ is selected from the group consisting of C₃₋₁₀ cycloalkyl, C₃₋₁₀ cycloalkyl containing at least one heteroatom as a ring-forming atom, phenyl; R₆₂ is selected from the group consisting of C₁₋₁₀ alkyl, phenyl unsubstituted or substituted by at least one of the groups in hydroxyl, halogen, C₁₋₈ alkyl, C₁₋₈ alkoxy and trimethylsilyl, C₃₋₁₀ cycloalkyl, C₃₋₁₀ cycloalkyl containing at least one heteroatom as a ring-forming atom; R₆₃, R₆₄ are independently selected from C₁₋₁₀ alkyl; each of the heteroatoms is independently at least one selected from N, O, S; each n is independently 1 or 2; R⁶ is selected from the group consisting of H, C₁₋₁₀ alkyl unsubstituted or substituted by at least one group from hydroxyl, halogen, C₁₋₈ alkyl, C₁₋₈ alkoxy and trimethylsilyl,, C₁₋₁₀ alkoxy unsubstituted or substituted by at least one group from hydroxyl, halogen, C₁₋₈ alkyl, C₁₋₈ alkoxy and trimethylsilyl,, C₃₋₁₀ cycloalkyl unsubstituted or substituted by at least one group from hydroxyl, halogen, C₁₋₈ alkyl, C₁₋₈ alkoxy and trimethylsilyl, phenyl unsubstituted or substituted by at least one group from hydroxyl, halogen, C₁₋₈ alkyl, C₁₋₈ alkoxy and trimethylsilyl,, C₃₋₁₀ cycloalkyl containing at least one heteroatom as a ring-forming atom, C₂₋₁₀ hydrocarbon containing at least one unsaturated carbon-carbon double bond or unsaturated carbon-carbon triple bond which is unsubstituted or substituted by at least one group from hydroxyl, halogen, C₁₋₈ alkyl, C₁₋₈ alkoxy and trimethylsilyl,, -(CH₂)ₙ-R₆₁, -CO-R₆₂, -COO-R₆₂, -CO-N(R₆₃R₆₄), - S(=O)₂-R₆₂, -(CH₂)ₙ-CO-R₆₂; R₆₁ is selected from the group consisting of C₃₋₁₀ cycloalkyl, C₃₋₁₀ cycloalkyl containing at least one heteroatom as a ring-forming atom, phenyl; R₆₂ is selected from the group consisting of C₁₋₁₀ alkyl, phenyl unsubstituted or substituted by at least one of the groups in hydroxyl, halogen, C₁₋₈ alkyl, C₁₋₈ alkoxy and trimethylsilyl, C₃₋₁₀ cycloalkyl, C₃₋₁₀ cycloalkyl containing at least one heteroatom as a ring-forming atom; R₆₃, R₆₄ are independently selected from C₁₋₁₀ alkyl; each of the heteroatoms is independently at least one selected from N, O, S; each n is independently 1 or 2.

Preferably, in the formula (I),
R¹ is selected from H, halogen, at least one halogen substituted C₁₋₆ alkyl, at least one halogen substituted C₁₋₆ alkoxy;
R² is selected from C₁₋₈ alkyl, C₁₋₈ alkoxy, halogen, at least one halogen-substituted C₁₋₆ alkyl, at least one halogen-substituted C₁₋₆ alkoxy and cyano;
R³ is selected from H, C₁₋₈ alkyl, C₁₋₈ alkoxy, C₃₋₁₀ cycloalkyl, -CO-R₃₁, -CO-CH₂-R₃₁; R₃₁ is selected from C₁₋₈ alkyl, C₃₋₁₀ cycloalkyl and C₁₋₈ alkoxy;
R⁴, R⁵ are respectively and independently selected from H, C₁₋₈ alkyl, C₁₋₈ alkoxy, halogen, at least one halogen-substituted C₁₋₆ alkyl and at least one halogen-substituted C₁₋₆ alkoxy;
R⁶ is selected from the group consisting of H, C₁₋₁₀ alkyl unsubstituted or substituted by at least one group from hydroxyl, halogen, C₁₋₈ alkyl, C₁₋₈ alkoxy and trimethylsilyl, C₁₋₁₀ alkoxy unsubstituted or substituted by at least one group from hydroxyl, halogen, C₁₋₈ alkyl, C₁₋₈ alkoxy and trimethylsilyl, C₃₋₁₀ cycloalkyl unsubstituted or substituted by at least one group from hydroxyl, halogen, C₁₋₈ alkyl, C₁₋₈ alkoxy and trimethylsilyl, phenyl unsubstituted or substituted by at least one group from hydroxyl, halogen, C₁₋₈ alkyl, C₁₋₈ alkoxy and trimethylsilyl, C₃₋₁₀ cycloalkyl containing at least one heteroatom as a ring-forming atom, C₂₋₁₀ hydrocarbon containing at least one unsaturated carbon-carbon double bond or unsaturated carbon-carbon triple bond which is unsubstituted or substituted by at least one group from hydroxyl, halogen, C₁₋₈ alkyl, C₁₋₈ alkoxy and trimethylsilyl, -(CH₂)ₙ-R₆₁, -CO-R₆₂, -COO-R₆₂, -CO-N(R₆₃R₆₄), -S(=O)₂-R₆₂, -(CH₂)ₙ-CO-R₆₂; R₆₁ is selected from the group consisting of C₃₋₁₀ cycloalkyl, C₃₋₁₀ cycloalkyl containing at least one heteroatom as a ring-forming atom, phenyl; R₆₂ is selected from the group consisting of C₁₋₁₀ alkyl, phenyl unsubstituted or substituted by at least one of the groups in hydroxyl, halogen, C₁₋₈ alkyl, C₁₋₈ alkoxy and trimethylsilyl, C₃₋₁₀ cycloalkyl, C₃₋₁₀ cycloalkyl containing at least one heteroatom as a ring-forming atom; R₆₃ and R₆₄ are independently selected from C₁₋₁₀ alkyl; each of the heteroatoms is independently at least one selected from N, O, S; each n is independently 1 or 2.

More preferably, in formula (I),
R¹ is selected from H, F, chloro, bromo;
R² is selected from methyl, ethyl, n-propyl, isopropyl, n-butyl, tert-butyl, cyano;
R³ is selected from the group consisting of H, methyl, ethyl, n-propyl, isopropyl, n-butyl, tert-butyl, methoxy, ethoxy, n-propoxy, isopropoxy, n-butoxy, tert-butoxy, cyclopropyl, cyclobutyl, cyclopentyl, cyclohexyl, -CO-R₃₁, -CO-CH₂-R₃₁; R₃₁ is selected from C₁₋₆ alkyl, C₃₋₆ cycloalkyl and C₁₋₆ alkoxy;
R⁴, R⁵ are each independently selected from H, methyl, ethyl, n-propyl, isopropyl, n-butyl, tert-butyl;
R⁶ is selected from the group consisting of H, C₁₋₁₀ alkyl unsubstituted or substituted by at least one group from hydroxyl, halogen, C₁₋₈ alkyl, C₁₋₈ alkoxy and trimethylsilyl, C₁₋₁₀ alkoxy unsubstituted or substituted by at least one group from hydroxyl, halogen, C₁₋₈ alkyl, C₁₋₈ alkoxy and trimethylsilyl, C₃₋₁₀ cycloalkyl unsubstituted or substituted by at least one group from hydroxyl, halogen, C₁₋₈ alkyl, C₁₋₈ alkoxy and trimethylsilyl, phenyl unsubstituted or substituted by at least one group from hydroxyl, halogen, C₁₋₈ alkyl, C₁₋₈ alkoxy and trimethylsilyl, C₃₋₁₀ cycloalkyl containing at least one heteroatom as a ring-forming atom, C₂₋₁₀ hydrocarbon containing at least one unsaturated carbon-carbon double bond or unsaturated carbon-carbon triple bond which is unsubstituted or substituted by at least one group from hydroxyl, halogen, C₁₋₈ alkyl, C₁₋₈ alkoxy and trimethylsilyl, -(CH₂)ₙ-R₆₁, -CO-R₆₂, -COO-R₆₂, -CO-N(R₆₃R₆₄), -S(=O)₂-R₆₂, -(CH₂)ₙ-CO-R₆₂; R₆₁ is selected from the group consisting of C₃₋₁₀ cycloalkyl, C₃₋₁₀ cycloalkyl containing at least one heteroatom as a ring-forming atom, phenyl; R₆₂ is selected from the group consisting of C₁₋₁₀ alkyl, phenyl unsubstituted or substituted by at least one of the groups in hydroxyl, halogen, C₁₋₈ alkyl, C₁₋₈ alkoxy and trimethylsilyl, C₃₋₁₀ cycloalkyl, C₃₋₁₀ cycloalkyl containing at least one heteroatom as a ring-forming atom; R₆₃, R₆₄ are independently selected from C₁₋₁₀ alkyl; each of the heteroatoms is independently at least one selected from N, O, S; each n is independently 1 or 2.

According to a particularly preferred embodiment, the compound is selected from any one of the following:
Number A1: R¹ is H, R² is CH₃, R³ is H, R⁴ is H, R⁵ is H, R⁶ is -H;
Number A2: R¹ is H, R² is CH₃, R³ is H, R⁴ is H, R⁵ is H, R⁶ is -CH₃;
Number A3: R¹ is H, R² is CH₃, R³ is H, R⁴ is H, R⁵ is H, R⁶ is -CHF₂;
Number A4: R¹ is H, R² is CH₃, R³ is H, R⁴ is H, R⁵ is H, R⁶ is -CF₃;
Number A5: R¹ is H, R² is CH₃, R³ is H, R⁴ is H, R⁵ is H, R⁶ is -CH₂CH₃;
Number A6: R¹ is H, R² is CH₃, R³ is H, R⁴ is H, R⁵ is H, R⁶ is -CH₂CH₂CH₃;
Number A7: R¹ is H, R² is CH₃, R³ is H, R⁴ is H, R⁵ is H, R⁶ is -CH(CH₃)₂;
Number A8: R¹ is H, R² is CH₃, R³ is H, R⁴ is H, R⁵ is H, R⁶ is -CH₂CH₂OH;
Number A9: R¹ is H, R² is CH₃, R³ is H, R⁴ is H, R⁵ is H, R⁶ is -CH₂CF₃;
Number A10: R¹ is H, R² is CH₃, R³ is H, R⁴ is H, R⁵ is H, R⁶ is -CH₂CHF₂;
Number A11: R¹ is H, R² is CH₃, R³ is H, R⁴ is H, R⁵ is H, R⁶ is -CH₂CH₂F;
Number A12: R¹ is H, R² is CH₃, R³ is H, R⁴ is H, R⁵ is H, R⁶ is -CH₂CH₂CF₃;
Number A13: R¹ is H, R² is CH₃, R³ is H, R⁴ is H, R⁵ is H, R⁶ is -CH₂CH₂CH₂F;
Number A14: R¹ is H, R² is CH₃, R³ is H, R⁴ is H, R⁵ is H, R⁶ is -CH₂CH₂OCH₃;
Number A15: R¹ is H, R² is CH₃, R³ is H, R⁴ is H, R⁵ is H, R⁶ is -CH₂CH₂OCH₂CH₃;
Number A16: R¹ is H, R² is CH₃, R³ is H, R⁴ is H, R⁵ is H, R⁶ is -CH₂(CH₂)₂OCH₃;
Number A17: R¹ is H, R² is CH₃, R³ is H, R⁴ is H, R⁵ is H, R⁶ is -CH₂CH₂CH₂CH₃;
Number A18: R¹ is H, R² is CH₃, R³ is H, R⁴ is H, R⁵ is H, R⁶ is -CH(CH₃)CH₂CH₃;
Number A19: R¹ is H, R² is CH₃, R³ is H, R⁴ is H, R⁵ is H, R⁶ is -CH₂(CH₂)₃CH₃;
Number A20: R¹ is H, R² is CH₃, R³ is H, R⁴ is H, R⁵ is H, R⁶ is 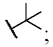
Number A21: R¹ is H, R² is CH₃, R³ is H, R⁴ is H, R⁵ is H, R⁶ is 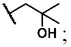
Number A22: R¹ is H, R² is CH₃, R³ is H, R⁴ is H, R⁵ is H, R⁶ is 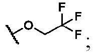
Number A23: R¹ is H, R² is CH₃, R³ is H, R⁴ is H, R⁵ is H, R⁶ is 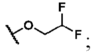
Number A24: R¹ is H, R² is CH₃, R³ is H, R⁴ is H, R⁵ is H, R⁶ is 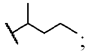
Number A25: R¹ is H, R² is CH₃, R³ is H, R⁴ is H, R⁵ is H, R⁶ is 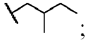
Number A26: R¹ is H, R² is CH₃, R³ is H, R⁴ is H, R⁵ is H, R⁶ is 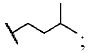
Number A27: R¹ is H, R² is CH₃, R³ is H, R⁴ is H, R⁵ is H, R⁶ is 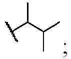
Number A28: R¹ is H, R² is CH₃, R³ is H, R⁴ is H, R⁵ is H, R⁶ is 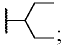
Number A29: R¹ is H, R² is CH₃, R³ is H, R⁴ is H, R⁵ is H, R⁶ is 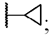
Number A30: R¹ is H, R² is CH₃, R³ is H, R⁴ is H, R⁵ is H, R⁶ is 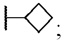
Number A31: R¹ is H, R² is CH₃, R³ is H, R⁴ is H, R⁵ is H, R⁶ is 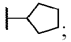
Number A32: R¹ is H, R² is CH₃, R³ is H, R⁴ is H, R⁵ is H, R⁶ is 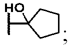
Number A33: R¹ is H, R² is CH₃, R³ is H, R⁴ is H, R⁵ is H, R⁶ is 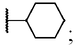
Number A34: R¹ is H, R² is CH₃, R³ is H, R⁴ is H, R⁵ is H, R⁶ is 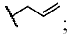
Number A35: R¹ is H, R² is CH₃, R³ is H, R⁴ is H, R⁵ is H, R⁶ is 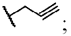
Number A36: R¹ is H, R² is CH₃, R³ is H, R⁴ is H, R⁵ is H, R⁶ is 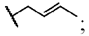
Number A37: R¹ is H, R² is CH₃, R³ is H, R⁴ is H, R⁵ is H, R⁶ is 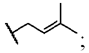
Number A38: R¹ is H, R² is CH₃, R³ is H, R⁴ is H, R⁵ is H, R⁶ is 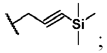
Number A39: R¹ is H, R² is CH₃, R³ is H, R⁴ is H, R⁵ is H, R⁶ is 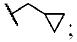
Number A40: R¹ is H, R² is CH₃, R³ is H, R⁴ is H, R⁵ is H, R⁶ is 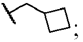
Number A41: R¹ is H, R² is CH₃, R³ is H, R⁴ is H, R⁵ is H, R⁶ is 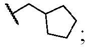
Number A42: R¹ is H, R² is CH₃, R³ is H, R⁴ is H, R⁵ is H, R⁶ is 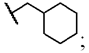
Number A43: R¹ is H, R² is CH₃, R³ is H, R⁴ is H, R⁵ is H, R⁶ is 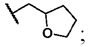
Number A44: R¹ is H, R² is CH₃, R³ is H, R⁴ is H, R⁵ is H, R⁶ is 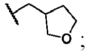
Number A45: R¹ is H, R² is CH₃, R³ is H, R⁴ is H, R⁵ is H, R⁶ is 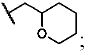
Number A46: R¹ is H, R² is CH₃, R³ is H, R⁴ is H, R⁵ is H, R⁶ is 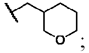
Number A47: R¹ is H, R² is CH₃, R³ is H, R⁴ is H, R⁵ is H, R⁶ is 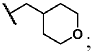
Number A48: R¹ is H, R² is CH₃, R³ is H, R⁴ is H, R⁵ is H, R⁶ is 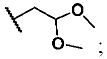
Number A49: R¹ is H, R² is CH₃, R³ is H, R⁴ is H, R⁵ is H, R⁶ is 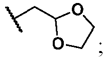
Number A50: R¹ is H, R² is CH₃, R³ is H, R⁴ is H, R⁵ is H, R⁶ is 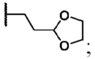
Number A51: R¹ is H, R² is CH₃, R³ is H, R⁴ is H, R⁵ is H, R⁶ is 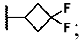
Number A52: R¹ is H, R² is CH₃, R³ is H, R⁴ is H, R⁵ is H, R⁶ is 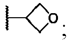
Number A53: R¹ is H, R² is CH₃, R³ is H, R⁴ is H, R⁵ is H, R⁶ is 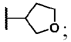
Number A54: R¹ is H, R² is CH₃, R³ is H, R⁴ is H, R⁵ is H, R⁶ is 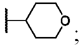
Number A55: R¹ is H, R² is CH₃, R³ is H, R⁴ is H, R⁵ is H, R⁶ is 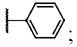
Number A56: R¹ is H, R² is CH₃, R³ is H, R⁴ is H, R⁵ is H, R⁶ is 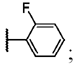
Number A57: R¹ is H, R² is CH₃, R³ is H, R⁴ is H, R⁵ is H, R⁶ is 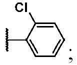
Number A58: R¹ is H, R² is CH₃, R³ is H, R⁴ is H, R⁵ is H, R⁶ is 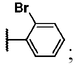
Number A59: R¹ is H, R² is CH₃, R³ is H, R⁴ is H, R⁵ is H, R⁶ is 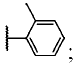
Number A60: R¹ is H, R² is CH₃, R³ is H, R⁴ is H, R⁵ is H, R⁶ is 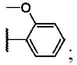
Number A61: R¹ is H, R² is CH₃, R³ is H, R⁴ is H, R⁵ is H, R⁶ is 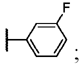
Number A62: R¹ is H, R² is CH₃, R³ is H, R⁴ is H, R⁵ is H, R⁶ is 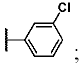
Number A63: R¹ is H, R² is CH₃, R³ is H, R⁴ is H, R⁵ is H, R⁶ is 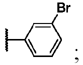
Number A64: R¹ is H, R² is CH₃, R³ is H, R⁴ is H, R⁵ is H, R⁶ is 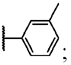
Number A65: R¹ is H, R² is CH₃, R³ is H, R⁴ is H, R⁵ is H, R⁶ is 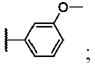
Number A66: R¹ is H, R² is CH₃, R³ is H, R⁴ is H, R⁵ is H, R⁶ is 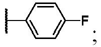
Number A67: R¹ is H, R² is CH₃, R³ is H, R⁴ is H, R⁵ is H, R⁶ is 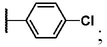
Number A68: R¹ is H, R² is CH₃, R³ is H, R⁴ is H, R⁵ is H, R⁶ is 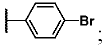
Number A69: R¹ is H, R² is CH₃, R³ is H, R⁴ is H, R⁵ is H, R⁶ is 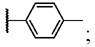
Number A70: R¹ is H, R² is CH₃, R³ is H, R⁴ is H, R⁵ is H, R⁶ is 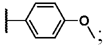
Number A71: R¹ is H, R² is CH₃, R³ is H, R⁴ is H, R⁵ is H, R⁶ is 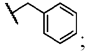
Number A72: R¹ is H, R² is CH₃, R³ is H, R⁴ is H, R⁵ is H, R⁶ is -COCH₃;
Number A73: R¹ is H, R² is CH₃, R³ is H, R⁴ is H, R⁵ is H, R⁶ is -COCH₂CH₃;
Number A74: R¹ is H, R² is CH₃, R³ is H, R⁴ is H, R⁵ is H, R⁶ is -COCH₂CH₂CH₃;
Number A75: R¹ is H, R² is CH₃, R³ is H, R⁴ is H, R⁵ is H, R⁶ is -COCH(CH₃)₂;
Number A76: R¹ is H, R² is CH₃, R³ is H, R⁴ is H, R⁵ is H, R⁶ is -COC₆H₅;
Number A77: R¹ is H, R² is CH₃, R³ is H, R⁴ is H, R⁵ is H, R⁶ is 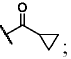
Number A78: R¹ is H, R² is CH₃, R³ is H, R⁴ is H, R⁵ is H, R⁶ is 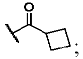
Number A79: R¹ is H, R² is CH₃, R³ is H, R⁴ is H, R⁵ is H, R⁶ is 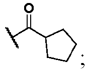
Number A80: R¹ is H, R² is CH₃, R³ is H, R⁴ is H, R⁵ is H, R⁶ is 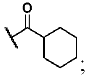
Number A81: R¹ is H, R² is CH₃, R³ is H, R⁴ is H, R⁵ is H, R⁶ is 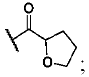
Number A82: R¹ is H, R² is CH₃, R³ is H, R⁴ is H, R⁵ is H, R⁶ is 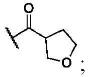
Number A83: R¹ is H, R² is CH₃, R³ is H, R⁴ is H, R⁵ is H, R⁶ is 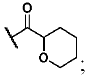
Number A84: R¹ is H, R² is CH₃, R³ is H, R⁴ is H, R⁵ is H, R⁶ is 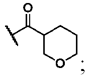
Number A85: R¹ is H, R² is CH₃, R³ is H, R⁴ is H, R⁵ is H, R⁶ is 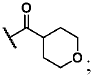
Number A86: R¹ is H, R² is CH₃, R³ is H, R⁴ is H, R⁵ is H, R⁶ is 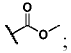
Number A87: R¹ is H, R² is CH₃, R³ is H, R⁴ is H, R⁵ is H, R⁶ is 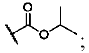
Number A88: R¹ is H, R² is CH₃, R³ is H, R⁴ is H, R⁵ is H, R⁶ is 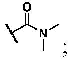
Number A89: R¹ is H, R² is CH₃, R³ is H, R⁴ is H, R⁵ is H, R⁶ is 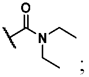
Number A90: R¹ is H, R² is CH₃, R³ is H, R⁴ is H, R⁵ is H, R⁶ is -S(=O)₂CH₃;
Number A91: R¹ is H, R² is CH₃, R³ is H, R⁴ is H, R⁵ is H, R⁶ is -S(=O)₂CH₂CH₃;
Number A92: R¹ is H, R² is CH₃, R³ is H, R⁴ is H, R⁵ is H, R⁶ is -S(=O)₂CH₂CH₂CH₃;
Number A93: R¹ is H, R² is CH₃, R³ is H, R⁴ is H, R⁵ is H, R⁶ is -S(=O)₂CH(CH₃)₂;
Number A94: R¹ is H, R² is CH₃, R³ is H, R⁴ is H, R⁵ is H, R⁶ is -S(=O)₂C₆H₅;
Number A95: R¹ is H, R² is CH₃, R³ is H, R⁴ is H, R⁵ is H, R⁶ is 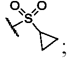
Number A96: R¹ is H, R² is CH₃, R³ is H, R⁴ is H, R⁵ is H, R⁶ is 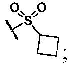
Number A97: R¹ is H, R² is CH₃, R³ is H, R⁴ is H, R⁵ is H, R⁶ is 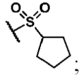
Number A98: R¹ is H, R² is CH₃, R³ is H, R⁴ is H, R⁵ is H, R⁶ is 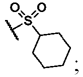
Number A99: R¹ is H, R² is CH₃, R³ is H, R⁴ is H, R⁵ is H, R⁶ is 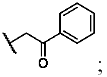
Number A100: R¹ is H, R² is CH₃, R³ is H, R⁴ is H, R⁵ is H, R⁶ is 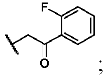
Number A101: R¹ is H, R² is CH₃, R³ is H, R⁴ is H, R⁵ is H, R⁶ is 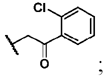
Number A102: R¹ is H, R² is CH₃, R³ is H, R⁴ is H, R⁵ is H, R⁶ is 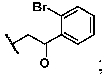
Number A103: R¹ is H, R² is CH₃, R³ is H, R⁴ is H, R⁵ is H, R⁶ is 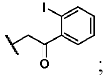
Number A104: R¹ is H, R² is CH₃, R³ is H, R⁴ is H, R⁵ is H, R⁶ is 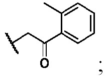
Number A105: R¹ is H, R² is CH₃, R³ is H, R⁴ is H, R⁵ is H, R⁶ is 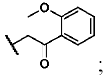
Number A106: R¹ is H, R² is CH₃, R³ is H, R⁴ is H, R⁵ is H, R⁶ is 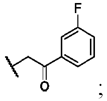
Number A107: R¹ is H, R² is CH₃, R³ is H, R⁴ is H, R⁵ is H, R⁶ is 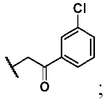
Number A108: R¹ is H, R² is CH₃, R³ is H, R⁴ is H, R⁵ is H, R⁶ is 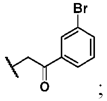
Number A109: R¹ is H, R² is CH₃, R³ is H, R⁴ is H, R⁵ is H, R⁶ is 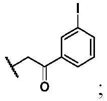
Number A110: R¹ is H, R² is CH₃, R³ is H, R⁴ is H, R⁵ is H, R⁶ is 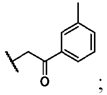
Number A111: R¹ is H, R² is CH₃, R³ is H, R⁴ is H, R⁵ is H, R⁶ is 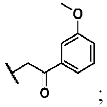
Number A112: R¹ is H, R² is CH₃, R³ is H, R⁴ is H, R⁵ is H, R⁶ is 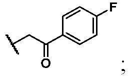
Number A113: R¹ is H, R² is CH₃, R³ is H, R⁴ is H, R⁵ is H, R⁶ is 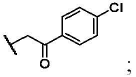
Number A114: R¹ is H, R² is CH₃, R³ is H, R⁴ is H, R⁵ is H, R⁶ is 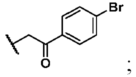
Number A115: R¹ is H, R² is CH₃, R³ is H, R⁴ is H, R⁵ is H, R⁶ is 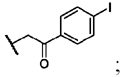
Number A116: R¹ is H, R² is CH₃, R³ is H, R⁴ is H, R⁵ is H, R⁶ is 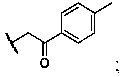
Number A117: R¹ is H, R² is CH₃, R³ is H, R⁴ is H, R⁵ is CH₃, R⁶ is 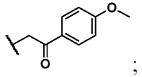
Number A118: R¹ is H, R² is CH₃, R³ is H, R⁴ is H, R⁵ is CH₃, R⁶ is -H;
Number A119: R^{l} is H, R² is CH₃, R³ is H, R⁴ is H, R⁵ is CH₃, R⁶ is -CH₃;
Number A120: R¹ is H, R² is CH₃, R³ is H, R⁴ is H, R⁵ is CH₃, R⁶ is -CH₂CH₃;
Number A121: R¹ is H, R² is CH₃, R³ is H, R⁴ is H, R⁵ is CH₃, R⁶ is -CH₂CH₂CH₃;
Number A122: R¹ is H, R² is CH₃, R³ is H, R⁴ is H, R⁵ is CH₃, R⁶ is -CH(CH₃)₂;
Number A123: R¹ is H, R² is CH₃, R³ is H, R⁴ is H, R⁵ is CH₃, R⁶ is 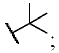
Number A124: R¹ is H, R² is CH₃, R³ is H, R⁴ is H, R⁵ is CH₃, R⁶ is 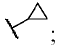
Number A125: R¹ is H, R² is CH₃, R³ is H, R⁴ is H, R⁵ is CH₃, R⁶ is 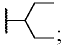
Number A126: R¹ is H, R² is CH₃, R³ is H, R⁴ is H, R⁵ is CH₃, R⁶ is 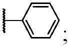
Number A127: R¹ is H, R² is CH₃, R³ is H, R⁴ is H, R⁵ is CH₃, R⁶ is 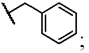
Number A128: R¹ is H, R² is CH₃, R³ is H, R⁴ is H, R⁵ is CH₃, R⁶ is -COCH₃;
Number A129: R¹ is H, R² is CH₃, R³ is H, R⁴ is H, R⁵ is CH₃, R⁶ is -COCH₂CH₃;
Number A130: R¹ is H, R² is CH₃, R³ is H, R⁴ is H, R⁵ is CH₃, R⁶ is -COCH₂CH₂CH₃;
Number A131: R¹ is H, R² is CH₃, R³ is H, R⁴ is H, R⁵ is CH₃, R⁶ is -COCH(CH₃)₂;
Number A132: R¹ is H, R² is CH₃, R³ is H, R⁴ is H, R⁵ is CH₃, R⁶ is -COC₆H₅;
Number A133: R¹ is H, R² is CH₃, R³ is H, R⁴ is H, R⁵ is CH₃, R⁶ is 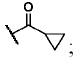
Number A134: R¹ is H, R² is CH₃, R³ is H, R⁴ is H, R⁵ is CH₃, R¹ is 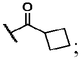
Number A135: R¹ is H, R² is CH₃, R³ is H, R⁴ is H, R⁵ is CH₃, R⁶ is 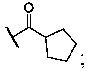
Number A136: R¹ is H, R² is CH₃, R³ is H, R⁴ is H, R⁵ is CH₃, R⁶ is 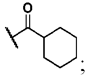
Number A137: R¹ is H, R² is CH₃, R³ is H, R⁴ is H, R⁵ is CH₃, R⁶ is 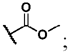
Number A138: R¹ is H, R² is CH₃, R³ is H, R⁴ is H, R⁵ is CH₃, R⁶ is 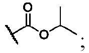
Number A139: R¹ is H, R² is CH₃, R³ is H, R⁴ is H, R⁵ is CH₃, R⁶ is 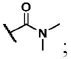
Number A140: R¹ is H, R² is CH₃, R³ is H, R⁴ is H, R⁵ is CH₃, R⁶ is 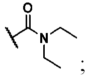
Number A141: R¹ is H, R² is CH₃, R³ is H, R⁴ is H, R⁵ is CH₃, R⁶ is -S(=O)₂CH₃;
Number A142: R¹ is H, R² is CH₃, R³ is H, R⁴ is H, R⁵ is CH₃, R⁶ is -S(=O)₂CH₂CH₃;
Number A143: R¹ is H, R² is CH₃, R³ is H, R⁴ is H, R⁵ is CH₃, R⁶ is -S(=O)₂CH₂CH₂CH₃;
Number A144: R¹ is H, R² is CH₃, R³ is H, R⁴ is H, R⁵ is CH₃, R⁶ is -S(=O)₂CH(CH₃)₂;
Number A145: R¹ is H, R² is CH₃, R³ is H, R⁴ is H, R⁵ is CH₃, R⁶ is -S(=O)₂C₆H₅;
Number A146: R¹ is H, R² is CH₃, R³ is H, R⁴ is H, R⁵ is CH₃, R⁶ is 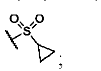
Number A147: R¹ is H, R² is CH₃, R³ is H, R⁴ is H, R⁵ is CH₃, R⁶ is 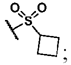
Number A148: R¹ is H, R² is CH₃, R³ is H, R⁴ is H, R⁵ is CH₃, R⁶ is 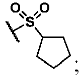
Number A149: R¹ is H, R² is CH₃, R³ is H, R⁴ is H, R⁵ is CH₃, R⁶ is 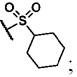
Number A150: R¹ is H, R² is CH₃, R³ is H, R⁴ is CH₃, R⁵ is CH₃, R⁶ is -H;
Number A151: R¹ is H, R² is CH₃, R³ is H, R⁴ is CH₃, R⁵ is CH₃, R⁶ is -CH₃;
Number A152: R¹ is H, R² is CH₃, R³ is H, R⁴ is CH₃, R⁵ is CH₃, R⁶ is -CH₂CH₃;
Number A153: R¹ is H, R² is CH₃, R³ is H, R⁴ is CH₃, R⁵ is CH₃, R⁶ is -CH₂CH₂CH₃;
Number A154: R¹ is H, R² is CH₃, R³ is H, R⁴ is CH₃, R⁵ is CH₃, R⁶ is -CH(CH₃)₂;
Number A155: R¹ is H, R² is CH₃, R³ is H, R⁴ is CH₃, R⁵ is CH₃, R⁶ is 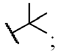
Number A156: R¹ is H, R² is CH₃, R³ is H, R⁴ is CH₃, R⁵ is CH₃, R⁶ is 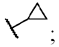
Number A157: R¹ is H, R² is CH₃, R³ is H, R⁴ is CH₃, R⁵ is CH₃, R⁶ is 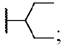
Number A158: R¹ is H, R² is CH₃, R³ is H, R⁴ is CH₃, R⁵ is CH₃, R⁶ is 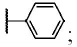
Number A159: R¹ is H, R² is CH₃, R³ is H, R⁴ is CH₃, R⁵ is CH₃, R⁶ is 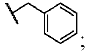
Number A160: R¹ is H, R² is CH₃, R³ is H, R⁴ is CH₃, R⁵ is CH₃, R⁶ is -COCH₃;
Number A161: R¹ is H, R² is CH₃, R³ is H, R⁴ is CH₃, R⁵ is CH₃, R⁶ is -COCH₂CH₃;
Number A162: R¹ is H, R² is CH₃, R³ is H, R⁴ is CH₃, R⁵ is CH₃, R⁶ is -COCH₂CH₂CH₃;
Number A163: R¹ is H, R² is CH₃, R³ is H, R⁴ is CH₃, R⁵ is CH₃, R⁶ is -COCH(CH₃)₂;
Number A164: R¹ is H, R² is CH₃, R³ is H, R⁴ is CH₃, R⁵ is CH₃, R⁶ is -COC₆H₅;
Number A165: R¹ is H, R² is CH₃, R³ is H, R⁴ is CH₃, R⁵ is CH₃, R⁶ is 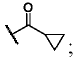
Number A166: R¹ is H, R² is CH₃, R³ is H, R⁴ is CH₃, R⁵ is CH₃, R⁶ is 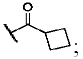
Number A167: R¹ is H, R² is CH₃, R³ is H, R⁴ is CH₃, R⁵ is CH₃, R⁶ is 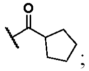
Number A168: R¹ is H, R² is CH₃, R³ is H, R⁴ is CH₃, R⁵ is CH₃, R⁶ is 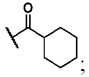
Number A169: R¹ is H, R² is CH₃, R³ is H, R⁴ is CH₃, R⁵ is CH₃, R⁶ is 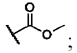
Number A170: R¹ is H, R² is CH₃, R³ is H, R⁴ is CH₃, R⁵ is CH₃, R⁶ is 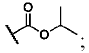
Number A171: R¹ is H, R² is CH₃, R³ is H, R⁴ is CH₃, R⁵ is CH₃, R⁶ is 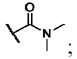
Number A172: R¹ is H, R² is CH₃, R³ is H, R⁴ is CH₃, R⁵ is CH₃, R⁶ is 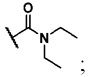
Number A173: R¹ is H, R² is CH₃, R³ is H, R⁴ is CH₃, R⁵ is CH₃, R⁶ is -S(=O)₂CH₃;
Number A174: R¹ is H, R² is CH₃, R³ is H, R⁴ is CH₃, R⁵ is CH₃, R⁶ is -S(=O)₂CH₂CH₃;
Number A175: R¹ is H, R² is CH₃, R³ is H, R⁴ is CH₃, R⁵ is CH₃, R⁶ is -S(=O)₂CH₂CH₂CH₃;
Number A176: R¹ is H, R² is CH₃, R³ is H, R⁴ is CH₃, R⁵ is CH₃, R⁶ is -S(=O)₂CH(CH₃)₂;
Number A177: R¹ is H, R² is CH₃, R³ is H, R⁴ is CH₃, R⁵ is CH₃, R⁶ is -S(=O)₂C₆H₅;
Number A178: R¹ is H, R² is CH₃, R³ is H, R⁴ is CH₃, R⁵ is CH₃, R⁶ is 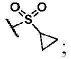
Number A179: R¹ is H, R² is CH₃, R³ is H, R⁴ is CH₃, R⁵ is CH₃, R⁶ is 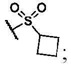
Number A180: R¹ is H, R² is CH₃, R³ is H, R⁴ is CH₃, R⁵ is CH₃, R⁶ is 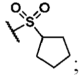
Number A181: R¹ is H, R² is CH₃, R³ is H, R⁴ is CH₃, R⁵ is CH₃, R⁶ is 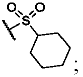
Number A182: R¹ is H, R² is CH₃, R³ is CH₃, R⁴ is H, R⁵ is H, R⁶ is 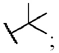
Number A183: R¹ is H, R² is CH₃, R³ is OCH₃, R⁴ is H, R⁵ is H, R⁶ is 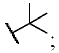
Number A184: R¹ is H, R² is CH₃, R³ is 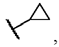 R⁴ is H, R⁵ is H, R⁶ is 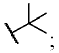
Number A185: R¹ is H, R² is CH₃, R³ is COCH₃, R⁴ is H, R⁵ is H, R⁶ is 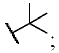
Number A186: R¹ is H, R² is CH₃, R³ is 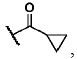 R⁴ is H, R⁵ is H, R⁶ is 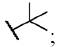
Number A187: R¹ is H, R² is CH₃, R³ is 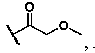 R⁴ isH,R⁵ is H, R⁶ is 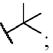
Number A188: R¹ is H, R² is CH₃, R³ is 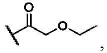 R⁴ is H, R⁵ is H, R⁶ is 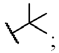
Number A189: R¹ is H, R² is CN, R³ is H, R⁴ is H, R⁵ is H, R⁶ is 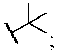
Number A190: R¹ is F, R² is CH₃, R³ is H, R⁴ is H, R⁵ is H, R⁶ is -H;
Number A191: R¹ is F, R² is CH₃, R³ is H, R⁴ is H, R⁵ is H, R⁶ is -CH₃;
Number A192: R¹ is F, R² is CH₃, R³ is H, R⁴ is H, R⁵ is H, R⁶ is -CH₂CH₃;
Number A193: R¹ is F, R² is CH₃, R³ is H, R⁴ is H, R⁵ is H, R⁶ is -CH₂CH₂CH₃;
Number A194: R¹ is F, R² is CH₃, R³ is H, R⁴ is H, R⁵ is H, R⁶ is -CH(CH₃)₂;
Number A195: R¹ is F, R² is CH₃, R³ is H, R⁴ is H, R⁵ is H, R⁶ is 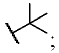
Number A196: R¹ is F, R² is CH₃, R³ is H, R⁴ is H, R⁵ is H, R⁶ is 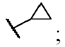
Number A197: R¹ is F, R² is CH₃, R³ is H, R⁴ is H, R⁵ is H, R⁶ is 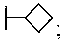
Number A198: R¹ is F, R² is CH₃, R³ is H, R⁴ is H, R⁵ is H, R⁶ is 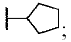
Number A199: R¹ is F, R² is CH₃, R³ is H, R⁴ is H, R⁵ is H, R⁶ is 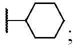
Number A200: R¹ is F, R² is CH₃, R³ is H, R⁴ is H, R⁵ is H, R⁶ is 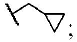
Number A201: R¹ is F, R² is CH₃, R³ is H, R⁴ is H, R⁵ is H, R⁶ is 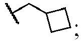
Number A202: R¹ is F, R² is CH₃, R³ is H, R⁴ is H, R⁵ is H, R⁶ is 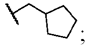
Number A203: R¹ is F, R² is CH₃, R³ is H, R⁴ is H, R⁵ is H, R⁶ is 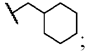
Number A204: R¹ is F, R² is CH₃, R³ is H, R⁴ is H, R⁵ is H, R⁶ is 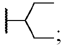
Number A205: R¹ is F, R² is CH₃, R³ is H, R⁴ is H, R⁵ is H, R⁶ is 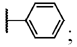
Number A206: R¹ is F, R² is CH₃, R³ is H, R⁴ is H, R⁵ is H, R⁶ is 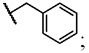
Number A207: R¹ is F, R² is CH₃, R³ is H, R⁴ is H, R⁵ is H, R⁶ is -COCH₃;
Number A208: R¹ is F, R² is CH₃, R³ is H, R⁴ is H, R⁵ is H, R⁶ is -COCH₂CH₃;
Number A209: R¹ is F, R² is CH₃, R³ is H, R⁴ is H, R⁵ is H, R⁶ is -COCH₂CH₂CH₃;
Number A210: R¹ is F, R² is CH₃, R³ is H, R⁴ is H, R⁵ is H, R⁶ is -COCH(CH₃)₂;
Number A211: R¹ is F, R² is CH₃, R³ is H, R⁴ is H, R⁵ is H, R⁶ is -COC₆H₅;
Number A212: R¹ is F, R² is CH₃, R³ is H, R⁴ is H, R⁵ is H, R⁶ is 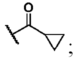
Number A213: R¹ is F, R² is CH₃, R³ is H, R⁴ is H, R⁵ is H, R⁶ is 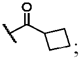
Number A214: R¹ is F, R² is CH₃, R³ is H, R⁴ is H, R⁵ is H, R⁶ is 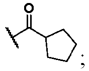
Number A215: R¹ is F, R² is CH₃, R³ is H, R⁴ is H, R⁵ is H, R⁶ is 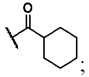
Number A216: R¹ is F, R² is CH₃, R³ is H, R⁴ is H, R⁵ is H, R⁶ is 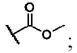
Number A217: R¹ is F, R² is CH₃, R³ is H, R⁴ is H, R⁵ is H, R⁶ is 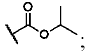
Number A218: R¹ is F, R² is CH₃, R³ is H, R⁴ is H, R⁵ is H, R⁶ is 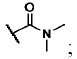
Number A219: R¹ is F, R² is CH₃, R³ is H, R⁴ is H, R⁵ is H, R⁶ is 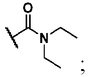
Number A220: R¹ is F, R² is CH₃, R³ is H, R⁴ is H, R⁵ is H, R⁶ is -S(=O)₂CH₃;
Number A221: R¹ is F, R² is CH₃, R³ is H, R⁴ is H, R⁵ is H, R⁶ is -S(=O)₂CH₂CH₃;
Number A222: R¹ is F, R² is CH₃, R³ is H, R⁴ is H, R⁵ is H, R⁶ is -S(=O)₂CH₂CH₂CH₃;
Number A223: R¹ is F, R² is CH₃, R³ is H, R⁴ is H, R⁵ is H, R⁶ is -S(=O)₂CH(CH₃)₂;
Number A224: R¹ is F, R² is CH₃, R³ is H, R⁴ is H, R⁵ is H, R⁶ is -S(=O)₂C₆H₅;
Number A225: R¹ is F, R² is CH₃, R³ is H, R⁴ is H, R⁵ is H, R⁶ is 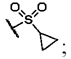
Number A226: R¹ is F, R² is CH₃, R³ is H, R⁴ is H, R⁵ is H, R⁶ is 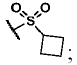
Number A227: R¹ is F, R² is CH₃, R³ is H, R⁴ is H, R⁵ is H, R⁶ is 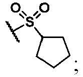
Number A228: R¹ is F, R² is CH₃, R³ is H, R⁴ is H, R⁵ is H, R⁶ is 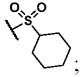
Number A229: R¹ is F, R² is CH₃, R³ is H, R⁴ is H, R⁵ is CH₃, R⁶ is -H;
Number A230: R¹ is F, R² is CH₃, R³ is H, R⁴ is H, R⁵ is CH₃, R⁶ is -CH₃;
Number A231: R¹ is F, R² is CH₃, R³ is H, R⁴ is H, R⁵ is CH₃, R⁶ is -CH₂CH₃;
Number A232: R¹ is F, R² is CH₃, R³ is H, R⁴ is H, R⁵ is CH₃, R⁶ is -CH₂CH₂CH₃;
Number A233: R¹ is F, R² is CH₃, R³ is H, R⁴ is H, R⁵ is CH₃, R⁶ is -CH(CH₃)₂;
Number A234: R¹ is F, R² is CH₃, R³ is H, R⁴ is H, R⁵ is CH₃, R⁶ is 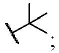
Number A235: R¹ is F, R² is CH₃, R³ is H, R⁴ is H, R⁵ is CH₃, R⁶ is 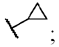
Number A236: R¹ is F, R² is CH₃, R³ is H, R⁴ is H, R⁵ is CH₃, R⁶ is 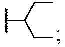
Number A237: R¹ is F, R² is CH₃, R³ is H, R⁴ is H, R⁵ is CH₃, R⁶ is 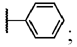
Number A238: R¹ is F, R² is CH₃, R³ is H, R⁴ is H, R⁵ is CH₃, R⁶ is 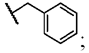
Number A239: R¹ is F, R² is CH₃, R³ is H, R⁴ is H, R⁵ is CH₃, R⁶ is -COCH₃;
Number A240: R¹ is F, R² is CH₃, R³ is H, R⁴ is H, R⁵ is CH₃, R⁶ is -COCH₂CH₃;
Number A241: R¹ is F, R² is CH₃, R³ is H, R⁴ is H, R⁵ is CH₃, R⁶ is -COCH₂CH₂CH₃;
Number A242: R¹ is F, R² is CH₃, R³ is H, R⁴ is H, R⁵ is CH₃, R⁶ is -COCH(CH₃)₂;
Number A243: R¹ is F, R² is CH₃, R³ is H, R⁴ is H, R⁵ is CH₃, R⁶ is -COC₆H₅;
Number A244: R¹ is F, R² is CH₃, R³ is H, R⁴ is H, R⁵ is CH₃, R⁶ is 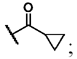
Number A245: R¹ is F, R² is CH₃, R³ is H, R⁴ is H, R⁵ is CH₃, R⁶ is 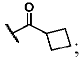
Number A246: R¹ is F, R² is CH₃, R³ is H, R⁴ is H, R⁵ is CH₃, R⁶ is 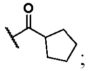
Number A247: R¹ is F, R² is CH₃, R³ is H, R⁴ is H, R⁵ is CH₃, R⁶ is 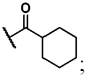
Number A248: R¹ is F, R² is CH₃, R³ is H, R⁴ is H, R⁵ is CH₃, R⁶ is 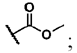
Number A249: R¹ is F, R² is CH₃, R³ is H, R⁴ is H, R⁵ is CH₃, R⁶ is 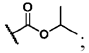
Number A250: R¹ is F, R² is CH₃, R³ is H, R⁴ is H, R⁵ is CH₃, R⁶ is 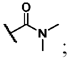
Number A251: R¹ is F, R² is CH₃, R³ is H, R⁴ is H, R⁵ is CH₃, R⁶ is 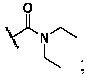
Number A252: R¹ is F, R² is CH₃, R³ is H, R⁴ is H, R⁵ is CH₃, R⁶ is -S(=O)₂CH₃;
Number A253: R¹ is F, R² is CH₃, R³ is H, R⁴ is H, R⁵ is CH₃, R⁶ is -S(=O)₂CH₂CH₃;
Number A254: R¹ is F, R² is CH₃, R³ is H, R⁴ is H, R⁵ is CH₃, R⁶ is -S(=O)₂CH₂CH₂CH₃;
Number A255: R¹ is F, R² is CH₃, R³ is H, R⁴ is H, R⁵ is CH₃, R⁶ is -S(=O)₂CH(CH₃)₂;
Number A256: R¹ is F, R² is CH₃, R³ is H, R⁴ is H, R⁵ is CH₃, R⁶ is -S(=O)₂C₆H₅;
Number A257: R¹ is F, R² is CH₃, R³ is H, R⁴ is H, R⁵ is CH₃, R⁶ is 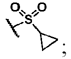
Number A258: R¹ is F, R² is CH₃, R³ is H, R⁴ is H, R⁵ is CH₃, R⁶ is 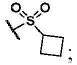
Number A259: R¹ is F, R² is CH₃, R³ is H, R⁴ is H, R⁵ is CH₃, R⁶ is 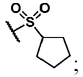
Number A260: R¹ is F, R² is CH₃, R³ is H, R⁴ is H, R⁵ is CH₃, R⁶ is 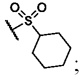
Number A261: R¹ is F, R² is CH₃, R³ is H, R⁴ is CH₃, R⁵ is CH₃, R⁶ is -H;
Number A262: R¹ is F, R² is CH₃, R³ is H, R⁴ is CH₃, R⁵ is CH₃, R⁶ is -CH₃;
Number A263: R¹ is F, R² is CH₃, R³ is H, R⁴ is CH₃, R⁵ is CH₃, R⁶ is -CH₂CH₃;
Number A264: R¹ is F, R² is CH₃, R³ is H, R⁴ is CH₃, R⁵ is CH₃, R⁶ is -CH₂CH₂CH₃;
Number A265: R¹ is F, R² is CH₃, R³ is H, R⁴ is CH₃, R⁵ is CH₃, R⁶ is -CH(CH₃)₂;
Number A266: R¹ is F, R² is CH₃, R³ is H, R⁴ is CH₃, R⁵ is CH₃, R⁶ is 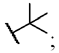
Number A267: R¹ is F, R² is CH₃, R³ is H, R⁴ is CH₃, R⁵ is CH₃, R⁶ is 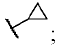
Number A268: R¹ is F, R² is CH₃, R³ is H, R⁴ is CH₃, R⁵ is CH₃, R⁶ is 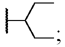
Number A269: R¹ is F, R² is CH₃, R³ is H, R⁴ is CH₃, R⁵ is CH₃, R⁶ is 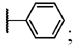
Number A270: R¹ is F, R² is CH₃, R³ is H, R⁴ is CH₃, R⁵ is CH₃, R⁶ is 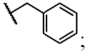
Number A271: R¹ is F, R² is CH₃, R³ is H, R⁴ is CH₃, R⁵ is CH₃, R⁶ is -COCH₃;
Number A272: R¹ is F, R² is CH₃, R³ is H, R⁴ is CH₃, R⁵ is CH₃, R⁶ is -COCH₂CH₃;
Number A273: R¹ is F, R² is CH₃, R³ is H, R⁴ is CH₃, R⁵ is CH₃, R⁶ is -COCH₂CH₂CH₃;
Number A274: R¹ is F, R² is CH₃, R³ is H, R⁴ is CH₃, R⁵ is CH₃, R⁶ is -COCH(CH₃)₂;
Number A275: R¹ is F, R² is CH₃, R³ is H, R⁴ is CH₃, R⁵ is CH₃, R⁶ is -COC₆H₅;
Number A276: R¹ is F, R² is CH₃, R³ is H, R⁴ is CH₃, R⁵ is CH₃, R⁶ is 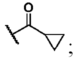
Number A277: R¹ is F, R² is CH₃, R³ is H, R⁴ is CH₃, R⁵ is CH₃, R⁶ is 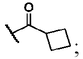
Number A278: R¹ is F, R² is CH₃, R³ is H, R⁴ is CH₃, R⁵ is CH₃, R⁶ is 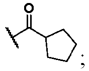
Number A279: R¹ is F, R² is CH₃, R³ is H, R⁴ is CH₃, R⁵ is CH₃, R⁶ is 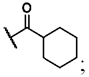
Number A280: R¹ is F, R² is CH₃, R³ is H, R⁴ is CH₃, R⁵ is CH₃, R⁶ is 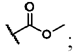
Number A281: R¹ is F, R² is CH₃, R³ is H, R⁴ is CH₃, R⁵ is CH₃, R⁶ is 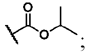
Number A282: R¹ is F, R² is CH₃, R³ is H, R⁴ is CH₃, R⁵ is CH₃, R⁶ is 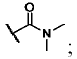
Number A283: R¹ is F, R² is CH₃, R³ is H, R⁴ is CH₃, R⁵ is CH₃, R⁶ is 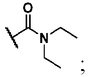
Number A284: R¹ is F, R² is CH₃, R³ is H, R⁴ is CH₃, R⁵ is CH₃, R⁶ is -S(=O)₂CH₃;
Number A285: R¹ is F, R² is CH₃, R³ is H, R⁴ is CH₃, R⁵ is CH₃, R⁶ is -S(=O)₂CH₂CH₃;
Number A286: R¹ is F, R² is CH₃, R³ is H, R⁴ is CH₃, R⁵ is CH₃, R⁶ is -S(=O)₂CH₂CH₂CH₃;
Number A287: R¹ is F, R² is CH₃, R³ is H, R⁴ is CH₃, R⁵ is CH₃, R⁶ is -S(=O)₂CH(CH₃)₂;
Number A288: R¹ is F, R² is CH₃, R³ is H, R⁴ is CH₃, R⁵ is CH₃, R⁶ is -S(=O)₂C₆H₅;
Number A289: R¹ is F, R² is CH₃, R³ is H, R⁴ is CH₃, R⁵ is CH₃, R⁶ is 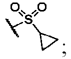
Number A290: R¹ is F, R² is CH₃, R³ is H, R⁴ is CH₃, R⁵ is CH₃, R⁶ is 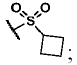
Number A291: R¹ is F, R² is CH₃, R³ is H, R⁴ is CH₃, R⁵ is CH₃, R⁶ is 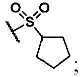
Number A292: R¹ is F, R² is CH₃, R³ is H, R⁴ is CH₃, R⁵ is CH₃, R⁶ is 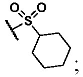
Number A293: R¹ is F, R² is CH₃, R³ is CH₃, R⁴ is H, R⁵ is H, R⁶ is 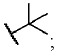
Number A294: R¹ is F, R² is CH₃, R³ is OCH₃, R⁴ is H, R⁵ is H, R⁶ is 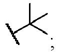
Number A295: R¹ is F, R² is CH₃, R³ is 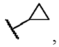 R⁴ is H, R⁵ is H, R⁶ is 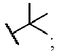
Number A296: R¹ is F, R² is CH₃, R³ is COCH₃, R⁴ is H, R⁵ is H, R⁶ is 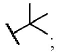
Number A297: R¹ is F, R² is CH₃, R³ is 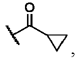 R⁴ is H, R⁵ is H, R⁶ is 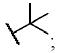
Number A298: R¹ is F, R² is CH₃, R³ is 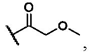 R⁴ is H, R⁵ is H, R⁶ is 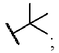
Number A299: R¹ is F, R² is CH₃, R³ is 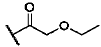 , R⁴is H, R⁵ is H, R⁶ is 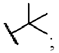
Number A300: R¹ is F, R² is CN, R³ is H, R⁴ is H, R⁵ is H, R⁶ is

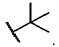

The present invention does not require any particular method for preparing the compounds described in the first aspect, and a person skilled in the art can determine a suitable synthetic route to obtain the compounds described in the first aspect of the invention based on the structural formulae provided herein in combination with knowledge known in the art of organic synthesis.

In a second aspect, the present invention provides the non-therapeutic use of the compound containing a pyrazole structure according to the first aspect, or an agrochemically acceptable salt, hydrate, and solvate thereof, for at least one of insect killing, mite killing, nematode killing, and piercing-sucking insects killing.

A third aspect of the present invention provides the non-therapeutic use of the aforementioned pyrazole structurecontaining compound or an agrochemically acceptable salt, hydrate and solvate thereof as an agrochemical insecticide.

A fourth aspect of the present invention provides an insecticide containing a pyrazole structure or an agrochemically acceptable salt, hydrate and solvate thereof as an active ingredient.

Preferably, the content of said active ingredient in the insecticide is 1-99.9% by weight.
more preferably, the content of the active ingredient in the pesticide is 1-99.9 wt%, and may be, for example, 5 wt%, 10 wt%, 15 wt%, 20 wt%, 25 wt%, 30 wt%, 35 wt%, 40 wt%, 45 wt%, 50 wt%, 55 wt%, 60 wt%, 65 wt%, 70 wt%, 75 wt%, 80 wt%, 85 wt%, 90 wt%, 95 wt%, etc.

Particularly preferably, the content of the active ingredient in the insecticide is 5 to 95% by weight.

Preferably, the formulation of the pesticide is at least one selected from the group consisting of emulsifiable concentrate, suspension, wettable powder, dustpowder, granule, aqueous solution, poison bait, mother liquor and mother powder.

Where not specifically recited, all of the following examples are racemates.

The present invention will be described in detail below by way of examples. In the following examples, various raw materials used are commercially available unless otherwise specified.

Unless otherwise specified, the room temperature or the normal temperatures described below means 25 ±3 °C.

The numbering of the compounds provided in the examples section below of the present invention is different from the numbering of the compounds in the preceding, i.e., there are instances where one structural formula has two numbering, which does not affect the clear manner of presentation of the invention. It will be clear that any reference numeral refers to the same structure.

First, the characterization data of some compounds of the present invention are listed in table 1.

**TABLE 1**

| **Compound number** | **structure** | **nuclear magnetism** |
|---|---|---|
| 1 | 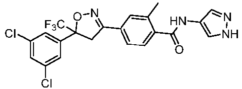 | ¹H NMR (600 MHz, DMSO-*d*₆) δ 12.66 (s, 1H), 10.44 (s, 1H), 7.97 (s, 1H), 7.82 (d, *J =* 2.3 Hz, 1H), 7.68 - 7.61 (m, 5H), 7.55 (d, *J =* 8.0 Hz, 1H), 4.40 (d, *J =* 18.4 Hz, 1H), 4.32 (d, *J =* 18.4 Hz, 1H), 2.41 (s, 3H). |
| 2 | 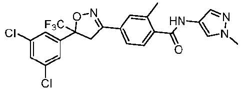 | ¹H NMR (600 MHz, DMSO-*d*₆) δ 10.44 (s, 1H), 8.01 (s, 1H), 7.83 (s, 1H), 7.68 - 7.61 (m, 4H), 7.55 (d, *J =* 7.9 Hz, 1H), 7.49 (s, 1H), 4.40 (d, *J =* 18.4 Hz, 1H), 4.32 (d, *J =* 18.4 Hz, 1H), 3.82 (s, 3H), 2.41 (s, 3H). |
| 3 | 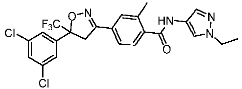 | ¹H NMR (600 MHz, DMSO-*d*₆) δ 10.45 (s, 1H), 8.04 (s, 1H), 7.83 (s, 1H), 7.70 - 7.60 (m, 4H), 7.55 (d, *J =* 7.9 Hz, 1H), 7.50 (s, 1H), 4.41 (d, *J =* 18.4 Hz, 1H), 4.33 (d, *J* = 18.4 Hz, 1H), 4.12 (q, *J =* 7.3 Hz, 2H), 2.41 (s, 3H), 1.36 (t, *J =* 7.3 Hz, 3H). |
| 4 | 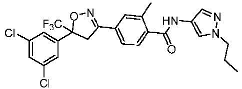 | ¹H NMR (600 MHz, DMSO-*d*₆) δ 10.44 (s, 1H), 8.02 (s, 1H), 7.83 (s, 1H), 7.67 - 7.62 (m, 4H), 7.55 (d, *J =* 8.0 Hz, 1H), 7.50 (s, 1H), 4.40 (d, *J =* 18.4 Hz, 1H), 4.32 (d, *J =* 18.4 Hz, 1H), 4.04 (t, *J =* 6.9 Hz, 2H), 2.41 (s, 3H), 1.80 - 1.73 (m, 2H), 0.83 (t, *J =* 7.4 Hz, 3H). |
| 5 | 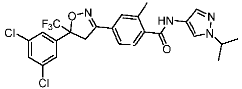 | ¹H NMR (600 MHz, DMSO-*d*₆) δ 10.43 (s, 1H), 8.03 (s, 1H), 7.83 (s, 1H), 7.68 - 7.62 (m, 4H), 7.54 (d, *J* = 7.9 Hz, 1H), 7.50 (s, 1H), 4.53 - 4.45 (m, 1H), 4.40 (d, *J =* 18.4 Hz, 1H), 4.32 (d, *J =* 18.4 Hz, 1H), 2.41 (s, 3H), 1.41 (d, *J =* 6.7 Hz, 6H). |
| 6 | 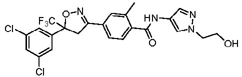 | ¹H NMR (400 MHz, Chloroform-*d*) δ 8.14 (s, 1H), 7.77 (s, 1H), 7.56 - 7.46 (m, 6H), 7.44 (t, *J* = 1.9 Hz, 1H), 4.26 (t, *J* = 4.7 Hz, 2H), 4.10 (d, *J =* 17.4 Hz, 1H), 4.01 (t, *J =* 4.7 Hz, 2H), 3.72 (d, *J =* 17.4 Hz, 1H), 2.49 (s, 3H), 2.29 (s, 1H). |
| 7 | 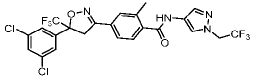 | ¹H NMR (600 MHz, DMSO-*d*₆) δ 10.59 (s, 1H), 8.23 (s, 1H), 7.82 (d, *J* = 2.0 Hz, 1H), 7.69 - 7.61 (m, 5H), 7.59 (d, *J =* 7.9 Hz, 1H), 5.14 (q, *J =* 9.1 Hz, 2H), 4.41 *(d, J=* 18.4 Hz, 1H), 4.33 (d, *J =* 18.4 Hz, 1H), 2.42 (s, 3H). |
| 8 | 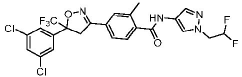 | ¹H NMR (600 MHz, Chloroform-*d*) δ 8.16 (s, 1H), 7.62 - 7.47 (m, 7H), 7.44 (t, *J* = 2.0 Hz, 1H), 6.23 - 5.97 (m, 1H), 4.52 - 4.40 (m, 2H), 4.09 (d, *J* = 17.4 Hz, 1H), 3.71 (d, *J =* 17.4 Hz, 1H), 2.52 (s, 3H). |
| 9 | 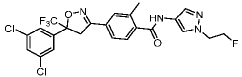 | ¹H NMR (400 MHz, DMSO-*d*₆) δ 10.50 (s, 1H), 8.12 (s, 1H), 7.83 (t, *J* = 1.9 Hz, 1H), 7.70 - 7.64 (m, 4H), 7.57 (d, *J* = 7.6 Hz, 2H), 4.83 (t, *J* = 4.7 Hz, 1H), 4.71 (t, *J =* 4.7 Hz, 1H), 4.47 (t, *J =* 4.7 Hz, 1H), 4.45 - 4.38 (m, 2H), 4.33 (d, *J =* 18.4 Hz, 1H), 2.43 (s, 3H). |
| 10 | 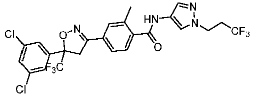 | ¹H NMR (400 MHz, Chloroform-*d*) δ 8.16 (s, 1H), 7.70 (s, 1H), 7.59 - 7.50 (m, 6H), 7.47 *(t, J=* 1.8 Hz, 1H), 4.40 (t, *J =* 7.2 Hz, 2H), 4.13 (d, *J =* 17.4 Hz, 1H), 3.75 (d, *J* = 17.4 Hz, 1H), 2.87 - 2.71 (m, 2H), 2.53 (s, 3H). |
| 11 | 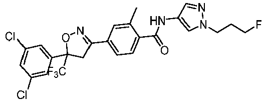 | ¹H NMR (400 MHz, Chloroform-*d*) δ 8.10 (s, 1H), 7.73 (s, 1H), 7.56 - 7.47 (m, 6H), 7.44 (t, *J* = 1.9 Hz, 1H), 4.52 (t, *J =* 5.6 Hz, 1H), 4.40 (t, *J =* 5.6 Hz, 1H), 4.27 (t, *J* = 6.8 Hz, 2H), 4.10 (d, *J =* 17.4 Hz, 1H), 3.72 (d, *J =* 17.4 Hz, 1H), 2.50 (s, 3H), 2.36 - 2.18 (m, 2H). |
| 12 | 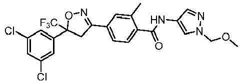 | ¹H NMR (600 MHz, Chloroform-*d*) δ 8.24 (s, 1H), 7.60 - 7.50 (m, 7H), 7.44 (s, 1H), 5.38 (s, 2H), 4.10 (d, *J =* 17.4 Hz, 1H), 3.71 (d, *J* = 17.4 Hz, 1H), 3.35 (s, 3H), 2.52 (s, 3H). |
| 13 | 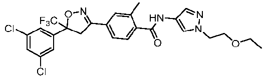 | ¹H NMR (400 MHz, DMSO-*d*₆) δ 10.46 (s, 1H), 8.05 (s, 1H), 7.84 (q, *J* = 2.1 Hz, 1H), 7.70 - 7.64 (m, 4H), 7.60 - 7.53 (m, 2H), 4.43 (d, *J =* 18.4 Hz, 1H), 4.34 (d, *J* = 18.4 Hz, 1H), 4.25 (t, *J* = 5.4 Hz, 2H), 3.73 (t, *J* = 5.4 Hz, 2H), 3.45 (q, *J =* 7.0 Hz, 2H), 2.43 (s, 3H), 1.10 *(t, J=* 7.0 Hz, 3H). |
| 14 | 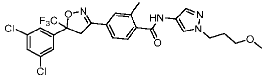 | ¹H NMR (400 MHz, DMSO-*d*₆) δ 10.46 (s, 1H), 8.02 (d, *J* = 0.7 Hz, 1H), 7.83 (t, *J =* 1.9 Hz, 1H), 7.69 - 7.62 (m, 4H), 7.56 (d, *J =* 7.9 Hz, 1H), 7.53 (d, *J =* 0.8 Hz, 1H), 4.41 (d, *J =* 18.4 Hz, 1H), 4.32 (d, *J =* 18.4 Hz, 1H), 4.13 (t, *J =* 7.0 Hz, 2H), 3.29 (t, *J =* 6.2 Hz, 2H), 3.24 (s, 3H), 2.41 (s, 3H), 2.02 - 1.94 (m, 2H). |
| 15 | 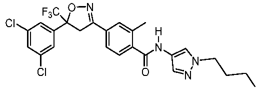 | ¹H NMR (400 MHz, Chloroform-*d*) δ 8.08 (s, 1H), 8.05 (s, 1H), 7.51 (d, *J* = 1.9 Hz, 2H), 7.48 (d, *J* = 3.4 Hz, 1H), 7.46 - 7.41 (m, 4H), 4.13 - 4.04 (m, 3H), 3.72 (d, *J* = 17.4 Hz, 1H), 2.46 (s, 3H), 1.88 - 1.79 (m, 2H), 1.40 - 1.28 (m, 2H), 0.93 (t, *J* = 7.4 Hz, 3H). |
| 16 | 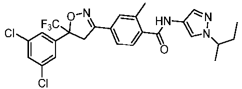 | ¹H NMR (400 MHz, Chloroform-*d*) δ 8.12 (s, 1H), 7.76 (s, 1H), 7.53 (d, *J =* 9.7 Hz, 6H), 7.44 (d, *J =* 1.9 Hz, 1H), 4.28 - 4.19 (m, 1H), 4.10 (d, *J =* 17.4 Hz, 1H), 3.72 (d, *J* = 17.4 Hz, 1H), 2.52 (s, 3H), 1.99 - 1.74 (m, 2H), 1.51 (d, *J* = 6.8 Hz, 3H), 0.84 (t, *J =* 7.4 Hz, 3H). |
| 17 | 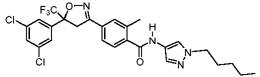 | ¹H NMR (600 MHz, DMSO-*d*₆) δ 10.42 (s, 1H), 8.00 (d, *J =* 2.8 Hz, 1H), 7.80 (s, 1H), 7.63 (d, *J =* 13.1 Hz, 4H), 7.55 - 7.51 (m, 1H), 7.48 (d, *J* = 3.0 Hz, 1H), 4.38 (d, *J =* 18.4 Hz, 1H), 4.30 (d, *J =* 18.4 Hz, 1H), 4.04 (t, *J =* 7.0 Hz, 2H), 2.39 (s, 3H), 1.76 - 1.69 (m, 2H), 1.32 - 1.21 (m, 2H), 1.22 - 1.16 (m, 2H), 0.83 (t, *J =* 7.5 Hz, 3H). |
| 18 | 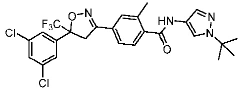 | ¹H NMR (400 MHz, DMSO-*d*₆) δ 10.43 (s, 1H), 8.06 (s, 1H), 7.83 (t, *J* = 1.9 Hz, 1H), 7.68 - 7.62 (m, 4H), 7.54 (d, *J =* 6.9 Hz, 2H), 4.41 (d, *J =* 18.4 Hz, 1H), 4.32 (d, *J* = 18.4 Hz, 1H), 2.41 (s, 3H), 1.52 (s, 9H). |
| 19 | 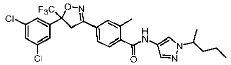 | ¹H NMR (400 MHz, Chloroform-*d*) δ 8.13 (s, 1H), 7.73 - 7.65 (m, 1H), 7.58 - 7.48 (m, 6H), 7.44 (t, *J* = 1.9 Hz, 1H), 4.40 - 4.26 (m, 1H), 4.10 (d, *J =* 17.4 Hz, 1H), 3.71 (d, *J =* 17.4 Hz, 1H), 2.53 (s, 3H), 1.94 - 1.86 (m, 1H), 1.81 - 1.64 (m, 1H), 1.51 (d, *J* = 6.7 Hz, 3H), 1.37 - 1.13 (m, 2H), 0.91 (t, *J* = 7.3 Hz, 3H). |
| 20 | 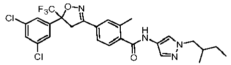 | ¹H NMR (400 MHz, DMSO-*d*₆) δ 10.47 (s, 1H), 8.03 (s, 1H), 7.87 - 7.82 (m, 1H), 7.71 - 7.64 (m, 4H), 7.58 (d, *J =* 7.9 Hz, 1H), 7.53 (d, *J =* 1.1 Hz, 1H), 4.43 (d, *J* = 18.4 Hz, 1H), 4.34 (d, *J =* 18.4 Hz, 1H), 4.06 - 3.97 (m, 1H), 3.95 - 3.85 (m, 1H), 2.44 (s, 3H), 1.96 - 1.86 (m, 1H), 1.38 - 1.24 (m, 1H), 1.20 - 1.06 (m, 1H), 0.89 (t, *J =* 7.4 Hz, 3H), 0.82 (d, *J =* 6.7 Hz, 3H). |
| 21 | 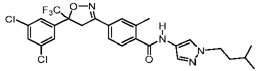 | ¹H NMR (400 MHz, Chloroform-*d*) δ 8.11 (s, 1H), 7.71 (s, 1H), 7.58 - 7.47 (m, 6H), 7.46 - 7.41 (m, 1H), 4.18 - 4.06 (m, 3H), 3.71 (d, *J =* 17.4 Hz, 1H), 2.51 (s, 3H), 1.83 - 1.74 (m, 2H), 1.68 - 1.53 (m, 1H), 0.96 (d, *J =* 6.6 Hz, 6H). |
| 22 | 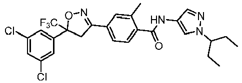 | ¹H NMR (600 MHz, Chloroform-*d*) δ 8.09 (s, 1H), 7.59 - 7.46 (m, 7H), 7.43 (t, *J=* 1.9 Hz, 1H), 4.09 *(d, J=* 17.4 Hz, 1H), 3.96 - 3.83 (m, 1H), 3.71 (d, *J=* 17.4 Hz, 1H), 2.54 (s, 3H), 1.95 - 1.77 (m, 4H), 0.81 (t, *J =* 7.3 Hz, 6H). |
| 23 | 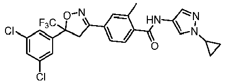 | ¹H NMR (600 MHz, DMSO-*d*₆) δ 10.41 (s, 1H), 8.01 (s, 1H), 7.80 (d, *J* = 1.7 Hz, 1H), 7.65 - 7.60 (m, 4H), 7.51 (d, *J* = 7.9 Hz, 1H), 7.46 (s, 1H), 4.37 (d,*J* = 18.4 Hz, 1H), 4.29 (d, *J* = 18.4 Hz, 1H), 3.72 - 3.66 (m, 1H), 2.38 (s, 3H), 1.02 - 0.98 (m, 2H), 0.94 - 0.89 (m, 2H). |
| 24 | 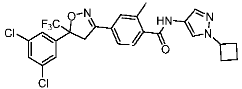 | ¹H NMR (600 MHz, DMSO-*d*₆) δ 10.45 (s, 1H), 8.07 (s, 1H), 7.83 (s, 1H), 7.69 - 7.60 (m, 4H), 7.54 (d, *J =* 7.8 Hz, 2H), 4.86 - 4.78 (m, 1H), 4.40 (d, *J* = 18.4 Hz, 1H), 4.32 (d, *J =* 18.4 Hz, 1H), 2.47 - 2.42 (m, 2H), 2.40 (s, 3H), 2.37 - 2.33 (m, 2H), 1.82 - 1.70 (m, 2H). |
| 25 | 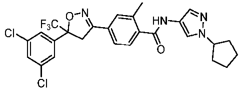 | ¹H NMR (600 MHz, DMSO-*d*₆) δ 10.42 (s, 1H), 8.00 (s, 1H), 7.81 (s, 1H), 7.66 - 7.57 (m, 4H), 7.52 (d, *J* = 7.9 Hz, 1H), 7.48 (s, 1H), 4.69 - 4.63 (m, 1H), 4.38 (d, *J =* 18.4 Hz, 1H), 4.30 (d, *J* = 18.4 Hz, 1H), 2.38 (s, 3H), 2.08 - 2.00 (m, 2H), 1.92 - 1.84 (m, 2H), 1.80 - 1.71 (m, 2H), 1.67 - 1.57 (m, 2H). |
| 26 | 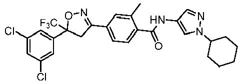 | ¹H NMR (600 MHz, DMSO-*d*₆) δ 10.44 (s, 1H), 8.01 (s, 1H), 7.83 (s, 1H), 7.68 - 7.62 (m, 4H), 7.54 (d, *J =* 7.9 Hz, 1H), 7.51 (s, 1H), 4.40 (d, *J =* 18.4 Hz, 1H), 4.32 (d, *J =* 18.4 Hz, 1H), 4.15 - 4.08 (m, 1H), 2.41 (s, 3H), 2.03 - 1.95 (m, 2H), 1.84 - 1.77 (m, 2H), 1.74 - 1.67 (m, 2H), 1.43 - 1.34 (m, 2H), 1.26 - 1.19 (m, 2H). |
| 27 | 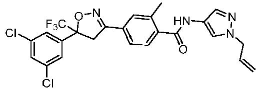 | ¹H NMR (600 MHz, DMSO-*d*₆) δ 10.47 (s, 1H), 8.03 (s, 1H), 7.83 (s, 1H), 7.68 - 7.61 (m, 4H), 7.58 - 7.51 (m, 2H), 6.05 - 5.96 (m, 1H), 5.23 - 5.12 (m, 2H), 4.74 (d, *J* = 4.2 Hz, 2H), 4.40 (d, *J* = 18.4 Hz, 1H), 4.32 (d, *J =* 18.4 Hz, 1H), 2.41 (s, 3H). |
| 28 | 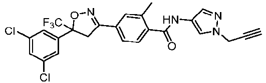 | ¹H NMR (600 MHz, DMSO-*d*₆) δ 10.59 (s, 1H), 8.23 (s, 1H), 7.82 (s, 1H), 7.67 (s, 2H), 7.65 (s, 1H), 7.64 (s, 2H), 7.57 (d, *J =* 7.9 Hz, 1H), 5.48 (s, 2H), 4.41 (d, *J =* 18.4 Hz, 1H), 4.32 (d, *J =* 18.4 Hz, 1H) , 2.61 (s, 1H), 2.42 (s, 3H). |
| 29 | 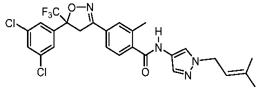 | ¹H NMR (600 MHz, DMSO-*d*₆) δ 10.46 (s, 1H), 8.00 (s, 1H), 7.82 (s, 1H), 7.66 (d, *J =* 120 Hz, 4H), 7.56 (d, *J =* 7.8 Hz, 1H), 7.50 (s, 1H), 5.39 (t, *J* = 7.3 Hz, 1H), 4.70 (d, *J =* 7.1 Hz, 2H), 4.41 (d, *J =* 18.4 Hz, 1H), 4.33 (d, *J =* 18.4 Hz, 1H), 2.42 (s, 3H), 1.75 (d, *J* = 10.7 Hz, 6H). |
| 30 | 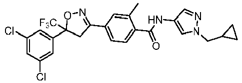 | ¹H NMR (600 MHz, DMSO-*d*₆) δ 10.46 (s, 1H), 8.08 (s, 1H), 7.83 (d, *J* = 2.1 Hz, 1H), 7.68 - 7.62 (m, 4H), 7.55 (d, *J* = 7.8 Hz, 2H), 4.86 - 4.79 (m, 1H), 4.41 (d, *J =* 18.4 Hz, 1H), 4.33 (d, *J* = 18.4 Hz, 1H), 2.45 (d, *J =* 7.3 Hz, 2H), 2.41 (s, 3H), 2.39 - 2.32 (m, 2H), 1.82 - 1.72 (m, 2H). |
| 31 | 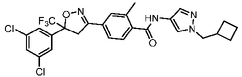 | ¹H NMR (600 MHz, DMSO-*d*₆) δ 10.44 (s, 1H), 8.00 (s, 1H), 7.83 (s, 1H), 7.67 - 7.61 (m, 4H), 7.55 (d, *J =* 7.8 Hz, 1H), 7.50 (s, 1H), 4.40 (d, *J =* 18.4 Hz, 1H), 4.32 (d, *J =* 18.4 Hz, 1H), 4.10 (d, *J =* 7.3 Hz, 2H), 2.77 - 2.66 (m, 1H), 2.41 (s, 3H), 2.03 - 1.92 (m, 2H), 1.88 - 1.80 (m, 2H), 1.80 - 1.69 (m, 2H). |
| 32 | 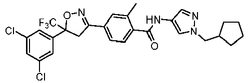 | ¹H NMR (600 MHz, DMSO-*d*₆) δ 10.40 (s, 1H), 8.00 (s, 1H), 7.80 (s, 1H), 7.63 (s, 1H), 7.61 (s, 3H), 7.53 (d, *J =* 7.7 Hz, 1H), 7.47 (s, 1H), 4.38 (d, *J =* 18.4 Hz, 1H), 4.29 (d, *J =* 18.4 Hz, 1H), 3.97 (d, *J* = 7.2 Hz, 2H), 2.39 (s, 3H), 1.99-1.94 (m, 1H), 1.61-1.55 (m, 4H), 1.51-1.45 (m, 2H), 1.25-1.20 (m, 2H). |
| 33 | 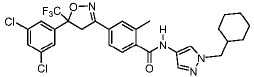 | ¹H NMR (400 MHz, Chloroform-*d*) δ 8.10 (s, 1H), 7.74 (s, 1H), 7.55 (d, *J* = 4.5 Hz, 4H), 7.53 - 7.50 (m, 2H), 7.44 (q, *J =* 1.6 Hz, 1H), 4.10 (d, *J =* 17.4 Hz, 1H), 3.96 (d, *J =* 7.2 Hz, 2H), 3.71 (d, *J* = 17.4 Hz, 1H), 2.53 (s, 3H), 1.95 - 1.85 (m, 1H), 1.78 - 1.59 (m, 4H), 1.34 - 1.10 (m, 4H), 1.06 - 0.91 (m, 2H). |
| 34 | 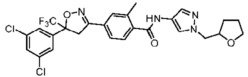 | ¹H NMR (600 MHz, DMSO-*d*₆) δ 10.44 (s, 1H), 8.03 (s, 1H), 7.83 (d, *J* = 2.4 Hz, 1H), 7.66 (d, *J =* 4.7 Hz, 1H), 7.64 (s, 3H), 7.56 (d, *J =* 7.9 Hz, 1H), 7.52 (s, 1H), 4.41 (d, *J* = 18.4 Hz, 1H), 4.32 (d, *J* = 18.4 Hz, 1H), 4.17 - 4.08 (m, 3H), 3.76 (q, *J =* 7.2 Hz, 1H), 3.64 (q, *J =* 7.2 Hz, 1H), 2.42 (s, 3H), 1.95 - 1.87 (m, 1H), 1.82 - 1.75 (m, 2H), 1.61 - 1.54 (m, 1H). |
| 35 | 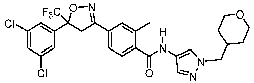 | ¹H NMR (400 MHz, DMSO-*d*₆) δ 10.45 (s, 1H), 8.03 (s, 1H), 7.82 (s, 1H), 7.69 - 7.60 (m, 4H), 7.59 - 7.48 (m, 2H), 4.40 (d, *J* = 18.4 Hz, 1H), 4.32 (d, *J* = 18.4 Hz, 1H), 4.05 - 3.94 (m, 2H), 3.88 - 3.76 (m, 2H), 3.30 - 3.18 (m, 2H), 2.41 (s, 3H), 2.01 - 1.96 (m, 1H), 1.46 - 1.32 (m, 2H), 1.30 - 1.18 (m, 2H). |
| 36 | 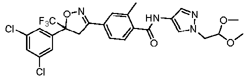 | ¹H NMR (600 MHz, DMSO-*d*₆) δ 10.45 (s, 1H), 8.03 (s, 1H), 7.83 (s, 1H), 7.65 (d, *J* = 13.0 Hz, 4H), 7.56 (d, *J* = 8.2 Hz, 1H), 7.54 (s, 1H), 4.69 (t, *J* = 5.4 Hz ,1H), 4.40 (d, *J =* 18.4 Hz, 1H), 4.32 (d, *J =* 18.4 Hz, 1H), 4.20 (d, *J* = 5.4 Hz, 2H), 3.28 (s, 6H), 2.41 (s, 3H). |
| 37 | 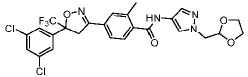 | ¹H NMR (600 MHz, DMSO-*d*₆) δ 10.45 (s, 1H), 8.03 (s, 1H), 7.81 (s, 1H), 7.65 (d, *J =* 4.4 Hz, 2H), 7.63 (s, 2H), 7.54 (d, *J =* 7.9 Hz, 1H), 7.52 (s, 1H), 5.13 (t, *J* = 4.4 Hz, 1H), 4.39 (d, *J =* 18.4 Hz, 1H), 4.31 (d, *J* = 18.4 Hz, 1H), 4.21 (d, *J* = 4.4 Hz, 2H), 3.87 (d, *J* = 4.7 Hz, 2H), 3.83 - 3.79 (m, 2H), 2.40 (s, 3H). |
| 38 | 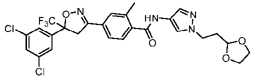 | ¹H NMR (400 MHz, DMSO-*d*₆) δ 10.47 (s, 1H), 8.05 (s, 1H), 7.84 (t, *J* = 1.9 Hz, 1H), 7.70 - 7.63 (m, 4H), 7.56 (d, *J =* 13.1 Hz, 2H), 4.83 (t, *J =* 4.7 Hz, 1H), 4.43 (d, *J* = 18.4 Hz, 1H), 4.34 (d, *J* = 18.4 Hz, 1H), 4.19 (t, *J =* 4.7 Hz, 2H), 3.96 - 3.90 (m, 2H), 3.83 - 3.76 (m, 2H), 2.43 (s, 3H), 2.15 - 2.06 (m, 2H). |
| 39 | 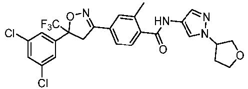 | ¹H NMR (400 MHz, DMSO-*d*₆) δ 10.48 (s, 1H), 8.08 (s, 1H), 7.83 (t, *J* = 1.9 Hz, 1H), 7.68 - 7.62 (m, 4H), 7.55 (d, *J* = 6.1 Hz, 2H), 5.07 - 4.98 (m, 1H), 4.41 (d, *J =* 18.4 Hz, 1H), 4.32 (d, *J =* 18.4 Hz, 1H), 4.01 - 3.92 (m, 2H), 3.90 - 3.78 (m, 2H), 2.41 (s, 3H), 2.40 - 2.32 (m, 1H), 2.28 - 2.18 (m, 1H). |
| 40 | 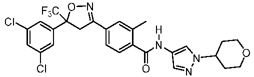 | ¹H NMR (400 MHz, Chloroform-*d*) δ 8.21 (s, 1H), 7.84 (s, 1H), 7.60 (s, 1H), 7.58 - 7.52 (m, 3H), 7.51 (d, *J* = 1.9 Hz, 2H), 7.44 (t, *J* = 1.9 Hz, 1H), 4.48 - 4.34 (m, 1H), 4.17 - 4.04 (m, 3H), 3.72 (d, *J =* 17.4 Hz, 1H), 3.61 - 3.48 (m, 2H), 2.52 (s, 3H), 2.17 - 2.07 (m, 4H). |
| 41 | 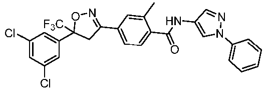 | ¹H NMR (600 MHz, DMSO-*d*₆) δ 10.68 (s, 1H), 8.67 (s, 1H), 7.88 - 7.79 (m, 4H), 7.74 - 7.59 (m, 5H), 7.50 (t, *J* = 7.8 Hz, 2H), 7.31 (t, *J* = 7.4 Hz, 1H), 4.42 (d, *J =* 18.4 Hz, 1H), 4.33 (d, *J =* 18.4 Hz, 1H), 2.45 (s, 3H). |
| 42 | 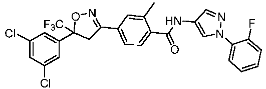 | ¹H NMR (400 MHz, DMSO-*d*₆) δ 10.71 (s, 1H), 8.51 (d, *J* = 2.9 Hz, 1H), 7.91 (s, 1H), 7.86 - 7.82 (m, 2H), 7.69 (s, 1H), 7.68 - 7.60 (m, 4H), 7.52 - 7.34 (m, 3H), 4.41 (d, *J =* 18.4 Hz, 1H), 4.33 (d, *J =* 18.4 Hz, 1H), 2.45 (s, 3H). |
| 43 | 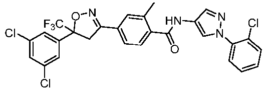 | ¹H NMR (400 MHz, DMSO-*d*₆) δ 10.52 (s, 1H), 8.14 (s, 1H), 7.83 (t, *J* = 1.9 Hz, 1H), 7.68 - 7.61 (m, 4H), 7.61 - 7.54 (m, 2H), 7.53 - 7.48 (m, 1H), 7.39 - 7.31 (m, 2H), 7.06 - 7.02 (m, 1H), 4.41 (d, *J =* 18.4 Hz, 1H), 4.32 (d, *J =* 18.4 Hz, 1H), 2.41 (s, 3H). |
| 44 | 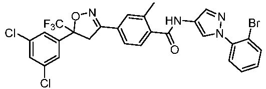 | ¹H NMR (600 MHz, DMSO-*d*₆) δ 10.49 (s, 1H), 8.13 (s, 1H), 7.81 (s, 1H), 7.69 - 7.59 (m, 6H), 7.57 (d, *J* = 7.9 Hz, 1H), 7.38 (t, *J* = 7.5 Hz, 1H), 7.28 (t, *J* = 7.7 Hz, 1H), 6.99 (d, *J* = 7.7 Hz, 1H), 4.40 (d, *J* = 18.4 Hz, 1H), 4.31 (d, *J* = 18.4 Hz, 1H), 2.42 (s, 3H). |
| 45 | 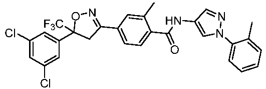 | ¹H NMR (600 MHz, DMSO-*d*₆) δ 10.45 (s, 1H), 8.00 (s, 1H), 7.84 - 7.79 (m, 1H), 7.69 - 7.59 (m, 4H), 7.55 (d, *J =* 8.1 Hz, 2H), 7.25 - 7.14 (m, 3H), 7.01 (d, *J* = 7.5 Hz, 1H), 4.39 (d, *J =* 18.4 Hz, 1H), 4.31 (d, *J =* 18.4 Hz, 1H), 2.41 (s, 3H), 2.31 (s, 3H). |
| 46 | 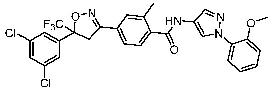 | ¹H NMR (600 MHz, DMSO-*d*₆) δ 10.54 (s, 1H), 8.14 (s, 1H), 7.93 - 7.90 (m, 1H), 7.81 (d, *J =* 3.8 Hz, 1H), 7.65 (t, *J =* 12.7 Hz, 5H), 7.59 (d, *J =* 7.3 Hz, 1H), 7.41 - 7.36 (m, 1H), 7.11 - 7.06 (m, 1H), 6.92 (d, *J =* 7.5 Hz, 1H), 4.42 (d, *J* = 18.3 Hz, 1H), 4.33 (d, *J =* 18.3 Hz, 1H) , 3.69 (s, 3H), 2.43 (s, 3H). |
| 47 | 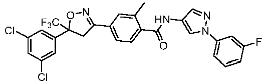 | ¹H NMR (600 MHz, DMSO-*d*₆) δ 10.73 (s, 1H), 8.76 (s, 1H), 7.88 (s, 1H), 7.82 (t, *J* = 1.9 Hz, 1H), 7.76 - 7.67 (m, 4H), 7.65 (d, *J* = 2.0 Hz, 2H), 7.63 (d, *J =* 8.0 Hz, 1H), 7.56 - 7.50 (m, 1H), 7.17 - 7.11 (m, 1H), 4.43 (d, *J =* 18.4 Hz, 1H), 4.34 (d, *J* = 18.4 Hz, 1H), 2.46 (s, 3H). |
| 48 | 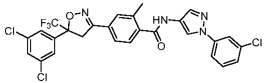 | ¹H NMR (400 MHz, DMSO-*d*₆) δ 10.50 (s, 1H), 8.19 (s, 1H), 7.83 (t, *J* = 1.9 Hz, 1H), 7.70 - 7.60 (m, 4H), 7.60 - 7.53 (m, 2H), 7.43 - 7.34 (m, 2H), 7.32 (d, *J* = 2.3 Hz, 1H), 7.28 - 7.19 (m, 1H), 4.41 (d, *J =* 18.4 Hz, 1H), 4.32 (d, *J* = 18.4 Hz, 1H), 2.41 (s, 3H). |
| 49 | 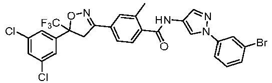 | ¹H NMR (600 MHz, DMSO-*d*₆) δ 10.52 (d, *J =* 6.0 Hz, 1H), 8.24 - 8.18 (m, 1H), 7.81 (d, *J =* 9.9 Hz, 1H), 7.67 (s, 1H), 7.65 (s, 3H), 7.61 - 7.56 (m, 2H), 7.50 *(d, J=* 7.9 Hz, 1H), 7.48 (s, 1H), 7.33 (t, *J* = 7.6 Hz, 1H), 7.27 (d, *J = 7.7* Hz, 1H), 4.41 (d, *J =* 18.4 Hz, 1H), 4.33 (d, *J =* 18.4 Hz, 1H), 2.42 (s, 3H). |
| 50 | 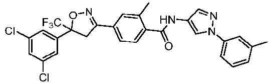 | ¹H NMR (600 MHz, DMSO-*d*₆) δ 10.52 (s, 1H), 8.16 (s, 1H), 7.79 (s, 1H), 7.69 (s, 1H), 7.67 (s, 3H), 7.60 (d, *J =* 4.3 Hz, 1H), 7.58 (s, 1H), 7.23 (d, *J =* 7.7 Hz, 1H), 7.11 (d, *J =* 8.1 Hz, 2H), 7.08 (d, *J =* 8.0 Hz, 1H), 4.43 (d, *J =* 18.3 Hz, 1H), 4.34 (d, *J =* 18.3 Hz, 1H), 2.44 (s, 3H), 2.29 (s, 3H). |
| 51 | 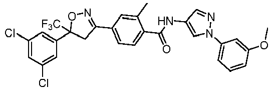 | ¹H NMR (600 MHz, DMSO-*d*₆) δ 10.47 (s, 1H), 8.12 (d, *J* = 2.6 Hz, 1H), 7.79 - 7.74 (m, 1H), 7.63 (d, *J =* 13.1 Hz, 4H), 7.57 - 7.52 (m, 2H), 7.24 (t, *J =* 7.9 Hz, 1H), 6.83 (s, 2H), 6.80 (d, *J* = 7.4 Hz, 1H), 4.38 (d, *J* = 18.4 Hz, 1H), 4.30 (d, *J* = 18.4 Hz, 1H), 3.71 (s, 3H), 2.40 (s, 3H). |
| 52 | 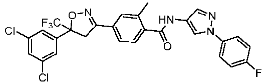 | ¹H NMR (600 MHz, DMSO-*d*₆) δ 10.70 (s, 1H), 8.67 (s, 1H), 7.90 - 7.85 (m, 2H), 7.85 (s, 1H), 7.83 (s, 1H), 7.69 (d, *J =* 10.1 Hz, 2H), 7.65 (d, *J =* 1.9 Hz, 2H), 7.62 (d, *J =* 7.8 Hz, 1H), 7.34 (t, *J =* 8.6 Hz, 2H), 4.42 (d, *J =* 18.4 Hz, 1H), 4.34 (d, *J =* 18.4 Hz, 1H), 2.46 (s, 3H). |
| 53 | 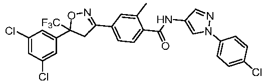 | ¹H NMR (600 MHz, DMSO-*d*₆) δ 10.51 (s, 1H), 8.10 (s, 1H), 7.85 (d, *J* = 8.2 Hz, 2H), 7.81 (s, 1H), 7.67 - 7.58 (m, 6H), 7.54 (t, *J =* 4.0 Hz, 2H), 4.40 (d, *J* = 18.4 Hz, 1H), 4.31 (d, *J* = 18.4 Hz, 1H), 2.44 (s, 3H). |
| 54 | 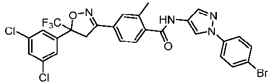 | ¹H NMR (600 MHz, DMSO-*d*₆) δ 10.51 (s, 1H), 8.18 (s, 1H), 7.81 (s, 1H), 7.67 (s, 2H), 7.65 (s, 2H), 7.59 - 7.56 (m, 3H), 7.55 (s, 1H), 7.23 (s, 1H), 7.22 (s, 1H), 4.41 (d, *J* = 18.4 Hz, 1H), 4.33 (d, *J* = 18.4 Hz, 1H), 2.42 (s, 3H). |
| 55 | 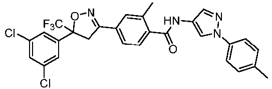 | ¹H NMR (600 MHz, DMSO-*d*₆) δ 10.48 (s, 1H), 8.10 (s, 1H), 7.81 (s, 1H), 7.67 (s, 2H), 7.65 (s, 2H), 7.56 (s, 1H), 7.55 (s, 1H), 7.17 (q, *J =* 8.0 Hz, 4H), 4.41 (d, *J* = 18.4 Hz, 1H), 4.32 (d, *J* = 18.4 Hz, 1H), 2.41 (s, 3H), 2.28 (s, 3H). |
| 56 | 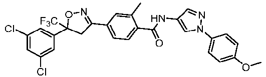 | ¹H NMR (600 MHz, DMSO-*d*₆) δ 10.46 (s, 1H), 8.08 (d, *J =* 2.3 Hz, 1H), 7.82 (s, 1H), 7.66 (s, 1H), 7.64 (s, 3H), 7.55 (d, *J =* 8.1 Hz, 1H), 7.52 (s, 1H), 7.25 (d, *J =* 80 Hz, 2H), 6.92 (s, 1H), 6.91 (s, 1H), 4.40 (d, *J =* 18.4 Hz, 1H), 4.32 (d, *J =* 18.4 Hz, 1H), 3.74 (s, 3H), 2.40 (s, 3H). |
| 57 | 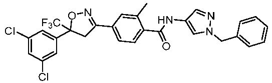 | ¹H NMR (600 MHz, DMSO-*d*₆) δ 10.49 (s, 1H), 8.13 (s, 1H), 7.83 (s, 1H), 7.69 - 7.60 (m, 4H), 7.55 (d, *J =* 8.9 Hz, 2H), 7.36 (t, *J =* 7.5 Hz, 2H), 7.30 (d, *J =* 7.2 Hz, 1H), 7.27 (d, *J =* 7.5 Hz, 2H), 5.31 (s, 2H), 4.40 (d, *J =* 18.4 Hz, 1H), 4.32 (d, *J =* 18.4 Hz, 1H), 2.40 (s, 3H). |
| 58 | 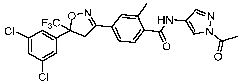 | ¹H NMR (400 MHz, DMSO-*d*₆) δ 10.41 (s, 1H), 8.55 (s, 1H), 7.86 (d, *J* = 0.7 Hz, 1H), 7.82 (t, *J =* 1.9 Hz, 1H), 7.72 (d, *J =* 1.6 Hz, 1H), 7.70 - 7.66 (m, 1H), 7.64 (d, *J* = 1.9 Hz, 2H), 7.60 (d, *J =* 8.0 Hz, 1H), 4.43 (d, *J* = 18.4 Hz, 1H), 4.34 (d, *J* = 18.4 Hz, 1H), 2.25 (s, 3H), 2.06 (s, 3H). |
| 59 | 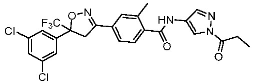 | ¹H NMR (400 MHz, DMSO-*d*₆) δ 10.33 (s, 1H), 8.56 (s, 1H), 7.87 (s, 1H), 7.83 (t, *J* = 1.9 Hz, 1H), 7.71 (s, 1H), 7.69 - 7.65 (m, 1H), 7.64 (d, *J =* 1.9 Hz, 2H), 7.60 (d, *J =* 8.0 Hz, 1H), 4.43 (d, *J* = 18.4 Hz, 1H), 4.34 (d, *J =* 18.4 Hz, 1H), 2.33 (q, *J =* 7.6 Hz, 2H), 2.25 (s, 3H), 1.09 (t, *J =* 7.6 Hz, 3H). |
| 60 | 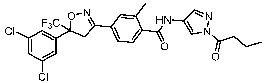 | ¹H NMR (400 MHz, DMSO-*d*₆) δ 10.80 (s, 1H), 8.56 (s, 1H), 7.96 (d, *J =* 0.7 Hz, 1H), 7.82 (t, *J =* 1.9 Hz, 1H), 7.68 (d, *J =* 7.4 Hz, 2H), 7.64 (d, *J* = 1.8 Hz, 2H), 7.61 (d, *J =* 8.2 Hz, 1H), 4.41 (d, *J =* 18.4 Hz, 1H), 4.32 (d, *J =* 18.4 Hz, 1H), 3.07 (t, *J* = 7.3 Hz, 2H), 2.43 (s, 3H), 1.76 - 1.64 (m, 2H), 0.96 (t, *J* = 7.4 Hz, 3H). |
| 61 | 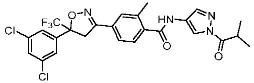 | ¹H NMR (400 MHz, DMSO-*d*₆) δ 10.54 (s, 1H), 8.57 (s, 1H), 7.90 (s, 1H), 7.84 (t, *J* = 1.7 Hz, 1H), 7.78 (s, 1H), 7.72 - 7.67 (m, 1H), 7.63 (d, *J =* 1.7 Hz, 2H), 7.57 (d, *J* = 8.0 Hz, 1H), 4.43 (d, *J* = 18.4 Hz, 1H), 4.35 (d, *J =* 18.4 Hz, 1H), 3.54-3.38 (m, 1H), 2.45 (s, 3H), 1.34 (d, *J =* 6.7 Hz, 6H). |
| 62 | 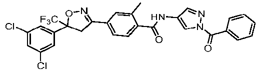 | ¹H NMR (400 MHz, DMSO-*d*₆) δ 10.90 (s, 1H), 8.75 (s, 1H), 8.07 (s, 1H), 8.03 (d,J= 1.2 Hz, 1H), 8.01 (d,J= 1.6 Hz, 1H), 7.82 *(t, J=* 1.9 Hz, 1H), 7.72 - 7.67 (m, 3H), 7.67 - 7.64 (m, 3H), 7.58 (t, *J =* 7.7 Hz, 2H), 4.43 (d, *J =* 18.4 Hz, 1H), 4.34 (d, *J =* 18.4 Hz, 1H), 2.46 (s, 3H). |
| 63 | 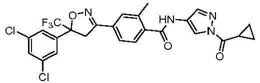 | ¹H NMR (600 MHz, DMSO-*d*₆) δ 10.64 (s, 1H), 8.52 (s, 1H), 7.88 (s, 1H), 7.82 (s, 1H), 7.71 (s, 1H), 7.66 (d, *J =* 8.2 Hz, 1H), 7.63 (s, 2H), 7.60 (d, *J =* 8.0 Hz, 1H), 4.42 *(d, J=* 18.4 Hz, 1H), 4.33 *(d, J=* 18.4 Hz, 1H), 2.24 (s, 3H), 1.78-1.72 (m, 1H), 0.83 (d, *J =* 6.0 Hz, 4H). |
| 64 | 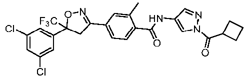 | ¹H NMR (600 MHz, DMSO-*d*₆) δ 10.53 (s, 1H), 8.57 (s, 1H), 7.83 (s, 1H), 7.73 - 7.60 (m, 4H), 7.56 (d, *J* = 7.8 Hz, 2H), 4.43 (d, *J* = 18.4 Hz, 1H), 4.36 (d, *J =* 18.4 Hz, 1H), 3.52 - 3.37 (m, 1H), 2.43 (s, 3H), 1.65 - 1.54 (m, 2H), 1.48 - 1.40 (m, 2H), 1.21 - 1.10 (m, 2H). |
| 65 | 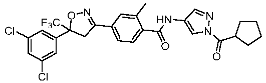 | ¹H NMR (400 MHz, DMSO-*d*₆) δ 10.36 (s, 1H), 8.56 (s, 1H), 7.88 (s, 1H), 7.83 (t, *J* = 1.9 Hz, 1H), 7.71 (s, 1H), 7.67 (d, *J =* 8.0, 1.6 Hz, 1H), 7.64 (d, *J* = 1.9 Hz, 2H), 7.60 (d, *J =* 8.0 Hz, 1H), 4.43 (d, *J =* 18.4 Hz, 1H), 4.34 (d, *J =* 18.4 Hz, 1H), 2.76 (p, *J* = 7.9 Hz, 1H) , 2.46 (s, 3H), 1.88 - 1.80 (m, 2H), 1.74 - 1.64 (m, 4H), 1.58 - 1.51 (m, 2H). |
| 66 | 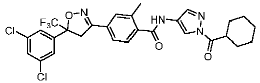 | ¹H NMR (600 MHz, DMSO-*d*₆) δ 10.44 (s, 1H), 8.41 (s, 1H), 7.97 (s, 1H), 7.82 - 7.77 (m, 4H), 7.68 (d, *J =* 7.8 Hz, 1H), 7.55 (s, 1H), 4.41 (d, *J =* 18.4 Hz, 1H), 4.34 (d, *J =* 18.4 Hz, 1H), 3.34 - 3.28 (m, 1H), 2.41 (s, 3H), 1.56 - 1.48 (m, 2H), 1.41 - 1.37 (m, 2H), 1.28 - 1.24 (m, 2H), 1.14 - 1.10 (m, 2H), 0.95 - 0.88 (m, 2H). |
| 67 | 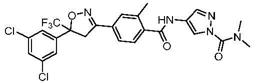 | ¹H NMR (600 MHz, DMSO-*d*₆) δ 10.69 (s, 1H), 8.40 (s, 1H), 7.85 (s, 1H), 7.83 (s, 1H), 7.68 (s, 1H), 7.66 (s, 1H), 7.64 (s, 2H), 7.60 (d, *J =* 8.4 Hz, 1H), 4.41 (d, *J =* 18.4 Hz, 1H), 4.33 (d, *J =* 18.4 Hz, 1H), 3.13 (s, 6H), 2.43 (s, 3H). |
| 68 | 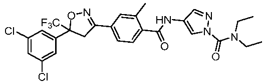 | ¹H NMR (400 MHz, Chloroform-*d*) δ 8.45 (s, 1H), 7.85 (s, 1H), 7.72 (s, 1H), 7.54 (s, 2H), 7.51 (t, *J =* 2.6 Hz, 4H), 4.10 (d, *J =* 17.4 Hz, 1H), 3.72 (d, *J =* 17.4 Hz, 1H), 3.58 *(q, J* = 6.9Hz, 4H), 2.51 (s, 3H), 1.27 *(t, J =* 6.9 Hz, 6H). |
| 69 | 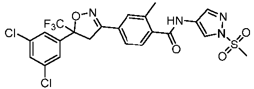 | ¹H NMR (600 MHz, DMSO-*d*₆) δ 10.87 (s, 1H), 8.47 (s, 1H), 8.02 (s, 1H), 7.83 (t, *J =* 1.9 Hz, 1H), 7.68 (d, *J =* 9.6 Hz, 2H), 7.65 - 7.58 (m, 3H), 4.42 (d, *J= 18.4* Hz, 1H), 4.33 (d, *J =* 18.4 Hz, 1H), 3.55 (s, 3H), 2.43 (s, 3H). |
| 70 | 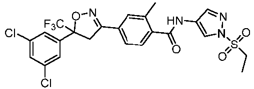 | ¹H NMR (600 MHz, DMSO-*d*₆) δ 10.86 (s, 1H), 8.47 (s, 1H), 8.04 (s, 1H), 7.83 (t, *J* = 1.9 Hz, 1H), 7.68 (d, *J =* 10.0 Hz, 2H), 7.66 - 7.60 (m, 3H), 4.42 (d, *J =* 18.4 Hz, 1H), 4.33 (d, *J =* 18.4 Hz, 1H), 3.71 (q, *J =* 7.3 Hz, 2H), 2.44 (s, 3H), 1.12 (t, *J =* 7.3 Hz, 3H). |
| 71 | 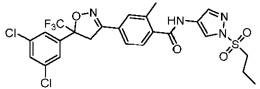 | ¹H NMR (600 MHz, DMSO-*d*₆) δ 10.85 (s, 1H), 8.46 (s, 1H), 8.03 (s, 1H), 7.83 (s, 1H), 7.68 (d, *J =* 10.1 Hz, 2H), 7.65 - 7.60 (m, 3H), 4.41 (d, *J =* 18.4 Hz, 1H), 4.33 (d, *J =* 18.4 Hz, 1H), 3.68 (t, *J =* 7.6 Hz, 2H), 2.43 (s, 3H), 1.62 - 1.53 (m, 2H), 0.92 (t, *J =* 7.4 Hz, 3H). |
| 72 | 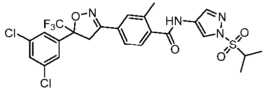 | ¹H NMR (600 MHz, DMSO-*d*₆) δ 10.85 (s, 1H), 8.46 (s, 1H), 8.04 (s, 1H), 7.83 (s, 1H), 7.68 (d, *J =* 10.2 Hz, 2H), 7.65 - 7.61 (m, 3H), 4.41 (d, *J =* 18.4 Hz, 1H), 4.33 (d, *J* = 18.4 Hz, 1H), 3.94 - 3.86 (m, 1H), 2.44 (s, 3H), 1.24 (d, *J =* 6.8 Hz, 6H). |
| 73 | 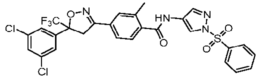 | ¹H NMR (600 MHz, DMSO-*d*₆) δ 10.83 (s, 1H), 8.59 (s, 1H), 8.01 (d, *J* = 7.9 Hz, 2H), 7.96 (s, 1H), 7.84 - 7.79 (m, 2H), 7.71 - 7.64 (m, 4H), 7.63 (s, 2H), 7.59 *(d, J* = 7.9 Hz, 1H), 4.40 (d, *J =* 18.4 Hz, 1H), 4.32 (d, *J* = 18.4 Hz, 1H), 2.42 (s, 3H). |
| 74 | 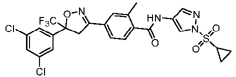 | ¹H NMR (600 MHz, DMSO-*d*₆) δ 10.86 (s, 1H), 8.46 (s, 1H), 8.03 (s, 1H), 7.83 (d, *J =* 2.0 Hz, 1H), 7.68 (d, *J =* 10.2 Hz, 2H), 7.65 - 7.60 (m, 3H), 4.42 *(d, J* = 18.4 Hz, 1H), 4.33 (d, *J =* 18.4 Hz, 1H), 3.19 - 3.12 (m, 1H), 2.44 (s, 3H), 1.29 - 1.25 (m, 2H), 1.25 - 1.22 (m, 2H). |
| 75 | 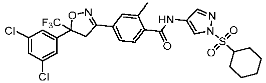 | ¹H NMR (600 MHz, DMSO-*d*₆) δ 10.72 (s, 1H), 8.30 (s, 1H), 7.89 (s, 1H), 7.65 (s, 1H), 7.53 (d, *J =* 8.3 Hz, 2H), 7.48 (d, *J =* 13.0 Hz, 3H), 4.26 (d, *J =* 18.4 Hz, 1H), 4.16 (d, *J =* 18.4 Hz, 1H), 3.58 - 3.52 (m, 1H), 2.29 (s, 3H), 1.72 - 1.66 (m, 2H), 1.63 - 1.58 (m, 2H), 1.46 - 1.40 (m, 1H), 1.28 - 1.20 (m, 2H), 1.15 - 1.07 (m, 2H), 1.00 - 0.91 (m, 1H). |
| 76 | 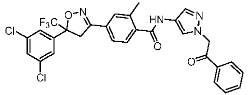 | ¹H NMR (600 MHz, DMSO-*d*₆) δ 10.49 (s, 1H), 8.33 (s, 1H), 8.17 (s, 1H), 7.95 - 7.87 (m, 4H), 7.74 (d, *J =* 8.9 Hz, 2H), 7.56 (t, *J* = 7.5 Hz, 2H), 7.47 (d, *J =* 7.2 Hz, 1H), 7.38 (d, *J =* 7.5 Hz, 2H), 5.81 (s, 2H), 4.40 (d, *J =* 18.4 Hz, 1H), 4.32 (d, *J =* 18.4 Hz, 1H), 2.40 (s, 3H). |
| 77 | 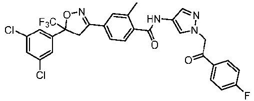 | ¹H NMR (600 MHz, DMSO-*d*₆) δ 10.51 (s, 1H), 8.16 - 8.11 (m, 2H), 8.10 (s, 1H), 7.83 (s, 1H), 7.67 (s, 1H), 7.66 - 7.63 (m, 3H), 7.60 - 7.57 (m, 2H), 7.43 (t, *J* = 8.7 Hz, 2H), 5.82 (s, 2H), 4.41 (d, *J =* 18.4 Hz, 1H), 4.33 (d, *J =* 18.4 Hz, 1H), 2.43 (s, 3H). |
| 78 | 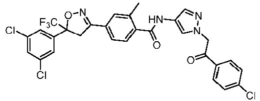 | ¹H NMR (600 MHz, DMSO-*d*₆) δ 10.51 (s, 1H), 8.10 (s, 1H), 8.05 (d, *J =* 8.2 Hz, 2H), 7.83 (s, 1H), 7.70 - 7.61 (m, 6H), 7.58 (t, *J =* 4.0 Hz, 2H), 5.82 (s, 2H), 4.41 (d, *J* = 18.4 Hz, 1H), 4.33 (d, *J* = 18.4 Hz, 1H), 2.43 (s, 3H). |
| 79 | 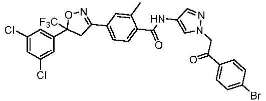 | ¹H NMR (600 MHz, DMSO-*d*₆) δ 10.51 (s, 1H), 8.18 (s, 1H), 8.01 (s, 1H), 7.67 (s, 2H), 7.65 (s, 2H), 7.59 - 7.56 (m, 3H), 7.55 (s, 1H), 7.23 (s, 1H), 7.22 (s, 1H), 5.82 (s, 2H), 4.41 (d, *J =* 18.4 Hz, 1H), 4.33 (d, *J =* 18.4 Hz, 1H), 2.42 (s, 3H). |
| 80 | 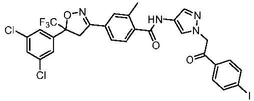 | ¹H NMR (600 MHz, DMSO-*d*₆) δ 10.54 (s, 1H), 8.24 (s, 1H), 8.13 - 8.10 (m, 1H), 7.91 (d, *J =* 3.8 Hz, 1H), 7.81 (t, *J =* 12.7 Hz, 5H), 7.71 (d, *J =* 7.3 Hz, 1H), 7.61 - 7.56 (m, 1H), 7.31 - 7.26 (m, 1H), 7.05 (d, *J =* 7.5 Hz, 1H), 5.85 (s, 2H), 4.42 (d, *J* = 18.4 Hz, 1H), 4.33 (d, *J =* 18.4 Hz, 1H), 2.43 (s, 3H). |
| 81 | 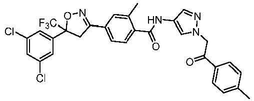 | ¹H NMR (600 MHz, DMSO-*d*₆) δ 10.48 (s, 1H), 8.30 (s, 1H), 8.11 (s, 1H), 7.67 (s, 2H), 7.65 (s, 2H), 7.56 (s, 1H), 7.55 (s, 1H), 7.25-7.11 (m, 4H), 5.76 (s, 2H), 4.41 (d, *J =* 18.4 Hz, 1H), 4.32 (d, *J =* 18.4 Hz, 1H), 2.41 (s, 3H), 2.28 (s, 3H). |
| 82 | 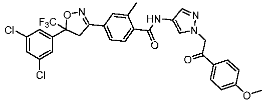 | ¹H NMR (600 MHz, DMSO-*d*₆) δ 10.46 (s, 1H), 8.18 (d, *J* = 2.3 Hz, 1H), 8.02 (s, 1H), 7.66 (s, 1H), 7.64 (s, 3H), 7.55 (d, *J =* 8.1 Hz, 1H), 7.52 (s, 1H), 7.25 (d, *J =* 8.0 Hz, 2H), 6.94 (s, 1H), 6.91 (s, 1H), 5.73 (s, 2H), 4.40 (d, *J* = 18.4 Hz, 1H), 4.32 (d, *J =* 18.4 Hz, 1H), 3.74 (s, 3H), 2.40 (s, 3H). |
| 83 | 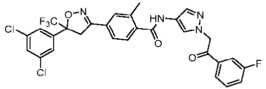 | ¹H NMR (600 MHz, DMSO-*d*₆) δ 10.51 (s, 1H), 8.31 (s, 1H), 8.15 (s, 1H), 8.11 (s, 1H), 7.85 (s, 2H), 7.71 (d, *J =* 6.2 Hz, 2H), 7.42 (d, *J =* 7.5 Hz, 1H), 7.37 (s, 1H), 7.30 (t, *J =* 11.7 Hz, 3H), 5.86 (s, 2H), 4.42 (d, *J =* 18.4 Hz, 1H), 4.34 (d, J= 18.4 Hz, 1H), 2.42 (s, 3H). |
| 84 | 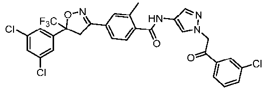 | ¹H NMR (400 MHz, DMSO-*d*₆) δ 10.50 (s, 1H), 8.32 (s, 1H), 8.17 (t, *J* = 1.9 Hz, 1H), 7.93 - 7.83 (m, 4H), 7.70 - 7.63 (m, 2H), 7.53 - 7.44 (m, 2H), 7.39 *(d, J* = 2.3 Hz, 1H), 7.34 - 7.25 (m, 1H), 5.84 (s, 2H), 4.41 (d, *J =* 18.4 Hz, 1H), 4.32 (d, *J =* 18.4 Hz, 1H), 2.41 (s, 3H). |
| 85 | 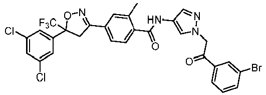 | ¹H NMR (600 MHz, DMSO-*d*₆) δ 10.52 (s, 1H), 8.34 - 8.28 (m, 1H), 8.21 (d, *J =* 9.9 Hz, 1H), 7.97 (s, 1H), 7.81 (s, 3H), 7.61 - 7.76 (m, 2H), 7.64 (d, *J =* 7.9 Hz, 1H), 7.58 (s, 1H), 7.51 (t, *J =* 7.6 Hz, 1H), 7.45 (d, *J =* 7.7 Hz, 1H), 5.84 (s, 2H), 4.41 (d, *J =* 18.4 Hz, 1H), 4.33 (d, *J =* 18.4 Hz, 1H), 2.42 (s, 3H). |
| 86 | 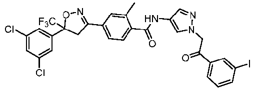 | ¹H NMR (600 MHz, DMSO-*d*₆) δ 10.52 (s, 1H), 8.25 (s, 1H), 8.17-8.14 (m, 1H), 7.97 - 7.85 (m, 4H), 7.74 - 7.67 (m, 2H), 7.62 - 7.47 (m, 2H), 7.41 (d, *J* = 2.3 Hz, 1H), 7.28 (s, 1H), 5.81 (s, 2H), 4.42 (d, *J =* 18.4 Hz, 1H), 4.32 (d, *J* = 18.4 Hz, 1H), 2.42 (s, 3H). |
| 87 | 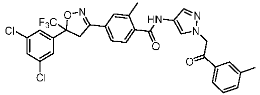 | ¹H NMR (600 MHz, DMSO-*d*₆) δ 10.52 (s, 1H), 8.26 (s, 1H), 8.13 (s, 1H), 7.95 (s, 1H), 7.84 (s, 3H), 7.70 (d, *J =* 4.3 Hz, 1H), 7.67 (s, 1H), 7.43 (d, *J =* 7.7 Hz, 1H), 7.35 (d, *J =* 8.1 Hz, 2H), 7.28 (d, *J =* 8.0 Hz, 1H), 5.79 (s, 2H), 4.43 (d, *J* = 18.3 Hz, 1H), 4.34 (d, *J* = 18.3 Hz, 1H), 3.43 (s, 3H), 2.29 (s, 3H). |
| 88 | 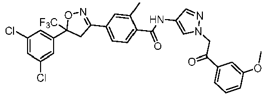 | ¹H NMR (600 MHz, DMSO-*d*₆) δ 10.47 (s, 1H), 8.33 (d, *J* = 2.6 Hz, 1H), 8.19 - 8.14 (m, 1H), 7.95 (d, *J =* 13.1 Hz, 4H), 7.83 - 7.75 (m, 2H), 7.64 (t, *J =* 7.9 Hz, 1H), 7.41 (s, 2H), 7.24 (d, *J =* 7.4 Hz, 1H), 5.76 (s, 2H), 4.38 (d, *J =* 18.4 Hz, 1H), 4.30 (d, *J =* 18.4 Hz, 1H), 3.71 (s, 3H), 2.40 (s, 3H). |
| 89 | 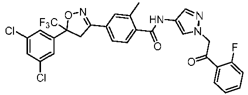 | ¹H NMR (600 MHz, DMSO-*d*₆) δ 10.47 (s, 1H), 8.26 (s, 1H), 8.17 (s, 1H), 8.02 (d, *J =* 12.7 Hz, 4H), 7.85 (d, *J =* 6.9 Hz, 2H), 7.68 - 7.62 (m, 1H), 7.59 - 7.46 (m, 3H), 5.88 (s, 2H), 4.41 (d, *J =* 18.4 Hz, 1H), 4.32 (d, *J =* 18.4 Hz, 1H), 2.44 (s, 3H). |
| 90 | 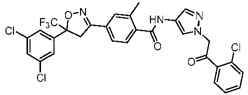 | ¹H NMR (400 MHz, DMSO-*d*₆) δ 10.52 (s, 1H), 8.35 (s, 1H), 8.26 (s, 1H), 8.18 - 8.01 (m, 4H), 7.91 - 7.84 (m, 2H), 7.74 (s, 1H), 7.56 - 7.48 (m, 2H), 7.36 - 7.29 (m, 1H), 5.93 (s, 2H), 4.40 (d, *J =* 18.4 Hz, 1H), 4.34 (d, *J =* 18.4 Hz, 1H), 2.45 (s, 3H). |
| 91 | 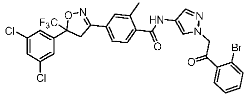 | ¹H NMR (600 MHz, DMSO-*d*₆) δ 10.49 (s, 1H), 8.31 (s, 1H), 8.25 (s, 1H), 8.11 - 8.03 (m, 6H), 7.94 (d, *J =* 7.9 Hz, 1H), 7.79 (t, *J =* 7.4 Hz, 1H), 7.68 (t, *J =* 7.7 Hz, 1H), 7.42 (d, *J =* 7.7 Hz, 1H), 5.91 (s, 2H), 4.42 (d, *J =* 18.4 Hz, 1H), 4.35 (d, *J =* 18.4 Hz, 1H), 2.41 (s, 3H). |
| 92 | 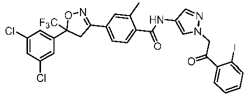 | ¹H NMR (600 MHz, DMSO-*d*₆) δ 10.54 (s, 1H), 8.35 (s, 1H), 8.23 - 8.18 (m, 1H), 8.04 (d, *J =* 3.8 Hz, 1H), 7.85 *(t, J =* 12.7 Hz, 5H), 7.79 *(d, J =* 7.3 Hz, 1H), 7.61 - 7.55 (m, 1H), 7.41 - 7.36 (m, 1H), 7.22 (d, *J =* 7.5 Hz, 1H), 5.85 (s, 2H), 4.45 (d, *J* = 18.3 Hz, 1H), 4.32 (d, *J =* 18.3 Hz, 1H), 2.41 (s, 3H). |
| 93 | 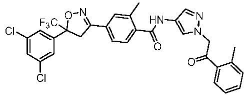 | ¹H NMR (600 MHz, DMSO-*d*₆) δ 10.45 (s, 1H), 8.28 (s, 1H), 8.13 - 8.08 (m, 1H), 7.89 - 7.81 (m, 4H), 7.62 (d, *J =* 8.1 Hz, 2H), 7.41 - 7.31 (m, 3H), 7.27 *(d, J=* 7.5 Hz, 1H), 5.82 (s, 2H), 4.39 (d, *J* = 18.4 Hz, 1H), 4.32 (d, *J* = 18.4 Hz, 1H), 2.42 (s, 3H), 2.33 (s, 3H). |
| 94 | 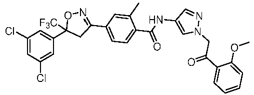 | ¹H NMR (600 MHz, DMSO-*d*₆) δ 10.45 (s, 1H), 8.23 (d, *J =* 2.8 Hz, 1H), 8.21 - 8.11 (m, 1H), 7.88 (d, *J =* 13.5 Hz, 4H), 7.78 - 7.70 (m, 2H), 7.63 (t, *J* =8.0 Hz, 1H), 7.50 (s, 2H), 7.33 (d, *J =* 7.6 Hz, 1H), 5.79 (s, 2H), 4.37 (d, *J =* 18.4 Hz, 1H), 4.30 (d, *J* = 18.4 Hz, 1H) , 3.72 (s, 3H), 2.42 (s, 3H). |
| 95 | 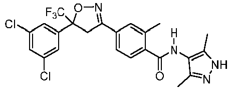 | ¹H NMR (600 MHz, DMSO-*d*₆) δ 12.16 (s, 1H), 9.43 (s, 1H), 7.83 (t, *J =* 1.9 Hz, 1H), 7.67 - 7.63 (m, 4H), 7.56 (d, *J =* 7.8 Hz, 1H), 4.40 (d, *J =* 18.4 Hz, 1H), 4.33 (d, *J =* 18.4 Hz, 1H), 2.45 (s, 3H), 2.09 (s, 6H). |
| 96 | 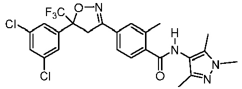 | ¹H NMR (600 MHz, DMSO-*d*₆) δ 9.47 (s, 1H), 7.82 (d, *J* = 13.0 Hz, 1H), 7.65 (d, *J =* 10.3 Hz, 4H), 7.57 (d, *J* = 7.4 Hz, 1H), 4.40 (d, *J =* 18.4 Hz, 1H), 4.32 (d, *J* = 18.4 Hz, 1H), 3.65 (s, 3H), 2.44 (s, 3H), 2.12 (s, 3H), 2.03 (s, 3H). |
| 97 | 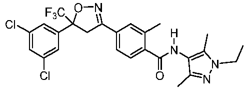 | ¹H NMR (600 MHz, DMSO-*d*₆) δ 9.46 (s, 1H), 7.83 (s, 1H), 7.66 (d, *J =* 11.1 Hz, 4H), 7.57 (d, *J =* 7.8 Hz, 1H), 4.41 (d, *J =* 18.4 Hz, 1H), 4.33 (d, *J =* 18.4 Hz, 1H), 3.98 (q, *J =* 7.2 Hz, 2H), 2.45 (s, 3H), 2.14 (s, 3H), 2.05 (s, 3H), 1.28 (t, *J* = 7.2 Hz, 3H). |
| 98 | 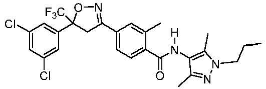 | ¹H NMR (600 MHz, DMSO-*d*₆) δ 9.47 (s, 1H), 7.83 (t, *J =* 1.9 Hz, 1H), 7.66 (d, *J =* 11.2 Hz, 4H), 7.58 (d, *J* = 7.8 Hz, 1H), 4.41 (d, *J =* 18.4 Hz, 1H), 4.33 (d, *J* = 18.4 Hz, 1H), 3.90 (t, *J =* 7.2 Hz, 2H), 2.45 (s, 3H), 2.14 (s, 3H), 2.06 (s, 3H), 1.78 - 1.64 (m, 2H), 0.86 (t, *J =* 7.4 Hz, 3H). |
| 99 | 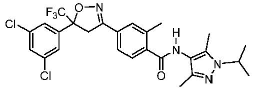 | ¹H NMR (600 MHz, DMSO-*d*₆) δ 9.44 (s, 1H), 7.83 (s, 1H), 7.66 (d, *J =* 11.0 Hz, 4H), 7.57 (d, *J =* 7.9 Hz, 1H), 4.46 - 4.42 (m, 1H), 4.41 (d, *J =* 18.4 Hz, 1H), 4.33 (d, *J* = 18.4 Hz, 1H), 2.45 (s, 3H), 2.15 (s, 3H), 2.06 (s, 3H), 1.35 (d, *J =* 6.6 Hz, 6H). |
| 100 | 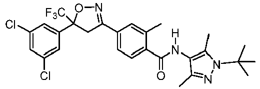 | ¹H NMR (600 MHz, DMSO-*d*₆) δ 9.41 (s, 1H), 7.83 (s, 1H), 7.65 (d, *J* = 11.1 Hz, 4H), 7.57 (d, *J* = 7.9 Hz, 1H), 4.40 (d, *J* = 18.4 Hz, 1H), 4.32 (d, *J=* 18.4 Hz, 1H), 2.45 (s, 3H), 2.28 (s, 3H), 2.04 (s, 3H), 1.55 (s, 9H). |
| 101 | 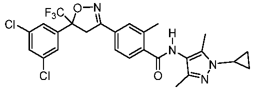 | ¹H NMR (400 MHz, Chloroform-d) δ 7.56 (d, *J =* 2.2 Hz, 3H), 7.52 (d, *J* = 1.8 Hz, 2H), 7.44 (t, *J* = 1.9 Hz, 1H), 7.00 (s, 1H), 4.10 (d, *J* = 17.4 Hz, 1H), 3.72 (d, *J =* 17.4 Hz, 1H), 3.33 - 3.25 (m, 1H), 2.54 (s, 3H), 2.31 (s, 3H), 2.20 (s, 3H), 1.20 - 1.14 (m, 2H), 1.09 - 1.02 (m, 2H). |
| 102 | 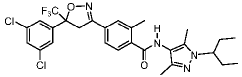 | ¹H NMR (600 MHz, DMSO-*d*₆) δ 9.44 (s, 1H), 7.83 (s, 1H), 7.69 - 7.52 (m, 5H), 4.41 (d, *J* = 18.4 Hz, 1H), 4.33 (d, *J* = 18.4 Hz, 1H), 3.93 - 3.80 (m, 1H), 2.45 (s, 3H), 2.14 (s, 3H), 2.08 (s, 3H), 1.85 - 1.75 (m, 2H), 1.75 - 1.63 (m, 2H), 0.68 (t, *J =* 7.2 Hz, 6H). |
| 103 | 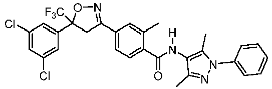 | ¹H NMR (400 MHz, DMSO-*d*₆) δ 9.68 (s, 1H), 7.83 (t, *J =* 1.9 Hz, 1H), 7.71 - 7.59 (m, 5H), 7.53 (d, *J* = 4.3 Hz, 4H), 7.45 - 7.37 (m, 1H), 4.42 (d, *J* = 18.4 Hz, 1H), 4.33 (d, *J* = 18.4 Hz, 1H), 2.48 (s, 3H), 2.25 (s, 3H), 2.17 (s, 3H). |
| 104 | 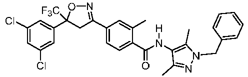 | ¹H NMR (400 MHz, Chloroform-d) δ 7.55 (s, 3H), 7.52 (d, *J =* 1.9 Hz, 2H), 7.44 (t, *J* = 1.9 Hz, 1H), 7.32 (d, *J =* 1.8 Hz, 1H), 7.31 - 7.26 (m, 2H), 7.16 - 7.11 (m, 2H), 6.95 (s, 1H), 5.23 (s, 2H), 4.10 (d, *J =* 17.4 Hz, 1H), 3.72 (d, *J* = 17.4 Hz, 1H), 2.54 (s, 3H), 2.23 (s, 3H), 2.14 (s, 3H). |
| 105 | 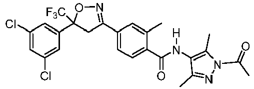 | ¹H NMR (600 MHz, DMSO-*d*₆) δ 9.85 (s, 1H), 7.83 (s, 1H), 7.68 (d, *J =* 11.0 Hz, 2H), 7.65 (s, 3H), 4.42 (d, *J =* 18.4 Hz, 1H), 4.34 (d, *J =* 18.4 Hz, 1H), 2.60 (s, 3H), 2.51 (s, 3H), 2.41 (s, 3H), 2.18 (s, 3H). |
| 106 | 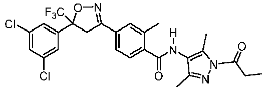 | ¹H NMR (400 MHz, DMSO-*d*₆) δ 9.83 (s, 1H), 7.85 (s, 1H), 7.73-7.65 (m, 5H) 4.43 (d, J= 18.4 Hz, 1H), 4.35 (d, *J* = 18.4 Hz, 1H), 3.15 (q, *J =* 7.6 Hz, 2H), 2.52 (s, 3H), 2.25 (s, 3H), 2.18 (s, 3H), 1.59 (t, *J =* 7.6 Hz, 3H). |
| 107 | 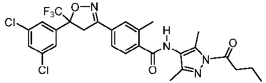 | ¹H NMR (400 MHz, DMSO-*d*₆) δ 9.84 (s, 1H), 7.85 (t, *J* = 1.9 Hz, 1H), 7.71 - 7.63 (m, 5H), 4.43 (d, *J =* 18.4 Hz, 1H), 4.34 (d, *J =* 18.4 Hz, 1H), 3.07 (t, *J* = 7.3 Hz, 2H), 2.47 (s, 3H), 2.43 (s, 3H), 2.19 (s, 3H), 1.75 - 1.63 (m, 2H), 0.98 (t, *J* = 7.4 Hz, 3H). |
| 108 | 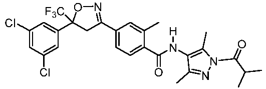 | ¹H NMR (400 MHz, DMSO-*d*₆) δ 9.84 (s, 1H), 7.82 (s, 1H), 7.78-7.72 (m, 5H) 4.44 (d, *J* = 18.4 Hz, 1H), 4.35 (d, *J* = 18.4 Hz, 1H), 3.48 - 3.42 (m, 1H), 2.48 (s, 3H), 2.40 (s, 3H), 2.17 (s, 3H), 1.37 (d, *J =* 6.7 Hz, 6H). |
| 109 | 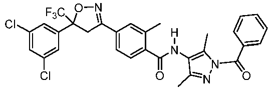 | ¹H NMR (400 MHz, DMSO-*d*₆) δ 10.90 (s, 1H), 8.52 (s, 1H), 8.04 (s, 1H), 8.01 (d, *J =* 1.2 Hz, 1H),7.86 (d, *J* = 1.6 Hz, 1H), 7.82 (t, *J =* 1.9 Hz, 1H), 7.77 - 7.68 (m, 2H), 7.63 - 7.60 (m, 2H), 7.49 (t, *J =* 7.6 Hz, 2H), 4.43 (d, *J =* 18.4 Hz, 1H), 4.34 (d, *J =* 18.4 Hz, 1H), 2.48 (s, 3H), 2.41 (s, 3H), 2.19 (s, 3H). |
| 110 | 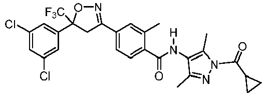 | ¹H NMR (600 MHz, DMSO-*d*₆) δ 9.86 (s, 1H), 7.83 (d, *J =* 2.7 Hz, 1H), 7.69 (s, 1H), 7.67 (s, 1H), 7.65 (s, 3H), 4.42 (d, *J =* 18.4 Hz, 1H), 4.33 (d, *J =* 18.4 Hz, 1H), 3.16 - 3.10 (m, 1H), 2.46 (s, 3H), 2.40 (s, 3H), 2.21 (s, 3H), 1.13 - 1.09 (m, 2H), 1.08 - 1.04 (m, 2H). |
| 111 | 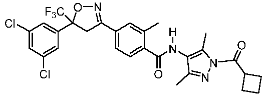 | ¹H NMR (600 MHz, DMSO-*d*₆) δ 9.81 (s, 1H), 7.82 (s, 1H), 7.67 (d, *J* = 9.9 Hz, 2H), 7.64 (d, *J =* 6.1 Hz, 3H), 4.41 (d, *J =* 18.4 Hz, 1H), 4.33 (d, *J =* 18.4 Hz, 1H), 4.26-4.19 (m, 1H), 2.45 (s, 3H), 2.42 (s, 3H), 2.32 - 2.27 (m, 2H), 2.25 - 2.21 (m, 2H), 2.16 (s, 3H), 2.06 - 1.98 (m, 2H). |
| 112 | 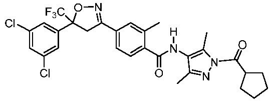 | ¹H NMR (600 MHz, DMSO-*d*₆) δ 9.81 (s, 1H), 7.81 (d, *J =* 2.5 Hz, 1H), 7.65 (d, *J =* 9.9 Hz, 2H), 7.62 (s, 3H), 4.38 (d, *J =* 18.4 Hz, 1H), 4.31 (d, *J =* 18.4 Hz, 1H), 3.98 - 3.91 (m, 1H), 2.43 (s, 3H), 2.39 (s, 3H), 2.15 (s, 3H), 1.79 - 1.71 (m, 2H), 1.71 - 1.64 (m, 2H), 1.63 - 1.56 (m, 2H), 0.86 - 0.78 (m, 2H). |
| 113 | 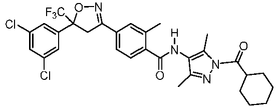 | ¹H NMR (600 MHz, DMSO-*d*₆) δ 9.84 (s, 1H), 7.83 (s, 1H), 7.68 (d, *J =* 9.8 Hz, 2H), 7.66 - 7.61 (m, 3H), 4.42 (d, *J =* 18.4 Hz, 1H), 4.33 (d, *J* = 18.4 Hz, 1H), 3.68 - 3.58 (m, 1H), 2.46 (s, 3H), 2.40 (s, 3H), 2.19 (s, 3H), 2.11 - 2.06 (m, 1H), 1.93 - 1.86 (m, 2H), 1.81 - 1.72 (m, 2H), 1.71 - 1.62 (m, 1H), 1.48 - 1.40 (m, 2H), 1.40 - 1.31 (m, 2H). |
| 114 | 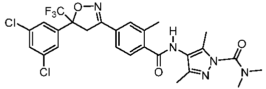 | ¹H NMR (600 MHz, DMSO-*d*₆) δ 9.68 (s, 1H), 7.84 - 7.80 (m, 1H), 7.68 (d,*J* = 10.6 Hz, 2H), 7.65 (s, 2H), 7.61 (d, *J* = 7.9 Hz, 1H), 4.41 (d, *J* = 18.4 Hz, 1H), 4.33 (d, *J =* 18.4 Hz, 1H), 3.03 (s, 6H), 2.46 (s, 3H), 2.25 (s, 3H), 2.13 (s, 3H). |
| 115 | 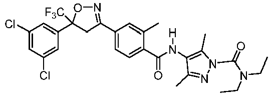 | ¹H NMR (400 MHz, DMSO-*d*₆) δ 9.68 (s, 1H), 7.83 (t, *J =* 1.9 Hz, 1H), 7.68 (s, 1H), 7.65 (d, *J =* 5.7, 3H), 7.61 (d, *J =* 7.9 Hz, 1H), 4.39 (d, *J =* 18.4 Hz, 1H), 4.33 (d, *J =* 18.4 Hz, 1H), 3.39 (q, *J =* 7.5 Hz, 4H), 2.46 (s, 3H), 2.23 (s, 3H), 2.13 (s, 3H), 1.16 (t, *J* = 7.5 Hz, 6H). |
| 116 | 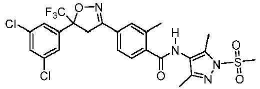 | ¹H NMR (400 MHz, Chloroform-d) δ 7.58 (s, 3H), 7.52 (d, *J =* 1.9 Hz, 2H), 7.44 (t, *J =* 1.9 Hz, 1H), 7.10 (s, 1H), 4.11 (d, *J =* 17.4 Hz, 1H), 3.73 (d, *J =* 17.4 Hz, 1H), 3.32 (s, 3H), 2.54 (s, 3H), 2.44 (s, 3H), 2.26 (s, 3H). |
| 117 | 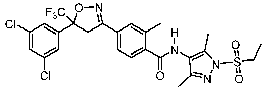 | ¹H NMR (600 MHz, DMSO-*d*₆) δ 9.86 (s, 1H), 7.83 (t, *J =* 2.3 Hz, 1H), 7.68 (d, *J* = 10.8 Hz, 2H), 7.66 - 7.63 (m, 3H), 4.42 (d, *J =* 18.4 Hz, 1H), 4.34 (d, *J* = 18.4 Hz, 1H), 3.65 (q, *J =* 7.4 Hz, 2H), 2.46 (s, 3H), 2.37 (s, 3H), 2.18 (s, 3H), 1.13 (t, *J =* 7.4 Hz, 3H). |
| 118 | 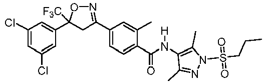 | ¹H NMR (600 MHz, DMSO-*d*₆) δ 9.84 (s, 1H), 7.83 (s, 1H), 7.67 (d, *J =* 11.0 Hz, 2H), 7.64 (s, 3H), 4.41 (d, *J =* 18.4 Hz, 1H), 4.33 (d, *J =* 18.4 Hz, 1H), 3.62 (t, *J =* 8.7 Hz, 2H), 2.46 (s, 3H), 2.36 (s, 3H), 2.17 (s, 3H), 1.62 - 1.53 (m, 2H), 0.93 (t, *J =* 6.5, 3H). |
| 119 | 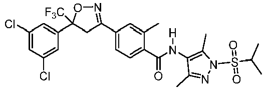 | ¹H NMR (600 MHz, DMSO-*d*₆) δ 9.85 (s, 1H), 7.84 (s, 1H), 7.68 (d, *J =* 11.9 Hz, 2H), 7.64 (s, 3H), 4.42 (d, *J =* 18.4 Hz, 1H), 4.33 (d, *J =* 18.4 Hz, 1H), 3.82 (m, 1H), 2.46 (s, 3H), 2.36 (s, 3H), 2.18 (s, 3H), 1.22 (d, *J =* 6.6 Hz, 6H). |
| 120 | 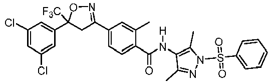 | ¹H NMR (600 MHz, DMSO-*d*₆) δ 9.80 (s, 1H), 7.98 (d, *J =* 7.4 Hz, 2H), 7.83 (s, 1H), 7.79 (d, *J* = 7.9 Hz, 1H), 7.69 (d, *J* = 7.6 Hz, 1H), 7.66 (s, 2H), 7.63 (s, 3H), 7.60 (d, *J =* 7.8 Hz, 1H), 4.40 (d, *J =* 18.4 Hz, 1H), 4.32 (d, *J =* 18.4 Hz, 1H), 2.42 (d, J= 2.8 Hz, 6H), 2.11 (s, 3H). |
| 121 | 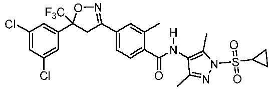 | ¹H NMR (600 MHz, DMSO-*d*₆) δ 9.81 (s, 1H), 7.80 (s, 1H), 7.65 *(d, J =* 10.6 Hz, 2H), 7.62 (d, *J =* 2.3 Hz, 3H), 4.39 (d, *J =* 18.4 Hz, 1H), 4.30 (d, *J =* 18.4 Hz, 1H), 3.17 - 3.12 (m, 1H), 2.44 (s, 3H), 2.34 (s, 3H), 2.15 (s, 3H), 1.22 - 1.20 (m, 2H), 1.16 - 1.13 (m, 2H). |
| 122 | 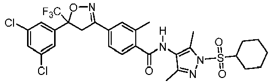 | ¹H NMR (600 MHz, DMSO-*d*₆) δ 9.83 (s, 1H), 7.83 (d, *J =* 2.0 Hz, 1H), 7.68 (d, *J =* 11.4 Hz, 2H), 7.65 (d, *J =* 4.6 Hz, 3H), 4.41 (d, *J =* 18.4 Hz, 1H), 4.33 (d, *J* = 18.4 Hz, 1H), 3.67 - 3.61 (m, 1H), 2.46 (s, 3H), 2.36 (s, 3H), 2.18 (s, 3H), 2.18-2.11 (m, 1H), 2.14-2.08 (m, 1H), 1.82 - 1.76 (m, 4H), 1.76 - 1.72 (m, 4H). |
| 123 | 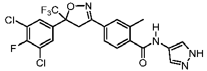 | ¹H NMR (600 MHz, DMSO-*d*₆) δ 12.69 (s, 1H), 10.44 (s, 1H), 7.82 (d, *J* = 5.9 Hz, 3H), 7.64 (d, *J =* 10.2 Hz, 3H), 7.56 (d, *J =* 7.9 Hz, 1H), 4.40 (d, *J =* 18.4 Hz, 1H), 4.34 (d, *J =* 18.4 Hz, 1H), 2.42 (s, 3H). |
| 124 | 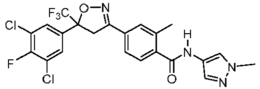 | ¹H NMR (600 MHz, DMSO-*d*₆) δ 10.44 (s, 1H), 8.01 (s, 1H), 7.82 (d, *J=* 5.9 Hz, 2H), 7.64 (d, J= 10.3 Hz, 2H), 7.55 (d, *J =* 7.9 Hz, 1H), 7.49 (s, 1H), 4.40 (d, *J* = 18.4 Hz, 1H), 4.34 (d, *J =* 18.4 Hz, 1H), 3.82 (s, 3H), 2.41 (s, 3H). |
| 125 | 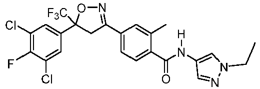 | ¹H NMR (600 MHz, DMSO-*d*₆) δ 10.44 (s, 1H), 8.04 (s, 1H), 7.82 (d, *J =* 6.1 Hz, 2H), 7.64 (d, J= 10.2 Hz, 2H), 7.55 (d, *J =* 7.9 Hz, 1H), 7.50 (s, 1H), 4.39 (d, *J =* 18.4 Hz, 1H), 4.34 (d, *J =* 18.4 Hz, 1H), 4.11 (q, *J =* 7.3 Hz, 2H), 2.41 (s, 3H), 1.36 (t, *J =* 7.3 Hz, 3H). |
| 126 | 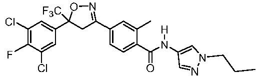 | ¹H NMR (600 MHz, DMSO-*d*₆) δ 10.44 (s, 1H), 8.03 (s, 1H), 7.82 (d, *J =* 6.0 Hz, 2H), 7.64 (d, *J =* 10.7 Hz, 2H), 7.56 (d, *J =* 7.9 Hz, 1H), 7.51 (s, 1H), 4.40 (d, *J =* 18.4 Hz, 1H), 4.34 (d, *J =* 18.4 Hz, 1H), 4.04 (t, *J =* 7.0 Hz, 2H), 2.42 (s, 3H), 1.83 - 1.72 (m, 2H), 0.83 (t, *J =* 7.3 Hz, 3H). |
| 127 | 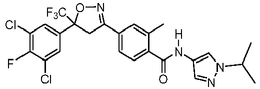 | ¹H NMR (600 MHz, DMSO-*d*₆) δ 10.44 (s, 1H), 8.04 (s, 1H), 7.83 (d, *J =* 6.0 Hz, 2H), 7.65 (d, *J =* 10.0 Hz, 2H), 7.56 (d, *J =* 7.8 Hz, 1H), 7.52 (s, 1H), 4.54 - 4.45 (m, 1H), 4.40 (d, *J =* 18.4 Hz, 1H), 4.34 (d, *J =* 18.4 Hz, 1H), 2.42 (s, 3H), 1.41 (d, *J =* 6.6 Hz, 6H). |
| 128 | 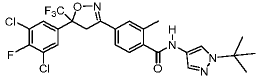 | ¹H NMR (600 MHz, DMSO-*d*₆) δ 10.42 (s, 1H), 8.06 (s, 1H), 7.82 (d, *J =* 6.0 Hz, 2H), 7.64 (d, *J =* 10.2 Hz, 2H), 7.54 (d, *J =* 9.0 Hz, 2H), 4.39 (d, *J =* 18.4 Hz, 1H), 4.33 (d, *J =* 18.4 Hz, 1H), 2.41 (s, 3H), 1.52 (s, 9H). |
| 129 | 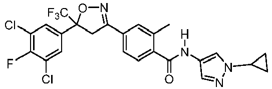 | ¹H NMR (600 MHz, DMSO-*d*₆) δ 10.42 (s, 1H), 8.00 (s, 1H), 7.79 (d, *J =* 6.1 Hz, 2H), 7.61 (d, *J* = 10.3 Hz, 2H), 7.52 (d, *J =* 8.0 Hz, 1H), 7.45 (s, 1H), 4.37 (d, *J =* 18.4 Hz, 1H), 4.31 (d, *J =* 18.4 Hz, 1H), 3.73 - 3.64 (m, 1H), 2.38 (s, 3H), 1.01 - 0.97 (m, 2H), 0.94 - 0.89 (m, 2H). |
| 130 | 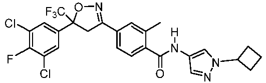 | ¹H NMR (600 MHz, DMSO-*d*₆) δ 10.46 (s, 1H), 8.08 (s, 1H), 7.82 (d, *J =* 6.0 Hz, 2H), 7.64 (d, *J* = 9.6 Hz, 2H), 7.55 (d, *J* = 9.0 Hz, 2H), 4.86 - 4.78 (m, 1H), 4.40 (d, *J =* 18.4 Hz, 1H), 4.34 (d, *J =* 18.4 Hz, 1H), 2.48 - 2.42 (m, 2H), 2.41 (s, 3H), 2.39 - 2.31 (m, 2H), 1.82 - 1.71 (m, 2H). |
| 131 | 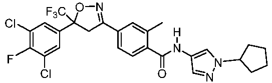 | ¹H NMR (600 MHz, DMSO-*d*₆) δ 10.43 (s, 1H), 8.03 (s, 1H), 7.82 (d, *J =* 6.1 Hz, 2H), 7.64 (d, *J =* 10.0 Hz, 2H), 7.55 (d, *J =* 7.8 Hz, 1H), 7.51 (s, 1H), 4.72 - 4.65 (m, 1H), 4.39 (d, *J =* 18.4 Hz, 1H), 4.34 (d, *J =* 18.4 Hz, 1H), 2.41 (s, 3H), 2.10 -2.01 (m, 2H), 1.95 - 1.84 (m, 2H), 1.84 - 1.72 (m, 2H), 1.69 - 1.58 (m, 2H). |
| 132 | 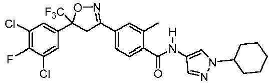 | ¹H NMR (600 MHz, DMSO-*d*₆) δ 10.44 (s, 1H), 8.01 (s, 1H), 7.82 (d, *J =* 6.0 Hz, 2H), 7.64 (d, *J =* 10.4 Hz, 2H), 7.55 (d, *J =* 7.9 Hz, 1H), 7.51 (s, 1H), 4.39 (d, *J =* 18.4 Hz, 1H), 4.34 (d, *J* = 18.4 Hz, 1H), 4.15 - 4.08 (m, 1H), 2.41 (s, 3H), 2.00 - 1.95 (m, 2H), 1.84 - 1.76 (m, 2H), 1.75 - 1.61 (m, 2H), 1.46 - 1.33 (m, 2H), 1.29 - 1.15 (m, 2H). |
| 133 | 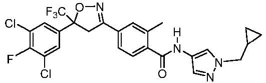 | ¹H NMR (600 MHz, DMSO-*d*₆) δ 10.45 (s, 1H), 8.09 (s, 1H), 7.83 (d, *J =* 6.0 Hz, 2H), 7.65 (d, *J =* 10.3 Hz, 2H), 7.56 (d, *J =* 7.9 Hz, 1H), 7.51 (s, 1H), 4.40 (d, *J =* 18.4 Hz, 1H), 4.34 (d, *J =* 18.4 Hz, 1H), 3.95 (d, *J =* 7.1 Hz, 2H), 2.42 (s, 3H), 1.28 - 1.20 (m, 1H), 0.57 - 0.49 (m, 2H), 0.40 - 0.33 (m, 2H). |
| 134 | 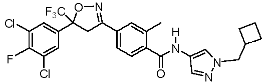 | ¹H NMR (600 MHz, DMSO-*d*₆) δ 10.42 (s, 1H), 7.98 (s, 1H), 7.80 (d, *J =* 6.0 Hz, 2H), 7.64 - 7.58 (m, 2H), 7.53 (d, *J =* 7.9 Hz, 1H), 7.47 (s, 1H), 4.37 (d, *J* = 18.4 Hz, 1H), 4.31 (d, *J* = 18.4 Hz, 1H), 4.08 (d, *J* = 7.2 Hz, 2H), 2.74 - 2.66 (m, 1H), 2.39 (s, 3H), 1.96 - 1.90 (m, 2H), 1.85 - 1.77 (m, 2H), 1.77 - 1.69 (m, 2H). |
| 135 | 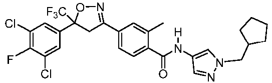 | ¹H NMR (600 MHz, DMSO-*d*₆) δ 10.44 (s, 1H), 8.03 (s, 1H), 7.82 (d, *J =* 6.0 Hz, 2H), 7.64 (d, *J =* 10.9 Hz, 2H), 7.56 (d, *J =* 7.9 Hz, 1H), 7.50 (s, 1H), 4.40 (d, *J =* 18.4 Hz, 1H), 4.34 (d, *J =* 18.4 Hz, 1H), 4.00 (d, *J =* 7.4 Hz, 2H), 2.41 (s, 3H), 2.38 - 2.31 (m, 1H), 1.65 - 1.55 (m, 4H), 1.54 - 1.44 (m, 2H), 1.28 - 1.20 (m, 2H). |
| 136 | 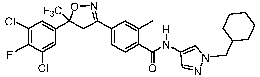 | ¹H NMR (600 MHz, DMSO-*d*₆) δ 10.44 (s, 1H), 7.99 (s, 1H), 7.82 (d, *J =* 6.0 Hz, 2H), 7.64 (d, *J =* 10.7 Hz, 2H), 7.56 (d, *J =* 7.9 Hz, 1H), 7.51 (s, 1H), 4.40 (d, *J* = 18.4 Hz, 1H), 4.34 (d, *J =* 18.4 Hz, 1H), 4.07 - 3.98 (m, 1H), 3.92 (d, *J* = 7.1 Hz, 2H), 2.42 (s, 3H), 1.83 - 1.57 (m, 4H), 1.56 - 1.46 (m, 2H), 1.20 - 1.17 (m, 2H), 1.00 - 0.88 (m, 2H). |
| 137 | 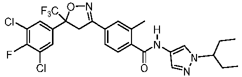 | ¹H NMR (600 MHz, DMSO-*d*₆) δ 10.43 (s, 1H), 8.01 (s, 1H), 7.82 (d, *J =* 6.1 Hz, 2H), 7.67 - 7.61 (m, 2H), 7.58 (d, *J =* 7.9 Hz, 1H), 7.54 (s, 1H), 4.39 (d, *J* = 18.4 Hz, 1H), 4.34 (d, *J* = 18.4 Hz, 1H), 4.00 - 3.92 (m, 1H), 2.42 (s, 3H), 1.84 - 1.69 (m, 4H), 0.70 (t, *J =* 7.4 Hz, 6H). |
| 138 | 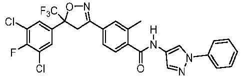 | ¹H NMR (600 MHz, DMSO-*d*₆) δ 10.66 (s, 1H), 8.65 (s, 1H), 7.84 - 7.77 (m, 5H), 7.65 (d, *J* = 9.1 Hz, 2H), 7.60 (d, *J* = 7.8 Hz, 1H), 7.48 (t, *J* = 7.9 Hz, 2H), 7.28 (t, *J =* 7.5 Hz, 1H), 4.38 (d, *J* = 18.4 Hz, 1H), 4.33 (d, *J =* 18.4 Hz, 1H), 2.43 (s, 3H). |
| 139 | 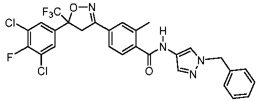 | ¹H NMR (600 MHz, DMSO-*d*₆) δ 10.48 (s, 1H), 8.14 (s, 1H), 7.82 (d, *J =* 6.0 Hz, 2H), 7.65 (d, *J =* 10.1 Hz, 2H), 7.57 (d, *J* = 7.3 Hz, 2H), 7.36 (t, *J* = 7.4 Hz, 2H), 7.31 (d, *J =* 7.3 Hz, 1H), 7.27 (d, *J* = 7.5 Hz, 2H), 5.32 (s, 2H), 4.40 (d, *J =* 18.4 Hz, 1H), 4.34 (d, *J* = 18.4 Hz, 1H), 2.42 (s, 3H). |
| 140 | 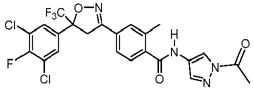 | ¹H NMR (400 MHz, DMSO-*d*₆) δ 10.42 (s, 1H), 8.55 (s, 1H), 7.85 (s, 1H), *7.82* (d, *J* = 6.1 Hz, 2H), 7.70 (s, 1H), 7.66 (d, *J* = 8.3 Hz, 1H), 7.61 (d, *J* = 8.1 Hz, 1H), 4.43 (d, *J =* 18.4 Hz, 1H), 4.35 (d, *J =* 18.4 Hz, 1H), 2.25 (s, 3H), 2.06 (s, 3H). |
| 141 | 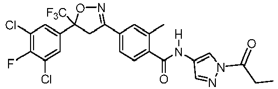 | ¹H NMR (600 MHz, DMSO-*d*₆) δ 10.34 (s, 1H), 8.56 (s, 1H), 7.87 (s, 1H), *7.82* (d, *J =* 6.0 Hz, 2H), 7.70 (s, 1H), 7.66 (d, *J* = 8.0 Hz, 1H), 7.61 (d, *J =* 8.1 Hz, 1H), 4.42 (d, *J =* 18.4 Hz, 1H), 4.35 (d, *J =* 18.4 Hz, 1H), 2.33 (q, *J =* 7.6 Hz, 2H), 2.25 (s, 3H), 1.09 (t, *J =* 7.6 Hz, 3H). |
| 142 | 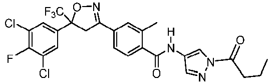 | ¹H NMR (600 MHz, DMSO-*d*₆) δ 10.37 (s, 1H), 8.57 (s, 1H), 7.85 (s, 1H), 7.81 (d, *J* = 6.2 Hz, 2H), 7.68 (s, 1H), 7.65 (d, *J =* 8.2 Hz, 1H), 7.63 (d, *J* = 8.0 Hz, 1H), 4.41 (d, *J* = 18.4 Hz, 1H), 4.35 (d, *J =* 18.4 Hz, 1H), 3.07 (t, *J =* 7.3 Hz, 2H), 2.43 (s, 3H), 1.76 - 1.64 (m, 2H), 0.96 (t, *J =* 7.4 Hz, 3H). |
| 143 | 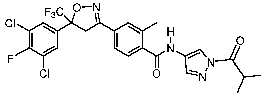 | ¹H NMR (600 MHz, DMSO-*d*₆) δ 10.35 (s, 1H), 8.55 (s, 1H), 7.87 (s, 1H), 7.83 (d, *J* = 6.1 Hz, 2H), 7.66 (s, 1H), 7.63 (d, *J =* 7.9 Hz, 1H), 7.61 (d, *J* = 8.1 Hz, 1H), 4.43 (d, *J =* 18.4 Hz, 1H), 4.36 (d, *J =* 18.4 Hz, 1H), 3.56-3.34 (m, 1H), 2.47 (s, 3H), 1.32 (d, *J* = 6.7 Hz, 6H). |
| 144 | 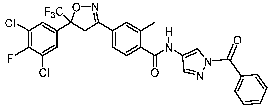 | ¹H NMR (600 MHz, DMSO-*d*₆) δ 10.31 (s, 1H), 8.61 (s, 1H), 8.04 (d, *J = 7.7* Hz, 2H), 7.94 (s, 1H), 7.83 - 7.79 (m, 3H), 7.74 - 7.65 (m, 4H), 7.60 (d, *J =* 8.1 Hz, 1H), 4.41 (d, *J* = 18.4 Hz, 1H), 4.33 (d, *J* = 18.4 Hz, 1H), 2.42 (s, 3H). |
| 145 | 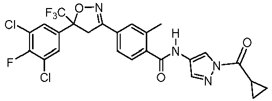 | ¹H NMR (600 MHz, DMSO-*d*₆) δ 10.33 (s, 1H), 8.73 (s, 1H), 7.86 (s, 1H), 7.81 (d, *J =* 6.3 Hz, 2H), 7.64 (s, 1H), 7.61 (d, *J =* 8.1 Hz, 1H), 7.58 (d, *J =* 8.2 Hz, 1H), 4.41 (d, *J =* 18.4 Hz, 1H), 4.35 (d, *J =* 18.4 Hz, 1H), 3.13 - 3.04 (m, 1H), 2.43 (s, 3H), 0.99 - 0.93 (m, 2H), 0.91 - 0.85 (m, 2H). |
| 146 | 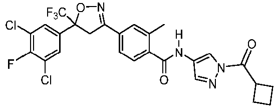 | ¹H NMR (400 MHz, DMSO-*d*₆) δ 10.41 (s, 1H), 9.82 (s, 1H), 7.86 (s, 1H), 7.83 (s, 1H), 7.63 (s, 2H), 7.61 (d, *J =* 1.9 Hz, 1H), 7.56 (s, 1H), 4.42 (d, *J =* 18.4 Hz, 1H), 4.35 (d, *J* = 18.4 Hz, 1H), 2.43 (s, 3H), 2.03 - 1.95 (m, 1H), 1.92 - 1.86 (m, 2H), 1.82 - 1.77 (m, 2H), 1.72 - 1.65 (m, 2H). |
| 147 | 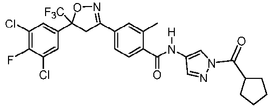 | ¹H NMR (400 MHz, DMSO-*d*₆) δ 10.45 (s, 1H), 9.84 (s, 1H), 7.83 (s, 1H), 7.82 (s, 1H), 7.66 (s, 2H), 7.63 (d, *J =* 1.7 Hz, 1H), 7.58 (s, 1H), 4.41 (d, *J =* 18.4 Hz, 1H), 4.34 (d, *J =* 18.4 Hz, 1H), 2.71-2.66 (m, 1H), 2.42 (s, 3H), 1.86 - 1.75 (m, 2H), 1.72 - 1.60 (m, 4H), 1.59 - 1.48 (m, 2H). |
| 148 | 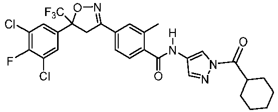 | ¹H NMR (400 MHz, DMSO-*d*₆) δ 10.43 (s, 1H), 9.83 (s, 1H), 7.84 (s, 1H), 7.81 (s, 1H), 7.68 (s, 2H), 7.65 (d, *J =* 1.7 Hz, 1H), 7.59 (s, 1H), 4.39 (d, *J =* 18.4 Hz, 1H), 4.34 (d, *J* = 18.4 Hz, 1H), 2.75 - 2.69 (m, 1H), 2.46 (s, 3H), 1.52 - 1.45 (m, 2H), 1.42 - 1.36 (m, 2H), 1.32 - 1.26 (m, 4H), 1.12 - 1.05 (m, 2H). |
| 149 | 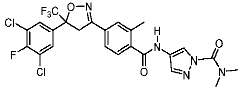 | ¹H NMR (600 MHz, DMSO-*d*₆) δ 10.69 (s, 1H), 8.41 (s, 1H), 7.94 - 7.77 (m, 3H), 7.73 - 7.53 (m, 3H), 4.41 (d, *J =* 18.4 Hz, 1H), 4.35 (d, *J* = 18.4 Hz, 1H), 3.14 (s, 6H), 2.44 (s, 3H). |
| 150 | 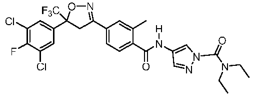 | ¹H NMR (600 MHz, DMSO-*d*₆) δ 10.72 (s, 1H), 8.43 (d, *J* = 4.1 Hz, 1H), 7.87 - 7.79 (m, 3H), 7.67 (d, *J =* 7.6 Hz, 2H), 7.61 (d, *J =* 7.4 Hz, 1H), 4.41 (d, *J =* 18.4 Hz, 1H), 4.35 (d, *J =* 18.4 Hz, 1H), 3.52 (q, *J =* 6.3 Hz, 4H), 2.43 (s, 3H), 1.20 (t, *J =* 6.3 Hz, 6H). |
| 151 | 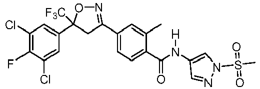 | ¹H NMR (600 MHz, DMSO-*d*₆) δ 10.86 (s, 1H), 8.47 (s, 1H), 8.02 (s, 1H), 7.82 (d, *J* = 5.9 Hz, 2H), 7.67 (d, *J* = 8.6 Hz, 2H), 7.62 (d, *J* = 7.9 Hz, 1H), 4.40 (d, *J* = 18.4 Hz, 1H), 4.34 (d, J= 18.4 Hz, 1H), 3.55 (s, 3H), 2.44 (s, 3H). |
| 152 | 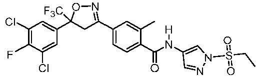 | ¹H NMR (600 MHz, DMSO-*d*₆) δ 10.85 (s, 1H), 8.47 (s, 1H), 8.04 (s, 1H), 7.82 (d, *J* = 6.0 Hz, 2H), 7.67 (d, *J* = 8.6 Hz, 2H), 7.63 (d, *J* = 7.9 Hz, 1H), 4.41 (d, *J=* 18.4 Hz, 1H), 4.34 (d, *J =* 18.4 Hz, 1H), 3.72 (q, *J* = 7.3 Hz, 2H), 2.44 (s, 3H), 1.13 (t, *J* = 7.3 Hz, 3H). |
| 153 | 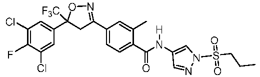 | ¹H NMR (600 MHz, DMSO-*d*₆) δ 10.86 (s, 1H), 8.47 (s, 1H), 8.03 (s, 1H), 7.82 (d, *J =* 4.4 Hz, 2H), 7.68 (d, *J* = 8.4 Hz, 2H), 7.63 (d, *J* = 6.6 Hz, 1H), 4.40 (d, *J =* 18.4 Hz, 1H), 4.35 (d, *J =* 18.4 Hz, 1H), 3.68 (t, *J =* 8.8 Hz, 2H), 2.44 (s, 3H), 1.61 - 1.53 (m, 2H), 0.92 (t, J = 7.8 Hz, 3H). |
| 154 | 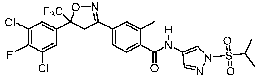 | ¹H NMR (600 MHz, DMSO-*d*₆) δ 10.87 (s, 1H), 8.48 (s, 1H), 8.06 (s, 1H), 7.83 (d, *J =* 5.9 Hz, 2H), 7.68 (d, *J =* 8.4 Hz, 2H), 7.64 (d, *J =* 8.0 Hz, 1H), 4.41 (d, *J=* 18.4 Hz, 1H), 4.35 (d, *J =* 18.4 Hz, 1H), 3.95 - 3.87 (m, 1H), 2.45 (s, 3H), 1.25 (d, *J =* 6.9 Hz, 6H). |
| 155 | 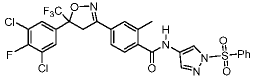 | ¹H NMR (600 MHz, DMSO-*d*₆) δ 10.84 (s, 1H), 8.60 (s, 1H), 8.01 *(d, J= 7.8* Hz, 2H), 7.97 (s, 1H), 7.84 - 7.78 (m, 3H), 7.72 - 7.63 (m, 4H), 7.60 (d, *J =* 8.2 Hz, 1H), 4.40 (d, *J* = 18.4 Hz, 1H), 4.33 (d, *J* = 18.4 Hz, 1H), 2.42 (s, 3H). |
| 156 | 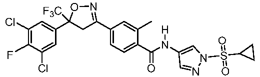 | ¹H NMR (600 MHz, DMSO-*d*₆) δ 10.87 (s, 1H), 8.47 (s, 1H), 8.04 (s, 1H), 7.83 (d, *J* = 6.0 Hz, 2H), 7.68 (d, *J* = 8.6 Hz, 2H), 7.64 (d, *J* = 8.0 Hz, 1H), 4.41 (d, *J=* 18.4 Hz, 1H), 4.35 (d, *J =* 18.4 Hz, 1H), 3.20 - 3.13 (m, 1H), 2.45 (s, 3H), 1.30 - 1.26 (m, 2H), 1.22 - 1.20 (m, 2H). |
| 157 | 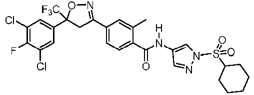 | ¹H NMR (600 MHz, DMSO-*d*₆) δ 10.87 (s, 1H), 8.45 (s, 1H), 8.04 (s, 1H), 7.86 - 7.76 (m, 2H), 7.74 - 7.58 (m, 3H), 4.41 (d, *J =* 18.4 Hz, 1H), 4.35 (d, *J* = 18.4 Hz, 1H), 3.76 - 3.64 (m, 1H), 2.44 (s, 3H), 1.87 - 1.80 (m, 2H), 1.80 - 1.72 (m, 2H), 1.43 - 1.33 (m, 2H), 1.32 - 1.22 (m, 2H), 1.21 - 1.13 (m, 2H). |
| 158 | 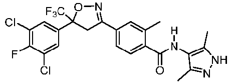 | ¹H NMR (600 MHz, DMSO-*d*₆) δ 12.16 (s, 1H), 9.43 (s, 1H), 7.83 (d, *J =* 6.0 Hz, 2H), 7.64 (d, *J =* 9.2 Hz, 2H), 7.57 (d, *J =* 7.8 Hz, 1H), 4.40 (d, *J =* 18.4 Hz, 1H), 4.34 (d, *J =* 18.4 Hz, 1H), 2.45 (s, 3H), 2.09 (s, 6H). |
| 159 | 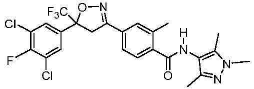 | ¹H NMR (600 MHz, DMSO-*d*₆) δ 9.48 (s, 1H), 7.83 (d, *J =* 6.0 Hz, 2H), 7.64 (d, *J* = 9.3 Hz, 2H), 7.57 (d, *J* = 7.9 Hz, 1H), 4.40 (d, *J* = 18.4 Hz, 1H), 4.34 (d, *J =* 18.4 Hz, 1H), 3.66 (s, 3H), 2.45 (s, 3H), 2.13 (s, 3H), 2.04 (s, 3H). |
| 160 | 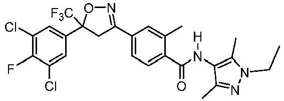 | ¹H NMR (600 MHz, DMSO-*d*₆) δ 9.46 (s, 1H), 7.83 (d, *J =* 6.1 Hz, 2H), 7.65 (d, *J* = 9.3 Hz, 2H), 7.58 (d, *J* = 7.8 Hz, 1H), 4.40 (d, *J* = 18.4 Hz, 1H), 4.34 (d, *J =* 18.4 Hz, 1H), 3.98 (q, *J =* 7.2 Hz, 2H), 2.45 (s, 3H), 2.14 (s, 3H), 2.05 (s, 3H), 1.28 (t, *J =* 7.2 Hz, 3H). |
| 161 | 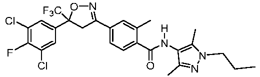 | ¹H NMR (600 MHz, DMSO-*d*₆) δ 9.45 (s, 1H), 7.82 (d, *J* = 6.0 Hz, 2H), 7.64 (d, *J* = 9.6 Hz, 2H), 7.57 (d, *J =* 7.8 Hz, 1H), 4.39 (d, *J* = 18.4 Hz, 1H), 4.34 (d, *J* = 18.4 Hz, 1H), 3.90 (t, *J =* 6.8 Hz, 2H), 2.45 (s, 3H), 2.14 (s, 3H), 2.05 (s, 3H), 1.74 - 1.68 (m, 2H), 0.85 (t, *J =* 7.4 Hz, 3H). |
| 162 | 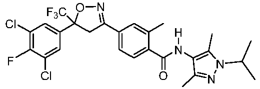 | ¹H NMR (600 MHz, DMSO-*d*₆) δ 9.44 (s, 1H), 7.83 (d, *J =* 6.0 Hz, 2H), 7.65 (d, *J =* 9.3 Hz, 2H), 7.57 (d, *J =* 7.8 Hz, 1H), 4.46 - 4.42 (m, 1H), 4.40 (d, *J =* 18.4 Hz, 1H), 4.34 (d, *J =* 18.4 Hz, 1H), 2.45 (s, 3H), 2.15 (s, 3H), 2.06 (s, 3H), 1.35 (d, *J =* 6.6 Hz, 6H). |
| 163 | 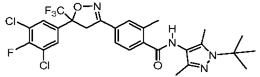 | ¹H NMR (600 MHz, Deuterium Oxide) δ 9.42 (s, 1H), 7.83 (d, *J* = 6.0 Hz, 2H), 7.65 (d, *J =* 9.1 Hz, 2H), 7.57 (d, *J =* 7.8 Hz, 1H), 4.39 (d, *J =* 18.4 Hz, 1H), 4.34 (d, *J =* 18.4 Hz, 1H), 2.45 (s, 3H), 2.28 (s, 3H), 2.04 (s, 3H), 1.55 (s, 9H). |
| 164 | 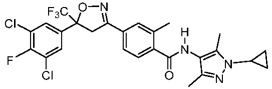 | ¹H NMR (400 MHz, DMSO-*d*₆) δ 9.48 (s, 1H), 7.86 - 7.80 (m, 2H), 7.64 (d, *J* = 7.5 Hz, 2H), 7.57 (d, *J =* 7.9 Hz, 1H), 4.40 (d, *J =* 18.4 Hz, 1H), 4.33 (d, *J =* 18.4 Hz, 1H), 3.46 - 3.37 (m, 1H), 2.44 (s, 3H), 2.21 (s, 3H), 2.03 (s, 3H), 1.02 - 0.97 (m, 2H), 0.97 - 0.93 (m, 2H). |
| 165 | 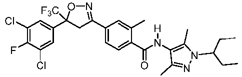 | ¹H NMR (600 MHz, DMSO-*d*₆) δ 9.45 (s, 1H), 7.83 (d, *J =* 6.1 Hz, 2H), 7.64 (d, *J =* 10.5 Hz, 2H), 7.58 (d, *J =* 7.9 Hz, 1H), 4.40 (d, *J =* 18.4 Hz, 1H), 4.34 (d, *J =* 18.4 Hz, 1H), 3.92 - 3.82 (m, 1H), 2.46 (s, 3H), 2.13 (s, 3H), 2.08 (s, 3H), 1.85 - 1.77 (m, 2H), 1.74 - 1.67 (m, 2H), 0.68 (t, *J =* 7.3 Hz, 6H). |
| 166 | 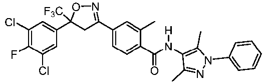 | ¹H NMR (600 MHz, DMSO-*d*₆) δ 9.70 (s, 1H), 7.83 (d, *J =* 6.1 Hz, 2H), 7.68 (d, *J =* 8.6 Hz, 2H), 7.63 (d, *J =* 7.8 Hz, 1H), 7.56 - 7.50 (m, 4H), 7.43 - 7.38 (m, 1H), 4.41 (d, *J* = 18.4 Hz, 1H), 4.35 (d, *J* = 18.4 Hz, 1H), 2.49 (s, 3H), 2.26 (s, 3H), 2.18 (s, 3H). |
| 167 | 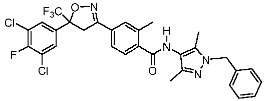 | ¹H NMR (400 MHz, Chloroform-d) δ 7.59 (d, *J =* 6.0 Hz, 2H), 7.56 (s, 3H), 7.36 - 7.27 (m, 3H), 7.17 - 7.11 (m, 2H), 6.90 (s, 1H), 5.25 (s, 2H), 4.10 (d, *J* = 17.4 Hz, 1H), 3.71 (d, *J* = 17.4 Hz, 1H), 2.55 (s, 3H), 2.24 (s, 3H), 2.15 (s, 3H). |
| 168 | 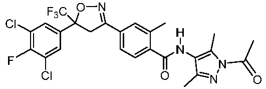 | ¹H NMR (600 MHz, DMSO-*d*₆) δ 9.85 (s, 1H), 7.83 (d, *J =* 6.0 Hz, 2H), 7.65 (s, 3H), 4.41 (d, *J* = 18.4 Hz, 1H), 4.35 (d, *J* = 18.4 Hz, 1H), 2.60 (s, 3H), 2.46 (s, 3H), 2.41 (s, 3H), 2.18 (s, 3H). |
| 169 | 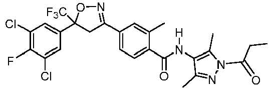 | ¹H NMR (400 MHz, DMSO-*d*₆) δ 9.84 (s, 1H), 7.85 (d, *J =* 6.2 Hz, 2H), 7.73 - 7.65 (m, 3H), 4.41 (d, *J =* 18.4 Hz, 1H), 4.35 (d, *J =* 18.4 Hz, 1H), 2.96 (q, *J* = 7.3 Hz, 2H), 2.46 (s, 3H), 2.42 (s, 3H), 2.16 (s, 3H), 1.32 (t, *J =* 7.4 Hz, 3H). |
| 170 | 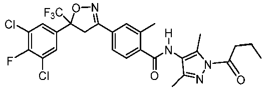 | ¹H NMR (400 MHz, DMSO-*d*₆) δ 9.85 (s, 1H), 7.84 (d, *J =* 6.2 Hz, 2H), 7.71 - 7.63 (m, 3H), 4.42 (d, *J =* 18.4 Hz, 1H), 4.35 (d, *J =* 18.4 Hz, 1H), 3.07 (t, *J* = 7.3 Hz, 2H), 2.47 (s, 3H), 2.43 (s, 3H), 2.19 (s, 3H), 1.76 - 1.61 (m, 2H), 0.98 (t, *J* = 7.4 Hz, 3H). |
| 171 | 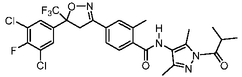 | ¹H NMR (400 MHz, DMSO-*d*₆) δ 9.83 (s, 1H), 7.86 (d, *J =* 6.2 Hz, 2H), 7.69 - 7.61 (m, 3H), 4.41 (d, *J =* 18.4 Hz, 1H), 4.33 (d, *J =* 18.4 Hz, 1H), 3.28 - 3.16 (m, 1H), 2.49 (s, 3H), 2.44 (s, 3H), 2.21 (s, 3H), 1.27 (d, *J =* 6.6 Hz, 6H). |
| 172 | 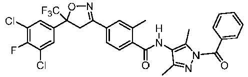 | ¹H NMR (600 MHz, DMSO-*d*₆) δ 9.83 (s, 1H), 7.96 (d, *J =* 7.6 Hz, 2H), 7.80 (d, *J =* 5.4 Hz, 2H), 7.79 (s, 1H), 7.71 (s, 2H), 7.66 (d, *J =* 10.8 Hz, 2H), 7.60 (d, *J =* 7.8 Hz, 1H), 4.41 (d, *J =* 18.4 Hz, 1H), 4.35 (d, *J =* 18.4 Hz, 1H), 2.47 (s, 3H), 2.43 (s, 3H), 2.19 (s, 3H). |
| 173 | 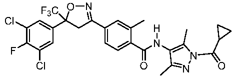 | ¹H NMR (600 MHz, DMSO-*d*₆) δ 9.86 (s, 1H), 7.83 (d, *J =* 6.3 Hz, 2H), 7.68 (s, 1H), 7.67 - 7.63 (m, 2H), 4.41 (d, *J =* 18.4 Hz, 1H), 4.35 (d, *J =* 18.4 Hz, 1H), 3.15 - 3.10 (m, 1H), 2.46 (s, 3H), 2.40 (s, 3H), 2.21 (s, 3H), 1.13 - 1.08 (m, 2H), 1.08 - 1.04 (m, 2H). |
| 174 | 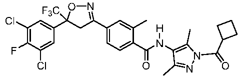 | ¹H NMR (600 MHz, DMSO-*d*₆) δ 9.82 (s, 1H), 7.84 - 7.80 (m, 2H), 7.66 (d, *J* = 8.9 Hz, 2H), 7.63 (d, *J =* 7.8 Hz, 1H), 4.40 (d, *J =* 18.4 Hz, 1H), 4.34 (d, *J =* 18.4 Hz, 1H), 4.25 - 4.20 (m, 1H), 2.45 (s, 3H), 2.42 (s, 3H), 2.32 - 2.27 (m, 2H), 2.27 - 2.19 (m, 2H), 2.15 (s, 3H), 2.06 - 2.00 (m, 1H), 1.87 - 1.79 (m, 1H). |
| 175 | 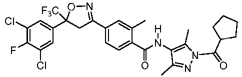 | ¹H NMR (600 MHz, DMSO-*d*₆) δ 9.83 (s, 1H), 7.82 (d, *J =* 5.7 Hz, 2H), 7.67 (s, 1H), 7.66 (s, 1H), 7.64 (s, 1H), 4.40 (d, *J =* 18.4 Hz, 1H), 4.35 (d, *J* = 18.4 Hz, 1H), 4.00 - 3.94 (m, 1H), 2.46 (s, 3H), 2.41 (s, 3H), 2.18 (s, 3H), 1.81 - 1.73 (m, 2H), 1.73 - 1.67 (m, 2H), 1.66 - 1.60 (m, 2H), 0.89 - 0.80 (m, 2H). |
| 176 | 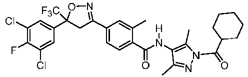 | ¹H NMR (600 MHz, DMSO-*d*₆) δ 9.84 (s, 1H), 7.83 (d, *J =* 6.0 Hz, 2H), 7.67 *(d, J=* 9.2 Hz, 2H), 7.63 (d, *J* = 7.8 Hz, 1H), 4.40 (d, *J* = 18.4 Hz, 1H), 4.35 (d, *J* = 18.4 Hz, 1H), 3.68 - 3.56 (m, 1H), 2.46 (s, 3H), 2.40 (s, 3H), 2.18 (s, 3H), 1.94 - 1.88 (m, 2H), 1.80 - 1.74 (m, 2H), 1.72 - 1.65 (m, 2H), 1.45 - 1.40 (m, 2H), 1.39 - 1.33 (m, 2H). |
| 177 | 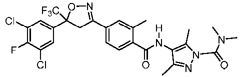 | ¹H NMR (600 MHz, DMSO-*d*₆) δ 9.68 (s, 1H), 7.83 (d, *J =* 6.1 Hz, 2H), 7.67 (d, *J =* 9.4 Hz, 2H), 7.62 (d, *J =* 7.8 Hz, 1H), 4.40 (d, *J =* 18.4 Hz, 1H), 4.34 (d, *J =* 18.4 Hz, 1H), 3.03 (s, 6H), 2.47 (s, 3H), 2.25 (s, 3H), 2.13 (s, 3H). |
| 178 | 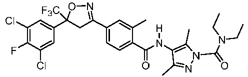 | ¹H NMR (400 MHz, DMSO-*d*₆) δ 9.68 (s, 1H), 7.83 (s, 1H), 7.82 (s, 1H), 7.67 (s, 1H), 7.65 (d, *J* = 1.6 Hz, 1H), 7.63 - 7.60 (m, 1H), 4.38 (d, *J* = 18.4 Hz, 1H), 4.34 (d, *J =* 18.4 Hz, 1H), 3.38 (q, *J =* 7.0 Hz, 4H), 2.46 (s, 3H), 2.23 (s, 3H), 2.13 (s, 3H), 1.16 (t, *J =* 7.0 Hz, 6H). |
| 179 | 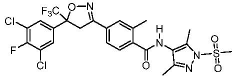 | ¹H NMR (600 MHz, DMSO-*d*₆) δ 9.85 (s, 1H), 7.83 (d, *J =* 6.1 Hz, 2H), 7.68 (s, 1H), 7.65 (t, *J =* 9.0 Hz, 2H), 4.41 (d, *J =* 18.4 Hz, 1H), 4.35 (d, *J =* 18.4 Hz, 1H), 3.51 (s, 3H), 2.47 (s, 3H), 2.38 (s, 3H), 2.18 (s, 3H). |
| 180 | 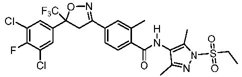 | ¹H NMR (600 MHz, DMSO-*d*₆) δ 9.86 (s, 1H), 7.83 (d, *J =* 6.0 Hz, 2H), 7.68 (s, 1H), 7.66 (d, *J =* 5.2 Hz, 2H), 4.41 (d, *J =* 18.4 Hz, 1H), 4.35 (d, *J =* 18.4 Hz, 1H), 3.65 (q, *J =* 7.3 Hz, 2H), 2.46 (s, 3H), 2.37 (s, 3H), 2.18 (s, 3H), 1.13 (t, *J =* 7.3 Hz, 3H). |
| 181 | 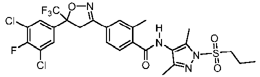 | ¹H NMR (600 MHz, DMSO-*d*₆) δ 9.84 (s, 1H), 7.85 - 7.80 (m, 2H), 7.66 (d, *J* = 13.4 Hz, 3H), 4.40 (d, *J =* 18.4 Hz, 1H), 4.34 (d, *J =* 18.4 Hz, 1H), 3.62 (t, *J* = 8.6 Hz, 2H), 2.46 (s, 3H), 2.37 (s, 3H), 2.17 (s, 3H), 1.62-1.56 (m, 2H), 0.94 (t, *J* = 7.7 Hz, 3H). |
| 182 | 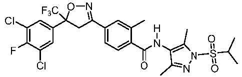 | ¹H NMR (600 MHz, DMSO-*d*₆) δ 9.83 (s, 1H), 7.80 (d, *J =* 5.9 Hz, 2H), 7.64 (d, *J* = 11.4 Hz, 3H), 4.38 (d, *J* = 18.4 Hz, 1H), 4.32 (d, *J* = 18.4 Hz, 1H), 3.85 - 3.75 (m, 1H), 2.44 (s, 3H), 2.34 (s, 3H), 2.15 (s, 3H), 1.20 (t, *J =* 5.2 Hz, 6H). |
| 183 | 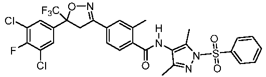 | ¹H NMR (600 MHz, DMSO-*d*₆) δ 9.79 (s, 1H), 7.98 (d, *J =* 7.7 Hz, 2H), 7.81 (d, *J =* 5.2 Hz, 2H), 7.79 (s, 1H), 7.69 (s, 2H), 7.64 (d, *J =* 11.2 Hz, 2H), 7.61 (d, *J =* 7.8 Hz, 1H), 4.39 (d, *J =* 18.4 Hz, 1H), 4.33 (d, *J =* 18.4 Hz, 1H), 2.42 (s, 6H), 2.10 (s, 3H). |
| 184 | 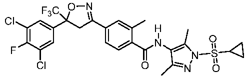 | ¹H NMR (600 MHz, DMSO-*d*₆) δ 9.83 (s, 1H), 7.82 (d, *J =* 6.1 Hz, 2H), 7.68 (s, 1H), 7.65 (t, *J =* 7.5 Hz, 2H), 4.40 (d, *J =* 18.4 Hz, 1H), 4.34 (d, *J =* 18.4 Hz, 1H), 3.19 - 3.14 (m, 1H), 2.46 (s, 3H), 2.37 (s, 3H), 2.27-2.20 (m, 1H), 2.18 (s, 3H), 2.12-2.07 (m, 1H), 1.19 - 1.15 (m, 2H). |
| 185 | 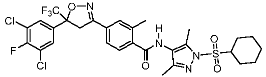 | ¹H NMR (400 MHz, DMSO-*d*₆) δ 9.86 (s, 1H), 7.84 (d, *J =* 6.2 Hz, 2H), 7.71 - 7.66 (m, 3H), 4.43 (d, *J =* 18.4 Hz, 1H), 4.36 (d, *J =* 18.4 Hz, 1H), 3.71 - 3.61 (m, 1H), 2.48 (s, 3H), 2.38 (s, 3H), 2.20 (s, 3H), 1.84 - 1.77 (m, 4H), 1.66 - 1.58 (m, 1H), 1.51 - 1.40 (m, 2H), 1.33 - 1.25 (m, 2H), 1.20 - 1.10 (m, 1H). |

### Preparation example 1

This preparation serves to illustrate the synthesis of compound 1:

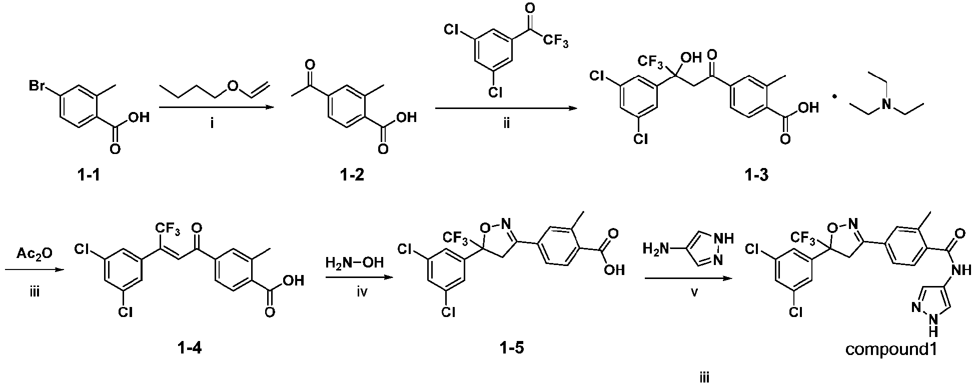

Preparation of intermediates 1-2 (step i): placing a compound (21.50g) shown in the formula 1-1, palladium acetate (0.23g) and potassium carbonate (27.64g) in a Schlenk tube in sequence, pumping and filling nitrogen for three times, then adding n-butyl vinyl ether (30.00g) and 100mL of n-butanol, carrying out reflux reaction at 120 °C for 6h, after the reaction is finished, evaporating the solvent to dryness under reduced pressure, adding a 1N HCl solution, acidifying to pH 2, carrying out suction filtration and drying to obtain an intermediate 1-2, wherein the yield is 92%.

Preparation of intermediates 1-3 (step ii): sequentially taking 1-2 (10.00g) of the intermediate, 1-(3,5-dichlorophenyl)-2,2,2-trifluoroethanone (16.38g), 11.37g of triethylamine and 100mL of toluene into a three-neck flask, reacting for 16h at 50 °C, cooling to room temperature after the reaction is finished, performing suction filtration, and drying the solid to obtain 1-3 of the intermediate, wherein the yield is 98%.

Preparation of intermediates 1-4 (step iii): sequentially taking the compound 1-3 (26.52g), 4-dimethylaminopyridine (6.20g) and toluene 100mL into a three-necked bottle, adding acetic anhydride (10.36 g), reacting at 60 °C for 12 hours, adding a 1N HCl solution after the reaction is finished, acidifying until the pH value is 2, carrying out suction filtration and drying to obtain the intermediate 1-4, wherein the yield is 95%.

Preparation of intermediates 1-5 (step iv): sequentially taking the intermediate 1-4 (16.70g), tetrabutylammonium bromide (4.01g) and 300mL of toluene into a three-neck bottle, adding 5.47g of 50% hydroxylamine aqueous solution, reacting at room temperature for 7 hours, adding 1N HCl solution after the reaction is finished, acidifying until the pH value is 2, carrying out suction filtration and drying to obtain the intermediate 1-5, wherein the yield is 95%.

Preparation of compound 1 (step v): putting the intermediates 1-5 (0.50g), CDI (0.39g), DIPEA (0.56g) and 10mL of dichloromethane into a three-necked bottle in sequence, adding 4-aminopyrazole (0.16g), reacting at normal temperature for 4 h, washing with saturated NaHCO₃ solution for 3 times after the reaction is finished, extracting an aqueous phase for 3 times with 20 mL of dichloromethane, merging organic phase after the extraction is finished, drying the organic phase with anhydrous Na₂SO₄, and purifying by column chromatography to obtain the compound 1 with the yield of 83%.

### Preparation example 2

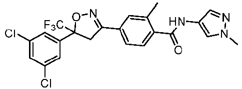

This preparation serves to illustrate the synthesis of compound 2: 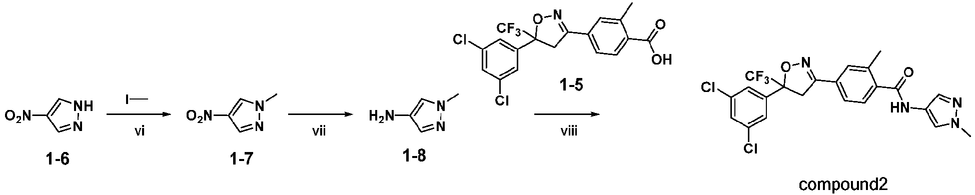 preparation of intermediates 1-7 (step vi): sequentially taking 20mL of intermediates 1-6 (1.00g), potassium carbonate (1.47g), methyl iodide (1.50g) and N, N-dimethylaminocarboxamide into a three-necked bottle, reacting at normal temperature for 3h, adding 100mL of ethyl acetate after the reaction is finished, washing the mixture with saturated saline solution for three times, drying an organic phase by using anhydrous Na₂SO₄, and purifying by column chromatography to obtain intermediates 1-7 with the yield of 93%.

Preparation of intermediates 1-8 (step vii): taking 1-7 intermediates (0.50g), ammonium chloride (0.95g), 5mL of water and 15mL of ethanol into a three-necked bottle in sequence, heating to boil, adding reduced iron powder (1.54g) in batches, refluxing for 30min, after the reaction is finished, adding 50 mL of ethyl acetate, washing with saturated saline solution for three times, drying an organic phase with anhydrous Na₂SO₄, and carrying out column chromatography purification to obtain 1-8 intermediates, wherein the yield is 78%.

Preparation of compound 2 (step viii): putting the intermediates 1-5 (0.50g), CDI (0.39g), DIPEA (0.56g) and dichloromethane (10 mL) in a three-necked bottle in sequence, adding the intermediates 1-8(0.18g), reacting at normal temperature for 4 h, after the reaction is finished, washing with saturated NaHCO₃ solution for 3 times, extracting an aqueous phase for 3 times with 20mL dichloromethane, combining organic phase after the extraction is finished, drying the organic phase with anhydrous Na₂SO₄, and purifying by column chromatography to obtain the compound 2, wherein the yield is 82%.

### Preparation of compound 3-compound 57, compounds 76-94:

Compound 3 to 57 and 76 to 94 were prepared according to a similar manner to that of preparation example 2.

### Preparation of compound 58:

Compound 58 is prepared similarly to preparation example 2, except that methyl iodide, potassium carbonate, and N, N-dimethylaminocarboxamide in step vi are replaced with acetyl chloride, triethylamine, and dichloromethane in equimolar amounts, and specifically, intermediates 1-7 are prepared by the steps comprising: sequentially taking the intermediate 1-6, triethylamine and dichloromethane into a three-necked bottle, dropwise adding acetyl chloride at 0 °C, reacting at normal temperature for 3h, after the reaction is finished, adding 100mL of ethyl acetate, washing with saturated saline solution for three times, drying an organic phase with anhydrous Na₂SO₄, and purifying by column chromatography to obtain the intermediate 1-7 with the yield of 68%.

### Preparation of compound 59-compound 75:

Compound 59-compound 75 were prepared according to a similar manner to that of compound 58.

### Preparation of compound 95:

Compound 95 is prepared analogously to preparation 1, except that the 4-aminopyrazole in step v is replaced with an equimolar amount of 3,5-dimethyl-1H-pyrazol-4-amine, specifically, in the preparation of compound 95: putting the intermediates 1-5 (0.50g), CDI (0.39g), DIPEA (0.56g) and 10mL of dichloromethane into a three-necked bottle in sequence, adding 3,5-dimethyl-1H-pyrazol-4-amine, reacting for 4H at normal temperature, washing 3 times with saturated NaHCO₃ solution after the reaction is finished, extracting an aqueous phase for 3 times with 20mL of dichloromethane, combining organic phase after the extraction is finished, drying the organic layer with anhydrous Na₂SO₄, and purifying by column chromatography to obtain the compound 95, wherein the yield is 89%.

### Preparation of compound 96:

Compound 96 was prepared similarly to preparation 2, except that intermediates 1-6 in step vi were replaced with equimolar 3,5-dimethyl-4-nitropyrazole, specifically the preparation of intermediates 1-7: 3, 5-dimethyl-4-nitropyrazole, potassium carbonate (1.47g), methyl iodide (1.50g) and N,N-dimethylaminocarboxamide 20mL are sequentially put into a three-necked bottle and reacted at normal temperature for 3h, after the reaction is finished, 100mL of ethyl acetate is added and washed with saturated saline three times, and then an organic phase is dried by anhydrous Na₂SO₄ and purified by column chromatography to obtain an intermediate 1-7 with the yield of 94%.

### Preparation of compound 97:

Compound 97 is prepared similarly to preparation 96, except that the iodomethane in step vi is replaced with an equimolar amount of iodoethane, and specifically, intermediates 1-7 are prepared by steps comprising:
sequentially taking 3,5-dimethyl-4-nitropyrazole, potassium carbonate, ethyl iodide and 20mL of N,N-dimethylaminocarboxamide into a three-necked bottle, reacting for 3 hours at normal temperature, adding 100mL of ethyl acetate after the reaction is finished, washing the mixture for three times by saturated saline solution, drying an organic phase by using anhydrous Na₂SO₄, and purifying by column chromatography to obtain an intermediate 1-7 with the yield of 96%.

### Preparation of compound 98-compound 104:

Compound 98 to compound 104 were prepared according to a similar method to that for compound 96.

### Preparation of compound 105:

Compound 105 was prepared similarly to compound 58, except that intermediates 1-6 in step vi were replaced with equimolar 3,5-dimethyl-4-nitropyrazole, specifically the preparation of intermediates 1-7: placing 3, 5-dimethyl-4-nitropyrazole, triethylamine and dichloromethane in sequence into a three-necked bottle, dropwise adding acetyl chloride (0.83g) at 0°C, reacting at normal temperature for 3h, adding 100mL of ethyl acetate after the reaction is finished, washing with saturated saline solution for three times, drying an organic phase with anhydrous Na₂SO₄, and purifying by column chromatography to obtain intermediates 1-7 with the yield of 63%.

### Preparation of compound 106-compound 122:

Compound 106-compound 122 were prepared according to a similar procedure as that for compound 105.

### Preparation of compound 123:

Compound 123 was prepared according to a similar manner to that of preparation 1, except that 1-(3,5-dichlorophenyl)-2,2,2-trifluoroethanone in step ii was replaced with an equimolar amount of 1-(3,5-dichloro-4-fluorophenyl)-2,2,2-trifluoroethanone, specifically, the preparation of intermediates 1-3 involved: sequentially taking 1-2 (10.00g) of the intermediate, 1-(3,5-dichloro-4-fluorophenyl)-2,2,2-trifluoroethanone, 11.37g of triethylamine and 100mL of toluene into a three-neck flask, reacting for 16h at 50 °C, cooling to room temperature after the reaction is finished, performing suction filtration, and drying the solid to obtain 1-3 of the intermediate, wherein the yield is 93%.

### Preparation of compound 124:

Compound 124 was prepared analogously to preparation 2, except that 1-(3,5-dichlorophenyl)-2,2,2-trifluoroethanone in step ii was replaced by an equimolar amount of 1-(3,5-dichloro-4-fluorophenyl)-2,2,2-trifluoroethanone, and in particular intermediates 1-3 were prepared by: sequentially taking the intermediates 1-2, 1-(3,5-dichloro-4-fluorophenyl)-2,2,2-trifluoroethanone, triethylamine and 100mL of toluene in a three-neck flask, reacting at 50 °C for 16 hours, cooling to room temperature after the reaction is finished, performing suction filtration, and drying the solid to obtain the intermediates 1-3 with the yield of 93%.

### Preparation of compound 125-compound 139:

Compound 125-compound 139 was prepared according to a similar procedure to that used to prepare compound 124.

### Preparation of compound 140:

Compound 140 is prepared similarly to compound 58, except that 1-(3,5-dichlorophenyl)-2,2,2-trifluoroethanone in step ii is replaced with an equimolar amount of 1-(3,5-dichloro-4-fluorophenyl)-2,2,2-trifluoroethanone, specifically, the preparation of intermediates 1-3 comprises: sequentially taking the intermediates 1-2, 1-(3,5-dichloro-4-fluorophenyl)-2,2,2-trifluoroethanone, triethylamine and 100mL of toluene in a three-neck flask, reacting at 50 °C for 16 hours, cooling to room temperature after the reaction is finished, performing suction filtration, and drying the solid to obtain the intermediates 1-3 with the yield of 93%.

### Preparation of compound 141-compound 157:

Compounds 141-157 were prepared according to a similar procedure to that for compound 140.

### Preparation of compound 158:

Compound 158 is prepared analogously to compound 123 by replacing 4-aminopyrazole in step v with 3,5-dimethyl-1H-pyrazol-4-amine, specifically by placing intermediate 1-5 (0.50g), CDI (0.39g), DIPEA (0.56g), and 10mL of methylene chloride in a three-necked flask, adding 3,5-dimethyl-1H-pyrazol-4-amine, reacting for 4h at room temperature, after completion of the reaction, washing 3 times with saturated NaHCO₃ solution, extracting the aqueous phase 3 times with 20mL of methylene chloride, combining the organic phase after completion of the extraction, drying the organic phase with anhydrous Na₂SO₄, and purifying the organic phase by column chromatography to give compound 158 in 88% yield.

### Preparation of compound 159:

Compound 159 was prepared similarly to compound 124, except that intermediates 1-6 in step vi were replaced with equimolar 3,5-dimethyl-4-nitropyrazole, specifically the preparation of intermediates 1-7: 3,5-dimethyl-4-nitropyrazole, potassium carbonate (1.47g), methyl iodide (1.50g) and N,N-dimethylaminocarboxamide 20mL are sequentially put into a three-necked bottle and reacted at normal temperature for 3h, after the reaction is finished, 100mL of ethyl acetate is added and washed with saturated saline three times, and then an organic phase is dried by anhydrous Na₂SO₄ and purified by column chromatography to obtain an intermediate 1-7 with the yield of 94%.

### Preparation of compound 160-compound 167:

Compound 160 to compound 167 were prepared according to a similar method to that for compound 159.

### Preparation of compound 168:

Compound 168 is prepared similarly to compound 140, except that intermediates 1-6 in step vi are replaced with equimolar 3,5-dimethyl-4-nitropyrazole, specifically intermediates 1-7 are prepared by: placing 3,5-dimethyl-4-nitropyrazole, triethylamine and dichloromethane in a three-necked bottle in sequence, dropwise adding acetyl chloride at 0 °C, reacting for 3 hours at normal temperature, adding 100mL of ethyl acetate after the reaction is finished, washing with saturated saline solution for three times, drying an organic phase with anhydrous Na₂SO₄, and purifying by column chromatography to obtain an intermediate 1-7 with the yield of 68%.

### Preparation of compound 169-compound 185:

Compound 169-compound 185 were prepared according to a similar procedure as that used to prepare compound 168.

### Test example 1:

Determining the toxicity activity of part of compounds on diamondback moth *(Plutella xylostella)* instar larvae by the leafdipping method.

The test agents of the compounds in Table 2 were weighed out accurately with an electronic analytical balance, and the original drug was dissolved in a 10000ppm solution with dimethyl sulfoxide as the solvent and then diluted with 0.1% of Triton solution to 1ppm and 0.25ppm solutions. Taking the cabbage not contacted with the liquid medicine, and cutting the leaves into 6 cm-diameter circular leaves. Soaking the leaves in the prepared liquid medicine for 15 seconds, taking out and drying, and placing the leaves into 6cm culture dishes, wherein 1 cabbage leaf is placed in each culture dish. The same standard second instar larvae were placed in petri dishes with 10 heads per dish and 4 replicates per concentration for a total of 40 heads. After inoculation, the culture dish is placed in a constant-temperature culture chamber with the temperature of 25 ± 1 °C and the illumination period of 16 (D) to 8 (L) h, and the number of dead and live larvae is investigated after 48h and 72 h. A blank and a control of compound K1 and compound K2 were also provided.

The mortality is calculated according to the following formula 1, then the corrected mortality is calculated according to the formula 2, and finally the classification is carried out according to the corrected mortality value and the rating standard shown in the table 2, and the specific results are shown in the tables 3 and 4.$mortality % = \left(number of dead insects/total number of insects\right) \times 100 % ;$$corrected mortality % = \left[\left(treatment-control mortality\right)/\left(1-control mortality\right)\right] \times 100 % .$

### Test example 2:

The toxicity activity of part of the compounds on the fourth instar larvae of the rice leaf rollers (*Cnaphalocrocis medinalis*) is determined by the leafdipping method.

The test agents of the compounds in Table 2 were weighed out accurately with an electronic analytical balance, and the original drug was dissolved completely in dimethylsulfoxide to 10000ppm solution and then diluted with 0.1% aqueous solution of Triton to 5ppm solution. And (4) selecting rice plants which are planted in a laboratory and are not contacted with the liquid medicine, and cutting leaves of the rice plants to about 8 cm. Soaking the leaves in the prepared liquid medicine for 15 seconds, taking out and drying, wrapping the roots with absorbent cotton, and putting the wrapped roots into 9cm culture dishes, wherein 3-5 rice leaves are placed in each culture dish. The water bamboo sections used as the feed for larvae are peeled off gently, and the four-instar larvae of the same standard are placed in culture dishes with 10 heads per dish and 4 repetitions per concentration for a total of 40 heads. After the inoculation, the culture dish is placed in a constant temperature culture room with the temperature of 26 ± 1 °C and the illumination period of 16 (D): 8 (L) h, and the number of dead and live larvae is investigated after 48 h. A blank and a control of compound K1 and compound K2 were also provided.

The mortality was calculated according to the following formula 1, then the corrected mortality was calculated according to the following formula 2, and finally the rating criteria shown in table 2 were combined for ranking according to the corrected mortality value, and the specific results are shown in table 5. $mortality % = \left(number of dead insects/total number of insects\right) \times 100 % ;$ $corrected mortality % = \left[\left(treatment-control mortality\right)/\left(1-control mortality\right)\right] \times 100 % .$

**TABLE 2**

| grade | mortality |
|---|---|
| A | 100% |
| B | 90% to less than 100% |
| C | 80% to less than 90% |
| D | 70% to less than 80% |
| E | 50% to less than 70% |
| F | less than 50% |

**TABLE 3**

| Compound number | mortality (1ppm) | | Compound number | mortality (1ppm) | |
|---|---|---|---|---|---|
| | 48h | 72h | | 48h | 72h |
| 1 | E | D | 2 | C | A |
| 3 | D | B | 4 | E | E |
| 5 | B | A | 8 | E | C |
| 7 | / | C | 10 | E | A |
| 9 | / | C | 12 | E | E |
| 11 | E | A | 16 | / | D |
| 23 | A | A | 24 | / | E |
| 27 | E | D | 28 | C | A |
| 33 | / | E | 30 | / | E |
| 39 | / | E | 36 | / | E |
| 47 | / | E | 40 | E | E |
| 51 | / | E | 42 | / | E |
| 59 | / | E | 44 | / | E |
| 67 | / | B | 52 | / | E |
| 69 | / | E | 58 | / | E |
| 71 | / | D | 60 | / | E |
| 73 | / | E | 64 | / | E |
| 75 | B | B | 68 | / | E |
| 97 | / | C | 72 | / | E |
| 123 | B | A | 98 | / | E |
| 125 | A | A | 102 | / | E |
| 127 | A | A | 104 | / | E |
| 129 | A | A | 124 | C | A |
| 131 | C | A | 130 | B | A |
| 133 | C | A | 132 | E | A |
| 137 | / | C | 134 | / | D |
| 139 | / | E | 140 | / | E |
| 149 | A | A | 150 | C | A |
| 151 | / | D | 154 | C | A |
| 153 | D | B | 158 | E | B |
| 155 | / | E | 160 | E | D |
| 157 | B | A | 162 | E | A |
| 159 | / | B | 168 | / | B |
| 161 | / | B | 170 | / | C |
| 169 | / | C | 174 | / | D |
| 171 | / | C | Compound K1 | F | F |
| 177 | / | C | | | |

| | | | | | |
|---|---|---|---|---|---|
| Note: "/" indicates that no test record was made. | | | | | |

**TABLE 4**

| Compound number | mortality(0.25ppm)(48h) |
|---|---|
| 127 | A |
| Compound K1 | F |
| Compound K2 | F |

**TABLE 5**

| Compound number | mortality(5ppm)(48h) |
|---|---|
| 127 | A |
| Compound K1 | F |
| Compound K2 | D |

The compound provided in the present invention can, at low concentrations, successfully kill insects, mites, nematodes and piercing-sucking insects.

In particular, the compound containing the pyrazole structure provided by the invention has excellent insecticidal activity on agricultural insects such as *Plutella xylostella, Ostrinia furnacalis, Helicoverpa armigera, Chilo suppressalis, Cnaphalocrocis medinalis, Spodoptera frugiperda, Spodoptera exigua, Spodoptera litura, Bemisia tabaci,* rice planthopper, aphid, thrips, flea beetle and the like.

Specifically, the lethality of the compound 127 containing a pyrazole structure to *Plutella xylostella* can reach 100% under the concentration of 0.25 ppm, and the effects of the control compounds K1 and K2 are both less than 50%. And the lethality of the compound 127 to *Cnaphalocrocis medinalis* at the concentration of 5ppm is 100 percent, which is far better than that of the control compounds K1 and K2.

## Claims

1. A compound having a pyrazole structure or an agrochemically acceptable salts, hydrates and solvates thereof, wherein the compound has the structure shown in formula (I): 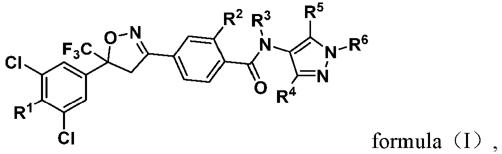
Wherein, in the formula (I),
R' is selected from H, halogen, at least one halogen-substituted C₁₋₆ alkyl, at least one halogen-substituted C₁₋₆ alkoxy;
R² is selected from C₁₋₁₂ alkyl, C₁₋₁₂ alkoxy, halogen, at least one halogen-substituted C₁₋₆ alkyl, at least one halogen-substituted C₁₋₆ alkoxy and cyano;
R³ is selected from H, C₁₋₁₂ alkyl, C₁₋₁₂ alkoxy, C₃₋₁₂ cycloalkyl, -CO-R₃₁, -CO-CH₂-R₃₁; R₃₁ is selected from C₁₋₁₂ alkyl, C₃₋₁₂ cycloalkyl and C₁₋₁₂ alkoxy;
R⁴, R⁵ are respectively and independently selected from H, C₁₋₁₂ alkyl, C₁₋₁₂ alkoxy, halogen, at least one halogen-substituted C₁₋₆ alkyl and at least one halogen-substituted C₁₋₆ alkoxy;
R⁶ is selected from the group consisting of H, C₁₋₁₂ alkyl unsubstituted or substituted by at least one group from hydroxyl, halogen, C₁₋₁₂ alkyl, C₁₋₁₂ alkoxy and trimethylsilyl, C₁₋₁₂ alkoxy unsubstituted or substituted by at least one group from hydroxyl, halogen, C₁₋₁₂ alkyl, C₁₋₁₂ alkoxy and trimethylsilyl, C₃₋₁₂ cycloalkyl unsubstituted or substituted by at least one group from hydroxyl, halogen, C₁₋₁₂ alkyl, C₁₋₁₂ alkoxy and trimethylsilyl, phenyl unsubstituted or substituted by at least one group from hydroxyl, halogen, C₁₋₁₂ alkyl, C₁₋₁₂ alkoxy and trimethylsilyl, C₃₋₁₂ cycloalkyl containing at least one hetero atom as a ring-forming atom, C₂₋₁₂ hydrocarbon containing at least one unsaturated carbon-carbon double bond or unsaturated carbon-carbon triple bond which is unsubstituted or substituted by at least one group from hydroxyl, halogen, C₁₋₁₂ alkyl, C₁₋₁₂ alkoxy and trimethylsilyl, -(CH₂)ₙ-R₆₁, -CO-R₆₂, -COO-R₆₂, -CO-N(R₆₃R₆₄), -S(=O)₂-R₆₂, - (CH₂)ₙ-CO-R₆₂, R₆₁ is selected from the group consisting of C₃₋₁₂ cycloalkyl, C₃₋₁₂ cycloalkyl containing at least one heteroatom as a ring-forming atom,phenyl, R₆₂ is selected from the group consisting of C₁₋₁₂ alkyl, phenyl unsubstituted or substituted by at least one of hydroxyl, halogen, C1-12 alkyl, C1-12 alkoxy and trimethylsilyl, C₃₋₁₂ cycloalkyl, C₃₋₁₂ cycloalkyl containing at least one heteroatom as a ring-forming atom; R₆₃, R₆₄ are independently selected from C₁₋₁₂ alkyl; each of the heteroatoms is independently at least one selected fromN, O, S; each n is independently 1 or 2.

2. The compound according to claim 1, wherein, in formula (I),
R¹ is selected from H, halogen, at least one halogen-substituted C₁₋₆ alkyl, at least one halogen-substituted C₁₋₆ alkoxy;
R² is selected from C₁₋₈ alkyl, C₁₋₈ alkoxy, halogen, at least one halogen-substituted C₁₋₆ alkyl, at least one halogen-substituted C₁₋₆ alkoxy and cyano;
R³ is selected from H, C₁₋₈ alkyl, C₁₋₈ alkoxy, C₃₋₁₀ cycloalkyl, -CO-R₃₁, -CO-CH₂-R₃₁; R₃₁ is selected from C₁₋₈ alkyl, C₃₋₁₀ cycloalkyl and C₁₋₈ alkoxy;
R⁴ and R⁵ are respectively and independently selected from H, C₁₋₈ alkyl, C₁₋₈ alkoxy, halogen, at least one halogen-substituted C₁₋₆ alkyl and at least one halogen-substituted C₁₋₆ alkoxy;
R⁶ is selected from the group consisting of H, C₁₋₁₀ alkyl unsubstituted or substituted by at least one group from hydroxyl, halogen, C₁₋₈ alkyl, C₁₋₈ alkoxy and trimethylsilyl, C₁₋₁₀ alkoxy unsubstituted or substituted by at least one group from hydroxyl, halogen, C₁₋₈ alkyl, C₁₋₈ alkoxy and trimethylsilyl, C₃₋₁₀ cycloalkyl unsubstituted or substituted by at least one group from hydroxyl, halogen, C₁₋₈ alkyl, C₁₋₈ alkoxy and trimethylsilyl, phenyl unsubstituted or substituted by at least one group from hydroxyl, halogen, C₁₋₈ alkyl, C₁₋₈ alkoxy and trimethylsilyl, C₃₋₁₀ cycloalkyl containing at least one heteroatom as a ring-forming atom, C₂₋₁₀ hydrocarbon containing at least one unsaturated carbon-carbon double bond or unsaturated carbon-carbon triple bond which is unsubstituted or substituted by at least one group from hydroxyl, halogen, C₁₋₈ alkyl, C₁₋₈ alkoxy and trimethylsilyl, -(CH₂)ₙ-R₆₁, -CO-R₆₂, -COO-R₆₂, -CO-N(R₆₃R₆₄), -S(=O)₂-R₆₂, -(CH₂)ₙ-CO-R₆₂; R₆₁ is selected from the group consisting of C₃₋₁₀ cycloalkyl, C₃₋₁₀ cycloalkyl containing at least one heteroatom as a ring-forming atom, phenyl; R₆₂ is selected from the group consisting of C₁₋₁₀ alkyl, phenyl unsubstituted or substituted by at least one of the groups in hydroxyl, halogen, C₁₋₈ alkyl, C₁₋₈ alkoxy and trimethylsilyl, C₃₋₁₀ cycloalkyl, C₃₋₁₀ cycloalkyl containing at least one heteroatom as a ring-forming atom; R₆₃ and R₆₄ are independently selected from C₁₋₁₀ alkyl of; each of the heteroatoms is independently at least one selected from N, O, S; each n is independently 1 or 2.

3. The compound according to claim 2, wherein, in formula (I),
R¹ is selected from H, F, chloro, bromo;
R² is selected from methyl, ethyl, n-propyl, isopropyl, n-butyl, tert-butyl, cyano;
R³ is selected from the group consisting of H, methyl, ethyl, n-propyl, isopropyl, n-butyl, tert-butyl, methoxy, ethoxy, n-propoxy, isopropoxy, n-butoxy, tert-butoxy, cyclopropyl, cyclobutyl, cyclopentyl, cyclohexyl, -CO-R₃₁, -CO-CH₂-R₃₁; R₃₁ is selected from C₁₋₆ alkyl, C₃₋₆ cycloalkyl and C₁₋₆ alkoxy;
R⁴, R⁵ are each independently selected from H, methyl, ethyl, n-propyl, isopropyl, n-butyl, tert-butyl;
R⁶ is selected from the group consisting of H, C₁₋₁₀ alkyl unsubstituted or substituted by at least one group from hydroxyl, halogen, C₁₋₈ alkyl, C₁₋₈ alkoxy and trimethylsilyl, C₁₋₁₀ alkoxy unsubstituted or substituted by at least one group from hydroxyl, halogen, C₁₋₈ alkyl, C₁₋₈ alkoxy and trimethylsilyl, C₃₋₁₀ cycloalkyl unsubstituted or substituted by at least one group from hydroxyl, halogen, C₁₋₈ alkyl, C₁₋₈ alkoxy and trimethylsilyl, phenyl unsubstituted or substituted by at least one group from hydroxyl, halogen, C₁₋₈ alkyl, C₁₋₈ alkoxy and trimethylsilyl, C₃₋₁₀ cycloalkyl containing at least one heteroatom as a ring-forming atom, C₂₋₁₀ hydrocarbon containing at least one unsaturated carbon-carbon double bond or unsaturated carbon-carbon triple bond which is unsubstituted or substituted by at least one group from hydroxyl, halogen, C₁₋₈ alkyl, C₁₋₈ alkoxy and trimethylsilyl, -(CH₂)ₙ-R₆₁, -CO-R₆₂, -COO-R₆₂, -CO-N(R₆₃R₆₄), -S(=O)₂-R₆₂, -(CH₂)ₙ-CO-R₆₂; R₆₁ is selected from the group consisting of C₃₋₁₀ cycloalkyl, C₃₋₁₀ cycloalkyl containing at least one heteroatom as a ring-forming atom, phenyl; R₆₂ is selected from the group consisting of C₁₋₁₀ alkyl, phenyl unsubstituted or substituted by at least one of the groups in hydroxyl, halogen, C₁₋₈ alkyl, C₁₋₈ alkoxy and trimethylsilyl, C₃₋₁₀ cycloalkyl, C₃₋₁₀ cycloalkyl containing at least one heteroatom as a ring-forming atom; R₆₃ and R₆₄ are independently selected from C₁₋₁₀ alkyl; each of the heteroatoms is independently at least one selected from N, O, S; each n is independently 1 or 2.

4. The compound according to claim 3, wherein the compound is selected from any one of the following:
Number A1: R¹ is H, R² is CH₃, R³ is H, R⁴ is H, R⁵ is H, R⁶ is -H;
Number A2: R¹ is H, R² is CH₃, R³ is H, R⁴ is H, R⁵ is H, R⁶ is -CH₃;
Number A3: R¹ is H, R² is CH₃, R³ is H, R⁴ is H, R⁵ is H, R⁶ is -CHF₂;
Number A4: R¹ is H, R² is CH₃, R³ is H, R⁴ is H, R⁵ is H, R⁶ is -CF₃;
Number A5: R¹ is H, R² is CH₃, R³ is H, R⁴ is H, R⁵ is H, R⁶ is -CH₂CH₃;
Number A6: R¹ is H, R² is CH₃, R³ is H, R⁴ is H, R⁵ is H, R⁶ is -CH₂CH₂CH₃;
Number A7: R¹ is H, R² is CH₃, R³ is H, R⁴ is H, R⁵ is H, R⁶ is -CH(CH₃)₂;
Number A8: R¹ is H, R² is CH₃, R³ is H, R⁴ is H, R⁵ is H, R⁶ is -CH₂CH₂OH;
Number A9: R¹ is H, R² is CH₃, R³ is H, R⁴ is H, R⁵ is H, R⁶ is -CH₂CF₃;
Number A10: R¹ is H, R² is CH₃, R³ is H, R⁴ is H, R⁵ is H, R⁶ is -CH₂CHF₂;
Number A11: R¹ is H, R² is CH₃, R³ is H, R⁴ is H, R⁵ is H, R⁶ is -CH₂CH₂F;
Number A12: R¹ is H, R² is CH₃, R³ is H, R⁴ is H, R⁵ is H, R⁶ is -CH₂CH₂CF₃;
Number A13: R¹ is H, R² is CH₃, R³ is H, R⁴ is H, R⁵ is H, R⁶ is -CH₂CH₂CH₂F;
Number A14: R¹ is H, R² is CH₃, R³ is H, R⁴ is H, R⁵ is H, R⁶ is -CH₂CH₂OCH₃;
Number A15: R¹ is H, R² is CH₃, R³ is H, R⁴ is H, R⁵ is H, R⁶ is -CH₂CH₂OCH₂CH₃;
Number A16: R¹ is H, R² is CH₃, R³ is H, R⁴ is H, R⁵ is H, R⁶ is -CH₂(CH₂)₂OCH₃;
Number A17: R¹ is H, R² is CH₃, R³ is H, R⁴ is H, R⁵ is H, R⁶ is -CH₂CH₂CH₂CH₃;
Number A18: R¹ is H, R² is CH₃, R³ is H, R⁴ is H, R⁵ is H, R⁶ is -CH(CH₃)CH₂CH₃;
Number A19: R¹ is H, R² is CH₃, R³ is H, R⁴ is H, R⁵ is H, R⁶ is -CH₂(CH₂)₃CH₃;
Number A20: R¹ is H, R² is CH₃, R³ is H, R⁴ is H, R⁵ is H, R⁶ is 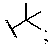
Number A21: R¹ is H, R² is CH₃, R³ is H, R⁴ is H, R⁵ is H, R⁶ is 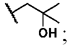
Number A22: R¹ is H, R² is CH₃, R³ is H, R⁴ is H, R⁵ is H, R⁶ is 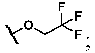
Number A23: R¹ is H, R² is CH₃, R³ is H, R⁴ is H, R⁵ is H, R⁶ is 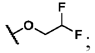
Number A24: R¹ is H, R² is CH₃, R³ is H, R⁴ is H, R⁵ is H, R⁶ is 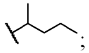
Number A25: R¹ is H, R² is CH₃, R³ is H, R⁴ is H, R⁵ is H, R⁶ is 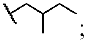
Number A26: R¹ is H, R² is CH₃, R³ is H, R⁴ is H, R⁵ is H, R⁶ is 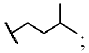
Number A27: R¹ is H, R² is CH₃, R³ is H, R⁴ is H, R⁵ is H, R⁶ is 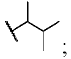
Number A28: R¹ is H, R² is CH₃, R³ is H, R⁴ is H, R⁵ is H, R⁶ is 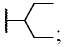
Number A29: R¹ is H, R² is CH₃, R³ is H, R⁴ is H, R⁵ is H, R⁶ is 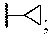
Number A30: R¹ is H, R² is CH₃, R³ is H, R⁴ is H, R⁵ is H, R⁶ is 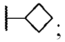
Number A31: R¹ is H, R² is CH₃, R³ is H, R⁴ is H, R⁵ is H, R⁶ is 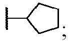
Number A32: R¹ is H, R² is CH₃, R³ is H, R⁴ is H, R⁵ is H, R⁶ is 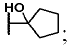
Number A33: R¹ is H, R² is CH₃, R³ is H, R⁴ is H, R⁵ is H, R⁶ is 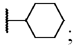
Number A34: R¹ is H, R² is CH₃, R³ is H, R⁴ is H, R⁵ is H, R⁶ is 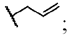
Number A35: R¹ is H, R² is CH₃, R³ is H, R⁴ is H, R⁵ is H, R⁶ is 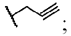
Number A36: R¹ is H, R² is CH₃, R³ is H, R⁴ is H, R⁵ is H, R⁶ is 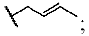
Number A37: R¹ is H, R² is CH₃, R³ is H, R⁴ is H, R⁵ is H, R⁶ is 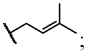
Number A38: R¹ is H, R² is CH₃, R³ is H, R⁴ is H, R⁵ is H, R⁶ is 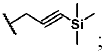
Number A39: R¹ is H, R² is CH₃, R³ is H, R⁴ is H, R⁵ is H, R⁶ is 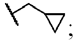
Number A40: R¹ is H, R² is CH₃, R³ is H, R⁴ is H, R⁵ is H, R⁶ is 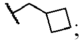
Number A41: R¹ is H, R² is CH₃, R³ is H, R⁴ is H, R⁵ is H, R⁶ is 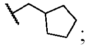
Number A42: R¹ is H, R² is CH₃, R³ is H, R⁴ is H, R⁵ is H, R⁶ is 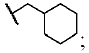
Number A43: R¹ is H, R² is CH₃, R³ is H, R⁴ is H, R⁵ is H, R⁶ is 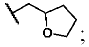
Number A44: R¹ is H, R² is CH₃, R³ is H, R⁴ is H, R⁵ is H, R⁶ is 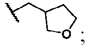
Number A45: R¹ is H, R² is CH₃, R³ is H, R⁴ is H, R⁵ is H, R⁶ is 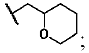
Number A46: R¹ is H, R² is CH₃, R³ is H, R⁴ is H, R⁵ is H, R⁶ is 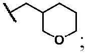
Number A47: R¹ is H, R² is CH₃, R³ is H, R⁴ is H, R⁵ is H, R⁶ is 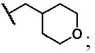
Number A48: R¹ is H, R² is CH₃, R³ is H, R⁴ is H, R⁵ is H, R⁶ is 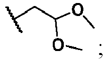
Number A49: R¹ is H, R² is CH₃, R³ is H, R⁴ is H, R⁵ is H, R⁶ is 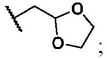
Number A50: R¹ is H, R² is CH₃, R³ is H, R⁴ is H, R⁵ is H, R⁶ is 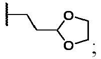
Number A51: R¹ is H, R² is CH₃, R³ is H, R⁴ is H, R⁵ is H, R⁶ is 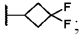
Number A52: R¹ is H, R² is CH₃, R³ is H, R⁴ is H, R⁵ is H, R⁶ is 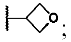
Number A53: R¹ is H, R² is CH₃, R³ is H, R⁴ is H, R⁵ is H, R⁶ is 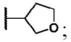
Number A54: R¹ is H, R² is CH₃, R³ is H, R⁴ is H, R⁵ is H, R⁶ is 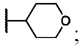
Number A55: R¹ is H, R² is CH₃, R³ is H, R⁴ is H, R⁵ is H, R⁶ is 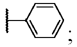
Number A56: R¹ is H, R² is CH₃, R³ is H, R⁴ is H, R⁵ is H, R⁶ is 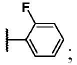
Number A57: R¹ is H, R² is CH₃, R³ is H, R⁴ is H, R⁵ is H, R⁶ is 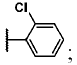
Number A58: R¹ is H, R² is CH₃, R³ is H, R⁴ is H, R⁵ is H, R⁶ is 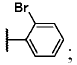
Number A59: R¹ is H, R² is CH₃, R³ is H, R⁴ is H, R⁵ is H, R⁶ is 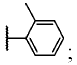
Number A60: R¹ is H, R² is CH₃, R³ is H, R⁴ is H, R⁵ is H, R⁶ is 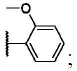
Number A61: R¹ is H, R² is CH₃, R³ is H, R⁴ is H, R⁵ is H, R⁶ is 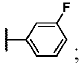
Number A62: R¹ is H, R² is CH₃, R³ is H, R⁴ is H, R⁵ is H, R⁶ is 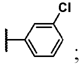
Number A63: R¹ is H, R² is CH₃, R³ is H, R⁴ is H, R⁵ is H, R⁶ is 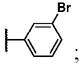
Number A64: R¹ is H, R² is CH₃, R³ is H, R⁴ is H, R⁵ is H, R⁶ is 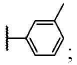
Number A65: R¹ is H, R² is CH₃, R³ is H, R⁴ is H, R⁵ is H, R⁶ is 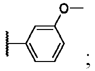
Number A66: R¹ is H, R² is CH₃, R³ is H, R⁴ is H, R⁵ is H, R⁶ is 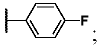
Number A67: R¹ is H, R² is CH₃, R³ is H, R⁴ is H, R⁵ is H, R⁶ is 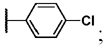
Number A68: R¹ is H, R² is CH₃, R³ is H, R⁴ is H, R⁵ is H, R⁶ is 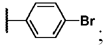
Number A69: R¹ is H, R² is CH₃, R³ is H, R⁴ is H, R⁵ is H, R⁶ is 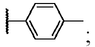
Number A70: R¹ is H, R² is CH₃, R³ is H, R⁴ is H, R⁵ is H, R⁶ is 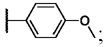
Number A71: R¹ is H, R² is CH₃, R³ is H, R⁴ is H, R⁵ is H, R⁶ is 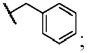
Number A72: R¹ is H, R² is CH₃, R³ is H, R⁴ is H, R⁵ is H, R⁶ is -COCH₃;
Number A73: R¹ is H, R² is CH₃, R³ is H, R⁴ is H, R⁵ is H, R⁶ is -COCH₂CH₃;
Number A74: R¹ is H, R² is CH₃, R³ is H, R⁴ is H, R⁵ is H, R⁶ is -COCH₂CH₂CH₃;
Number A75: R¹ is H, R² is CH₃, R³ is H, R⁴ is H, R⁵ is H, R⁶ is -COCH(CH₃)₂;
Number A76: R¹ is H, R² is CH₃, R³ is H, R⁴ is H, R⁵ is H, R⁶ is -COC₆H₅;
Number A77: R¹ is H, R² is CH₃, R³ is H, R⁴ is H, R⁵ is H, R⁶ is 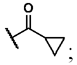
Number A78: R¹ is H, R² is CH₃, R³ is H, R⁴ is H, R⁵ is H, R⁶ is 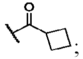
Number A79: R¹ is H, R² is CH₃, R³ is H, R⁴ is H, R⁵ is H, R⁶ is 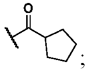
Number A80: R¹ is H, R² is CH₃, R³ is H, R⁴ is H, R⁵ is H, R⁶ is 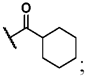
Number A81: R¹ is H, R² is CH₃, R³ is H, R⁴ is H, R⁵ is H, R⁶ is 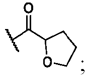
Number A82: R¹ is H, R² is CH₃, R³ is H, R⁴ is H, R⁵ is H, R⁶ is 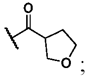
Number A83: R¹ is H, R² is CH₃, R³ is H, R⁴ is H, R⁵ is H, R⁶ is 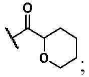
Number A84: R¹ is H, R² is CH₃, R³ is H, R⁴ is H, R⁵ is H, R⁶ is 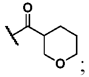
Number A85: R¹ is H, R² is CH₃, R³ is H, R⁴ is H, R⁵ is H, R⁶ is 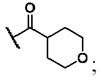
Number A86: R¹ is H, R² is CH₃, R³ is H, R⁴ is H, R⁵ is H, R⁶ is 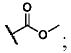
Number A87: R¹ is H, R² is CH₃, R³ is H, R⁴ is H, R⁵ is H, R⁶ is 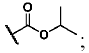
Number A88: R¹ is H, R² is CH₃, R³ is H, R⁴ is H, R⁵ is H, R⁶ is 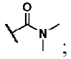
Number A89: R¹ is H, R² is CH₃, R³ is H, R⁴ is H, R⁵ is H, R⁶ is 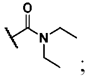
Number A90: R¹ is H, R² is CH₃, R³ is H, R⁴ is H, R⁵ is H, R⁶ is -S(=O)₂CH₃;
Number A91: R¹ is H, R² is CH₃, R³ is H, R⁴ is H, R⁵ is H, R⁶ is -S(=O)₂CH₂CH₃;
Number A92: R¹ is H, R² is CH₃, R³ is H, R⁴ is H, R⁵ is H, R⁶ is -S(=O)₂CH₂CH₂CH₃;
Number A93: R¹ is H, R² is CH₃, R³ is H, R⁴ is H, R⁵ is H, R⁶ is -S(=O)₂CH(CH₃)₂;
Number A94: R¹ is H, R² is CH₃, R³ is H, R⁴ is H, R⁵ is H, R⁶ is -S(=O)₂C₆H₅;
Number A95: R¹ is H, R² is CH₃, R³ is H, R⁴ is H, R⁵ is H, R⁶ is 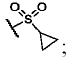
Number A96: R¹ is H, R² is CH₃, R³ is H, R⁴ is H, R⁵ is H, R⁶ is 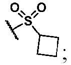
Number A97: R¹ is H, R² is CH₃, R³ is H, R⁴ is H, R⁵ is H, R⁶ is 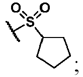
Number A98: R¹ is H, R² is CH₃, R³ is H, R⁴ is H, R⁵ is H, R⁶ is 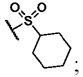
Number A99: R¹ is H, R² is CH₃, R³ is H, R⁴ is H, R⁵ is H, R⁶ is 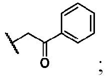
Number A100: R¹ is H, R² is CH₃, R³ is H, R⁴ is H, R⁵ is H, R⁶ is 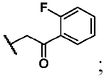
Number A101: R¹ is H, R² is CH₃, R³ is H, R⁴ is H, R⁵ is H, R⁶ is 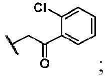
Number A102: R¹ is H, R² is CH₃, R³ is H, R⁴ is H, R⁵ is H, R⁶ is 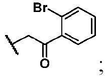
Number A103: R¹ is H, R² is CH₃, R³ is H, R⁴ is H, R⁵ is H, R⁶ is 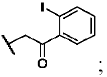
Number A104: R¹ is H, R² is CH₃, R³ is H, R⁴ is H, R⁵ is H, R⁶ is 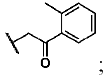
Number A105: R¹ is H, R² is CH₃, R³ is H, R⁴ is H, R⁵ is H, R⁶ is 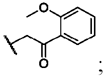
Number A106: R¹ is H, R² is CH₃, R³ is H, R⁴ is H, R⁵ is H, R⁶ is 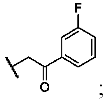
Number A107: R¹ is H, R² is CH₃, R³ is H, R⁴ is H, R⁵ is H, R⁶ is 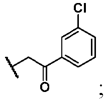
Number A108: R¹ is H, R² is CH₃, R³ is H, R⁴ is H, R⁵ is H, R⁶ is 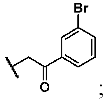
Number A109: R¹ is H, R² is CH₃, R³ is H, R⁴ is H, R⁵ is H, R⁶ is 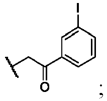
Number A110: R¹ is H, R² is CH₃, R³ is H, R⁴ is H, R⁵ is H, R⁶ is 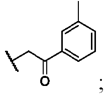
Number A111: R¹ is H, R² is CH₃, R³ is H, R⁴ is H, R⁵ is H, R⁶ is 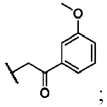
Number A112: R¹ is H, R² is CH₃, R³ is H, R⁴ is H, R⁵ is H, R⁶ is 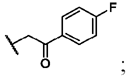
Number A113: R¹ is H, R² is CH₃, R³ is H, R⁴ is H, R⁵ is H, R⁶ is 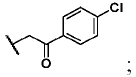
Number A114: R¹ is H, R² is CH₃, R³ is H, R⁴ is H, R⁵ is H, R⁶ is 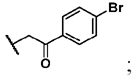
Number A115: R¹ is H, R² is CH₃, R³ is H, R⁴ is H, R⁵ is H, R⁶ is 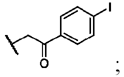
Number A116: R¹ is H, R² is CH₃, R³ is H, R⁴ is H, R⁵ is H, R⁶ is 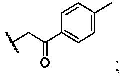
Number A117: R¹ is H, R² is CH₃, R³ is H, R⁴ is H, R⁵ is CH₃, R⁶ is 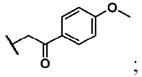
Number A118: R¹ is H, R² is CH₃, R³ is H, R⁴ is H, R⁵ is CH₃, R⁶ is -H;
Number A119: R¹ is H, R² is CH₃, R³ is H, R⁴ is H, R⁵ is CH₃, R⁶ is -CH₃;
Number A120: R¹ is H, R² is CH₃, R³ is H, R⁴ is H, R⁵ is CH₃, R⁶ is -CH₂CH₃;
Number A121: R¹ is H, R² is CH₃, R³ is H, R⁴ is H, R⁵ is CH₃, R⁶ is -CH₂CH₂CH₃;
Number A122: R¹ is H, R² is CH₃, R³ is H, R⁴ is H, R⁵ is CH₃, R⁶ is -CH(CH₃)₂;
Number A123: R¹ is H, R² is CH₃, R³ is H, R⁴ is H, R⁵ is CH₃, R⁶ is 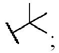
Number A124: R¹ is H, R² is CH₃, R³ is H, R⁴ is H, R⁵ is CH₃, R⁶ is 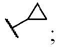
Number A125: R¹ is H, R² is CH₃, R³ is H, R⁴ is H, R⁵ is CH₃, R⁶ is 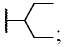
Number A126: R¹ is H, R² is CH₃, R³ is H, R⁴ is H, R⁵ is CH₃, R⁶ is 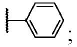
Number A127: R¹ is H, R² is CH₃, R³ is H, R⁴ is H, R⁵ is CH₃, R⁶ is 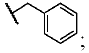
Number A128: R¹ is H, R² is CH₃, R³ is H, R⁴ is H, R⁵ is CH₃, R⁶ is -COCH₃;
Number A129: R¹ is H, R² is CH₃, R³ is H, R⁴ is H, R⁵ is CH₃, R⁶ is -COCH₂CH₃;
Number A130: R¹ is H, R² is CH₃, R³ is H, R⁴ is H, R⁵ is CH₃, R⁶ is -COCH₂CH₂CH₃;
Number A131: R¹ is H, R² is CH₃, R³ is H, R⁴ is H, R⁵ is CH₃, R⁶ is -COCH(CH₃)₂;
Number A132: R¹ is H, R² is CH₃, R³ is H, R⁴ is H, R⁵ is CH₃, R⁶ is -COC₆H₅;
Number A133: R¹ is H, R² is CH₃, R³ is H, R⁴ is H, R⁵ is CH₃, R⁶ is 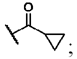
Number A134: R¹ is H, R² is CH₃, R³ is H, R⁴ is H, R⁵ is CH₃, R⁶ is 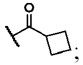
Number A135: R¹ is H, R² is CH₃, R³ is H, R⁴ is H, R⁵ is CH₃, R⁶ is 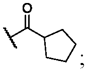
Number A136: R¹ is H, R² is CH₃, R³ is H, R⁴ is H, R⁵ is CH₃, R⁶ is 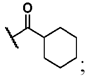
Number A137: R¹ is H, R² is CH₃, R³ is H, R⁴ is H, R⁵ is CH₃, R⁶ is 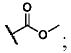
Number A138: R¹ is H, R² is CH₃, R³ is H, R⁴ is H, R⁵ is CH₃, R⁶ is 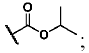
Number A139: R¹ is H, R² is CH₃, R³ is H, R⁴ is H, R⁵ is CH₃, R⁶ is 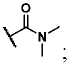
Number A140: R¹ is H, R² is CH₃, R³ is H, R⁴ is H, R⁵ is CH₃, R⁶ is 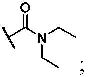
Number A141: R¹ is H, R² is CH₃, R³ is H, R⁴ is H, R⁵ is CH₃, R⁶ is -S(=O)₂CH₃;
Number A142: R¹ is H, R² is CH₃, R³ is H, R⁴ is H, R⁵ is CH₃, R⁶ is -S(=O)₂CH₂CH₃;
Number A143: R¹ is H, R² is CH₃, R³ is H, R⁴ is H, R⁵ is CH₃, R⁶ is -S(=O)₂CH₂CH₂CH₃;
Number A144: R¹ is H, R² is CH₃, R³ is H, R⁴ is H, R⁵ is CH₃, R⁶ is -S(=O)₂CH(CH₃)₂;
Number A145: R¹ is H, R² is CH₃, R³ is H, R⁴ is H, R⁵ is CH₃, R⁶ is -S(=O)₂C₆H₅;
Number A146: R¹ is H, R² is CH₃, R³ is H, R⁴ is H, R⁵ is CH₃, R⁶ is 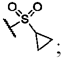
Number A147: R¹ is H, R² is CH₃, R³ is H, R⁴ is H, R⁵ is CH₃, R⁶ is 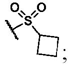
Number A148: R¹ is H, R² is CH₃, R³ is H, R⁴ is H, R⁵ is CH₃, R⁶ is 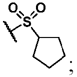
Number A149: R¹ is H, R² is CH₃, R³ is H, R⁴ is H, R⁵ is CH₃, R⁶ is 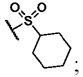
Number A150: R¹ is H, R² is CH₃, R³ is H, R⁴ is CH₃, R⁵ is CH₃, R⁶ is -H;
Number A151: R¹ is H, R² is CH₃, R³ is H, R⁴ is CH₃, R⁵ is CH₃, R⁶ is -CH₃;
Number A152: R¹ is H, R² is CH₃, R³ is H, R⁴ is CH₃, R⁵ is CH₃, R⁶ is -CH₂CH₃;
Number A153: R¹ is H, R² is CH₃, R³ is H, R⁴ is CH₃, R⁵ is CH₃, R⁶ is -CH₂CH₂CH₃;
Number A154: R¹ is H, R² is CH₃, R³ is H, R⁴ is CH₃, R⁵ is CH₃, R⁶ is -CH(CH₃)₂;
Number A155: R¹ is H, R² is CH₃, R³ is H, R⁴ is CH₃, R⁵ is CH₃, R⁶ is 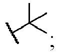
Number A156: R¹ is H, R² is CH₃, R³ is H, R⁴ is CH₃, R⁵ is CH₃, R⁶ is 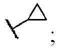
Number A157: R¹ is H, R² is CH₃, R³ is H, R⁴ is CH₃, R⁵ is CH₃, R⁶ is 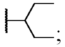
Number A158: R¹ is H, R² is CH₃, R³ is H, R⁴ is CH₃, R⁵ is CH₃, R⁶ is 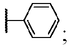
Number A159: R¹ is H, R² is CH₃, R³ is H, R⁴ is CH₃, R⁵ is CH₃, R⁶ is 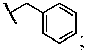
Number A160: R¹ is H, R² is CH₃, R³ is H, R⁴ is CH₃, R⁵ is CH₃, R⁶ is -COCH₃;
Number A161: R^{l} is H, R² is CH₃, R³ is H, R⁴ is CH₃, R⁵ is CH₃, R⁶ is -COCH₂CH₃;
Number A162: R¹ is H, R² is CH₃, R³ is H, R⁴ is CH₃, R⁵ is CH₃, R⁶ is -COCH₂CH₂CH₃;
Number A163: R¹ is H, R² is CH₃, R³ is H, R⁴ is CH₃, R⁵ is CH₃, R⁶ is -COCH(CH₃)₂;
Number A164: R¹ is H, R² is CH₃, R³ is H, R⁴ is CH₃, R⁵ is CH₃, R⁶ is -COC₆H₅;
Number A165: R¹ is H, R² is CH₃, R³ is H, R⁴ is CH₃, R⁵ is CH₃, R⁶ is 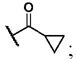
Number A166: R¹ is H, R² is CH₃, R³ is H, R⁴ is CH₃, R⁵ is CH₃, R⁶ is 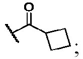
Number A167: R¹ is H, R² is CH₃, R³ is H, R⁴ is CH₃, R⁵ is CH₃, R⁶ is 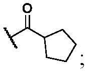
Number A168: R¹ is H, R² is CH₃, R³ is H, R⁴ is CH₃, R⁵ is CH₃, R⁶ is 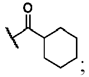
Number A169: R¹ is H, R² is CH₃, R³ is H, R⁴ is CH₃, R⁵ is CH₃, R⁶ is 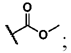
Number A170: R¹ is H, R² is CH₃, R³ is H, R⁴ is CH₃, R⁵ is CH₃, R⁶ is 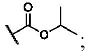
Number A171: R¹ is H, R² is CH₃, R³ is H, R⁴ is CH₃, R⁵ is CH₃, R⁶ is 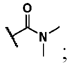
Number A172: R¹ is H, R² is CH₃, R³ is H, R⁴ is CH₃, R⁵ is CH₃, R⁶ is 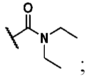
Number A173: R¹ is H, R² is CH₃, R³ is H, R⁴ is CH₃, R⁵ is CH₃, R⁶ is -S(=O)₂CH₃;
Number A174: R¹ is H, R² is CH₃, R³ is H, R⁴ is CH₃, R⁵ is CH₃, R⁶ is -S(=O)₂CH₂CH₃;
Number A175: R¹ is H, R² is CH₃, R³ is H, R⁴ is CH₃, R⁵ is CH₃, R⁶ is -S(=O)₂CH₂CH₂CH₃;
Number A176: R¹ is H, R² is CH₃, R³ is H, R⁴ is CH₃, R⁵ is CH₃, R⁶ is -S(=O)₂CH(CH₃)₂;
Number A177: R¹ is H, R² is CH₃, R³ is H, R⁴ is CH₃, R⁵ is CH₃, R⁶ is -S(=O)₂C₆H₅;
Number A178: R¹ is H, R² is CH₃, R³ is H, R⁴ is CH₃, R⁵ is CH₃, R⁶ is 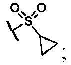
Number A179: R¹ is H, R² is CH₃, R³ is H, R⁴ is CH₃, R⁵ is CH₃, R⁶ is 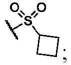
Number A180: R¹ is H, R² is CH₃, R³ is H, R⁴ is CH₃, R⁵ is CH₃, R⁶ is 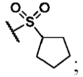
Number A181: R¹ is H, R² is CH₃, R³ is H, R⁴ is CH₃, R⁵ is CH₃, R⁶ is 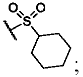
Number A182: R¹ is H, R² is CH₃, R³ is CH₃, R⁴ is H, R⁵ is H, R⁶ is 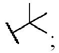
Number A183: R¹ is H, R² is CH₃, R³ is OCH₃, R⁴ is H, R⁵ is H, R⁶ is 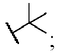
Number A184: R¹ is H, R² is CH₃, R³ is 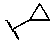 , R⁴ is H, R⁵ is H, R⁶ is 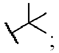
Number A185: R¹ is H, R² is CH₃, R³ is COCH₃, R⁴ is H, R⁵ is H, R⁶ is 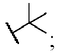
Number A186: R¹ is H, R² is CH₃, R³ is 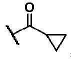 , R⁴ is H, R⁵ is H, R⁶ is 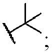
Number A187: R¹ is H, R² is CH₃, R³ is 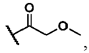 R⁴ is H, R⁵ is H, R⁶ is 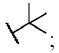
Number A188: R¹ is H, R² is CH₃, R³ is 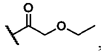 R⁴ is H, R⁵ is H, R⁶ is 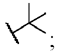
Number A189: R¹ is H, R² is CN, R³ is H, R⁴ is H, R⁵ is H, R⁶ is 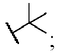
Number A190: R¹ is F, R² is CH₃, R³ is H, R⁴ is H, R⁵ is H, R⁶ is -H;
Number A191: R¹ is F, R² is CH₃, R³ is H, R⁴ is H, R⁵ is H, R⁶ is -CH₃;
Number A192: R¹ is F, R² is CH₃, R³ is H, R⁴ is H, R⁵ is H, R⁶ is -CH₂CH₃;
Number A193: R¹ is F, R² is CH₃, R³ is H, R⁴ is H, R⁵ is H, R⁶ is -CH₂CH₂CH₃;
Number A194: R¹ is F, R² is CH₃, R³ is H, R⁴ is H, R⁵ is H, R⁶ is -CH(CH₃)₂;
Number A195: R¹ is F, R² is CH₃, R³ is H, R⁴ is H, R⁵ is H, R⁶ is 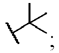
Number A196: R¹ is F, R² is CH₃, R³ is H, R⁴ is H, R⁵ is H, R⁶ is 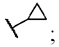
Number A197: R¹ is F, R² is CH₃, R³ is H, R⁴ is H, R⁵ is H, R⁶ is 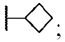
Number A198: R¹ is F, R² is CH₃, R³ is H, R⁴ is H, R⁵ is H, R⁶ is 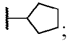
Number A199: R¹ is F, R² is CH₃, R³ is H, R⁴ is H, R⁵ is H, R⁶ is 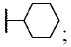
Number A200: R¹ is F, R² is CH₃, R³ is H, R⁴ is H, R⁵ is H, R⁶ is 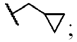
Number A201: R¹ is F, R² is CH₃, R³ is H, R⁴ is H, R⁵ is H, R⁶ is 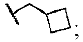
Number A202: R¹ is F, R² is CH₃, R³ is H, R⁴ is H, R⁵ is H, R⁶ is 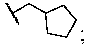
Number A203: R¹ is F, R² is CH₃, R³ is H, R⁴ is H, R⁵ is H, R⁶ is 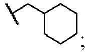
Number A204: R¹ is F, R² is CH₃, R³ is H, R⁴ is H, R⁵ is H, R⁶ is 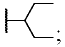
Number A205: R¹ is F, R² is CH₃, R³ is H, R⁴ is H, R⁵ is H, R⁶ is 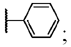
Number A206: R¹ is F, R² is CH₃, R³ is H, R⁴ is H, R⁵ is H, R⁶ is 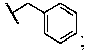
Number A207: R¹ is F, R² is CH₃, R³ is H, R⁴ is H, R⁵ is H, R⁶ is -COCH₃;
Number A208: R¹ is F, R² is CH₃, R³ is H, R⁴ is H, R⁵ is H, R⁶ is -COCH₂CH₃;
Number A209: R¹ is F, R² is CH₃, R³ is H, R⁴ is H, R⁵ is H, R⁶ is -COCH₂CH₂CH₃;
Number A210: R¹ is F, R² is CH₃, R³ is H, R⁴ is H, R⁵ is H, R⁶ is -COCH(CH₃)₂;
Number A211: R¹ is F, R² is CH₃, R³ is H, R⁴ is H, R⁵ is H, R⁶ is -COC₆H₅;
Number A212: R¹ is F, R¹ is CH₃, R³ is H, R⁴ is H, R⁵ is H, R⁶ is 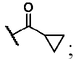
Number A213: R¹ is F, R² is CH₃, R³ is H, R⁴ is H, R⁵ is H, R⁶ is 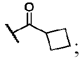
Number A214: R¹ is F, R² is CH₃, R³ is H, R⁴ is H, R⁵ is H, R⁶ is 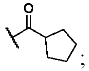
Number A215: R¹ is F, R² is CH₃, R³ is H, R⁴ is H, R⁵ is H, R⁶ is 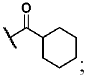
Number A216: R¹ is F, R² is CH₃, R³ is H, R⁴ is H, R⁵ is H, R⁶ is 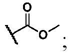
Number A217: R¹ is F, R² is CH₃, R³ is H, R⁴ is H, R⁵ is H, R⁶ is 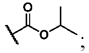
Number A218: R¹ is F, R² is CH₃, R³ is H, R⁴ is H, R⁵ is H, R⁶ is 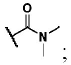
Number A219: R¹ is F, R² is CH₃, R³ is H, R⁴ is H, R⁵ is H, R⁶ is 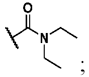
Number A220: R¹ is F, R² is CH₃, R³ is H, R⁴ is H, R⁵ is H, R⁶ is -S(=O)₂CH₃;
Number A221: R¹ is F, R² is CH₃, R³ is H, R⁴ is H, R⁵ is H, R⁶ is -S(=O)₂CH₂CH₃;
Number A222: R¹ is F, R² is CH₃, R³ is H, R⁴ is H, R⁵ is H, R⁶ is -S(=O)₂CH₂CH₂CH₃;
Number A223: R¹ is F, R² is CH₃, R³ is H, R⁴ is H, R⁵ is H, R⁶ is -S(=O)₂CH(CH₃)₂;
Number A224: R¹ is F, R² is CH₃, R³ is H, R⁴ is H, R⁵ is H, R⁶ is -S(=O)₂C₆H₅;
Number A225: R¹ is F, R² is CH₃, R³ is H, R⁴ is H, R⁵ is H, R⁶ is 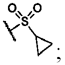
Number A226: R¹ is F, R² is CH₃, R³ is H, R⁴ is H, R⁵ is H, R⁶ is 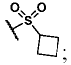
Number A227: R¹ is F, R² is CH₃, R³ is H, R⁴ is H, R⁵ is H, R⁶ is 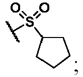
Number A228: R¹ is F, R² is CH₃, R³ is H, R⁴ is H, R⁵ is H, R⁶ is 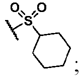
Number A229: R¹ is F, R² is CH₃, R³ is H, R⁴ is H, R⁵ is CH₃, R⁶ is -H;
Number A230: R¹ is F, R² is CH₃, R³ is H, R⁴ is H, R⁵ is CH₃, R⁶ is -CH₃;
Number A231: R¹ is F, R² is CH₃, R³ is H, R⁴ is H, R⁵ is CH₃, R⁶ is -CH₂CH₃;
Number A232: R¹ is F, R² is CH₃, R³ is H, R⁴ is H, R⁵ is CH₃, R⁶ is -CH₂CH₂CH₃;
Number A233: R¹ is F, R² is CH₃, R³ is H, R⁴ is H, R⁵ is CH₃, R⁶ is -CH(CH₃)₂;
Number A234: R¹ is F, R² is CH₃, R³ is H, R⁴ is H, R⁵ is CH₃, R⁶ is 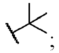
Number A235: R¹ is F, R² is CH₃, R³ is H, R⁴ is H, R⁵ is CH₃, R⁶ is 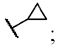
Number A236: R¹ is F, R² is CH₃, R³ is H, R⁴ is H, R⁵ is CH₃, R⁶ is 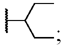
Number A237: R¹ is F, R² is CH₃, R³ is H, R⁴ is H, R⁵ is CH₃, R⁶ is 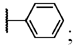
Number A238: R¹ is F, R² is CH₃, R³ is H, R⁴ is H, R⁵ is CH₃, R⁶ is 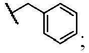
Number A239: R¹ is F, R² is CH₃, R³ is H, R⁴ is H, R⁵ is CH₃, R⁶ is -COCH₃;
Number A240: R¹ is F, R² is CH₃, R³ is H, R⁴ is H, R⁵ is CH₃, R⁶ is -COCH₂CH₃;
Number A241: R¹ is F, R² is CH₃, R³ is H, R⁴ is H, R⁵ is CH₃, R⁶ is -COCH₂CH₂CH₃;
Number A242: R¹ is F, R² is CH₃, R³ is H, R⁴ is H, R⁵ is CH₃, R⁶ is -COCH(CH₃)₂;
Number A243: R¹ is F, R² is CH₃, R³ is H, R⁴ is H, R⁵ is CH₃, R⁶ is -COC₆H₅;
Number A244: R¹ is F, R² is CH₃, R³ is H, R⁴ is H, R⁵ is CH₃, R⁶ is 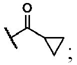
Number A245: R¹ is F, R² is CH₃, R³ is H, R⁴ is H, R⁵ is CH₃, R⁶ is 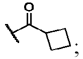
Number A246: R¹ is F, R² is CH₃, R³ is H, R⁴ is H, R⁵ is CH₃, R⁶ is 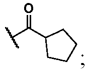
Number A247: R¹ is F, R² is CH₃, R³ is H, R⁴ is H, R⁵ is CH₃, R⁶ is 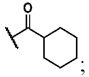
Number A248: R¹ is F, R² is CH₃, R³ is H, R⁴ is H, R⁵ is CH₃, R⁶ is 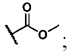
Number A249: R¹ is F, R² is CH₃, R³ is H, R⁴ is H, R⁵ is CH₃, R⁶ is 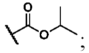
Number A250: R¹ is F, R² is CH₃, R³ is H, R⁴ is H, R⁵ is CH₃, R⁶ is 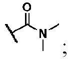
Number A251: R¹ is F, R² is CH₃, R³ is H, R⁴ is H, R⁵ is CH₃, R⁶ is 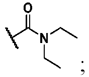
Number A252: R¹ is F, R² is CH₃, R³ is H, R⁴ is H, R⁵ is CH₃, R⁶ is -S(=O)₂CH₃;
Number A253: R¹ is F, R² is CH₃, R³ is H, R⁴ is H, R⁵ is CH₃, R⁶ is -S(=O)₂CH₂CH₃;
Number A254: R¹ is F, R² is CH₃, R³ is H, R⁴ is H, R⁵ is CH₃, R⁶ is -S(=O)₂CH₂CH₂CH₃;
Number A255: R¹ is F, R² is CH₃, R³ is H, R⁴ is H, R⁵ is CH₃, R⁶ is -S(=O)₂CH(CH₃)₂;
Number A256: R¹ is F, R² is CH₃, R³ is H, R⁴ is H, R⁵ is CH₃, R⁶ is -S(=O)₂C₆H₅;
Number A257: R¹ is F, R² is CH₃, R³ is H, R⁴ is H, R⁵ is CH₃, R⁶ is 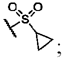
Number A258: R¹ is F, R² is CH₃, R³ is H, R⁴ is H, R⁵ is CH₃, R⁶ is 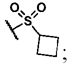
Number A259: R¹ is F, R² is CH₃, R³ is H, R⁴ is H, R⁵ is CH₃, R⁶ is 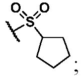
Number A260: R¹ is F, R² is CH₃, R³ is H, R⁴ is H, R⁵ is CH₃, R⁶ is 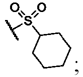
Number A261: R¹ is F, R² is CH₃, R³ is H, R⁴ is CH₃, R⁵ is CH₃, R⁶ is -H;
Number A262: R¹ is F, R² is CH₃, R³ is H, R⁴ is CH₃, R⁵ is CH₃, R⁶ is -CH₃;
Number A263: R¹ is F, R² is CH₃, R³ is H, R⁴ is CH₃, R⁵ is CH₃, R⁶ is -CH₂CH₃;
Number A264: R¹ is F, R² is CH₃, R³ is H, R⁴ is CH₃, R⁵ is CH₃, R⁶ is -CH₂CH₂CH₃;
Number A265: R¹ is F, R² is CH₃, R³ is H, R⁴ is CH₃, R⁵ is CH₃, R⁶ is -CH(CH₃)₂;
Number A266: R¹ is F, R² is CH₃, R³ is H, R⁴ is CH₃, R⁵ is CH₃, R⁶ is 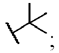
Number A267: R¹ is F, R² is CH₃, R³ is H, R⁴ is CH₃, R⁵ is CH₃, R⁶ is 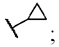
Number A268: R¹ is F, R² is CH₃, R³ is H, R⁴ is CH₃, R⁵ is CH₃, R⁶ is 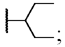
Number A269: R¹ is F, R² is CH₃, R³ is H, R⁴ is CH₃, R⁵ is CH₃, R⁶ is 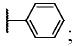
Number A270: R¹ is F, R² is CH₃, R³ is H, R⁴ is CH₃, R⁵ is CH₃, R⁶ is 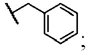
Number A271: R¹ is F, R² is CH₃, R³ is H, R⁴ is CH₃, R⁵ is CH₃, R⁶ is -COCH₃;
Number A272: R¹ is F, R² is CH₃, R³ is H, R⁴ is CH₃, R⁵ is CH₃, R⁶ is -COCH₂CH₃;
Number A273: R¹ is F, R² is CH₃, R³ is H, R⁴ is CH₃, R⁵ is CH₃, R⁶ is -COCH₂CH₂CH₃;
Number A274: R¹ is F, R² is CH₃, R³ is H, R⁴ is CH₃, R⁵ is CH₃, R⁶ is -COCH(CH₃)₂;
Number A275: R¹ is F, R² is CH₃, R³ is H, R⁴ is CH₃, R⁵ is CH₃, R⁶ is -COC₆H₅;
Number A276: R¹ is F, R² is CH₃, R³ is H, R⁴ is CH₃, R⁵ is CH₃, R⁶ is 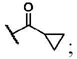
Number A277: R¹ is F, R² is CH₃, R³ is H, R⁴ is CH₃, R⁵ is CH₃, R⁶ is 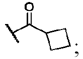
Number A278: R¹ is F, R² is CH₃, R³ is H, R⁴ is CH₃, R⁵ is CH₃, R⁶ is 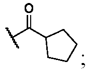
Number A279: R¹ is F, R² is CH₃, R³ is H, R⁴ is CH₃, R⁵ is CH₃, R⁶ is 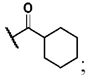
Number A280: R¹ is F, R² is CH₃, R³ is H, R⁴ is CH₃, R⁵ is CH₃, R⁶ is 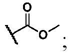
Number A281: R¹ is F, R² is CH₃, R³ is H, R⁴ is CH₃, R⁵ is CH₃, R⁶ is 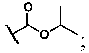
Number A282: R¹ is F, R² is CH₃, R³ is H, R⁴ is CH₃, R⁵ is CH₃, R⁶ is 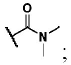
Number A283: R¹ is F, R² is CH₃, R³ is H, R⁴ is CH₃, R⁵ is CH₃, R⁶ is 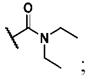
Number A284: R¹ is F, R² is CH₃, R³ is H, R⁴ is CH₃, R⁵ is CH₃, R⁶ is -S(=O)₂CH₃;
Number A285: R¹ is F, R² is CH₃, R³ is H, R⁴ is CH₃, R⁵ is CH₃, R⁶ is -S(=O)₂CH₂CH₃;
Number A286: R¹ is F, R² is CH₃, R³ is H, R⁴ is CH₃, R⁵ is CH₃, R⁶ is -S(=O)₂CH₂CH₂CH₃;
Number A287: R¹ is F, R² is CH₃, R³ is H, R⁴ is CH₃, R⁵ is CH₃, R⁶ is -S(=O)₂CH(CH₃)₂;
Number A288: R¹ is F, R² is CH₃, R³ is H, R⁴ is CH₃, R⁵ is CH₃, R⁶ is -S(=O)₂C₆H₅;
Number A289: R¹ is F, R² is CH₃, R³ is H, R⁴ is CH₃, R⁵ is CH₃, R⁶ is 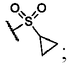
Number A290: R¹ is F, R² is CH₃, R³ is H, R⁴ is CH₃, R⁵ is CH₃, R⁶ is 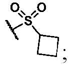
Number A291: R¹ is F, R² is CH₃, R³ is H, R⁴ is CH₃, R⁵ is CH₃, R⁶ is 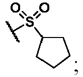
Number A292: R¹ is F, R² is CH₃, R³ is H, R⁴ is CH₃, R⁵ is CH₃, R⁶ is 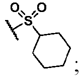
Number A293: R¹ is F, R² is CH₃, R³ is CH₃, R⁴ is H, R⁵ is H, R⁶ is 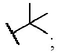
Number A294: R¹ is F, R² is CH₃, R³ is OCH₃, R⁴ is H, R⁵ is H, R⁶ is 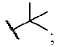
Number A295: R¹ is F, R² is CH₃, R³ is 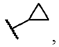 R⁴ is H, R⁵ is H, R⁶ is 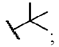
Number A296: R¹ is F, R² is CH₃, R³ is COCH₃, R⁴ is H, R⁵ is H, R⁶ is 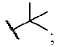
Number A297: R¹ is F, R² is CH₃, R³ is 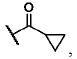 R⁴ is H, R⁵ is H, R⁶ is 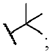
Number A298: R¹ is F, R² is CH₃, R³ is 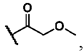 R⁴ is H, R⁵ is H, R⁶ is 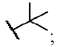
Number A299: R¹ is F, R² is CH₃, R³ is 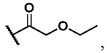 R⁴ is H, R⁵ is H, R⁶ is 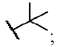
Number A300: R¹ is F, R² is CN, R³ is H, R⁴ is H, R⁵ is H, R⁶ is 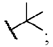
Number A301: R¹ is H, R² is CH₃, R³ is H, R⁴ is H, R⁵ is H, R⁶ is -CH₂OCH₃.

5. Non-therapeutic use of a pyrazole structure-containing compound according to any one of claims 1 to 4 or an agrochemically acceptable salt, hydrate and solvate thereof for at least one of insect killing, mite killing, nematode killing, and piercing-sucking insects killing.

6. Non-therapeutic use of a compound containing a pyrazole structure according to any one of claims 1 to 4 or an agrochemically acceptable salt, hydrate and solvate thereof as an agrochemical insecticide.

7. A insecticide which comprises, as an active ingredient, a pesticidally effective amount of at least one of a compound containing a pyrazole structure according to any one of claims 1 to 4 or an agrochemically acceptable salt, hydrate and solvate thereof.

8. The insecticide of claim 7, wherein the active ingredient is present in the insecticide in an amount of 1 to 99.9% by weight.

9. The insecticide of claim 8, wherein the active ingredient is present in the insecticide in an amount of 5 to 95% by weight.

10. The insecticide according to any one of claims 7 to 9, wherein the insecticide is in a form selected from at least one of emulsifiable concentrate, suspension, wettable powder, dustpowder, granules, aqueous solution, poison bait, mother liquor and mother powder.

## Patentansprüche

1. Eine Verbindung mit einer Pyrazolstruktur oder agrochemisch annehmbare Salze, Hydrate und Solvate davon, wobei die Verbindung die in Formel (I) gezeigte Struktur aufweist: 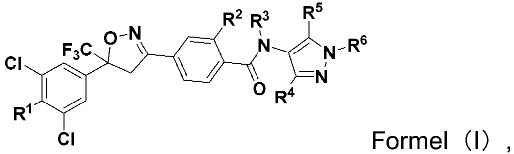
wobei in Formel (I)
R¹ aus H, Halogen, mindestens einem halogensubstituierten C₁₋₆-Alkylrest und mindestens einem halogensubstituierten C₁₋₆-Alkoxyrest ausgewählt ist; R² aus C₁₋₁₂-Alkyl, C₁₋₁₂-Alkoxy, Halogen, mindestens einem halogensubstituierten C₁₋₆-Alkyl, mindestens einem halogensubstituierten C₁₋₆-Alkoxy und Cyano ausgewählt ist;
R³ aus **H,** C₁₋₁₂-Alkyl, C₁₋₁₂-Alkoxy, C₃₋₁₂-Cycloalkyl, -CO-R₃₁ und -CO-CH₂-R₃₁ ausgewählt ist; wobei R₃₁ aus C₁₋₁₂-Alkyl, C₃₋₁₂-Cycloalkyl und C₁₋₁₂-Alkoxy ausgewählt ist;
R⁴ und R⁵ jeweils unabhängig voneinander ausgewählt sind aus **H,** C₁₋₁₂-Alkyl, C₁₋₁₂-Alkoxy, Halogen, mindestens einem halogensubstituierten C₁₋₆-Alkyl und mindestens einem halogensubstituierten C₁₋₆-Alkoxy;
R⁶ aus der Gruppe ausgewählt ist, bestehend aus **H,** C₁₋₁₂-Alkyl, das unsubstituiert oder durch mindestens eine Gruppe substituiert ist, die aus Hydroxyl, Halogen, C₁₋₁₂-Alkyl, C₁₋₁₂-Alkoxy und Trimethylsilyl ausgewählt ist; C₁₋₁₂-Alkoxy, das unsubstituiert oder durch mindestens eine Gruppe substituiert ist, die aus Hydroxyl, Halogen, C₁₋₁₂-Alkyl, C₁₋₁₂-Alkoxy und Trimethylsilyl ausgewählt ist; C₃₋₁₂-Cycloalkyl, das unsubstituiert ist oder durch mindestens eine Gruppe substituiert ist, die aus Hydroxyl, Halogen, C₁₋₁₂-Alkyl, C₁₋₁₂-Alkoxy und Trimethylsilyl ausgewählt ist; einem Phenyl, der unsubstituiert oder durch mindestens eine Gruppe substituiert ist, die aus Hydroxyl, Halogen, C₁₋₁₂-Alkyl, C₁₋₁₂-Alkoxy und Trimethylsilyl aus-gewählt ist; C₃₋₁₂-Cycloalkyl, das mindestens ein Heteroatom als ringbildendes Atom enthält; C₂₋₁₂-Kohlenwasserstoff, der mindestens eine ungesättigte Kohlenstoff-Kohlenstoff-Doppelbindung oder ungesättigte Kohlenstoff-Kohlenstoff-Dreifachbindung enthält und der unsubstituiert oder durch mindestens eine Gruppe substituiert ist, die aus Hydroxyl, Halogen, C₁₋₁₂-Alkyl, C₁₋₁₂-Alkoxy und Trimethylsilyl ausgewählt ist, -(CH₂)ₙ-R₆₁, -CO-R₆₂, -COO-R₆₂, -CO-N(R₆₃R₆₄), -S(=O)₂-R₆₂, -(CH₂)ₙ-CO-R₆₂, wobei R₆₁ aus der Gruppe ausgewählt ist, die aus C₃₋₁₂-Cycloalkyl, C₃₋₁₂-Cycloalkyl, das mindestens ein Heteroatom als ringbildendes Atom enthält, und Phenyl besteht; und R₆₂ aus der Gruppe ausgewählt ist, die aus C₁₋₁₂-Alkyl, Phenyl, das unsubstituiert oder durch mindestens eines von Hydroxyl, Halogen, C1-12-Alkyl, C1-12-Alkoxy und Trimethylsilyl substituiert ist, C₃₋₁₂-Cycloalkyl oder C₃₋₁₂-Cycloalkyl, das mindestens ein Heteroatom als ringbildendes Atom enthält; R₆₃ und R₆₄ unabhängig voneinander aus C₁₋₁₂-Alkyl ausgewählt sind, wobei jedes der Heteroatome unabhängig voneinander wenigstens eines aus N, O, S ist; und wobei jedes n unabhängig voneinander 1 oder 2 ist.

2. Verbindung gemäß Anspruch 1, wobei in Formel (I)
R¹ aus H, Halogen, mindestens einem halogensubstituierten C₁₋₆-Alkyl und mindestens einem halogensubstituierten C₁₋₆-Alkoxy ausgewählt ist;
R² aus C₁₋₈-Alkyl, C₁₋₈-Alkoxy, Halogen, mindestens einem halogensubstituierten C₁₋₆-Alkyl, mindestens einem halogensubstituierten C₁₋₆-Alkoxy und Cyano ausgewählt ist;
R³ aus H, C₁₋₈-Alkyl, C₁₋₈-Alkoxy, C₃₋₁₀-Cycloalkyl, -CO-R₃₁ und -CO-CH₂-R₃₁ ausgewählt ist, wobei R₃₁ aus C₁₋₈-Alkyl, C₃₋₁₀-Cycloalkyl und C₁₋₈-Alkoxy ausgewählt ist;
R⁴ und R⁵ jeweils unabhängig voneinander ausgewählt sind aus H, C₁₋₈-Alkyl, C₁₋₈-Alkoxy, Halogen, mindestens einem halogensubstituierten C₁₋₆-Alkyl und mindestens einem halogensubstituierten C₁₋₆-Alkoxy;
R⁶ aus der Gruppe ausgewählt ist, bestehend aus H, C₁₋₁₀-Alkyl, das unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist; C₁₋₁₀-Alkoxy, das unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist; C₃₋₁₀-Cycloalkyl, das unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist; Phenyl, das unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist; C₃₋₁₀-Cycloalkyl, das mindestens ein Heteroatom als ringbildendes Atom enthält; C₂₋₁₀-Kohlenwasserstoff, der mindestens eine ungesättigte Kohlenstoff-Kohlenstoff-Doppelbindung oder ungesättigte Kohlenstoff-Kohlenstoff-Dreifachbindung enthält und der unsubstituiert oder durch mindestens eine Gruppe substituiert ist, die aus Hydroxyl, Halogen, C₁₋₈-Alkyl, C₁₋₈-Alkoxy und Trimethylsilylausgewählt ist, -(CH₂)ₙ-R₆₁, -CO-R₆₂, -COO-R₆₂, -CO-N(R₆₃R₆₄), -S(=O)₂-R₆₂, -(CH₂)ₙ-CO-R₆₂; wobei R₆₁ aus der Gruppe ausgewählt ist, bestehend aus C₃₋₁₀-Cycloalkyl, C₃₋₁₀-Cycloalkyl, das mindestens ein Heteroatom als ringbildendes Atom enthält, und Phenyl; R₆₂ aus der Gruppe ausgewählt ist, bestehend aus C₁₋₁₀-Alkyl, Phenyl, das unsubstituiert oder durch mindestens eine der Gruppen Hydroxyl, Halogen, C₁₋₈-Alkyl, C₁₋₈-Alkoxy und Trimethylsilyl substituiert ist, C₃₋₁₀-Cycloalkyl und C₃₋₁₀-Cycloalkyl, das mindestens ein Heteroatom als ringbildendes Atom enthält; R₆₃ und R₆₄ unabhängig voneinander aus C₁₋₁₀-Alkyl ausgewählt sind; wobei jedes der Heteroatome unabhängig voneinander mindestens eines ist, das aus N, O, S ausgewählt ist; und wobei jedes n unabhängig voneinander 1 oder 2 ist.

3. Verbindung nach Anspruch 2, wobei in Formel (I)
R¹ aus H, F, Chlor und Brom ausgewählt ist;
R² aus Methyl, Ethyl, n-Propyl, Isopropyl, n-Butyl, tert-Butyl und Cyano ausgewählt ist;
R³ aus der Gruppe ausgewählt ist, bestehend aus H, Methyl, Ethyl, n-Propyl, Isopropyl, n-Butyl, tert-Butyl, Methoxy, Ethoxy, n-Propoxy, Isopropoxy, n-Butoxy, tert-Butoxy, Cyclopropyl, Cyclobutyl, Cyclopentyl, Cyclohexyl, -CO-R₃₁, und -CO-CH₂-R₃₁; wobei R₃₁ aus C₁₋₆-Alkyl, C₃₋₆-Cycloalkyl und C₁₋₆-Alkoxy ausgewählt ist; R⁴ und R⁵ jeweils unabhängig voneinander ausgewählt sind aus H, Methyl, Ethyl, n-Propyl, Isopropyl, n-Butyl und tert-Butyl;
R⁶ aus der Gruppe ausgewählt ist, bestehend aus H, C₁₋₁₀-Alkyl, das unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist; C₁₋₁₀-Alkoxy, das unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist; C₃₋₁₀-Cycloalkyl, das unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist; Phenyl, das unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist; C₃₋₁₀-Cycloalkyl, das mindestens ein Heteroatom als ringbildendes Atom enthält; C₂₋₁₀-Kohlenwasserstoff, der mindestens eine ungesättigte Kohlenstoff-Kohlenstoff-Doppelbindung oder ungesättigte Kohlenstoff-Kohlenstoff-Dreifachbindung enthält und unsubstituiert oder durch mindestens eine Gruppe substituiert ist, die aus Hydroxyl, Halogen, C₁₋₈-Alkyl, C₁₋₈-Alkoxy und Trimethylsilyl ausgewählt ist, -(CH₂)ₙ-R₆₁, -CO-R₆₂, -COO-R₆₂, -CO-N(R₆₃R₆₄), -S(=O)₂-R₆₂, und -(CH₂)ₙ-CO-R₆₂; wobei R₆₁ aus der Gruppe bestehend aus C₃₋₁₀-Cycloalkyl, C₃₋₁₀-Cycloalkyl, das mindestens ein Heteroatom als ringbildendes Atom enthält, und Phenyl ausgewählt ist; und R₆₂ aus der Gruppe bestehend aus C₁₋₁₀-Alkyl, Phenyl, das unsubstituiert oder durch mindestens eine der Gruppen Hydroxyl, Halogen, C₁₋₈-Alkyl, C₁₋₈-Alkoxy und Trimethylsilyl substituiert ist, C₃₋₁₀-Cycloalkyl und C₃₋₁₀-Cycloalkyl, das mindestens ein Heteroatom als ringbildendes Atom enthält ausgewählt ist; und R₆₃ und R₆₄ unabhängig voneinander aus C₁₋₁₀-Alkyl ausgewählt sind; wobei jedes Heteroatom unabhängig voneinander mindestens eines, ausgewählt aus N, O, S ist; und jedes n unabhängig voneinander 1 oder 2 ist.

4. Verbindung nach Anspruch 3, wobei die Verbindung aus einer der folgenden ausgewählt ist:
Nummer A1: R¹ ist ist H, R² ist ist CH₃, R³ ist H, R⁴ ist H, R⁵ ist H, R⁶ ist -H;
Nummer A2: R¹ ist H, R² ist CH₃, R³ ist H, R⁴ ist H, R⁵ ist H, R⁶ ist -CH₃;
Nummer A3: R¹ ist H, R² ist CH₃, R³ ist H, R⁴ ist H, R⁵ ist H, R⁶ ist -CHF₂;
Nummer A4: R¹ ist H, R² ist CH₃, R³ ist H, R⁴ ist H, R⁵ ist H, R⁶ ist -CF₃;
Nummer A5: R¹ ist H, R² ist CH₃, R³ ist H, R⁴ ist H, R⁵ ist H, R⁶ ist -CH₂CH₃;
Nummer A6: R¹ ist H, R² ist CH₃, R³ ist H, R⁴ ist H, R⁵ ist H, R⁶ ist -CH₂CH₂CH₃;
Nummer A7: R¹ ist H, R² ist CH₃, R³ ist H, R⁴ ist H, R⁵ ist H, R⁶ ist -CH(CH₃)₂;
Nummer A8: R¹ ist H, R² ist CH₃, R³ ist H, R⁴ ist H, R⁵ ist H, R⁶ ist -CH₂CH₂OH;
Nummer A9: R¹ ist H, R² ist CH₃, R³ ist H, R⁴ ist H, R⁵ ist H, R⁶ ist -CH₂CF₃;
Nummer A10: R¹ ist H, R² ist CH₃, R³ ist H, R⁴ ist H, R⁵ ist H, R⁶ ist -CH₂CHF₂;
Nummer A11: R¹ ist H, R² ist CH₃, R³ ist H, R⁴ ist H, R⁵ ist H, R⁶ ist -CH₂CH₂F;
Nummer A12: R¹ ist H, R² ist CH₃, R³ ist H, R⁴ ist H, R⁵ ist H, R⁶ ist -CH₂CH₂CF₃;
Nummer A13: R¹ ist H, R² ist CH₃, R³ ist H, R⁴ ist H, R⁵ ist H, R⁶ ist -CH₂CH₂CH₂F;
Nummer A14: R¹ ist H, R² ist CH₃, R³ ist H, R⁴ ist H, R⁵ ist H, R⁶ ist -CH₂CH₂OCH₃
Nummer A15: R¹ ist H, R² ist CH₃, R³ ist H, R⁴ ist H, R⁵ ist H, R⁶ ist -CH₂CH₂OCH₂CH₃
Nummer A16: R¹ ist H, R² ist CH₃, R³ ist H, R⁴ ist H, R⁵ ist H, R⁶ ist -CH₂(CH₂)₂OCH₃
Nummer A17: R¹ ist H, R² ist CH₃, R³ ist H, R⁴ ist H, R⁵ ist H, R⁶ ist -CH₂CH₂CH₂CH₃;
Nummer A18: R¹ ist H, R² ist CH₃, R³ ist H, R⁴ ist H, R⁵ ist H, R⁶ ist -CH(CH₃)CH₂CH₃;
Nummer A19: R¹ ist H, R² ist CH₃, R³ ist H, R⁴ ist H, R⁵ ist H, R⁶ ist -CH₂(CH₂)₃CH₃;
Nummer A20: R¹ ist H, R² ist CH₃, R³ ist H, R⁴ ist H, R⁵ ist H, R⁶ ist 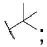
Nummer A21: R¹ ist H, R² ist CH₃, R³ ist H, R⁴ ist H, R⁵ ist H, R⁶ ist 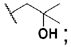
Nummer A22: R¹ ist H, R² ist CH₃, R³ ist H, R⁴ ist H, R⁵ ist H, R⁶ ist 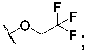
Nummer A23: R¹ ist H, R² ist CH₃, R³ ist H, R⁴ ist H, R⁵ ist H, R⁶ ist 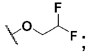
Nummer A24: R¹ ist H, R² ist CH₃, R³ ist H, R⁴ ist H, R⁵ ist H, R⁶ ist 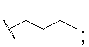
Nummer A25: R¹ ist H, R² ist CH₃, R³ ist H, R⁴ ist H, R⁵ ist H, R⁶ ist 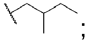
Nummer A26: R¹ ist H, R² ist CH₃, R³ ist H, R⁴ ist H, R⁵ ist H, R⁶ ist 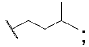
Nummer A27: R¹ ist H, R² ist CH₃, R³ ist H, R⁴ ist H, R⁵ ist H, R⁶ ist 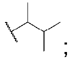
Nummer A28: R¹ ist H, R² ist CH₃, R³ ist H, R⁴ ist H, R⁵ ist H, R⁶ ist 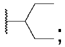
Nummer A29: R¹ ist H, R² ist CH₃, R³ ist H, R⁴ ist H, R⁵ ist H, R⁶ ist 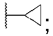
Nummer A30: R¹ ist H, R² ist CH₃, R³ ist H, R⁴ ist H, R⁵ ist H, R⁶ ist 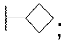
Nummer A31: R¹ ist H, R² ist CH₃, R³ ist H, R⁴ ist H, R⁵ ist H, R⁶ ist 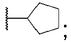
Nummer A32: R¹ ist H, R² ist CH₃, R³ ist H, R⁴ ist H, R⁵ ist H, R⁶ ist 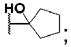
Nummer A33: R¹ ist H, R² ist CH₃, R³ ist H, R⁴ ist H, R⁵ ist H, R⁶ ist 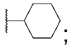
Nummer A34: R¹ ist H, R² ist CH₃, R³ ist H, R⁴ ist H, R⁵ ist H, R⁶ ist 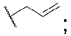
Nummer A35: R¹ ist H, R² ist CH₃, R³ ist H, R⁴ ist H, R⁵ ist H, R⁶ ist 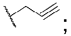
Nummer A36: R¹ ist H, R² ist CH₃, R³ ist H, R⁴ ist H, R⁵ ist H, R⁶ ist 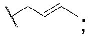
Nummer A37: R¹ ist H, R² ist CH₃, R³ ist H, R⁴ ist H, R⁵ ist H, R⁶ ist 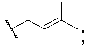
Nummer A38: R¹ ist H, R² ist CH₃, R³ ist H, R⁴ ist H, R⁵ ist H, R⁶ ist 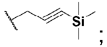
Nummer A39: R¹ ist H, R² ist CH₃, R³ ist H, R⁴ ist H, R⁵ ist H, R⁶ ist 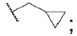
Nummer A40: R¹ ist H, R² ist CH₃, R³ ist H, R⁴ ist H, R⁵ ist H, R⁶ ist 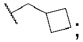
Nummer A41: R¹ ist H, R² ist CH₃, R³ ist H, R⁴ ist H, R⁵ ist H, R⁶ ist 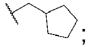
Nummer A42: R¹ ist H, R² ist CH₃, R³ ist H, R⁴ ist H, R⁵ ist H, R⁶ ist 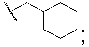
Nummer A43: R¹ ist H, R² ist CH₃, R³ ist H, R⁴ ist H, R⁵ ist H, R⁶ ist 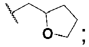
Nummer A44: R¹ ist H, R² ist CH₃, R³ ist H, R⁴ ist H, R⁵ ist H, R⁶ ist 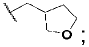
Nummer A45: R¹ ist H, R² ist CH₃, R³ ist H, R⁴ ist H, R⁵ ist H, R⁶ ist 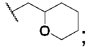
Nummer A46: R¹ ist H, R² ist CH₃, R³ ist H, R⁴ ist H, R⁵ ist H, R⁶ ist 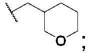
Nummer A47: R¹ ist H, R² ist CH₃, R³ ist H, R⁴ ist H, R⁵ ist H, R⁶ ist 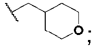
Nummer A48: R¹ ist H, R² ist CH₃, R³ ist H, R⁴ ist H, R⁵ ist H, R⁶ ist 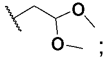
Nummer A49: R¹ ist H, R² ist CH₃, R³ ist H, R⁴ ist H, R⁵ ist H, R⁶ ist 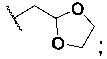
Nummer A50: R¹ ist H, R² ist CH₃, R³ ist H, R⁴ ist H, R⁵ ist H, R⁶ ist 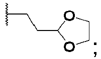
Nummer A51: R¹ ist H, R² ist CH₃, R³ ist H, R⁴ ist H, R⁵ ist H, R⁶ ist 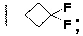
Nummer A52: R¹ ist H, R² ist CH₃, R³ ist H, R⁴ ist H, R⁵ ist H, R⁶ ist 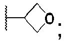
Nummer A53: R¹ ist H, R² ist CH₃, R³ ist H, R⁴ ist H, R⁵ ist H, R⁶ ist 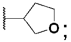
Nummer A54: R¹ ist H, R² ist CH₃, R³ ist H, R⁴ ist H, R⁵ ist H, R⁶ ist 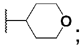
Nummer A55: R¹ ist H, R² ist CH₃, R³ ist H, R⁴ ist H, R⁵ ist H, R⁶ ist 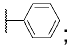
Nummer A56: R¹ ist H, R² ist CH₃, R³ ist H, R⁴ ist H, R⁵ ist H, R⁶ ist 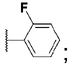
Nummer A57: R¹ ist H, R² ist CH₃, R³ ist H, R⁴ ist H, R⁵ ist H, R⁶ ist 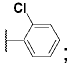
Nummer A58: R¹ ist H, R² ist CH₃, R³ ist H, R⁴ ist H, R⁵ ist H, R⁶ ist 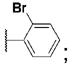
Nummer A59: R¹ ist H, R² ist CH₃, R³ ist H, R⁴ ist H, R⁵ ist H, R⁶ ist 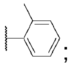
Nummer A60: R¹ ist H, R² ist CH₃, R³ ist H, R⁴ ist H, R⁵ ist H, R⁶ ist 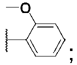
Nummer A61: R¹ ist H, R² ist CH₃, R³ ist H, R⁴ ist H, R⁵ ist H, R⁶ ist 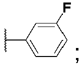
Nummer A62: R¹ ist H, R² ist CH₃, R³ ist H, R⁴ ist H, R⁵ ist H, R⁶ ist 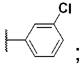
Nummer A63: R¹ ist H, R² ist CH₃, R³ ist H, R⁴ ist H, R⁵ ist H, R⁶ ist 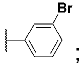
Nummer A64: R¹ ist H, R² ist CH₃, R³ ist H, R⁴ ist H, R⁵ ist H, R⁶ ist 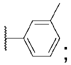
Nummer A65: R¹ ist H, R² ist CH₃, R³ ist H, R⁴ ist H, R⁵ ist H, R⁶ ist 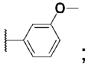
Nummer A66: R¹ ist H, R² ist CH₃, R³ ist H, R⁴ ist H, R⁵ ist H, R⁶ ist 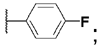
Nummer A67: R¹ ist H, R² ist CH₃, R³ ist H, R⁴ ist H, R⁵ ist H, R⁶ ist 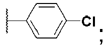
Nummer A68: R¹ ist H, R² ist CH₃, R³ ist H, R⁴ ist H, R⁵ ist H, R⁶ ist 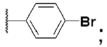
Nummer A69: R¹ ist H, R² ist CH₃, R³ ist H, R⁴ ist H, R⁵ ist H, R⁶ ist 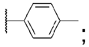
Nummer A70: R¹ ist H, R² ist CH₃, R³ ist H, R⁴ ist H, R⁵ ist H, R⁶ ist 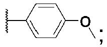
Nummer A71: R¹ ist H, R² ist CH₃, R³ ist H, R⁴ ist H, R⁵ ist H, R⁶ ist 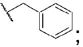
Nummer A72: R¹ ist H, R² ist CH₃, R³ ist H, R⁴ ist H, R⁵ ist H, R⁶ ist -COCH₃;
Nummer A73: R¹ ist H, R² ist CH₃, R³ ist H, R⁴ ist H, R⁵ ist H, R⁶ ist -COCH₂CH₃;
Nummer A74: R¹ ist H, R² ist CH₃, R³ ist H, R⁴ ist H, R⁵ ist H, R⁶ ist -COCH₂CH₂CH₃;
Nummer A75: R¹ ist H, R² ist CH₃, R³ ist H, R⁴ ist H, R⁵ ist H, R⁶ ist -COCH(CH₃)₂;
Nummer A76: R¹ ist H, R² ist CH₃, R³ ist H, R⁴ ist H, R⁵ ist H, R⁶ ist -COC₆H₅;
Nummer A77: R¹ ist H, R² ist CH₃, R³ ist H, R⁴ ist H, R⁵ ist H, R⁶ ist 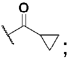
Nummer A78: R¹ ist H, R² ist CH₃, R³ ist H, R⁴ ist H, R⁵ ist H, R⁶ ist 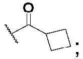
Nummer A79: R¹ ist H, R² ist CH₃, R³ ist H, R⁴ ist H, R⁵ ist H, R⁶ ist 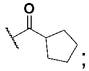
Nummer A80: R¹ ist H, R² ist CH₃, R³ ist H, R⁴ ist H, R⁵ ist H, R⁶ ist 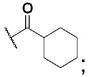
Nummer A81: R¹ ist H, R² ist CH₃, R³ ist H, R⁴ ist H, R⁵ ist H, R⁶ ist 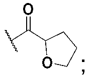
Nummer A82: R¹ ist H, R² ist CH₃, R³ ist H, R⁴ ist H, R⁵ ist H, R⁶ ist 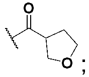
Nummer A83: R¹ ist H, R² ist CH₃, R³ ist H, R⁴ ist H, R⁵ ist H, R⁶ ist 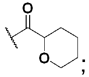
Nummer A84: R¹ ist H, R² ist CH₃, R³ ist H, R⁴ ist H, R⁵ ist H, R⁶ ist 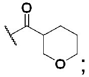
Nummer A85: R¹ ist H, R² ist CH₃, R³ ist H, R⁴ ist H, R⁵ ist H, R⁶ ist 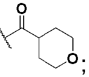
Nummer A86: R¹ ist H, R² ist CH₃, R³ ist H, R⁴ ist H, R⁵ ist H, R⁶ ist 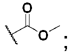
Nummer A87: R¹ ist H, R² ist CH₃, R³ ist H, R⁴ ist H, R⁵ ist H, R⁶ ist 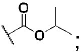
Nummer A88: R¹ ist H, R² ist CH₃, R³ ist H, R⁴ ist H, R⁵ ist H, R⁶ ist 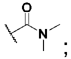
Nummer A89: R¹ ist H, R² ist CH₃, R³ ist H, R⁴ ist H, R⁵ ist H, R⁶ ist 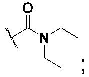
Nummer A90: R¹ ist H, R² ist CH₃, R³ ist H, R⁴ ist H, R⁵ ist H, R⁶ ist -S(=O)₂CH₃;
Nummer A91: R¹ ist H, R² ist CH₃, R³ ist H, R⁴ ist H, R⁵ ist H, R⁶ ist -S(=O)₂CH₂CH₃;
Nummer A92: R¹ ist H, R² ist CH₃, R³ ist H, R⁴ ist H, R⁵ ist H, R⁶ ist -S(=O)₂CH₂CH₂CH₃;
Nummer A93: R¹ ist H, R² ist CH₃, R³ ist H, R⁴ ist H, R⁵ ist H, R⁶ ist -S(=O)₂CH(CH₃)₂;
Nummer A94: R¹ ist H, R² ist CH₃, R³ ist H, R⁴ ist H, R⁵ ist H, R⁶ ist -S(=O)₂C₆H₅;
Nummer A95: R¹ ist H, R² ist CH₃, R³ ist H, R⁴ ist H, R⁵ ist H, R⁶ ist 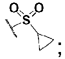
Nummer A96: R¹ ist H, R² ist CH₃, R³ ist H, R⁴ ist H, R⁵ ist H, R⁶ ist 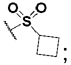
Nummer A97: R¹ ist H, R² ist CH₃, R³ ist H, R⁴ ist H, R⁵ ist H, R⁶ ist 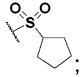
Nummer A98: R¹ ist H, R² ist CH₃, R³ ist H, R⁴ ist H, R⁵ ist H, R⁶ ist 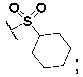
Nummer A99: R¹ ist H, R² ist CH₃, R³ ist H, R⁴ ist H, R⁵ ist H, R⁶ ist 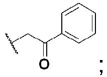
Nummer A100: R¹ ist H, R² ist CH₃, R³ ist H, R⁴ ist H, R⁵ ist H, R⁶ ist 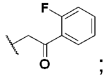
Nummer A101: R¹ ist H, R² ist CH₃, R³ ist H, R⁴ ist H, R⁵ ist H, R⁶ ist 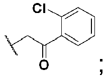
Nummer A102: R¹ ist H, R² ist CH₃, R³ ist H, R⁴ ist H, R⁵ ist H, R⁶ ist 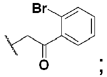
Nummer A103: R¹ ist H, R² ist CH₃, R³ ist H, R⁴ ist H, R⁵ ist H, R⁶ ist 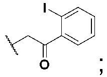
Nummer A104: R¹ ist H, R² ist CH₃, R³ ist H, R⁴ ist H, R⁵ ist H, R⁶ ist 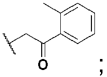
Nummer A105: R¹ ist H, R² ist CH₃, R³ ist H, R⁴ ist H, R⁵ ist H, R⁶ ist 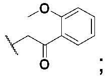
Nummer A106: R¹ ist H, R² ist CH₃, R³ ist H, R⁴ ist H, R⁵ ist H, R⁶ ist 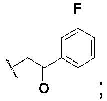
Nummer A107: R¹ ist H, R² ist CH₃, R³ ist H, R⁴ ist H, R⁵ ist H, R⁶ ist 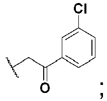
Nummer A108: R¹ ist H, R² ist CH₃, R³ ist H, R⁴ ist H, R⁵ ist H, R⁶ ist 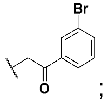
Nummer A109: R¹ ist H, R² ist CH₃, R³ ist H, R⁴ ist H, R⁵ ist H, R⁶ ist 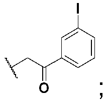
Nummer A110: R¹ ist H, R² ist CH₃, R³ ist H, R⁴ ist H, R⁵ ist H, R⁶ ist 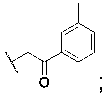
Nummer A111: R¹ ist H, R² ist CH₃, R³ ist H, R⁴ ist H, R⁵ ist H, R⁶ ist 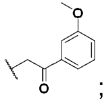
Nummer A112: R¹ ist H, R² ist CH₃, R³ ist H, R⁴ ist H, R⁵ ist H, R⁶ ist 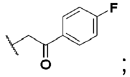
Nummer A113: R¹ ist H, R² ist CH₃, R³ ist H, R⁴ ist H, R⁵ ist H, R⁶ ist 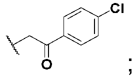 Nummer A114: R¹ ist H, R² ist CH₃, R³ ist H, R⁴ ist H, R⁵ ist H, R⁶ ist 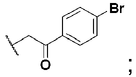
Nummer A115: R¹ ist H, R² ist CH₃, R³ ist H, R⁴ ist H, R⁵ ist H, R⁶ ist 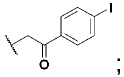
Nummer A116: R¹ ist H, R² ist CH₃, R³ ist H, R⁴ ist H, R⁵ ist H, R⁶ ist 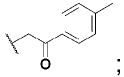
Nummer A117: R¹ ist H, R² ist CH₃, R³ ist H, R⁴ ist H, R⁵ ist CH₃, R⁶ ist 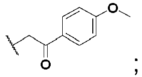
Nummer A118: R¹ ist H, R² ist CH₃, R³ ist H, R⁴ ist H, R⁵ ist CH₃, R⁶ ist -H;
Nummer A119: R¹ ist H, R² ist CH₃, R³ ist H, R⁴ ist H, R⁵ ist CH₃, R⁶ ist -CH₃;
Nummer A120: R¹ ist H, R² ist CH₃, R³ ist H, R⁴ ist H, R⁵ ist CH₃, R⁶ ist -CH₂CH₃;
Nummer A121: R¹ ist H, R² ist CH₃, R³ ist H, R⁴ ist H, R⁵ ist CH₃, R⁶ ist -CH₂CH₂CH₃;
Nummer A122: R¹ ist H, R² ist CH₃, R³ ist H, R⁴ ist H, R⁵ ist CH₃, R⁶ ist -CH(CH₃)₂;
Nummer A123: R¹ ist H, R² ist CH₃, R³ ist H, R⁴ ist H, R⁵ ist CH₃, R⁶ ist 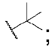
Nummer A124: R¹ ist H, R² ist CH₃, R³ ist H, R⁴ ist H, R⁵ ist CH₃, R⁶ ist 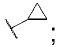
Nummer A125: R¹ ist H, R² ist CH₃, R³ ist H, R⁴ ist H, R⁵ ist CH₃, R⁶ ist 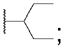
Nummer A126: R¹ ist H, R² ist CH₃, R³ ist H, R⁴ ist H, R⁵ ist CH₃, R⁶ ist 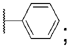
Nummer A127: R¹ ist H, R² ist CH₃, R³ ist H, R⁴ ist H, R⁵ ist CH₃, R⁶ ist 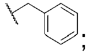
Nummer A128: R¹ ist H, R² ist CH₃, R³ ist H, R⁴ ist H, R⁵ ist CH₃, R⁶ ist -COCH₃;
Nummer A129: R¹ ist H, R² ist CH₃, R³ ist H, R⁴ ist H, R⁵ ist CH₃, R⁶ ist -COCH₂CH₃;
Nummer A130: R¹ ist H, R² ist CH₃, R³ ist H, R⁴ ist H, R⁵ ist CH₃, R⁶ ist -COCH₂CH₂CH₃;
Nummer A131: R¹ ist H, R² ist CH₃, R³ ist H, R⁴ ist H, R⁵ ist CH₃, R⁶ ist -COCH(CH₃)₂;
Nummer A132: R¹ ist H, R² ist CH₃, R³ ist H, R⁴ ist H, R⁵ ist CH₃, R⁶ ist -COC₆H₅;
Nummer A133: R¹ ist H, R² ist CH₃, R³ ist H, R⁴ ist H, R⁵ ist CH₃, R⁶ ist 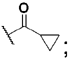
Nummer A134: R¹ ist H, R² ist CH₃, R³ ist H, R⁴ ist H, R⁵ ist CH₃, R⁶ ist 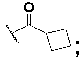
Nummer A135: R¹ ist H, R² ist CH₃, R³ ist H, R⁴ ist H, R⁵ ist CH₃, R⁶ ist 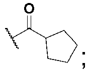
Nummer A136: R¹ ist H, R² ist CH₃, R³ ist H, R⁴ ist H, R⁵ ist CH₃, R⁶ ist 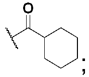
Nummer A137: R¹ ist H, R² ist CH₃, R³ ist H, R⁴ ist H, R⁵ ist CH₃, R⁶ ist 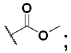
Nummer A138: R¹ ist H, R² ist CH₃, R³ ist H, R⁴ ist H, R⁵ ist CH₃, R⁶ ist 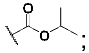
Nummer A139: R¹ ist H, R² ist CH₃, R³ ist H, R⁴ ist H, R⁵ ist CH₃, R⁶ ist 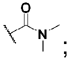
Nummer A140: R¹ ist H, R² ist CH₃, R³ ist H, R⁴ ist H, R⁵ ist CH₃, R⁶ ist 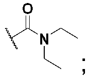
Nummer A141: R¹ ist H, R² ist CH₃, R³ ist H, R⁴ ist H, R⁵ ist CH₃, R⁶ ist -S(=O)₂CH₃;
Nummer A142: R¹ ist H, R² ist CH₃, R³ ist H, R⁴ ist H, R⁵ ist CH₃, R⁶ ist -S(=O)₂CH₂CH₃;
Nummer A143: R¹ ist H, R² ist CH₃, R³ ist H, R⁴ ist H, R⁵ ist CH₃, R⁶ ist -S(=O)₂CH₂CH₂CH₃;
Nummer A144: R¹ ist H, R² ist CH₃, R³ ist H, R⁴ ist H, R⁵ ist CH₃, R⁶ ist -S(=O)₂CH(CH₃)₂;
Nummer A145: R¹ ist H, R² ist CH₃, R³ ist H, R⁴ ist H, R⁵ ist CH₃, R⁶ ist -S(=O)₂C₆H₅;
Nummer A146: R¹ ist H, R² ist CH₃, R³ ist H, R⁴ ist H, R⁵ ist CH₃, R⁶ ist 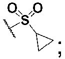
Nummer A147: R¹ ist H, R² ist CH₃, R³ ist H, R⁴ ist H, R⁵ ist CH₃, R⁶ ist 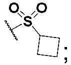
Nummer A148: R¹ ist H, R² ist CH₃, R³ ist H, R⁴ ist H, R⁵ ist CH₃, R⁶ ist 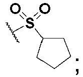
Nummer A149: R¹ ist H, R² ist CH₃, R³ ist H, R⁴ ist H, R⁵ ist CH₃, R⁶ ist 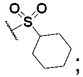
Nummer A150: R¹ ist H, R² ist CH₃, R³ ist H, R⁴ ist CH₃, R⁵ ist CH₃, R⁶ ist -H;
Nummer A151: R¹ ist H, R² ist CH₃, R³ ist H, R⁴ ist CH₃, R⁵ ist CH₃, R⁶ ist -CH₃;
Nummer A152: R¹ ist H, R² ist CH₃, R³ ist H, R⁴ ist CH₃, R⁵ ist CH₃, R⁶ ist -CH₂CH₃;
Nummer A153: R¹ ist H, R² ist CH₃, R³ ist H, R⁴ ist CH₃, R⁵ ist CH₃, R⁶ ist -CH₂CH₂CH₃;
Nummer A154: R¹ ist H, R² ist CH₃, R³ ist H, R⁴ ist CH₃, R⁵ ist CH₃, R⁶ ist -CH(CH₃)₂;
Nummer A155: R¹ ist H, R² ist CH₃, R³ ist H, R⁴ ist CH₃, R⁵ ist CH₃, R⁶ ist 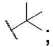
Nummer A156: R¹ ist H, R² ist CH₃, R³ ist H, R⁴ ist CH₃, R⁵ ist CH₃, R⁶ ist 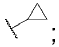
Nummer A157: R¹ ist H, R² ist CH₃, R³ ist H, R⁴ ist CH₃, R⁵ ist CH₃, R⁶ ist 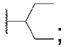
Nummer A158: R¹ ist H, R² ist CH₃, R³ ist H, R⁴ ist CH₃, R⁵ ist CH₃, R⁶ ist 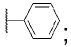
Nummer A159: R¹ ist H, R² ist CH₃, R³ ist H, R⁴ ist CH₃, R⁵ ist CH₃, R⁶ ist 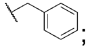
Nummer A160: R¹ ist H, R² ist CH₃, R³ ist H, R⁴ ist CH₃, R⁵ ist CH₃, R⁶ ist -COCH₃;
Nummer A161: R¹ ist H, R² ist CH₃, R³ ist H, R⁴ ist CH₃, R⁵ ist CH₃, R⁶ ist -COCH₂CH₃;
Nummer A162: R¹ ist H, R² ist CH₃, R³ ist H, R⁴ ist CH₃, R⁵ ist CH₃, R⁶ ist -COCH₂CH₂CH₃;
Nummer A163: R¹ ist H, R² ist CH₃, R³ ist H, R⁴ ist CH₃, R⁵ ist CH₃, R⁶ ist -COCH(CH₃)₂;
Nummer A164: R¹ ist H, R² ist CH₃, R³ ist H, R⁴ ist CH₃, R⁵ ist CH₃, R⁶ ist -COC₆H₅;
Nummer A165: R¹ ist H, R² ist CH₃, R³ ist H, R⁴ ist CH₃, R⁵ ist CH₃, R⁶ ist 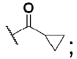
Nummer A166: R¹ ist H, R² ist CH₃, R³ ist H, R⁴ ist CH₃, R⁵ ist CH₃, R⁶ ist 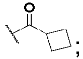
Nummer A167: R¹ ist H, R² ist CH₃, R³ ist H, R⁴ ist CH₃, R⁵ ist CH₃, R⁶ ist 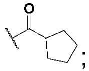
Nummer A168: R¹ ist H, R² ist CH₃, R³ ist H, R⁴ ist CH₃, R⁵ ist CH₃, R⁶ ist 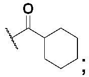
Nummer A169: R¹ ist H, R² ist CH₃, R³ ist H, R⁴ ist CH₃, R⁵ ist CH₃, R⁶ ist 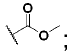
Nummer A170: R¹ ist H, R² ist CH₃, R³ ist H, R⁴ ist CH₃, R⁵ ist CH₃, R⁶ ist 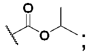
Nummer A171: R¹ ist H, R² ist CH₃, R³ ist H, R⁴ ist CH₃, R⁵ ist CH₃, R⁶ ist 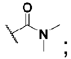
Nummer A172: R¹ ist H, R² ist CH₃, R³ ist H, R⁴ ist CH₃, R⁵ ist CH₃, R⁶ ist 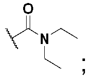
Nummer A173: R¹ ist H, R² ist CH₃, R³ ist H, R⁴ ist CH₃, R⁵ ist CH₃, R⁶ ist -S(=O)₂CH₃;
Nummer A174: R¹ ist H, R² ist CH₃, R³ ist H, R⁴ ist CH₃, R⁵ ist CH₃, R⁶ ist -S(=O)₂CH₂CH₃;
Nummer A175: R¹ ist H, R² ist CH₃, R³ ist H, R⁴ ist CH₃, R⁵ ist CH₃, R⁶ ist -S(=O)₂CH₂CH₂CH₃;
Nummer A176: R¹ ist H, R² ist CH₃, R³ ist H, R⁴ ist CH₃, R⁵ ist CH₃, R⁶ ist -S(=O)₂CH(CH₃)₂;
Nummer A177: R¹ ist H, R² ist CH₃, R³ ist H, R⁴ ist CH₃, R⁵ ist CH₃, R⁶ ist -S(=O)₂C₆H₅;
Nummer A178: R¹ ist H, R² ist CH₃, R³ ist H, R⁴ ist CH₃, R⁵ ist CH₃, R⁶ ist 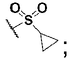
Nummer A179: R¹ ist H, R² ist CH₃, R³ ist H, R⁴ ist CH₃, R⁵ ist CH₃, R⁶ ist 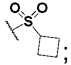
Nummer A180: R¹ ist H, R² ist CH₃, R³ ist H, R⁴ ist CH₃, R⁵ ist CH₃, R⁶ ist 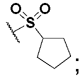
Nummer A181: R¹ ist H, R² ist CH₃, R³ ist H, R⁴ ist CH₃, R⁵ ist CH₃, R⁶ ist 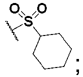
Nummer A182: R¹ ist H, R² ist CH₃, R³ ist CH₃, R⁴ ist H, R⁵ ist H, R⁶ ist 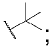
Nummer A183: R¹ ist H, R² ist CH₃, R³ ist OCH₃, R⁴ ist H, R⁵ ist H, R⁶ ist 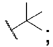
Nummer A184: R¹ ist H, R² ist CH₃, R³ ist 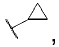 R⁴ ist H, R⁵ ist H, R⁶ ist 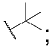
Nummer A185: R¹ ist H, R² ist CH₃, R³ ist COCH₃, R⁴ ist H, R⁵ ist H, R⁶ ist 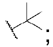
Nummer A186: R¹ ist H, R² ist CH₃, R³ ist 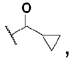 R⁴ ist H, R⁵ ist H, R⁶ ist 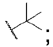
Nummer A187: R¹ ist H, R² ist CH₃, R³ ist 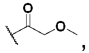 R⁴ ist H, R⁵ ist H, R⁶ ist 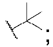
Nummer A188: R¹ ist H, R² ist CH₃, R³ ist 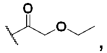 R⁴ ist H, R⁵ ist H, R⁶ ist 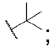
Nummer A189: R¹ ist H, R² ist CN, R³ ist H, R⁴ ist H, R⁵ ist H, R⁶ ist 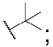
Nummer A190: R¹ ist F, R² ist CH₃, R³ ist H, R⁴ ist H, R⁵ ist H, R⁶ ist -H;
Nummer A191: R¹ ist F, R² ist CH₃, R³ ist H, R⁴ ist H, R⁵ ist H, R⁶ ist -CH₃;
Nummer A192: R¹ ist F, R² ist CH₃, R³ ist H, R⁴ ist H, R⁵ ist H, R⁶ ist -CH₂CH₃;
Nummer A193: R¹ ist F, R² ist CH₃, R³ ist H, R⁴ ist H, R⁵ ist H, R⁶ ist -CH₂CH₂CH₃;
Nummer A194: R¹ ist F, R² ist CH₃, R³ ist H, R⁴ ist H, R⁵ ist H, R⁶ ist -CH(CH₃)₂;
Nummer A195: R¹ ist F, R² ist CH₃, R³ ist H, R⁴ ist H, R⁵ ist H, R⁶ ist 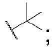
Nummer A196: R¹ ist F, R² ist CH₃, R³ ist H, R⁴ ist H, R⁵ ist H, R⁶ ist 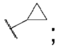
Nummer A197: R¹ ist F, R² ist CH₃, R³ ist H, R⁴ ist H, R⁵ ist H, R⁶ ist 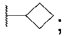
Nummer A198: R¹ ist F, R² ist CH₃, R³ ist H, R⁴ ist H, R⁵ ist H, R⁶ ist 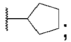
Nummer A199: R¹ ist F, R² ist CH₃, R³ ist H, R⁴ ist H, R⁵ ist H, R⁶ ist 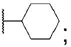
Nummer A200: R¹ ist F, R² ist CH₃, R³ ist H, R⁴ ist H, R⁵ ist H, R⁶ ist 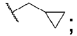
Nummer A201: R¹ ist F, R² ist CH₃, R³ ist H, R⁴ ist H, R⁵ ist H, R⁶ ist 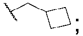
Nummer A202: R¹ ist F, R² ist CH₃, R³ ist H, R⁴ ist H, R⁵ ist H, R⁶ ist 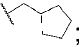
Nummer A203: R¹ ist F, R² ist CH₃, R³ ist H, R⁴ ist H, R⁵ ist H, R⁶ ist 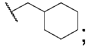
Nummer A204: R¹ ist F, R² ist CH₃, R³ ist H, R⁴ ist H, R⁵ ist H, R⁶ ist 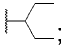
Nummer A205: R¹ ist F, R² ist CH₃, R³ ist H, R⁴ ist H, R⁵ ist H, R⁶ ist 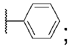
Nummer A206: R¹ ist F, R² ist CH₃, R³ ist H, R⁴ ist H, R⁵ ist H, R⁶ ist 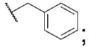
Nummer A207: R¹ ist F, R² ist CH₃, R³ ist H, R⁴ ist H, R⁵ ist H, R⁶ ist -COCH₃;
Nummer A208: R¹ ist F, R² ist CH₃, R³ ist H, R⁴ ist H, R⁵ ist H, R⁶ ist -COCH₂CH₃;
Nummer A209: R¹ ist F, R² ist CH₃, R³ ist H, R⁴ ist H, R⁵ ist H, R⁶ ist -COCH₂CH₂CH₃;
Nummer A210: R¹ ist F, R² ist CH₃, R³ ist H, R⁴ ist H, R⁵ ist H, R⁶ ist -COCH(CH₃)₂;
Nummer A211: R¹ ist F, R² ist CH₃, R³ ist H, R⁴ ist H, R⁵ ist H, R⁶ ist -COC₆H₅;
Nummer A212: R¹ ist F, R² ist CH₃, R³ ist H, R⁴ ist H, R⁵ ist H, R⁶ ist 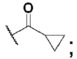
Nummer A213: R¹ ist F, R² ist CH₃, R³ ist H, R⁴ ist H, R⁵ ist H, R⁶ ist 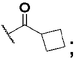
Nummer A214: R¹ ist F, R² ist CH₃, R³ ist H, R⁴ ist H, R⁵ ist H, R⁶ ist 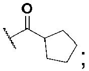
Nummer A215: R¹ ist F, R² ist CH₃, R³ ist H, R⁴ ist H, R⁵ ist H, R⁶ ist 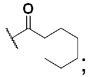
Nummer A216: R¹ ist F, R² ist CH₃, R³ ist H, R⁴ ist H, R⁵ ist H, R⁶ ist 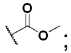
Nummer A217: R¹ ist F, R² ist CH₃, R³ ist H, R⁴ ist H, R⁵ ist H, R⁶ ist 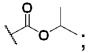
Nummer A218: R¹ ist F, R² ist CH₃, R³ ist H, R⁴ ist H, R⁵ ist H, R⁶ ist 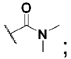
Nummer A219: R¹ ist F, R² ist CH₃, R³ ist H, R⁴ ist H, R⁵ ist H, R⁶ ist 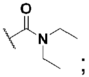
Nummer A220: R¹ ist F, R² ist CH₃, R³ ist H, R⁴ ist H, R⁵ ist H, R⁶ ist -S(=O)₂CH₃;
Nummer A221: R¹ ist F, R² ist CH₃, R³ ist H, R⁴ ist H, R⁵ ist H, R⁶ ist -S(=O)₂CH₂CH₃;
Nummer A222: R¹ ist F, R² ist CH₃, R³ ist H, R⁴ ist H, R⁵ ist H, R⁶ ist -S(=O)₂CH₂CH₂CH₃;
Nummer A223: R¹ ist F, R² ist CH₃, R³ ist H, R⁴ ist H, R⁵ ist H, R⁶ ist -S(=O)₂CH(CH₃)₂;
Nummer A224: R¹ ist F, R² ist CH₃, R³ ist H, R⁴ ist H, R⁵ ist H, R⁶ ist -S(=O)₂C₆H₅;
Nummer A225: R¹ ist F, R² ist CH₃, R³ ist H, R⁴ ist H, R⁵ ist H, R⁶ ist 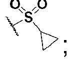
Nummer A226: R¹ ist F, R² ist CH₃, R³ ist H, R⁴ ist H, R⁵ ist H, R⁶ ist 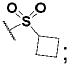
Nummer A227: R¹ ist F, R² ist CH₃, R³ ist H, R⁴ ist H, R⁵ ist H, R⁶ ist 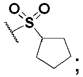
Nummer A228: R¹ ist F, R² ist CH₃, R³ ist H, R⁴ ist H, R⁵ ist H, R⁶ ist 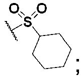
Nummer A229: R¹ ist F, R² ist CH₃, R³ ist H, R⁴ ist H, R⁵ ist CH₃, R⁶ ist -H;
Nummer A230: R¹ ist F, R² ist CH₃, R³ ist H, R⁴ ist H, R⁵ ist CH₃, R⁶ ist -CH₃;
Nummer A231: R¹ ist F, R² ist CH₃, R³ ist H, R⁴ ist H, R⁵ ist CH₃, R⁶ ist -CH₂CH₃;
Nummer A232: R¹ ist F, R² ist CH₃, R³ ist H, R⁴ ist H, R⁵ ist CH₃, R⁶ ist -CH₂CH₂CH₃;
Nummer A233: R¹ ist F, R² ist CH₃, R³ ist H, R⁴ ist H, R⁵ ist CH₃, R⁶ ist -CH(CH₃)₂;
Nummer A234: R¹ ist F, R² ist CH₃, R³ ist H, R⁴ ist H, R⁵ ist CH₃, R⁶ ist 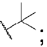
Nummer A235: R¹ ist F, R² ist CH₃, R³ ist H, R⁴ ist H, R⁵ ist CH₃, R⁶ ist 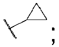
Nummer A236: R¹ ist F, R² ist CH₃, R³ ist H, R⁴ ist H, R⁵ ist CH₃, R⁶ ist 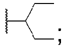
Nummer A237: R¹ ist F, R² ist CH₃, R³ ist H, R⁴ ist H, R⁵ ist CH₃, R⁶ ist 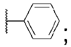
Nummer A238: R¹ ist F, R² ist CH₃, R³ ist H, R⁴ ist H, R⁵ ist CH₃, R⁶ ist 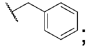
Nummer A239: R¹ ist F, R² ist CH₃, R³ ist H, R⁴ ist H, R⁵ ist CH₃, R⁶ ist -COCH₃;
Nummer A240: R¹ ist F, R² ist CH₃, R³ ist H, R⁴ ist H, R⁵ ist CH₃, R⁶ ist -COCH₂CH₃;
Nummer A241: R¹ ist F, R² ist CH₃, R³ ist H, R⁴ ist H, R⁵ ist CH₃, R⁶ ist -COCH₂CH₂CH₃;
Nummer A242: R¹ ist F, R² ist CH₃, R³ ist H, R⁴ ist H, R⁵ ist CH₃, R⁶ ist -COCH(CH₃)₂;
Nummer A243: R¹ ist F, R² ist CH₃, R³ ist H, R⁴ ist H, R⁵ ist CH₃, R⁶ ist -COC₆H₅;
Nummer A244: R¹ ist F, R² ist CH₃, R³ ist H, R⁴ ist H, R⁵ ist CH₃, R⁶ ist 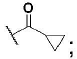
Nummer A245: R¹ ist F, R² ist CH₃, R³ ist H, R⁴ ist H, R⁵ ist CH₃, R⁶ ist 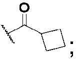
Nummer A246: R¹ ist F, R² ist CH₃, R³ ist H, R⁴ ist H, R⁵ ist CH₃, R⁶ ist 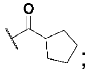
Nummer A247: R¹ ist F, R² ist CH₃, R³ ist H, R⁴ ist H, R⁵ ist CH₃, R⁶ ist 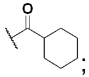
Nummer A248: R¹ ist F, R² ist CH₃, R³ ist H, R⁴ ist H, R⁵ ist CH₃, R⁶ ist 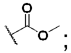
Nummer A249: R¹ ist F, R² ist CH₃, R³ ist H, R⁴ ist H, R⁵ ist CH₃, R⁶ ist 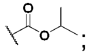
Nummer A250: R¹ ist F, R² ist CH₃, R³ ist H, R⁴ ist H, R⁵ ist CH₃, R⁶ ist 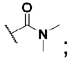
Nummer A251: R¹ ist F, R² ist CH₃, R³ ist H, R⁴ ist H, R⁵ ist CH₃, R⁶ ist 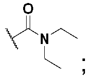
Nummer A252: R¹ ist F, R² ist CH₃, R³ ist H, R⁴ ist H, R⁵ ist CH₃, R⁶ ist -S(=O)₂CH₃;
Nummer A253: R¹ ist F, R² ist CH₃, R³ ist H, R⁴ ist H, R⁵ ist CH₃, R⁶ ist -S(=O)₂CH₂CH₃;
Nummer A254: R¹ ist F, R² ist CH₃, R³ ist H, R⁴ ist H, R⁵ ist CH₃, R⁶ ist -S(=O)₂CH₂CH₂CH₃;
Nummer A255: R¹ ist F, R² ist CH₃, R³ ist H, R⁴ ist H, R⁵ ist CH₃, R⁶ ist -S(=O)₂CH(CH₃)₂;
Nummer A256: R¹ ist F, R² ist CH₃, R³ ist H, R⁴ ist H, R⁵ ist CH₃, R⁶ ist -S(=O)₂C₆H₅;
Nummer A257: R¹ ist F, R² ist CH₃, R³ ist H, R⁴ ist H, R⁵ ist CH₃, R⁶ ist 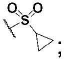
Nummer A258: R¹ ist F, R² ist CH₃, R³ ist H, R⁴ ist H, R⁵ ist CH₃, R⁶ ist 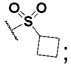
Nummer A259: R¹ ist F, R² ist CH₃, R³ ist H, R⁴ ist H, R⁵ ist CH₃, R⁶ ist 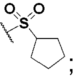
Nummer A260: R¹ ist F, R² ist CH₃, R³ ist H, R⁴ ist H, R⁵ ist CH₃, R⁶ ist 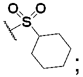
Nummer A261: R¹ ist F, R² ist CH₃, R³ ist H, R⁴ ist CH₃, R⁵ ist CH₃, R⁶ ist -H;
Nummer A262: R¹ ist F, R² ist CH₃, R³ ist H, R⁴ ist CH₃, R⁵ ist CH₃, R⁶ ist -CH₃;
Nummer A263: R¹ ist F, R² ist CH₃, R³ ist H, R⁴ ist CH₃, R⁵ ist CH₃, R⁶ ist -CH₂CH₃;
Nummer A264: R¹ ist F, R² ist CH₃, R³ ist H, R⁴ ist CH₃, R⁵ ist CH₃, R⁶ ist -CH₂CH₂CH₃;
Nummer A265: R¹ ist F, R² ist CH₃, R³ ist H, R⁴ ist CH₃, R⁵ ist CH₃, R⁶ ist -CH(CH₃)₂;
Nummer A266: R¹ ist F, R² ist CH₃, R³ ist H, R⁴ ist CH₃, R⁵ ist CH₃, R⁶ ist 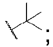
Nummer A267: R¹ ist F, R² ist CH₃, R³ ist H, R⁴ ist CH₃, R⁵ ist CH₃, R⁶ ist 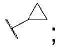
Nummer A268: R¹ ist F, R² ist CH₃, R³ ist H, R⁴ ist CH₃, R⁵ ist CH₃, R⁶ ist 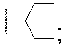
Nummer A269: R¹ ist F, R² ist CH₃, R³ ist H, R⁴ ist CH₃, R⁵ ist CH₃, R⁶ ist 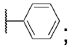
Nummer A270: R¹ ist F, R² ist CH₃, R³ ist H, R⁴ ist CH₃, R⁵ ist CH₃, R⁶ ist 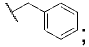
Nummer A271: R¹ ist F, R² ist CH₃, R³ ist H, R⁴ ist CH₃, R⁵ ist CH₃, R⁶ ist -COCH₃;
Nummer A272: R¹ ist F, R² ist CH₃, R³ ist H, R⁴ ist CH₃, R⁵ ist CH₃, R⁶ ist -COCH₂CH₃;
Nummer A273: R¹ ist F, R² ist CH₃, R³ ist H, R⁴ ist CH₃, R⁵ ist CH₃, R⁶ ist -COCH₂CH₂CH₃;
Nummer A274: R¹ ist F, R² ist CH₃, R³ ist H, R⁴ ist CH₃, R⁵ ist CH₃, R⁶ ist -COCH(CH₃)₂;
Nummer A275: R¹ ist F, R² ist CH₃, R³ ist H, R⁴ ist CH₃, R⁵ ist CH₃, R⁶ ist -COC₆H₅;
Nummer A276: R¹ ist F, R² ist CH₃, R³ ist H, R⁴ ist CH₃, R⁵ ist CH₃, R⁶ ist 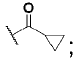
Nummer A277: R¹ ist F, R² ist CH₃, R³ ist H, R⁴ ist CH₃, R⁵ ist CH₃, R⁶ ist 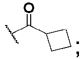
Nummer A278: R¹ ist F, R² ist CH₃, R³ ist H, R⁴ ist CH₃, R⁵ ist CH₃, R⁶ ist 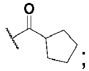
Nummer A279: R¹ ist F, R² ist CH₃, R³ ist H, R⁴ ist CH₃, R⁵ ist CH₃, R⁶ ist 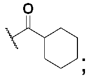
Nummer A280: R¹ ist F, R² ist CH₃, R³ ist H, R⁴ ist CH₃, R⁵ ist CH₃, R⁶ ist 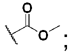
Nummer A281: R¹ ist F, R² ist CH₃, R³ ist H, R⁴ ist CH₃, R⁵ ist CH₃, R⁶ ist 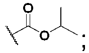
Nummer A282: R¹ ist F, R² ist CH₃, R³ ist H, R⁴ ist CH₃, R⁵ ist CH₃, R⁶ ist 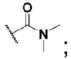
Nummer A283: R¹ ist F, R² ist CH₃, R³ ist H, R⁴ ist CH₃, R⁵ ist CH₃, R⁶ ist 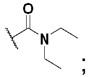
Nummer A284: R¹ ist F, R² ist CH₃, R³ ist H, R⁴ ist CH₃, R⁵ ist CH₃, R⁶ ist -S(=O)₂CH₃;
Nummer A285: R¹ ist F, R² ist CH₃, R³ ist H, R⁴ ist CH₃, R⁵ ist CH₃, R⁶ ist -S(=O)₂CH₂CH₃;
Nummer A286: R¹ ist F, R² ist CH₃, R³ ist H, R⁴ ist CH₃, R⁵ ist CH₃, R⁶ ist -S(=O)₂CH₂CH₂CH₃;
Nummer A287: R¹ ist F, R² ist CH₃, R³ ist H, R⁴ ist CH₃, R⁵ ist CH₃, R⁶ ist -S(=O)₂CH(CH₃)₂;
Nummer A288: R¹ ist F, R² ist CH₃, R³ ist H, R⁴ ist CH₃, R⁵ ist CH₃, R⁶ ist -S(=O)₂C₆H₅;
Nummer A289: R¹ ist F, R² ist CH₃, R³ ist H, R⁴ ist CH₃, R⁵ ist CH₃, R⁶ ist 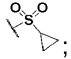
Nummer A290: R¹ ist F, R² ist CH₃, R³ ist H, R⁴ ist CH₃, R⁵ ist CH₃, R⁶ ist 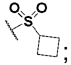
Nummer A291: R¹ ist F, R² ist CH₃, R³ ist H, R⁴ ist CH₃, R⁵ ist CH₃, R⁶ ist 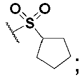
Nummer A292: R¹ ist F, R² ist CH₃, R³ ist H, R⁴ ist CH₃, R⁵ ist CH₃, R⁶ ist 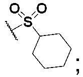
Nummer A293: R¹ ist F, R² ist CH₃, R³ ist CH₃, R⁴ ist H, R⁵ ist H, R⁶ ist 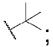
Nummer A294: R¹ ist F, R² ist CH₃, R³ ist OCH₃, R⁴ ist H, R⁵ ist H, R⁶ ist 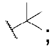
Nummer A295: R¹ ist F, R² ist CH₃, R³ ist 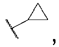 R⁴ ist H, R⁵ ist H, R⁶ ist 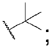
Nummer A296: R¹ ist F, R² ist CH₃, R³ ist COCH₃, R⁴ ist H, R⁵ ist H, R⁶ ist 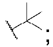
Nummer A297: R¹ ist F, R² ist CH₃, R³ ist 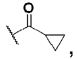 R⁴ ist H, R⁵ ist H, R⁶ ist 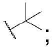
Nummer A298: R¹ ist F, R² ist CH₃, R³ ist 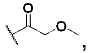 R⁴ ist H, R⁵ ist H, R⁶ ist 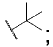
Nummer A299: R¹ ist F, R² ist CH₃, R³ ist 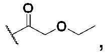 R⁴ ist H, R⁵ ist H, R⁶ ist 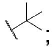
Nummer A300: R¹ ist F, R² ist CN, R³ ist H, R⁴ ist H, R⁵ ist H, R⁶ ist 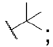
Nummer A301: R¹ ist H, R² ist CH₃, R³ ist H, R⁴ ist H, R⁵ ist H, R⁶ ist -CH₂OCH₃.

5. Nichttherapeutische Verwendung einer Verbindung, die eine Pyrazolstruktur gemäß einem der Ansprüche 1 bis 4 umfasst, oder eines agrochemisch akzeptablen Salzes, Hydrats oder Solvats davon zur Abtötung von mindestens einem der folgenden Schädlinge: Insekten, Milben, Nematoden und stechend-saugende Insekten.

6. Nichttherapeutische Verwendung einer Verbindung, die eine Pyrazolstruktur gemäß einem der Ansprüche 1 bis 4 umfasst, oder eines agrochemisch akzeptablen Salzes, Hydrats oder Solvats davon als agrochemisches Insektizid.

7. Ein Insektizid, das als Wirkstoff eine pestizidwirksame Menge mindestens einer Verbindung mit einer Pyrazolstruktur gemäß einem der Ansprüche 1 bis 4 oder eines agrochemisch akzeptablen Salzes, Hydrats oder Solvats davon enthält.

8. Das Insektizid nach Anspruch 7, wobei der Wirkstoff in dem Insektizid in einer Menge von 1 bis 99,9 Gew.-% vorliegt.

9. Das Insektizid nach Anspruch 8, wobei der Wirkstoff in dem Insektizid in einer Menge von 5 bis 95 Gew.-% vorliegt.

10. Insektizid nach einem der Ansprüche 7 bis 9, wobei das Insektizid in einer Form vorliegt, die aus mindestens einer der folgenden ausgewählt ist: emulgierbares Konzentrat, Suspension, benetzbares Pulver, Staubpulver, Granulat, wässrige Lösung, Giftköder, Mutterlauge und Mutterpulver.

## Revendications

1. Composé ayant une structure de pyrazole ou un de ses sels, hydrates et solvates acceptables sur le plan agrochimique, dans lequel le composé présente la structure représentée par la formule (I) : 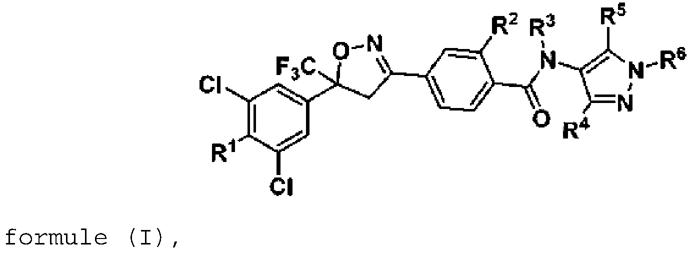
dans laquelle, dans la formule (I),
R¹ est sélectionné parmi H, un halogène, un alkyle en C₁₋₆ substitué avec au moins un halogène, un alcoxy en C₁₋₆ substitué avec au moins un halogène ;
R² est sélectionné parmi un alkyle en C₁₋₁₂, un alcoxy en C₁₋₁₂, un halogène, un alkyle en C₁₋₆ substitué avec au moins un halogène, un alcoxy en C₁₋₆ substitué avec au moins un halogène, et un cyano ;
R³ est sélectionné parmi H, un alkyle en C₁₋₁₂, un alcoxy en C₁₋₁₂, un cycloalkyle en C₃₋₁₂, -CO-R₃₁, -CO-CH₂ -R₃₁ ; R₃₁ est sélectionné parmi un alkyle en C₁₋₁₂, un cycloalkyle en C₃₋₁₂ et un alcoxy en C₁₋₁₂ ;
R⁴, R⁵ sont sélectionnés respectivement et indépendamment parmi H, un alkyle en C₁₋₁₂, un alcoxy en C₁₋₁₂, un halogène, un alkyle en C₁₋₆ substitué avec au moins un halogène, et un alcoxy en C₁₋₆ substitué avec au moins un halogène ;
R⁶ est sélectionné dans le groupe consistant en H, un alkyle en C₁₋₁₂ non substitué ou substitué avec au moins un groupe parmi un hydroxyle, un halogène, un alkyle en C₁₋₁₂, un alcoxy en C₁₋₁₂ et un triméthylsilyle, un alcoxy en C₁₋₁₂ non substitué ou substitué avec au moins un groupe parmi un hydroxyle, un halogène, un alkyle en C₁₋₁₂, un alcoxy en C₁₋₁₂ et un triméthylsilyle, un cycloalkyle en C₃₋₁₂ non substitué ou substitué avec au moins un groupe parmi un hydroxyle, un halogène, un alkyle en C₁₋₁₂, un alcoxy en C₁₋₁₂ et un triméthylsilyle, un phényle non substitué ou substitué avec au moins un groupe parmi un hydroxyle, un halogène, un alkyle en C₁₋₁₂, un alcoxy en C₁₋₁₂ et un triméthylsilyle, un cycloalkyle en C₃₋₁₂ contenant au moins un hétéroatome en tant qu'atome formant un cycle, un hydrocarbure en C₂₋₁₂ contenant au moins une double liaison carbone-carbone insaturée ou une triple liaison carbone-carbone insaturée qui est non substitué ou substitué avec au moins un groupe parmi un hydroxyle, un halogène, un alkyle en C₁₋₁₂, un alcoxy en C₁₋₁₂ et un triméthylsilyle, - (CH₂)ₙ-R₆₁, -CO-R₆₂, -COO-R₆₂, -CO-N(R₆₃R₆₄), -S(=O)₂-R₆₂, - (CH₂)ₙ-CO-R₆₂, R₆₁ est sélectionné dans le groupe consistant en un cycloalkyle en C₃₋₁₂, un cycloalkyle en C₃₋₁₂ contenant au moins un hétéroatome en tant qu'atome formant un cycle, un phényle, R₆₂ est sélectionné dans le groupe consistant en un alkyle en C₁₋₁₂, un phényle non substitué ou substitué avec au moins l'un parmi un hydroxyle, un halogène, un alkyle en C₁₋₁₂, un alcoxy en C₁₋₁₂ et un triméthylsilyle, un cycloalkyle en C₃₋₁₂, un cycloalkyle en C₃₋₁₂ contenant au moins un hétéroatome en tant qu'atome formant un cycle ; R₆₃, R₆₄ sont sélectionnés indépendamment parmi un alkyle en C₁₋₁₂ ; chacun des hétéroatomes est indépendamment au moins un sélectionné parmi N, S ; chaque n est indépendamment 1 ou 2.

2. Composé selon la revendication 1, dans lequel, dans la formule (I),
R¹ est sélectionné parmi H, un halogène, un alkyle en C₁₋₆ substitué avec au moins un halogène, un alcoxy en C₁₋₆ substitué avec au moins un halogène ;
R² est sélectionné parmi un alkyle en C₁₋₈, un alcoxy en C₁₋₈, un halogène, un alkyle en C₁₋₆ substitué avec au moins un halogène, un alcoxy en C₁₋₆ substitué avec au moins un halogène, et un cyano ;
R³ est sélectionné parmi H, un alkyle en C₁₋₈, un alcoxy en C₁₋₈, un cycloalkyle en C₃₋₁₀, -CO-R₃₁, -CO-CH₂ -R₃₁ ; R₃₁ est sélectionné parmi un alkyle en C₁₋₈, un cycloalkyle en C₃₋₁₀ et un alcoxy en C₁₋₈ ;
R⁴ et R⁵ sont sélectionnés respectivement et indépendamment parmi H, un alkyle en C₁₋₈, un alcoxy en C₁₋₈, un halogène, un alkyle en C₁₋₆ substitué avec au moins un halogène, et un alcoxy en C₁₋₆ substitué avec au moins un halogène ;
R⁶ est sélectionné dans le groupe consistant en H, un alkyle en C₁₋₁₀ non substitué ou substitué avec au moins un groupe parmi un hydroxyle, un halogène, un alkyle en C₁₋₈, un alcoxy en C₁₋₈ et un triméthylsilyle, un alcoxy en C₁₋₁₀ non substitué ou substitué avec au moins un groupe parmi un hydroxyle, un halogène, un alkyle en C₁₋₈, un alcoxy en C₁₋₈ et un triméthylsilyle, un cycloalkyle en C_{3 -10} non substitué ou substitué avec au moins un groupe parmi un hydroxyle, un halogène, un alkyle en C₁₋₈, un alcoxy en C₁₋₈ et un triméthylsilyle, un phényle non substitué ou substitué avec au moins un groupe parmi un hydroxyle, un halogène, un alkyle en C₁₋₈, un alcoxy en C₁₋₈ et un triméthylsilyle, un cycloalkyle en C₃₋₁₀ contenant au moins un hétéroatome en tant qu'atome formant un cycle, un hydrocarbure en C₂₋₁₀ contenant au moins une double liaison carbone-carbone insaturée ou une triple liaison carbone-carbone insaturée qui est non substitué ou substitué avec au moins un groupe parmi un hydroxyle, un halogène, un alkyle en C₁₋₈, un alcoxy en C₁₋₈ et un triméthylsilyle, - (CH₂)ₙ-R₆₁, -CO-R₆₂, -COO-R₆₂, -CO-N(R₆₃R₆₄), -S(=O)₂-R₆₂, - (CH₂)ₙ-CO-R₆₂ ; R₆₁ est sélectionné dans le groupe consistant en un cycloalkyle en C₃₋₁₀, un cycloalkyle en C₃₋₁₀ contenant au moins un hétéroatome en tant qu'atome formant un cycle, un phényle ; R₆₂ est sélectionné dans le groupe consistant en un alkyle en C₁₋₁₀, un phényle non substitué ou substitué avec au moins l'un des groupes hydroxyle, halogène, alkyle en C₁₋₈, alcoxy en C₁₋₈ et triméthylsilyle, un cycloalkyle en C₃₋₁₀, un cycloalkyle en C₃₋₁₀ contenant au moins un hétéroatome en tant qu'atome formant un cycle ; R₆₃ et R₆₄ sont sélectionnés indépendamment parmi un alkyle en C₁₋₁₀ ; chacun des hétéroatomes est indépendamment au moins un sélectionné parmi N, O, S ; chaque n est indépendamment 1 ou 2.

3. Composé selon la revendication 2, dans lequel, dans la formule (I),
R¹ est sélectionné parmi H, F, un chloro, un bromo ;
R² est sélectionné parmi un méthyle, un éthyle, un n-propyle, un isopropyle, un n-butyle, un tert-butyle, un cyano ;
R³ est sélectionné dans le groupe consistant en H, un méthyle, un éthyle, un n-propyle, un isopropyle, un n-butyle, un tert-butyle, un méthoxy, un éthoxy, un n-propoxy, un isopropoxy, un n-butoxy, un tert-butoxy, un cyclopropyle, un cyclobutyle, un cyclopentyle, un cyclohexyle, -CO-R₃₁, -CO-CH₂ -R₃₁ ; R₃₁ est sélectionné parmi un alkyle en C₁₋₆, un cycloalkyle en C₃₋₆ et un alcoxy en C₁₋₆ ;
R⁴, R⁵ sont sélectionnés chacun indépendamment parmi H, un méthyle, un éthyle, un n-propyle, un isopropyle, un n-butyle, un tert-butyle ;
R⁶ est sélectionné dans le groupe consistant en H, un alkyle en C₁₋₁₀ non substitué ou substitué avec au moins un groupe parmi un hydroxyle, un halogène, un alkyle en C₁₋₈, un alcoxy en C₁₋₈ et un triméthylsilyle, un alcoxy en C₁₋₁₀ non substitué ou substitué avec au moins un groupe parmi un hydroxyle, un halogène, un alkyle en C₁₋₈, un alcoxy en C₁₋₈ et un triméthylsilyle, un cycloalkyle en C_{3 -10} non substitué ou substitué avec au moins un groupe parmi un hydroxyle, un halogène, un alkyle en C₁₋₈, un alcoxy en C₁₋₈ et un triméthylsilyle, un phényle non substitué ou substitué avec au moins un groupe parmi un hydroxyle, un halogène, un alkyle en C₁₋₈, un alcoxy en C₁₋₈ et un triméthylsilyle, un cycloalkyle en C₃₋₁₀ contenant au moins un hétéroatome en tant qu'atome formant un cycle, un hydrocarbure en C₂₋₁₀ contenant au moins une double liaison carbone-carbone insaturée ou une triple liaison carbone-carbone insaturée qui est non substitué ou substitué avec au moins un groupe parmi un hydroxyle, un halogène, un alkyle en C₁₋₈, un alcoxy en C₁₋₈ et un triméthylsilyle, - (CH₂)ₙ-R₆₁, -CO-R₆₂, -COO-R₆₂, -CO-N(R₆₃R₆₄), -S(=O)₂-R₆₂, - (CH₂)ₙ-CO-R₆₂ ; R₆₁ est sélectionné dans le groupe consistant en un cycloalkyle en C₃₋₁₀, un cycloalkyle en C₃₋₁₀ contenant au moins un hétéroatome en tant qu'atome formant un cycle, un phényle ; R₆₂ est sélectionné dans le groupe consistant en un alkyle en C₁₋₁₀, un phényle non substitué ou substitué avec au moins l'un des groupes hydroxyle, halogène, alkyle en C₁₋₈, alcoxy en C₁₋₈ et triméthylsilyle, un cycloalkyle en C₃₋₁₀, un cycloalkyle en C₃₋₁₀ contenant au moins un hétéroatome en tant qu'atome formant un cycle ; R₆₃ et R₆₄ sont sélectionnés indépendamment parmi un alkyle en C₁₋₁₀ ; chacun des hétéroatomes est indépendamment au moins un sélectionné parmi N, O, S ; chaque n est indépendamment 1 ou 2.

4. Composé selon la revendication 3, dans lequel le composé est sélectionné parmi l'un quelconque des suivants :
Numéro A1 : R¹ est H, R² est CH₃, R³ est H, R⁴ est H, R⁵ est H, R⁶ est -H ;
Numéro A2 : R¹ est H, R² est CH₃, R³ est H, R⁴ est H, R⁵ est H, R⁶ est -CH₃ ;
Numéro A3 : R¹ est H, R² est CH₃, R³ est H, R⁴ est H, R⁵ est H, R⁶ est -CHF₂ ;
Numéro A4 : R¹ est H, R² est CH₃, R³ est H, R⁴ est H, R⁵ est H, R⁶ est -CF₃ ;
Numéro A5 : R¹ est H, R² est CH₃, R³ est H, R⁴ est H, R⁵ est H, R⁶ est -CH₂CH₃ ;
Numéro A6 : R¹ est H, R² est CH₃, R³ est H, R⁴ est H, R⁵ est H, R⁶ est -CH₂CH₂CH₃ ;
Numéro A7 : R₁ est H, R² est CH₃, R³ est H, R⁴ est H, R⁵ est H, R⁶ est -CH(CH₃)₂ ;
Numéro A8 : R¹ est H, R² est CH₃, R³ est H, R⁴ est H, R⁵ est H, R⁶ est -CH₂CH₂OH ;
Numéro A9 : R¹ est H, R² est CH₃, R³ est H, R⁴ est H, R⁵ est H, R⁶ est -CH₂CF₃ ;
Numéro A10 : R¹ est H, R² est CH₃, R³ est H, R⁴ est H, R⁵ est H, R⁶ est -CH₂CHF₂ ;
Numéro A11 : R¹ est H, R² est CH₃, R³ est H, R⁴ est H, R⁵ est H, R⁶ est -CH₂CH₂F ;
Numéro A12 : R¹ est H, R² est CH₃, R³ est H, R⁴ est H, R⁵ est H, R⁶ est -CH₂CH₂CF₃ ;
Numéro A13 : R¹ est H, R² est CH₃, R³ est H, R⁴ est H, R⁵ est H, R⁶ est -CH2CH2CH2F ;
Numéro A14 : R¹ est H, R² est CH₃, R³ est H, R⁴ est H, R⁵ est H, R⁶ est -CH₂CH₂OCH₃ ;
Numéro A15 : R¹ est H, R² est CH₃, R³ est H, R⁴ est H, R⁵ est H, R⁶ est -CH2CH2OCH2CH3 ;
Numéro A16 : R¹ est H, R² est CH₃, R³ est H, R⁴ est H, R⁵ est H, R⁶ est -CH₂(CH₂)₂OCH₃ ;
Numéro A17 : R¹ est H, R² est CH₃, R³ est H, R⁴ est H, R⁵ est H, R⁶ est -CH₂CH₂CH₂CH₃ ;
Numéro A18 : R¹ est H, R² est CH₃, R³ est H, R⁴ est H, R⁵ est H, R⁶ est -CH(CH₃)CH₂CH₃ ;
Numéro A19 : R¹ est H, R² est CH₃, R³ est H, R⁴ est H, R⁵ est H, R⁶ est -CH₂(CH₂)₃CH₃ ;
Numéro A20 : R¹ est H, R² est CH₃, R³ est H, R⁴ est H, R⁵ est H, R⁶ est 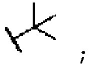
Numéro A21 : R¹ est H, R² est CH₃, R³ est H, R⁴ est H, R⁵ est H, R⁶ est 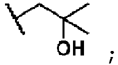
Numéro A22 : R¹ est H, R² est CH₃, R³ est H, R⁴ est H, R⁵ est H, R⁶ est 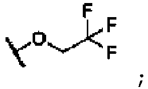
Numéro A23 : R¹ est H, R² est CH₃, R³ est H, R⁴ est H, R⁵ est H, R⁶ est 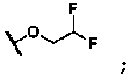
Numéro A24 : R¹ est H, R² est CH₃, R³ est H, R⁴ est H, R⁵ est H, R⁶ est 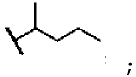
Numéro A25 : R¹ est H, R² est CH₃, R³ est H, R⁴ est H, R⁵ est H, R⁶ est 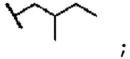
Numéro A26 : R¹ est H, R² est CH₃, R³ est H, R⁴ est H, R⁵ est H, R⁶ est 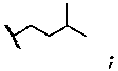
Numéro A27 : R¹ est H, R² est CH₃, R³ est H, R⁴ est H, R⁵ est H, R⁶ est 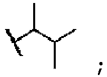
Numéro A28 : R¹ est H, R² est CH₃, R³ est H, R⁴ est H, R⁵ est H, R⁶ est 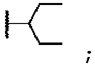
Numéro A29 : R¹ est H, R² est CH₃, R³ est H, R⁴ est H, R⁵ est H, R⁶ est 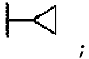
Numéro A30 : R¹ est H, R² est CH₃, R³ est H, R⁴ est H, R⁵ est H, R⁶ est 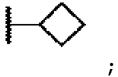
Numéro A31 : R¹ est H, R² est CH₃, R³ est H, R⁴ est H, R⁵ est H, R⁶ est 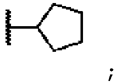
Numéro A32 : R¹ est H, R² est CH₃, R³ est H, R⁴ est H, R⁵ est H, R⁶ est 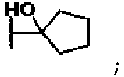
Numéro A33 : R¹ est H, R² est CH₃, R³ est H, R⁴ est H, R⁵ est H, R⁶ est 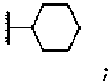
Numéro A34 : R¹ est H, R² est CH₃, R³ est H, R⁴ est H, R⁵ est H, R⁶ est 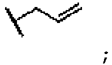
Numéro A35 : R¹ est H, R² est CH₃, R³ est H, R⁴ est H, R⁵ est H, R⁶ est 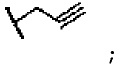
Numéro A36 : R¹ est H, R² est CH₃, R³ est H, R⁴ est H, R⁵ est H, R⁶ est 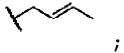
Numéro A37 : R¹ est H, R² est CH₃, R³ est H, R⁴ est H, R⁵ est H, R⁶ est 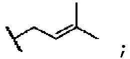
Numéro A38 : R¹ est H, R² est CH₃, R³ est H, R⁴ est H, R⁵ est H, R⁶ est 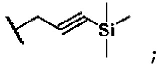
Numéro A39 : R¹ est H, R² est CH₃, R³ est H, R⁴ est H, R⁵ est H, R⁶ est 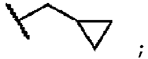
Numéro A40 : R¹ est H, R² est CH₃, R³ est H, R⁴ est H, R⁵ est H, R⁶ est 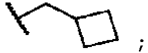
Numéro A41 : R¹ est H, R² est CH₃, R³ est H, R⁴ est H, R⁵ est H, R⁶ est 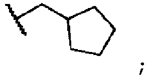
Numéro A42 : R¹ est H, R² est CH₃, R³ est H, R⁴ est H, R⁵ est H, R⁶ est 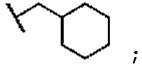
Numéro A43 : R¹ est H, R² est CH₃, R³ est H, R⁴ est H, R⁵ est H, R⁶ est 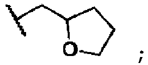
Numéro A44 : R¹ est H, R² est CH₃, R³ est H, R⁴ est H, R⁵ est H, R⁶ est 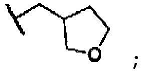
Numéro A45 : R¹ est H, R² est CH₃, R³ est H, R⁴ est H, R⁵ est H, R⁶ est 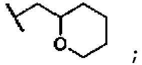
Numéro A46 : R¹ est H, R² est CH₃, R³ est H, R⁴ est H, R⁵ est H, R⁶ est 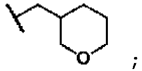
Numéro A47 : R¹ est H, R² est CH₃, R³ est H, R⁴ est H, R⁵ est H, R⁶ est 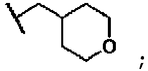
Numéro A48 : R¹ est H, R² est CH₃, R³ est H, R⁴ est H, R⁵ est H, R⁶ est 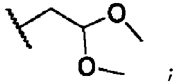
Numéro A49 : R¹ est H, R² est CH₃, R³ est H, R⁴ est H, R⁵ est H, R⁶ est 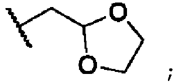
Numéro A50 : R¹ est H, R² est CH₃, R³ est H, R⁴ est H, R⁵ est H, R⁶ est 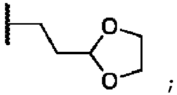
Numéro AS1 : R¹ est H, R² est CH₃, R³ est H, R⁴ est H, R⁵ est H, R⁶ est 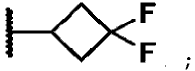
Numéro A52 : R¹ est H, R² est CH₃, R³ est H, R⁴ est H, R⁵ est H, R⁶ est 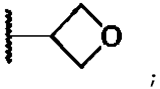
Numéro A53 : R¹ est H, R² est CH₃, R³ est H, R⁴ est H, R⁵ est H, R⁶ est 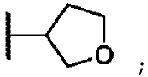
Numéro A54 : R¹ est H, R² est CH₃, R³ est H, R⁴ est H, R⁵ est H, R⁶ est 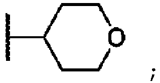
Numéro A55 : R¹ est H, R² est CH₃, R³ est H, R⁴ est H, R⁵ est H, R⁶ est 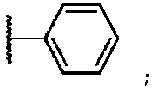
Numéro A56 : R¹ est H, R² est CH₃, R³ est H, R⁴ est H, R⁵ est H, R⁶ est 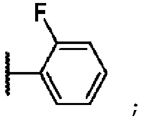
Numéro A57 : R¹ est H, R² est CH₃, R³ est H, R⁴ est H, R⁵ est H, R⁶ est 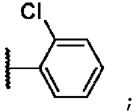
Numéro A58 : R¹ est H, R² est CH₃, R³ est H, R⁴ est H, R⁵ est H, R⁶ est 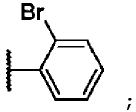
Numéro A59 : R¹ est H, R² est CH₃, R³ est H, R⁴ est H, R⁵ est H, R⁶ est 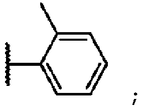
Numéro A60 : R¹ est H, R² est CH₃, R³ est H, R⁴ est H, R⁵ est H, R⁶ est 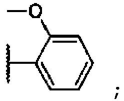
Numéro A61 : R¹ est H, R² est CH₃, R³ est H, R⁴ est H, R⁵ est H, R⁶ est 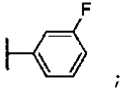
Numéro A62 : R¹ est H, R² est CH₃, R³ est H, R⁴ est H, R⁵ est H, R⁶ est 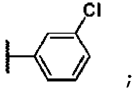
Numéro A63 : R¹ est H, R² est CH₃, R³ est H, R⁴ est H, R⁵ est H, R⁶ est 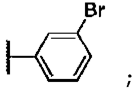
Numéro A64 : R¹ est H, R² est CH₃, R³ est H, R⁴ est H, R⁵ est H, R⁶ est 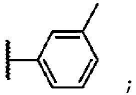
Numéro A65 : R¹ est H, R² est CH₃, R³ est H, R⁴ est H, R⁵ est H, R⁶ est 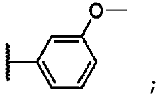
Numéro A66 : R¹ est H, R² est CH₃, R³ est H, R⁴ est H, R⁵ est H, R⁶ est 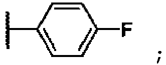
Numéro A67 : R¹ est H, R² est CH₃, R³ est H, R⁴ est H, R⁵ est H, R⁶ est 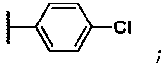
Numéro A68 : R¹ est H, R² est CH₃, R³ est H, R⁴ est H, R⁵ est H, R⁶ est 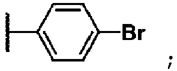
Numéro A69 : R¹ est H, R² est CH₃, R³ est H, R⁴ est H, R⁵ est H, R⁶ est 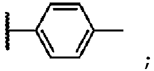
Numéro A70 : R¹ est H, R² est CH₃, R³ est H, R⁴ est H, R⁵ est H, R⁶ est 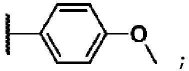
Numéro A71 : R¹ est H, R² est CH₃, R³ est H, R⁴ est H, R⁵ est H, R⁶ est 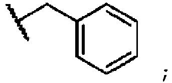
Numéro A72 : R¹ est H, R² est CH₃, R³ est H, R⁴ est H, R⁵ est H, R⁶ est -COCH₃ ;
Numéro A73 : R¹ est H, R² est CH₃, R³ est H, R⁴ est H, R⁵ est H, R⁶ est -COCH2CH3 ;
Numéro A74 : R¹ est H, R² est CH₃, R³ est H, R⁴ est H, R⁵ est H, R⁶ est -COCH2CH2CH3 ;
Numéro A75 : R¹ est H, R² est CH₃, R³ est H, R⁴ est H, R⁵ est H, R⁶ est -COCH(CH₃)₂ ;
Numéro A76 : R¹ est H, R² est CH₃, R³ est H, R⁴ est H, R⁵ est H, R⁶ est -COC₆H₅ ;
Numéro A77 : R¹ est H, R² est CH₃, R³ est H, R⁴ est H, R⁵ est H, R⁶ est 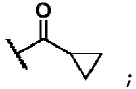
Numéro A78 : R¹ est H, R² est CH₃, R³ est H, R⁴ est H, R⁵ est H, R⁶ est 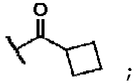
Numéro A79 : R¹ est H, R² est CH₃, R³ est H, R⁴ est H, R⁵ est H, R⁶ est 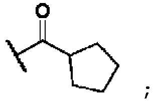
Numéro A80 : R¹ est H, R² est CH₃, R³ est H, R⁴ est H, R⁵ est H, R⁶ est 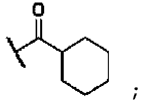
Numéro A81 : R¹ est H, R² est CH₃, R³ est H, R⁴ est H, R⁵ est H, R⁶ est 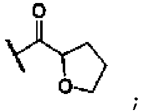
Numéro A82 : R¹ est H, R² est CH₃, R³ est H, R⁴ est H, R⁵ est H, R⁶ est 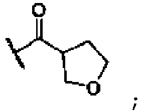
Numéro A83 : R¹ est H, R² est CH₃, R³ est H, R⁴ est H, R⁵ est H, R⁶ est 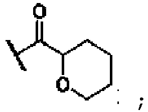
Numéro A84 : R¹ est H, R² est CH₃, R³ est H, R⁴ est H, R⁵ est H, R⁶ est 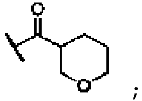
Numéro A85 : R¹ est H, R² est CH₃, R³ est H, R⁴ est H, R⁵ est H, R⁶ est 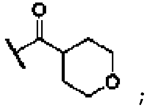
Numéro A86 : R¹ est H, R² est CH₃, R³ est H, R⁴ est H, R⁵ est H, R⁶ est 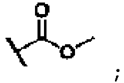
Numéro A87 : R¹ est H, R² est CH₃, R³ est H, R⁴ est H, R⁵ est H, R⁶ est 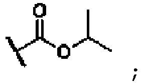
Numéro A88 : R¹ est H, R² est CH₃, R³ est H, R⁴ est H, R⁵ est H, R⁶ est 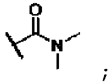
Numéro A89 : R¹ est H, R² est CH₃, R³ est H, R⁴ est H, R⁵ est H, R⁶ est 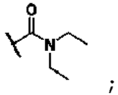
Numéro A90 : R¹ est H, R² est CH₃, R³ est H, R⁴ est H, R⁵ est H, R⁶ est -S(=O)₂CH₃ ;
Numéro A91 : R¹ est H, R² est CH₃, R³ est H, R⁴ est H, R⁵ est H, R⁶ est -S(=O)₂CH₂CH₃ ;
Numéro A92 : R¹ est H, R² est CH₃, R³ est H, R⁴ est H, R⁵ est H, R⁶ est -S(=O)₂CH₂CH₂CH₃ ;
Numéro A93 : R¹ est H, R² est CH₃, R³ est H, R⁴ est H, R⁵ est H, R⁶ est -S(=O)₂CH(CH₃)₂ ;
Numéro A94 : R¹ est H, R² est CH₃, R³ est H, R⁴ est H, R⁵ est H, R⁶ est -S (=O) ₂C₆H₅ ;
Numéro A95 : R¹ est H, R² est CH₃, R³ est H, R⁴ est H, R⁵ est H, R⁶ est 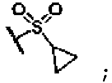
Numéro A96 : R¹ est H, R² est CH₃, R³ est H, R⁴ est H, R⁵ est H, R⁶ est 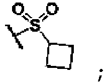
Numéro A97 : R¹ est H, R² est CH₃, R³ est H, R⁴ est H, R⁵ est H, R⁶ est 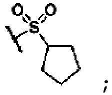
Numéro A98 : R¹ est H, R² est CH₃, R³ est H, R⁴ est H, R⁵ est H, R⁶ est 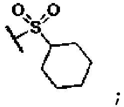
Numéro A99 : R¹ est H, R² est CH₃, R³ est H, R⁴ est H, R⁵ est H, R⁶ est 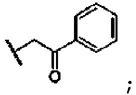
Numéro A100 : R¹ est H, R² est CH₃, R³ est H, R⁴ est H, R⁵ est H, R⁶ est 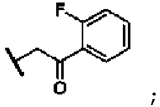
Numéro A1O1 : R¹ est H, R² est CH₃, R³ est H, R⁴ est H, R⁵ est H, R⁶ est 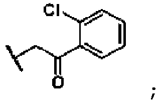
Numéro A102 : R¹ est H, R² est CH₃, R³ est H, R⁴ est H, R⁵ est H, R⁶ est 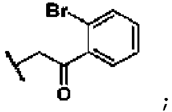
Numéro A103 : R¹ est H, R² est CH₃, R³ est H, R⁴ est H, R⁵ est H, R⁶ est 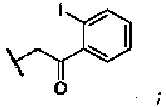
Numéro A104 : R¹ est H, R² est CH₃, R³ est H, R⁴ est H, R⁵ est H, R⁶ est 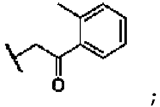
Numéro A105 : R¹ est H, R² est CH₃, R³ est H, R⁴ est H, R⁵ est H, R⁶ est 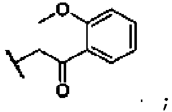
Numéro A106 : R¹ est H, R² est CH₃, R³ est H, R⁴ est H, R⁵ est H, R⁶ est 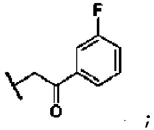
Numéro A107 : R¹ est H, R² est CH₃, R³ est H, R⁴ est H, R⁵ est H, R⁶ est 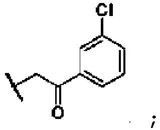
Numéro A108 : R¹ est H, R² est CH₃, R³ est H, R⁴ est H, R⁵ est H, R⁶ est 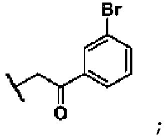
Numéro A109 : R¹ est H, R² est CH₃, R³ est H, R⁴ est H, R⁵ est H, R⁶ est 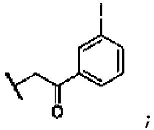
Numéro A110 : R¹ est H, R² est CH₃, R³ est H, R⁴ est H, R⁵ est H, R⁶ est 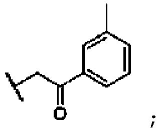
Numéro A111 : R¹ est H, R² est CH₃, R³ est H, R⁴ est H, R⁵ est H, R⁶ est 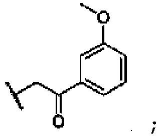
Numéro A112 : R¹ est H, R² est CH₃, R³ est H, R⁴ est H, R⁵ est H, R⁶ est 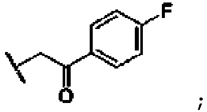
Numéro A113 : R¹ est H, R² est CH₃, R³ est H, R⁴ est H, R⁵ est H, R⁶ est 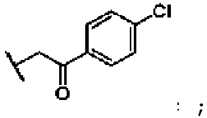
Numéro A114 : R¹ est H, R² est CH₃, R³ est H, R⁴ est H, R⁵ est H, R⁶ est 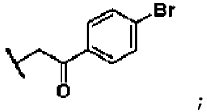
Numéro A115 : R¹ est H, R² est CH₃, R³ est H, R⁴ est H, R⁵ est H, R⁶ est 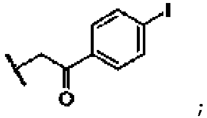
Numéro A116 : R¹ est H, R² est CH₃, R³ est H, R⁴ est H, R⁵ est H, R⁶ est 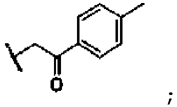
Numéro A117 : R¹ est H, R² est CH₃, R³ est H, R⁴ est H, R⁵ est CH₃, R⁶ est 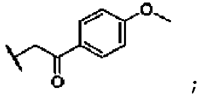
Numéro A118 : R¹ est H, R² est CH₃, R³ est H, R⁴ est H, R⁵ est CH₃, R⁶ est -H ;
Numéro A119 : R¹ est H, R² est CH₃, R³ est H, R⁴ est H, R⁵ est CH₃, R⁶ est -CH₃ ;
Numéro A120 : R¹ est H, R² est CH₃, R³ est H, R⁴ est H, R⁵ est CH₃, R⁶ est -CH2CH3 ;
Numéro A121 : R¹ est H, R² est CH₃, R³ est H, R⁴ est H, R⁵ est CH₃, R⁶ est -CH2CH2CH3 ;
Numéro A122 : R¹ est H, R² est CH₃, R³ est H, R⁴ est H, R⁵ est CH₃, R⁶ est -CH(CH3)2 ;
Numéro A123 : R¹ est H, R² est CH₃, R³ est H, R⁴ est H, R⁵ est CH₃, R⁶ est 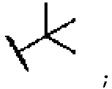
Numéro A124 : R¹ est H, R² est CH₃, R³ est H, R⁴ est H, R⁵ est CH₃, R⁶ est 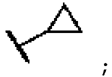
Numéro A125 : R¹ est H, R² est CH₃, R³ est H, R⁴ est H, R⁵ est CH₃, R⁶ est 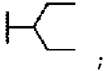
Numéro A126 : R¹ est H, R² est CH₃, R³ est H, R⁴ est H, R⁵ est CH3, R⁶ est 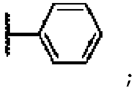
Numéro A127 : R¹ est H, R² est CH₃, R³ est H, R⁴ est H, R⁵ est CH₃, R⁶ est 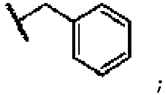
Numéro A128 : R¹ est H, R² est CH₃, R³ est H, R⁴ est H, R⁵ est CH₃, R⁶ est -COCH3 ;
Numéro A129 : R¹ est H, R² est CH₃, R³ est H, R⁴ est H, R⁵ est CH₃, R⁶ est -COCH2CH3 ;
Numéro A130 : R¹ est H, R² est CH₃, R³ est H, R⁴ est H, R⁵ est CH₃, R⁶ est -COCH2CH2CH3 ;
Numéro A131 : R¹ est H, R² est CH₃, R³ est H, R⁴ est H, R⁵ est CH₃, R⁶ est -COCH(CH₃)₂ ;
Numéro A132 : R¹ est H, R² est CH₃, R³ est H, R⁴ est H, R⁵ est CH₃, R⁶ est -COC₆H₅ ;
Numéro A133 : R¹ est H, R² est CH₃, R³ est H, R⁴ est H, R⁵ est CH₃, R⁶ est 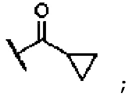
Numéro A134 : R¹ est H, R² est CH₃, R³ est H, R⁴ est H, R⁵ est CH₃, R⁶ est 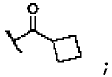
Numéro A135 : R¹ est H, R² est CH₃, R³ est H, R⁴ est H, R⁵ est CH₃, R⁶ est 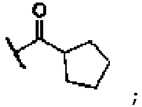
Numéro A136 : R¹ est H, R² est CH₃, R³ est H, R⁴ est H, R⁵ est CH₃, R⁶ est 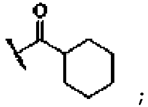
Numéro A137 : R¹ est H, R² est CH₃, R³ est H, R⁴ est H, R⁵ est CH₃, R⁶ est 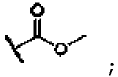
Numéro A138 : R¹ est H, R² est CH₃, R³ est H, R⁴ est H, R⁵ est CH₃, R⁶ est 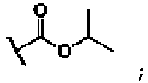
Numéro A139 : R¹ est H, R² est CH₃, R³ est H, R⁴ est H, R⁵ est CH₃, R⁶ est 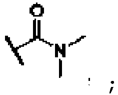
Numéro A140 : R¹ est H, R² est CH₃, R³ est H, R⁴ est H, R⁵ est CH₃, R⁶ est 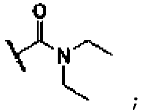
Numéro A141 : R¹ est H, R² est CH₃, R³ est H, R⁴ est H, R⁵ est CH₃, R⁶ est -S(=O)2CH3 ;
Numéro A142 : R¹ est H, R² est CH₃, R³ est H, R⁴ est H, R⁵ est CH₃, R⁶ est -S(=O)₂CH₂CH₃ ;
Numéro A143 : R¹ est H, R² est CH₃, R³ est H, R⁴ est H, R⁵ est CH₃, R⁶ est -S(=O)₂CH₂CH₂CH₃ ;
Numéro A144 : R¹ est H, R² est CH₃, R³ est H, R⁴ est H, R⁵ est CH₃, R⁶ est -S(=O)2CH(CH3)2 ;
Numéro A145 : R¹ est H, R² est CH₃, R³ est H, R⁴ est H, R⁵ est CH₃, R⁶ est -S (=O) ₂C₆H₅ ;
Numéro A146 : R¹ est H, R² est CH₃, R³ est H, R⁴ est H, R⁵ est CH₃, R⁶ est 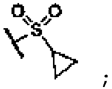
Numéro A147 : R¹ est H, R² est CH₃, R³ est H, R⁴ est H, R⁵ est CH₃, R⁶ est 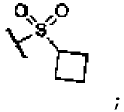
Numéro A148 : R¹ est H, R² est CH₃, R³ est H, R⁴ est H, R⁵ est CH₃, R⁶ est 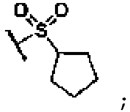
Numéro A149 : R¹ est H, R² est CH₃, R³ est H, R⁴ est H, R⁵ est CH₃, R⁶ est 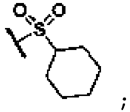
Numéro A150 : R¹ est H, R² est CH₃, R³ est H, R⁴ est CH₃, R⁵ est CH₃, R⁶ est -H ;
Numéro A151 : R¹ est H, R² est CH₃, R³ est H, R⁴ est CH₃, R⁵ est CH₃, R⁶ est -CH3 ;
Numéro A152 : R¹ est H, R² est CH₃, R³ est H, R⁴ est CH₃, R⁵ est CH₃, R⁶ est -CH₂CH₃ ;
Numéro A153 : R¹ est H, R² est CH₃, R³ est H, R⁴ est CH₃, R⁵ est CH₃, R⁶ est -CH₂CH₂CH₃ ;
Numéro A154 : R¹ est H, R² est CH₃, R³ est H, R⁴ est CH₃, R⁵ est CH₃, R⁶ est -CH(CH₃)₂ ;
Numéro A155 : R¹ est H, R² est CH₃, R³ est H, R⁴ est CH₃, R⁵ est CH₃, R⁶ est 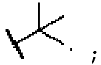
Numéro A156 : R¹ est H, R² est CH₃, R³ est H, R⁴ est CH₃, R⁵ est CH₃, R⁶ est 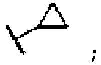
Numéro A157 : R¹ est H, R² est CH₃, R³ est H, R⁴ est CH₃, R⁵ est CH₃, R⁶ est 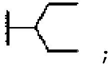
Numéro A158 : R¹ est H, R² est CH₃, R³ est H, R⁴ est CH₃, R⁵ est CH₃, R⁶ est 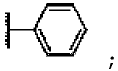
Numéro A159 : R¹ est H, R² est CH₃, R³ est H, R⁴ est CH₃, R⁵ est CH₃, R⁶ est 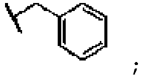
Numéro A160 : R¹ est H, R² est CH₃, R³ est H, R⁴ est CH₃, R⁵ est CH₃, R⁶ est -COCH3 ;
Numéro A161 : R¹ est H, R² est CH₃, R³ est H, R⁴ est CH₃, R⁵ est CH₃, R⁶ est -COCH2CH3 ;
Numéro A162 : R¹ est H, R² est CH₃, R³ est H, R⁴ est CH₃, R⁵ est CH₃, R⁶ est -COCH2CH2CH3 ;
Numéro A163 : R¹ est H, R² est CH₃, R³ est H, R⁴ est CH₃, R⁵ est CH₃, R⁶ est -COCH(CH₃)₂ ;
Numéro A164 : R¹ est H, R² est CH₃, R³ est H, R⁴ est CH₃, R⁵ est CH₃, R⁶ est -COC₆H₅ ;
Numéro A165 : R¹ est H, R² est CH₃, R³ est H, R⁴ est CH₃, R⁵ est CH₃, R⁶ est 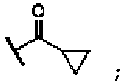
Numéro A166 : R¹ est H, R² est CH₃, R³ est H, R⁴ est CH₃, R⁵ est CH₃ ; R⁶ est 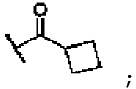
Numéro A167 : R¹ est H, R² est CH₃, R³ est H, R⁴ est CH₃, R⁵ est CH₃, R⁶ est 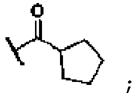
Numéro A168 : R¹ est H, R² est CH₃, R³ est H, R⁴ est CH₃, R⁵ est CH₃, R⁶ est 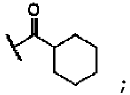
Numéro A169 : R¹ est H, R² est CH₃, R³ est H, R⁴ est CH₃, R⁵ est CH₃, R⁶ est 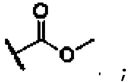
Numéro A170 : R¹ est H, R² est CH₃, R³ est H, R⁴ est CH₃, R⁵ est CH₃, R⁶ est 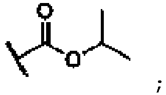
Numéro A171 : R¹ est H, R² est CH₃, R³ est H, R⁴ est CH₃, R⁵ est CH₃, R⁶ est 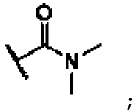
Numéro A172 : R¹ est H, R² est CH₃, R³ est H, R⁴ est CH₃, R⁵ est CH₃, R⁶ est 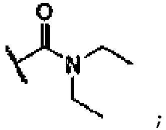
Numéro A173 : R¹ est H, R² est CH₃, R³ est H, R⁴ est CH₃, R⁵ est CH₃, R⁶ est -S(=O)₂CH₃ ;
Numéro A174 : R¹ est H, R² est CH₃, R³ est H, R⁴ est CH₃, R⁵ est CH₃, R⁶ est -S(=O)₂CH₂CH₃ ;
Numéro A175 : R¹ est H, R² est CH₃, R³ est H, R⁴ est CH₃, R⁵ est CH₃, R⁶ est -S(=O)₂CH₂CH₂CH₃ ;
Numéro A176 : R¹ est H, R² est CH₃, R³ est H, R⁴ est CH₃, R⁵ est CH₃, R⁶ est -S(=O)2CH(CH3)2 ;
Numéro A177 : R¹ est H, R² est CH₃, R³ est H, R⁴ est CH₃, R⁵ est CH₃, R⁶ est -S(=O)₂C₆H₅ ;
Numéro A178 : R¹ est H, R² est CH₃, R³ est H, R⁴ est CH₃, R⁵ est CH₃, R⁶ est 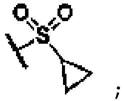
Numéro A179 : R¹ est H, R² est CH₃, R³ est H, R⁴ est CH₃, R⁵ est CH₃, R⁶ est 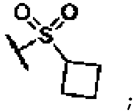
Numéro A180 : R¹ est H, R² est CH₃, R³ est H, R⁴ est CH₃, R⁵ est CH₃, R⁶ est 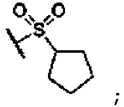
Numéro A181 : R¹ est H, R² est CH₃, R³ est H, R⁴ est CH₃, R⁵ est CH₃, R⁶ est 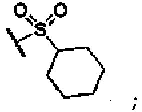
Numéro A182 : R¹ est H, R² est CH₃, R³ est CH₃, R⁴ est H, R⁵ est H, R⁶ est 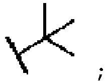
Numéro A183 : R¹ est H, R² est CH₃, R³ est OCH₃, R⁴ est H, R⁵ est H, R⁶ est 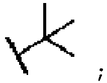
Numéro A184 : R¹ est H, R² est CH₃, R³ est 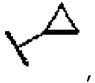 R⁴ est H, R⁵ est H, R⁶ est 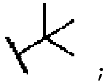
Numéro A185 : R¹ est H, R² est CH₃, R³ est COCH₃, R⁴ est H, R⁵ est H, R⁶ est 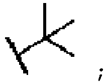
Numéro A186 : R¹ est H, R² est CH₃, R³ est 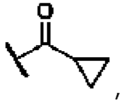 R⁴ est H, R⁵ est H, R⁶ est 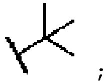
Numéro A187 : R¹ est H, R² est CH₃, R³ est 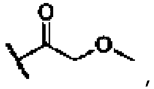 R⁴ est H, R⁵ est H, R⁶ est 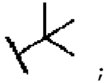
Numéro A188 : R¹ est H, R² est CH₃, R³ est 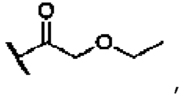 R⁴ est H, R⁵ est H, R⁶ est 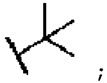
Numéro A189 : R¹ est H, R² est CN, R³ est H, R⁴ est H, R⁵ est H, R⁶ est 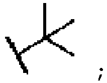
Numéro A190 : R¹ est F, R² est CH₃, R³ est H, R⁴ est H, R⁵ est H, R⁶ est -H ;
Numéro A191 : R¹ est F, R² est CH₃, R³ est H, R⁴ est H, R⁵ est H, R⁶ est -CH₃ ;
Numéro A192 : R¹ est F, R² est CH₃, R³ est H, R⁴ est H, R⁵ est H, R⁶ est -CH₂CH₃ ;
Numéro A193 : R¹ est F, R² est CH₃, R³ est H, R⁴ est H, R⁵ est H, R⁶ est -CH₂CH₂CH₃ ;
Numéro A194 : R¹ est F, R² est CH₃, R³ est H, R⁴ est H, R⁵ est H, R⁶ est -CH(CH₃)₂ ;
Numéro A195 : R¹ est F, R² est CH₃, R³ est H, R⁴ est H, R⁵ est H, R⁶ est 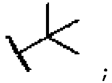
Numéro A196 : R¹ est F, R² est CH₃, R³ est H, R⁴ est H, R⁵ est H, R⁶ est 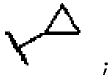
Numéro A197 : R¹ est F, R² est CH₃, R³ est H, R⁴ est H, R⁵ est H, R⁶ est 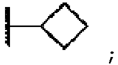
Numéro A198 : R¹ est F, R² est CH₃, R³ est H, R⁴ est H, R⁵ est H, R⁶ est 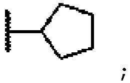
Numéro A199 : R¹ est F, R² est CH₃, R³ est H, R⁴ est H, R⁵ est H, R⁶ est 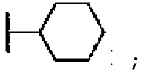
Numéro A200 : R¹ est F, R² est CH₃, R³ est H, R⁴ est H, R⁵ est H, R⁶ est 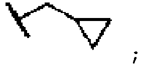
Numéro A201 : R¹ est F, R² est CH₃, R³ est H, R⁴ est H, R⁵ est H, R⁶ est 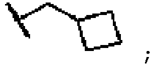
Numéro A202 : R¹ est F, R² est CH₃, R³ est H, R⁴ est H, R⁵ est H, R⁶ est 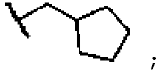
Numéro A203 : R¹ est F, R² est CH₃, R³ est H, R⁴ est H, R⁵ est H, R⁶ est 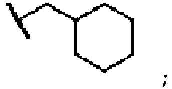
Numéro A204 : R¹ est F, R² est CH₃, R³ est H, R⁴ est H, R⁵ est H, R⁶ est 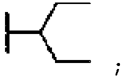
Numéro A205 : R¹ est F, R² est CH₃, R³ est H, R⁴ est H, R⁵ est H, R⁶ est 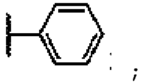
Numéro A206 : R¹ est F, R² est CH₃, R³ est H, R⁴ est H, R⁵ est H, R⁶ est 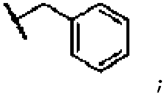
Numéro A207 : R¹ est F, R² est CH₃, R³ est H, R⁴ est H, R⁵ est H, R⁶ est -COCH₃ ;
Numéro A208 : R¹ est F, R² est CH₃, R³ est H, R⁴ est H, R⁵ est H, R⁶ est -COCH₂CH₃ ;
Numéro A209 : R¹ est F, R² est CH₃, R³ est H, R⁴ est H, R⁵ est H, R⁶ est -COCH2CH2CH3 ;
Numéro A210 : R¹ est F, R² est CH₃, R³ est H, R⁴ est H, R⁵ est H, R⁶ est -COCH(CH₃)₂ ;
Numéro A211 : R¹ est F, R² est CH₃, R³ est H, R⁴ est H, R⁵ est H, R⁶ est -COC₆H₅ ;
Numéro A212 : R¹ est F, R² est CH₃, R³ est H, R⁴ est H, R⁵ est H, R⁶ est 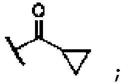
Numéro A213 : R¹ est F, R² est CH₃, R³ est H, R⁴ est H, R⁵ est H, R⁶ est 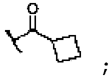
Numéro A214 : R¹ est F, R² est CH₃, R³ est H, R⁴ est H, R⁵ est H, R⁶ est 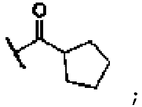
Numéro A215 : R¹ est F, R² est CH₃, R³ est H, R⁴ est H, R⁵ est H, R⁶ est 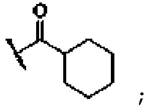
Numéro A216 : R¹ est F, R² est CH₃, R³ est H, R⁴ est H, R⁵ est H, R⁶ est 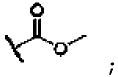
Numéro A217 : R¹ est F, R² est CH₃, R³ est H, R⁴ est H, R⁵ est H, R⁶ est 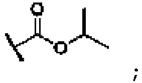
Numéro A218 : R¹ est F, R² est CH₃, R³ est H, R⁴ est H, R⁵ est H, R⁶ est ; 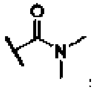
Numéro A219 : R¹ est F, R² est CH₃, R³ est H, R⁴ est H, R⁵ est H, R⁶ est 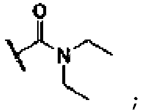
Numéro A220 : R¹ est F, R² est CH₃, R³ est H, R⁴ est H, R⁵ est H, R⁶ est -S(=O)₂CH₃ ;
Numéro A221 : R¹ est F, R² est CH₃, R³ est H, R⁴ est H, R⁵ est H, R⁶ est -S(=O)₂CH₂CH₃ ;
Numéro A222 : R¹ est F, R² est CH₃, R³ est H, R⁴ est H, R⁵ est H, R⁶ est -S(=O)₂CH₂CH₂CH₃ ;
Numéro A223 : R¹ est F, R² est CH₃, R³ est H, R⁴ est H, R⁵ est H, R⁶ est -S(=O)₂CH(CH₃)₂ ;
Numéro A224 : R¹ est F, R² est CH₃, R³ est H, R⁴ est H, R⁵ est H, R⁶ est -S(=O)₂C₆H₅ ;
Numéro A225 : R¹ est F, R² est CH₃, R³ est H, R⁴ est H, R⁵ est H, R⁶ est 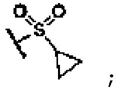
Numéro A226 : R¹ est F, R² est CH₃, R³ est H, R⁴ est H, R⁵ est H, R⁶ est 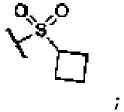
Numéro A227 : R¹ est F, R² est CH₃, R³ est H, R⁴ est H, R⁵ est H, R⁶ est 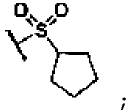
Numéro A228 : R¹ est F, R² est CH₃, R³ est H, R⁴ est H, R⁵ est H, R⁶ est 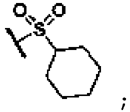
Numéro A229 : R¹ est F, R² est CH₃, R³ est H, R⁴ est H, R⁵ est CH₃, R⁶ est -H ;
Numéro A230 : R¹ est F, R² est CH₃, R³ est H, R⁴ est H, R⁵ est CH₃, R⁶ est -CH₃ ;
Numéro A231 : R¹ est F, R² est CH₃, R³ est H, R⁴ est H, R⁵ est CH₃, R⁶ est -CH₂CH₃ ;
Numéro A232 : R¹ est F, R² est CH₃, R³ est H, R⁴ est H, R⁵ est CH₃, R⁶ est -CH₂CH₂CH₃ ;
Numéro A233 : R¹ est F, R² est CH₃, R³ est H, R⁴ est H, R⁵ est CH₃, R⁶ est -CH(CH₃)₂ ;
Numéro A234 : R¹ est F, R² est CH₃, R³ est H, R⁴ est H, R⁵ est CH₃, R⁶ est 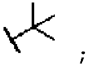
Numéro A235 : R¹ est F, R² est CH₃, R³ est H, R⁴ est H, R⁵ est CH₃, R⁶ est 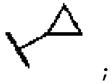
Numéro A236 : R¹ est F, R² est CH₃, R³ est H, R⁴ est H, R⁵ est CH₃, R⁶ est 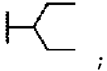
Numéro A237 : R¹ est F, R² est CH₃, R³ est H, R⁴ est H, R⁵ est CH₃, R⁶ est 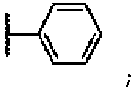
Numéro A238 : R¹ est F, R² est CH₃, R³ est H, R⁴ est H, R⁵ est CH₃, R⁶ est 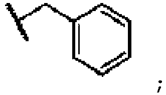
Numéro A239 : R¹ est F, R² est CH₃, R³ est H, R⁴ est H, R⁵ est CH₃, R⁶ est -COCH3 ;
Numéro A240 : R¹ est F, R² est CH₃, R³ est H, R⁴ est H, R⁵ est CH₃, R⁶ est -COCH2CH3 ;
Numéro A241 : R¹ est F, R² est CH₃, R³ est H, R⁴ est H, R⁵ est CH₃, R⁶ est -COCH2CH2CH3 ;
Numéro A242 : R¹ est F, R² est CH₃, R³ est H, R⁴ est H, R⁵ est CH₃, R⁶ est -COCH(CH₃)₂ ;
Numéro A243 : R¹ est F, R² est CH₃, R³ est H, R⁴ est H, R⁵ est CH₃, R⁶ est -COC₆H₅ ;
Numéro A244 : R¹ est F, R² est CH₃, R³ est H, R⁴ est H, R⁵ est CH₃, R⁶ est 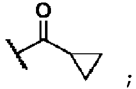
Numéro A245 : R¹ est F, R² est CH₃, R³ est H, R⁴ est H, R⁵ est CH₃, R⁶ est 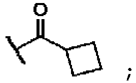
Numéro A246 : R¹ est F, R² est CH₃, R³ est H, R⁴ est H, R⁵ est CH₃, R⁶ est 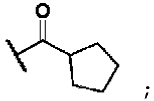
Numéro A247 : R¹ est F, R² est CH₃, R³ est H, R⁴ est H, R⁵ est CH₃, R⁶ est 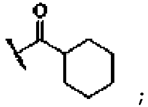
Numéro A248 : R¹ est F, R² est CH₃, R³ est H, R⁴ est H, R⁵ est CH₃, R⁶ est 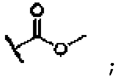
Numéro A249 : R¹ est F, R² est CH₃, R³ est H, R⁴ est H, R⁵ est CH₃, R⁶ est 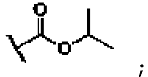
Numéro A250 : R¹ est F, R² est CH₃, R³ est H, R⁴ est H, R⁵ est CH₃, R⁶ est 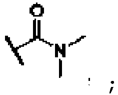
Numéro A251 : R¹ est F, R² est CH₃, R³ est H, R⁴ est H, R⁵ est CH₃, R⁶ est 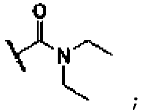
Numéro A252 : R¹ est F, R² est CH₃, R³ est H, R⁴ est H, R⁵ est CH₃, R⁶ est -S(=O)2CH3 ;
Numéro A253 : R¹ est F, R² est CH₃, R³ est H, R⁴ est H, R⁵ est CH₃, R⁶ est -S(=O)₂CH₂CH₃ ;
Numéro A254 : R¹ est F, R² est CH₃, R³ est H, R⁴ est H, R⁵ est CH₃, R⁶ est -S(=O)₂CH₂CH₂CH₃ ;
Numéro A255 : R¹ est F, R² est CH₃, R³ est H, R⁴ est H, R⁵ est CH₃, R⁶ est -S(=O)₂CH(CH₃)₂ ;
Numéro A256 : R¹ est F, R² est CH₃, R³ est H, R⁴ est H, R⁵ est CH₃, R⁶ est -S(=O)₂C₆H₅ ;
Numéro A257 : R¹ est F, R² est CH₃, R³ est H, R⁴ est H, R⁵ est CH₃, R⁶ est 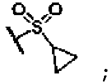
Numéro A258 : R¹ est F, R² est CH₃, R³ est H, R⁴ est H, R⁵ est CH₃, R⁶ est 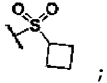
Numéro A259 : R¹ est F, R² est CH₃, R³ est H, R⁴ est H, R⁵ est CH₃, R⁶ est 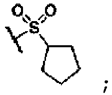
Numéro A260 : R¹ est F, R² est CH₃, R³ est H, R⁴ est H, R⁵ est CH₃, R⁶ est 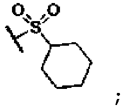
Numéro A261 : R¹ est F, R² est CH₃, R³ est H, R⁴ est CH₃, R⁵ est CH₃, R⁶ est -H ;
Numéro A262 : R¹ est F, R² est CH₃, R³ est H, R⁴ est CH₃, R⁵ est CH₃, R⁶ est -CH₃ ;
Numéro A263 : R¹ est F, R² est CH₃, R³ est H, R⁴ est CH₃, R⁵ est CH₃, R⁶ est -CH₂CH₃ ;
Numéro A264 : R¹ est F, R² est CH₃, R³ est H, R⁴ est CH₃, R⁵ est CH₃, R⁶ est -CH₂CH₂CH₃ ;
Numéro A265 : R¹ est F, R² est CH₃, R³ est H, R⁴ est CH₃, R⁵ est CH₃, R⁶ est -CH(CH₃)₂ ;
Numéro A266 : R¹ est F, R² est CH₃, R³ est H, R⁴ est CH₃, R⁵ est CH₃, R⁶ est 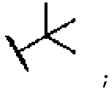
Numéro A267 : R¹ est F, R² est CH₃, R³ est H, R⁴ est CH₃, R⁵ est CH₃, R⁶ est 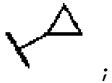
Numéro A268 : R¹ est F, R² est CH₃, R³ est H, R⁴ est CH₃, R⁵ est CH₃, R⁶ est 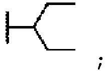
Numéro A269 : R¹ est F, R² est CH₃, R³ est H, R⁴ est CH₃, R⁵ est CH₃, R⁶ est 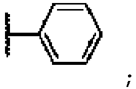
Numéro A270 : R¹ est F, R² est CH₃, R³ est H, R⁴ est CH₃, R⁵ est CH₃, R⁶ est 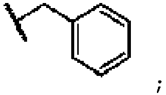
Numéro A271 : R¹ est F, R² est CH₃, R³ est H, R⁴ est CH₃, R⁵ est CH₃, R⁶ est -COCH3 ;
Numéro A272 : R¹ est F, R² est CH₃, R³ est H, R⁴ est CH₃, R⁵ est CH₃, R⁶ est -COCH2CH3 ;
Numéro A273 : R¹ est F, R² est CH₃, R³ est H, R⁴ est CH₃, R⁵ est CH₃, R⁶ est -COCH2CH2CH3 ;
Numéro A274 : R¹ est F, R² est CH₃, R³ est H, R⁴ est CH₃, R⁵ est CH₃, R⁶ est -COCH(CH₃)₂ ;
Numéro A275 : R¹ est F, R² est CH₃, R³ est H, R⁴ est CH₃, R⁵ est CH₃, R⁶ est -COC₆H₅ ;
Numéro A276 : R¹ est F, R² est CH₃, R³ est H, R⁴ est CH₃, R⁵
est CH₃, R⁶ est 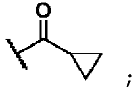 Numéro A277 : R¹ est F, R² est CH₃, R³ est H, R⁴ est CH₃, R⁵ est CH₃, R⁶ est 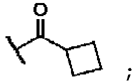
Numéro A278 : R¹ est F, R² est CH₃, R³ est H, R⁴ est CH₃, R⁵ est CH₃, R⁶ est 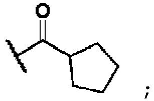
Numéro A279 : R¹ est F, R² est CH₃, R³ est H, R⁴ est CH₃, R⁵ est CH₃, R⁶ est 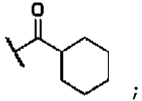
Numéro A280 : R¹ est F, R² est CH₃, R³ est H, R⁴ est CH₃, R⁵ est CH₃, R⁶ est 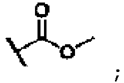
Numéro A281 : R¹ est F, R² est CH₃, R³ est H, R⁴ est CH₃, R⁵ est CH3, R⁶ est 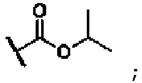
Numéro A282 : R¹ est F, R² est CH₃, R³ est H, R⁴ est CH₃, R⁵ est CH₃, R⁶ est 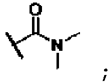
Numéro A283 : R¹ est F, R² est CH₃, R³ est H, R⁴ est CH₃, R⁵ est CH₃, R⁶ est 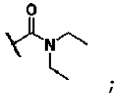
Numéro A284 : R¹ est F, R² est CH₃, R³ est H, R⁴ est CH₃, R⁵ est CH3, R⁶ est -S(=O)₂CH₃ ;
Numéro A285 : R¹ est F, R² est CH₃, R³ est H, R⁴ est CH₃, R⁵ est CH₃, R⁶ est -S(=O)₂CH₂CH₃ ;
Numéro A286 : R¹ est F, R² est CH₃, R³ est H, R⁴ est CH₃, R⁵ est CH₃, R⁶ est -S(=O)₂CH₂CH₂CH₃ ;
Numéro A287 : R¹ est F, R² est CH₃, R³ est H, R⁴ est CH₃, R⁵ est CH₃, R⁶ est -S(=O)_{2C}H(CH₃)₂ ;
Numéro A288 : R¹ est F, R² est CH₃, R³ est H, R⁴ est CH₃, R⁵ est CH₃, R⁶ est -S(=O)₂C₆H₅ ;
Numéro A289 : R¹ est F, R² est CH₃, R³ est H, R⁴ est CH₃, R⁵ est CH3, R⁶ est 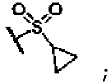
Numéro A290 : R¹ est F, R² est CH₃, R³ est H, R⁴ est CH₃, R⁵ est CH3, R⁶ est 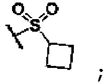
Numéro A291 : R¹ est F, R² est CH₃, R³ est H, R⁴ est CH₃, R⁵ est CH3, R⁶ est 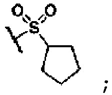
Numéro A292 : R¹ est F, R² est CH₃, R³ est H, R⁴ est CH₃, R⁵ est CH3, R⁶ est 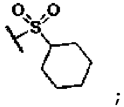
Numéro A293 : R¹ est F, R² est CH₃, R³ est CH₃, R⁴ est H, R⁵ est H, R⁶ est 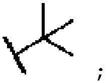
Numéro A294 : R¹ est F, R² est CH₃, R³ est OCH₃, R⁴ est H, R⁵ est H, R⁶ est 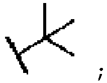
Numéro A295 : R¹ est F, R² est CH₃, R³ est 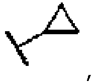 R⁴ est H, R⁵ est H, R⁶ est 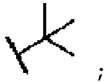
Numéro A296 : R¹ est F, R² est CH₃, R³ est COCH₃, R⁴ est H, R⁵ est H, R⁶ est 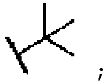
Numéro A297 : R¹ est F, R² est CH₃, R³ est 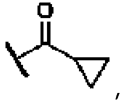 R⁴ est H, R⁵ est H, R⁶ est 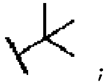
Numéro A298 : R¹ est F, R² est CH₃, R³ est 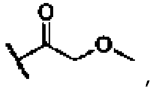 R⁴ est H, R⁵ est H, R⁶ est 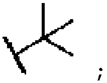
Numéro A299 : R¹ est F, R² est CH₃, R³ est 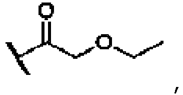 R⁴ est H, R⁵ est H, R⁶ est 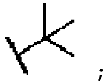
Numéro A300 : R¹ est F, R² est CN, R³ est H, R⁴ est H, R⁵ est H, R⁶ est 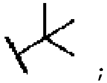
Numéro A301 : R¹ est H, R² est CH₃, R³ est H, R⁴ est H, R⁵ est H, R⁶ est -CH₂OCH₃.

5. Utilisation non thérapeutique d'un composé contenant une structure de pyrazole selon l'une quelconque des revendications 1 à 4 ou d'un de ses *sels,* hydrates et solvates acceptables sur le plan agrochimique pour au moins l'un parmi tuer des insectes, tuer des acariens, tuer des nématodes et tuer des insectes piqueurs-suceurs.

6. Utilisation non thérapeutique d'un composé contenant une structure de pyrazole selon l'une quelconque des revendications 1 à 4 ou d'un de ses *sels,* hydrates et solvates acceptables sur le plan agrochimique comme insecticide agrochimique.

7. Insecticide qui comprend, en tant qu'ingrédient actif, une quantité efficace sur le plan pesticide d'au moins l'un parmi un composé contenant une structure de pyrazole selon l'une quelconque des revendications 1 à 4 ou un de ses *sels,* hydrates et solvates acceptables sur le plan agrochimique.

8. Insecticide selon la revendication 7, dans lequel l'ingrédient actif est présent dans l'insecticide en une quantité de 1 à 99,9 % en poids.

9. Insecticide selon la revendication 8, dans lequel l'ingrédient actif est présent dans l'insecticide en une quantité de 5 à 95 % en poids.

10. Insecticide selon l'une quelconque des revendications 7 à 9, dans lequel l'insecticide est sous une forme sélectionnée parmi au moins l'un parmi un concentré émulsifiable, une suspension, une poudre mouillable, une poudre, des granulés, une solution aqueuse, un appât empoisonné, une liqueur mère et une poudre mère.
